# EUROPEAN PATENT APPLICATION

(11) **EP 4 748 839 A1**
(43) Date of publication of application: **27.05.2026**
(21) Application number: 24844739.3
(22) Date of filing: 19.07.2024
(51) Int. Cl.: C07D 405/14, C07D 471/04, C07D 491/00, A61K 31/343, A61K 31/4184, A61K 31/4188, A61P 3/04, A61P 3/10

(54) **POLYCYCLIC COMPOUND AND USE THEREOF**

(30) Priority: 21.07.2023 CN 202310900145; 25.08.2023 CN 202311081129; 31.05.2024 CN 202410698010
(71) Applicant: CSPC Zhongqi Pharmaceutical Technology (Shijiazhuang) Co., Ltd., Shijiazhuang, Hebei 050035 (CN)
(72) Inventor: WANG, Kuanglei, Shijiazhuang, Hebei 050035 (CN); GUO, Jianqiao, Shijiazhuang, Hebei 050035 (CN); ZHAO, Chuanwu, Shijiazhuang, Hebei 050035 (CN); SONG, Yunlong, Shijiazhuang, Hebei 050035 (CN); YANG, Hanyu, Shijiazhuang, Hebei 050035 (CN); CHU, Wenhao, Shijiazhuang, Hebei 050035 (CN); HAN, Yue, Shijiazhuang, Hebei 050035 (CN); ZHANG, Xueming, Shijiazhuang, Hebei 050035 (CN); WU, Bin, Shijiazhuang, Hebei 050035 (CN); XIAO, Linlin, Shijiazhuang, Hebei 050035 (CN)
(74) Representative: Gill Jennings & Every LLP
(86) International application number: PCT/CN2024/106558
(87) International publication number: WO 2025/021048

(57) **Abstract**

Provided are a compound represented by formula (1'), or a tautomer, stereoisomer or pharmaceutically acceptable salt thereof. The compound represented by formula (I') has a strong agonistic effect on GLP-1R, and can treat diseases such as diabetes, metabolic-associated fatty liver diseases, and Alzheimer's disease, or has a weight loss effect.

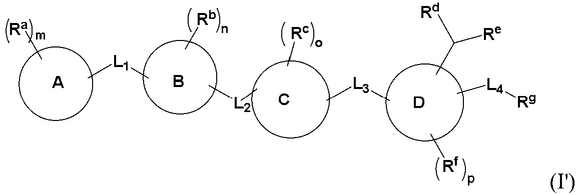

## Description

### CROSS-REFERENCE TO RELATED APPLICATIONS

The present application claims the priorities to and the benefit of Chinese Patent Application No. 202310900145.6 filed with the China National Intellectual Property Administration on July 21, 2023, Chinese Patent Application No. 202311081129.5 filed with the China National Intellectual Property Administration on August 25, 2023, and Chinese Patent Application No. 202410698010.0 filed with the China National Intellectual Property Administration on May 31, 2024, the contents of which are incorporated herein by reference in their entireties.

### THECNICAL FIELD

The present application relates to the field of medical technology, and in particular, to a class of novel compounds as GLP-1R agonists and a use thereof in the treatment and prevention of diseases, disorders, and conditions mediated by GLP-1R.

### BACKGROUND

Overweight and obesity have become global health concerns. Currently, approximately 50% of type 2 diabetes, 30% of ischemic cardiovascular and cerebrovascular diseases, and 10%-40% of cancers are attributable to obesity or overweight. China has become the country with the highest number of overweight and obese individuals in the world, and there is a huge unmet clinical need for obesity.

Type 2 diabetes is the most prevalent type of diabetes, accounting for more than 90% of the diabetic population. The pathogenesis of type 2 diabetes remains unclear, and it is currently believed that it is related to unhealthy lifestyles, genetic factors, environmental factors, and the toxic effects of lipotoxicity and glucotoxicity on pancreatic β cells. Diabetes can lead to various complications, including atherosclerosis, retinopathy and neuritis, renal insufficiency and proteinuria, myocarditis, heart failure, urinary tract infections, etc.

GLP-1 (Glucagon-like peptide-1) is primarily secreted by L cells, acts on its receptor GLP-1R (Glucagon-like peptide-1 receptor), which is predominantly distributed in the pancreas, gastrointestinal tract, central nervous system, cardiovascular system, etc. GLP-1R belongs to the glucagon receptor subfamily within the of G protein-coupled receptors cluster B, and it is typically characterized by its structure comprising a seven-transmembrane core domain, an extracellular N-terminal domain, and an intracellular C-terminal domain, interconnected by three intracellular loops and three extracellular loops on both sides of the membrane. Typically, binding to a peptide ligand occurs via a two-domain model, in which the C-terminal of the ligand first binds to the extracellular domain of GLP-1R, resulting in a change in the spatial conformation of GLP-1R to expose the binding site of the core domain, and then the N-terminal of the ligand binds to the core domain of GLP-1R, thereby activating GLP-1R. Generally, the primary function of the extracellular domain of GLP-1R is to recognize a specific ligand, while the core domain plays an important role in signal-specific transduction.

In pancreatic islet cells, the primary functions of GLP-1 are to promote the proliferation of pancreatic β cells, stimulate the synthesis and release of insulin, and inhibit the synthesis and release of glucagon; in the gastrointestinal tract, GLP-1 can inhibit gastric juice secretion and gastrointestinal motility, delay gastric emptying, increase satiety, and reduce food intake; in neural tissues, GLP-1 can protect nerve cells, counteract of appetite, and enhance memory; in the cardiovascular system, GLP-1 can improve cardiovascular functions and reduce inflammation; and in the liver, GLP-1 can increase insulin sensitivity and reduce gluconeogenesis. Therefore, GLP-1R activation can achieve the weight loss effect through multiple biological mechanisms.

It has been reported that defects in insulin signal transduction are related to the pathogenesis of Alzheimer's disease (AD). GLP-1 can counteract the significant increase in TLR4 expression in neuroglial cells induced by lipopolysaccharide (LPS) and reduce the expression of p-p38, p-JNK, and p-AKT, suggesting that GLP-1 can inhibit the inflammatory response by suppressing the MAPK signaling pathway. In addition, studies have shown that in AD, when glucose metabolism is impaired and mitochondrial function is compromised, the shuttle of lactate during glycolysis in glial cells becomes increasingly important for neuronal survival, acting as an energy substitute. GLP-1 can partially restore the glycolytic function of astrocytes and increase lactate flux, which helps alleviate the neuronal energy crisis. The neuroprotective mechanism of GLP-1 is closely related to its promotion of aerobic glycolysis, alleviation of oxidative phosphorylation activation, and activation of the PI3K/Akt pathway. Therefore, GLP-1 agonists present a promising strategy for the prevention and treatment of AD.

Metabolic-associated fatty liver disease (MAFLD), also known as non-alcoholic fatty liver disease (NAFLD), has a significantly increasing incidence rate, with a prevalence of up to 40%. It has now surpassed viral hepatitis to become the most common liver disease in the world, increasing the urgency for further basic and clinical research. MAFLD is classified into two categories: non-alcoholic fatty liver (NAFL) and non-alcoholic steatohepatitis (NASH). With excessive supply of metabolic substrates, excess fat accumulates in hepatocytes, accompanied by progressive accumulation of potentially toxic lipid species and increased de novo lipogenesis (DNL) in MAFLD. NASH, characterized by hepatocellular injury and inflammatory infiltration, is considered a more aggressive form of MAFLD that may progress to cirrhosis and hepatocellular carcinoma, with limited treatment options. Studies have shown that obesity and type 2 diabetes are two major risk factors for NASH, and individuals with a history of NASH have a significantly higher risk of developing liver and cardiovascular diseases. In addition, insulin resistance in the liver and adipose tissue is considered a key driver of the incidence and mortality of NASH, while GLP-1 analogs can improve blood glucose control, reduce weight, and activate liver enzyme levels in T2DM patients. It has been reported that liraglutide acts directly on human hepatocytes in vitro to reduce hepatic steatosis by decreasing the DNL level and increasing fatty acid oxidation. It has been shown that after treatment with liraglutide, steatosis and hepatocellular ballooning can be improved in most patients, indicating that liraglutide has the potential to reduce lipotoxicity by improving insulin sensitivity in adipose tissue. Only a small fraction of NASH patients experience disease progression after liraglutide therapy. In addition, semaglutide can reduce alanine transaminase and inflammatory markers levels, with a significant decrease in inflammatory biomarkers after treatment. Given the lack of GLP-1R expression in the liver, the potential mechanism of action of GLP-1R agonists in NASH may be related to their indirect benefits on weight loss, and the reduction of metabolic dysfunction, lipotoxic effects, and inflammation.

### DETAILED DESCRIPTION

In one aspect, the present application provides a compound represented by Formula (I'), or a tautomer, stereoisomer, or pharmaceutically acceptable salt thereof, and the compound has the following structure: 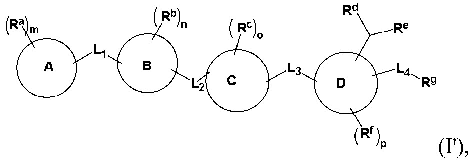 wherein,
ring A is independently selected from the group consisting of 3- to 10-membered heterocyclyl, C₆₋₁₄ aryl, and 5- to 12-membered heteroaryl;
ring B is independently selected from the group consisting of C₃₋₁₀ carbocyclyl, 3- to 12-membered heterocyclyl, C₆₋₁₄ aryl, and 5- to 12-membered heteroaryl;
ring C is independently selected from the group consisting of C₃₋₁₂ carbocyclyl, 3- to 12-membered heterocyclyl, C₆₋₁₄ aryl, and 5- to 12-membered heteroaryl;
ring D is independently selected from the group consisting of 3- to 12-membered heterocyclyl, C₆₋₁₄ aryl, and 5- to 12-membered heteroaryl;
R^{a} is, at each occurrence, independently selected from the group consisting of deuterium, halo, hydroxy, amino, nitro, mercapto, cyano, oxo, -OR^{a1}, -S(O)R^{a1}, -SO₂(R^{a1}), -C(O)R^{a1}, -C(O)OR^{a1}, -OC(O)R^{a1}, -N(R^{a1})(R^{a2}),-C(O)N(R^{a1})(R^{a2}), -N(R^{a1})C(O)(R^{a2}), -S(O)N(R^{a1})(R^{a2}), -SO₂N(R^{a1})(R^{a2}), -N(R^{a1})S(O)(R^{a2}), -N(R^{a1})SO₂(R^{a2}), C₁₋₆ alkyl, C₁₋₆ alkoxy, C₁₋₆ alkylaminyl, C₂₋₆ alkenyl, C₂₋₆ alkynyl, C₃₋₁₀ cycloalkyl, 3- to 10-membered heterocyclyl, C₆₋₁₄ aryl, and 5- to 12-membered heteroaryl, wherein the C₁₋₆ alkyl, the C₁₋₆ alkoxy, the C₁₋₆ alkylaminyl, the C₂₋₆ alkenyl, the C₂₋₆ alkynyl, the C₃₋₁₀ cycloalkyl, the 3- to 10-membered heterocyclyl, the C₆₋₁₄ aryl, and the 5- to 12-membered heteroaryl are optionally substituted with one or more R^{a3};
R^{a1} and R^{a2} are, at each occurrence, each independently selected from the group consisting of hydrogen, deuterium, C₁₋₆ alkyl, C₁₋₆ alkoxy, C₂₋₆ alkenyl, C₂₋₆ alkynyl, C₃₋₁₀ cycloalkyl, 3- to 10-membered heterocyclyl, C₆₋₁₄ aryl, and 5- to 12-membered heteroaryl, wherein the C₁₋₆ alkyl, the C₁₋₆ alkoxy, the C₂₋₆ alkenyl, the C₂₋₆ alkynyl, the C₃₋₁₀ cycloalkyl, the 3- to 10-membered heterocyclyl, the C₆₋₁₄ aryl, and the 5- to 12-membered heteroaryl are optionally substituted with one or more substituents selected from the group consisting of halo, hydroxy, amino, nitro, mercapto, cyano, oxo, C₁₋₆ alkyl, C₁₋₆ alkoxy, C₂₋₆ alkenyl, C₂₋₆ alkynyl, C₃₋₆ cycloalkyl, 3- to 6-membered heterocyclyl, phenyl, and 5- to 6-membered heteroaryl;
R^{a3} is, at each occurrence, independently selected from the group consisting of deuterium, halo, hydroxy, amino, nitro, mercapto, cyano, oxo, -R^{a4}, -OR^{a4}, -SR^{a4}, -S(O)R^{a4}, -SO₂(R^{a4}), ^{_}C(O)R^{a4}, -C(O)OR^{a4}, -OC(O)R^{a4}, -N(R^{a4})(R^{a5}), -C(O)N(R^{a4})(Ra⁵), -N(Ra⁴)C(O)(Ra⁵), -S(O)N(Ra⁴)(R^{a5}), -S0₂N(R^{a4})(R^{a5}), -N(R^{a4})S(O)(R^{a5}), and -N(R^{a4})SO₂(R^{a5});
R^{a4} and R^{a5} are, at each occurrence, each independently selected from the group consisting of hydrogen, deuterium, C₁₋₆ alkyl, C₁₋₆ alkoxy, C₂₋₆ alkenyl, C₂₋₆ alkynyl, C₃₋₁₀ cycloalkyl, 3- to 10-membered heterocyclyl, C₆₋₁₄ aryl, and 5- to 12-membered heteroaryl, wherein the C₁₋₆ alkyl, the C₁₋₆ alkoxy, the C₂₋₆ alkenyl, the C₂₋₆ alkynyl, the C₃₋₁₀ cycloalkyl, the 3- to 10-membered heterocyclyl, the C₆₋₁₄ aryl, and the 5- to 12-membered heteroaryl are optionally substituted with one or more substituents selected from the group consisting of halo, hydroxy, amino, nitro, mercapto, cyano, oxo, C₁₋₆ alkyl, C₁₋₆ alkoxy, C₂₋₆ alkenyl, C₂₋₆ alkynyl, C₃₋₇ cycloalkyl, 3- to 7-membered heterocyclyl, phenyl, and 5- to 6-membered heteroaryl; or
two R^{a}s, together with the atom(s) to which they are attached, form 3- to 10-membered heterocyclyl or 5- to 12-membered heteroaryl, each of which is optionally substituted with one or more substituents selected from the group consisting of deuterium, halo, hydroxy, amino, nitro, mercapto, cyano, oxo, C₁₋₆ alkyl, C₁₋₆ alkoxy, C₃₋₁₀ cycloalkyl, 3- to 10-membered heterocyclyl, C₆₋₁₄ aryl, and 5- to 12-membered heteroaryl;
R^{b} is, at each occurrence, independently selected from the group consisting of deuterium, halo, hydroxy, amino, nitro, mercapto, cyano, oxo, optionally substituted C₁₋₆ alkyl, optionally substituted C₁₋₆ alkoxy, optionally substituted C₁₋₆ alkylaminyl, optionally substituted C₂₋₆ alkenyl, optionally substituted C₂₋₆ alkynyl, optionally substituted C₃₋₁₀ cycloalkyl, optionally substituted 3- to 10-membered heterocyclyl, optionally substituted C₆₋₁₄ aryl, and optionally substituted 5- to 12-membered heteroaryl, wherein the term "optionally substituted" means that hydrogen on the substituted group is not substituted, or one or more substitutable sites of the substituted group are independently substituted with R^{b1}; or
two R^{b}s, together with the atom(s) to which they are attached, form 3- to 10-membered heterocyclyl or 5- to 12-membered heteroaryl, each of which is optionally substituted with one or more substituents selected from the group consisting of deuterium, halo, hydroxy, amino, nitro, mercapto, cyano, oxo, C₁₋₆ alkyl, C₁₋₆ alkoxy, C₃₋₁₀ cycloalkyl, 3- to 10-membered heterocyclyl, C₆₋₁₄ aryl, and 5- to 12-membered heteroaryl;
R^{b1} is, at each occurrence, independently selected from the group consisting of deuterium, halo, hydroxy, amino, nitro, mercapto, cyano, oxo, -R^{b2}, -OR^{b2}, -SR^{b2}, -S(O)R^{b2}, -SO₂(R^{b2}), -C(O)R^{b2} , -C(O)OR^{b2} , -OC(O)R^{b2},-N(R^{b2})(R^{b3}), -C(O)N(R^{b2})(R^{b3}), -N(R^{b2})C(O)(R^{b3}), -S(O)N(R^{b2})(R^{b3}), -SO₂N(R^{b2})(R^{b3}), -N(R^{b2})S(O)(R^{b3}), and-N(R^{b2})SO₂(R^{b3});
R^{b2} and R^{b3} are, at each occurrence, each independently selected from the group consisting of hydrogen, deuterium, C₁₋₆ alkyl, C₁₋₆ alkoxy, C₂₋₆ alkenyl, C₂₋₆ alkynyl, C₃₋₁₀ cycloalkyl, 3- to 10-membered heterocyclyl, C₆₋₁₄ aryl, and 5- to 12-membered heteroaryl, each of which is optionally substituted with one or more substituents selected from the group consisting of halo, hydroxy, amino, nitro, mercapto, cyano, oxo, C₁₋₆ alkyl, C₁₋₆ alkoxy, C₂₋₆ alkenyl, C₂₋₆ alkynyl, C₃₋₁₀ cycloalkyl, 3- to 10-membered heterocyclyl, C₆₋₁₄ aryl, and 5- to 12-membered heteroaryl;
R^{c} is, at each occurrence, independently selected from the group consisting of deuterium, halo, hydroxy, amino, nitro, mercapto, cyano, oxo, optionally substituted C₁₋₆ alkyl, optionally substituted C₁₋₆ alkoxy, optionally substituted C₁₋₆ alkylaminyl, optionally substituted C₂₋₆ alkenyl, optionally substituted C₂₋₆ alkynyl, optionally substituted C₃₋₁₀ cycloalkyl, optionally substituted 3- to 10-membered heterocyclyl, optionally substituted C₆₋₁₄ aryl, and optionally substituted 5- to 12-membered heteroaryl, wherein the term "optionally substituted" means that hydrogen on the substituted group is not substituted, or one or more substitutable sites of the substituted group are independently substituted with R^{c1}; or
two R^{c}s, together with the atom(s) to which they are attached, form 3- to 10-membered heterocyclyl or 5- to 12-membered heteroaryl, each of which is optionally substituted with one or more substituents selected from the group consisting of deuterium, halo, hydroxy, amino, nitro, mercapto, cyano, oxo, C₁₋₆ alkyl, C₁₋₆ alkoxy, C₃₋₁₀ cycloalkyl, 3- to 10-membered heterocyclyl, C₆₋₁₄ aryl, and 5- to 12-membered heteroaryl;
R^{c1} is, at each occurrence, independently selected from the group consisting of deuterium, halo, hydroxy, amino, nitro, mercapto, cyano, oxo, -R^{c2}, -OR^{c2}, -SR^{c2}, -S(O)R^{c2}, -SO₂(R^{c2}), -C(O)R^{c2}, -C(O)OR^{c2}, -OC(O)R^{c2}, -N(R^{c2})(R^{c3}), -C(O)N(R^{c2})(R^{c3}), -N(R^{c2})C(O)(R^{c3}), -S(O)N(R^{c2})(R^{c3}), -SO₂N(R^{c2})(R^{c3}), -N(R^{c2})S(O)(R^{c3}), and -N(R^{c2})SO₂(R^{c3});
R^{c2} and R^{c3} are, at each occurrence, each independently selected from the group consisting of hydrogen, deuterium, C₁₋₆ alkyl, C₁₋₆ alkoxy, C₂₋₆ alkenyl, C₂₋₆ alkynyl, C₃₋₁₀ cycloalkyl, 3- to 10-membered heterocyclyl, C₆₋₁₄ aryl, and 5- to 12-membered heteroaryl, each of which is optionally substituted with one or more substituents selected from the group consisting of halo, hydroxy, amino, nitro, mercapto, cyano, oxo, C₁₋₆ alkyl, C₁₋₆ alkoxy, C₂₋₆ alkenyl, C₂₋₆ alkynyl, C₃₋₁₀ cycloalkyl, 3- to 10-membered heterocyclyl, C₆₋₁₄ aryl, and 5- to 12-membered heteroaryl;
R^{d} and R^{e} are, at each occurrence, each independently selected from the group consisting of hydrogen, deuterium, halo, hydroxy, amino, nitro, mercapto, cyano, oxo, optionally substituted C₁₋₆ alkyl, optionally substituted C₁₋₆ alkoxy, optionally substituted C₁₋₆ alkylaminyl, optionally substituted C₂₋₆ alkenyl, optionally substituted C₂₋₆ alkynyl, optionally substituted C₃₋₁₀ cycloalkyl, optionally substituted 3- to 10-membered heterocyclyl, optionally substituted C₆₋₁₄ aryl, and optionally substituted 5- to 12-membered heteroaryl, wherein the term "optionally substituted" means that hydrogen on the substituted group is not substituted, or one or more substitutable sites of the substituted group are independently substituted with R^{d1}; or
R^{d} and R^{e}, together with the carbon atom to which they are attached, form 3- to 10-membered heterocyclyl or 5- to 12-membered heteroaryl, each of which is optionally substituted with one or more substituents selected from the group consisting of deuterium, halo, hydroxy, amino, nitro, mercapto, cyano, oxo, C₁₋₆ alkyl, C₁₋₆ alkoxy, C₃₋₁₀ cycloalkyl, 3- to 10-membered heterocyclyl, C₆₋₁₄ aryl, and 5- to 12-membered heteroaryl;
R^{d1} is, at each occurrence, independently selected from the group consisting of deuterium, halo, hydroxy, amino, nitro, mercapto, cyano, oxo, -OR^{d2}, -SR^{d2}, -S(O)R^{d2}, -SO₂(R^{d2}), -C(O)R^{d2}, -C(O)OR^{d2}, -OC(O)R^{d2}, -N(R^{d2})(R^{d3}),-C(O)N(R^{d2})(R^{d3}), -N(R^{d2})C(O)(R^{d3}), -S(O)N(R^{d2})(R^{d3}), -SO₂N(R^{d2})(R^{d3}), -N(R^{d2})S(O)(R^{d3}), -N(R^{d2})SO₂(R^{d3}), C₁₋₆ alkyl, C₁₋₆ alkoxy, C₂₋₆ alkenyl, C₂₋₆ alkynyl, C₃₋₁₀ cycloalkyl, 3- to 10-membered heterocyclyl, C₆₋₁₄ aryl, and 5- to 12-membered heteroaryl, wherein the C₁₋₆ alkyl, the C₁₋₆ alkoxy, the C₂₋₆ alkenyl, the C₂₋₆ alkynyl, the C₃₋₁₀ cycloalkyl, the 3- to 10-membered heterocyclyl, the C₆₋₁₄ aryl, and the 5- to 12-membered heteroaryl are optionally substituted with one or more substituents selected from the group consisting of deuterium, halo, hydroxy, amino, nitro, mercapto, cyano, oxo, C₁₋₆ alkyl, C₁₋₆ haloalkyl, C₁₋₆ alkoxy, C₂₋₆ alkenyl, C₂₋₆ alkynyl, C₃₋₆ cycloalkyl, 3- to 6-membered heterocyclyl, phenyl, and 5- to 6-membered heteroaryl;
R^{d2} and R^{d3} are, at each occurrence, each independently selected from the group consisting of hydrogen, deuterium, C₁₋₆ alkyl, C₁₋₆ alkoxy, C₂₋₆ alkenyl, C₂₋₆ alkynyl, C₃₋₁₀ cycloalkyl, 3- to 10-membered heterocyclyl, C₆₋₁₄ aryl, and 5- to 12-membered heteroaryl, wherein the C₁₋₆ alkyl, the C₁₋₆ alkoxy, the C₂₋₆ alkenyl, the C₂₋₆ alkynyl, the C₃₋₁₀ cycloalkyl, the 3- to 10-membered heterocyclyl, the C₆₋₁₄ aryl, and the 5- to 12-membered heteroaryl are optionally substituted with one or more substituents selected from the group consisting of halo, hydroxy, amino, nitro, mercapto, cyano, oxo, C₁₋₆ alkyl, C₁₋₆ alkoxy, C₂₋₆ alkenyl, C₂₋₆ alkynyl, C₃₋₆ cycloalkyl, 3- to 6-membered heterocyclyl, phenyl, and 5- to 6-membered heteroaryl;
R^{f} is, at each occurrence, independently selected from the group consisting of deuterium, halo, hydroxy, amino, nitro, mercapto, cyano, oxo, -OR^{f4}, -SR^{f4}, -S(O)R^{f4}, -SO₂(R^{f4}), -C(O)R^{f4}, -C(O)OR^{f4}, -OC(O)R^{f4}, -N(R^{f4})(R^{f5}),-C(O)N(R^{f4})(R^{f5}), -N(R^{f4})C(O)(R^{f5}), -S(O)N(R^{f4})(R^{f5}), -SO₂N(R^{f4})(R^{f5}), -N(R^{f4})S(O)(R^{f5}), -N(R^{f4})SO₂(R^{f5}), C₁₋₆ alkyl, C₁₋₆ alkoxy, C₁₋₆ alkylaminyl, C₂₋₆ alkenyl, C₂₋₆ alkynyl, C₃₋₁₀ cycloalkyl, 3- to 10-membered heterocyclyl, C₆₋₁₄ aryl, and 5- to 12-membered heteroaryl, wherein the C₁₋₆ alkyl, the C₁₋₆ alkoxy, the C₁₋₆ alkylaminyl, the C₂₋₆ alkenyl, the C₂₋₆ alkynyl, the C₃₋₁₀ cycloalkyl, the 3- to 10-membered heterocyclyl, the C₆₋₁₄ aryl, and the 5- to 12-membered heteroaryl are optionally substituted with one or more R^{f1}; or
two R^{f}s, together with the atom(s) to which they are attached, form 3- to 10-membered heterocyclyl or 5- to 12-membered heteroaryl, each of which is optionally substituted with one or more substituents selected from the group consisting of deuterium, halo, hydroxy, amino, nitro, mercapto, cyano, oxo, C₁₋₆ alkyl, C₁₋₆ alkoxy, C₃₋₁₀ cycloalkyl, 3- to 10-membered heterocyclyl, C₆₋₁₄ aryl, and 5- to 12-membered heteroaryl;
R^{f1} is, at each occurrence, independently selected from the group consisting of deuterium, halo, hydroxy, amino, nitro, mercapto, cyano, oxo, -R^{f2}, -OR^{f2}, -SR^{f2}, -S(O)R^{f2}, -SO₂(R^{f2}), -C(O)R^{f2}, -C(O)OR^{f2}, -OC(O)R^{f2}, -N(R^{f2})(R^{f3}), -C(O)N(R^{f2})(R^{f3}), -N(R^{f2})C(O)(R^{f3}), -S(O)N(R^{f2})(R^{f3}), -S0₂N(R^{f2})(R^{f3}), -N(R^{f2})S(O)(R^{f3}), and -N(R^{C})SO₂(R^{f3});
R^{f2} and R^{f3} are, at each occurrence, each independently selected from the group consisting of hydrogen, deuterium, C₁₋₆ alkyl, C₁₋₆ alkoxy, C₂₋₆ alkenyl, C₂₋₆ alkynyl, C₃₋₁₀ cycloalkyl, 3- to 10-membered heterocyclyl, C₆₋₁₄ aryl, and 5- to 12-membered heteroaryl, each of which is optionally substituted with one or more substituents selected from the group consisting of halo, hydroxy, amino, nitro, mercapto, cyano, oxo, C₁₋₆ alkyl, C₁₋₆ alkoxy, C₂₋₆ alkenyl, C₂₋₆ alkynyl, C₃₋₁₀ cycloalkyl, 3- to 10-membered heterocyclyl, C₆₋₁₄ aryl, and 5- to 12-membered heteroaryl; or when R^{f2} and R^{f3} are attached to the same nitrogen atom, R^{f2} and R^{f3}, together with the nitrogen atom to which they are attached, form 3- to 10-membered heterocyclyl or 5- to 12-membered heteroaryl, each of which is optionally substituted with one or more substituents selected from the group consisting of deuterium, halo, hydroxy, amino, nitro, mercapto, cyano, oxo, C₁₋₆ alkyl, C₁₋₆ alkoxy, C₃₋₁₀ cycloalkyl, 3- to 10-membered heterocyclyl, C₆₋₁₄ aryl, and 5- to 12-membered heteroaryl;
R^{f4} and R^{f5} are, at each occurrence, each independently selected from the group consisting of hydrogen, deuterium, C₁₋₆ alkyl, C₁₋₆ alkoxy, C₂₋₆ alkenyl, C₂₋₆ alkynyl, C₃₋₁₀ cycloalkyl, 3- to 10-membered heterocyclyl, C₆₋₁₄ aryl, and 5- to 12-membered heteroaryl, each of which is optionally substituted with one or more substituents selected from the group consisting of halo, hydroxy, amino, nitro, mercapto, cyano, oxo, C₁₋₆ alkyl, C₁₋₆ alkoxy, C₂₋₆ alkenyl, C₂₋₆ alkynyl, C₃₋₁₀ cycloalkyl, 3- to 10-membered heterocyclyl, C₆₋₁₄ aryl, and 5- to 12-membered heteroaryl; or when R^{f4} and R^{f5} are attached to the same nitrogen atom, R^{f4} and R^{f5}, together with the nitrogen atom to which they are attached, form 3- to 10-membered heterocyclyl or 5- to 12-membered heteroaryl, each of which is optionally substituted with one or more substituents selected from the group consisting of deuterium, halo, hydroxy, amino, nitro, mercapto, cyano, oxo, C₁₋₆ alkyl, C₁₋₆ alkoxy, C₃₋₁₀ cycloalkyl, 3- to 10-membered heterocyclyl, C₆₋₁₄ aryl, and 5- to 12-membered heteroaryl;
R^{g} is, at each occurrence, independently selected from the group consisting of -R^{g1}, -OR^{g1}, -SR^{g1}, -S(O)R^{g1},-SO₂(R^{g1}), -C(O)R^{g1}, -C(O)OR^{g1}, -OC(O)R^{g1}, -N(R^{g1})(R^{g2}), -C(O)N(R^{g1})(R^{g1}), -C(O)N(R^{g1})SO₂N(R^{g1})(R^{g2}),-C(O)N(R^{g1})SO₂R^{g2}, -P(O)(R^{g1})R^{g2}, -N(R^{g1})C(O)(R^{g2}), -S(O)N(R^{g1})(R^{g2}), -SO₂N(R^{g1})(R^{g2}), -N(R^{g1})S(O)(R^{g2}), and -N(R^{g1})SO₂(R^{g2});
R^{g1} and R^{g2} are, at each occurrence, each independently selected from the group consisting of hydrogen, deuterium, halo, hydroxy, amino, nitro, mercapto, cyano, C₁₋₆ alkyl, C₁₋₆ alkoxy, C₂₋₆ alkenyl, C₂₋₆ alkynyl, C₃₋₁₀ carbocyclyl, 3- to 10-membered heterocyclyl, C₆₋₁₄ aryl, and 5- to 12-membered heteroaryl, each of which is optionally substituted with one or more substituents selected from the group consisting of deuterium, halo, hydroxy, amino, nitro, mercapto, cyano, oxo, C₁₋₆ alkyl, C₁₋₆ alkoxy, C₂₋₆ alkenyl, C₂₋₆ alkynyl, C₃₋₁₀ cycloalkyl, 3- to 10-membered heterocyclyl, C₆₋₁₄ aryl, and 5- to 12-membered heteroaryl;
L₁ is independently selected from the group consisting of a bond, -C(R^{L1})₂-, -O-, -C(R^{L1})₂O-, -S-, -C(O)-,-C(O)O-, -OC(O)-, -N(R^{L1})C(O)-, -C(O)N(R^{L1})-, and -N(R^{L1})-, wherein R^{L1} is independently selected from the group consisting of hydrogen, deuterium, C₁₋₆ alkyl, C₁₋₆ alkoxy, C₂₋₆ alkenyl, C₂₋₆ alkynyl, C₃₋₁₀ cycloalkyl, and 3- to 10-membered heterocyclyl, each of which is optionally substituted with one or more substituents selected from the group consisting of deuterium, halo, hydroxy, amino, nitro, mercapto, cyano, C₁₋₆ alkyl, C₁₋₆ alkoxy, C₁₋₆ alkylaminyl, C₂₋₆ alkenyl, C₂₋₆ alkynyl, C₃₋₁₀ cycloalkyl, and 3- to 10-membered heterocyclyl;
L₂ is independently selected from the group consisting of a bond, -C(R^{L2})₂-, -O-, -C(R^{L1})₂O-, -S-, -C(O)-,-C(O)O-, -OC(O)-, -N(R^{L2})C(O)-, -C(O)N(R^{L2})-, and -N(R^{L2})-, wherein R^{L2} is independently selected from the group consisting of hydrogen, deuterium, C₁₋₆ alkyl, C₁₋₆ alkoxy, C₂₋₆ alkenyl, C₂₋₆ alkynyl, C₃₋₁₀ cycloalkyl, and 3- to 10-membered heterocyclyl, each of which is optionally substituted with one or more substituents selected from the group consisting of deuterium, halo, hydroxy, amino, nitro, mercapto, cyano, C₁₋₆ alkyl, C₁₋₆ alkoxy, C₁₋₆ alkylaminyl, C₂₋₆ alkenyl, C₂₋₆ alkynyl, C₃₋₁₀ cycloalkyl, and 3- to 10-membered heterocyclyl;
L³ is independently selected from the group consisting of a bond, -C(R^{L3})₂-, -O-, -S-, -C(O)-, -C(O)O-, -OC(O)-, -N(R^{L3})C(O)-, -C(O)N(R^{L3})-, and -N(R^{L3})-, wherein R^{L3} is independently selected from the group consisting of hydrogen, deuterium, C₁₋₆ alkyl, C₁₋₆ alkoxy, C₃₋₆ alkenyl, C₃₋₆ alkynyl, C₃₋₁₀ cycloalkyl, and 3- to 10-membered heterocyclyl, each of which is optionally substituted with one or more substituents selected from the group consisting of deuterium, halo, hydroxy, amino, nitro, mercapto, cyano, C₁₋₆ alkyl, C₁₋₆ alkoxy, C₁₋₆ alkylaminyl, C₃₋₆ alkenyl, C₃₋₆ alkynyl, C₃₋₁₀ cycloalkyl, and 3- to 10-membered heterocyclyl;
L₄ is independently selected from the group consisting of a bond, -C(R^{L4})₂-, -O-, -S-, -C(O)-, -C(O)O-, -OC(O)-, -N(R^{L4})C(O)-, -C(O)N(R^{L4})-, -N(R^{L4})-, -CH=CH-, -C(R^{L4})=C-, and -CH=C(R^{L4})-, wherein R^{L4} is independently selected from the group consisting of hydrogen, deuterium, optionally substituted C₁₋₆ alkyl, optionally substituted C₁₋₆ alkoxy, optionally substituted C₂₋₆ alkenyl, optionally substituted C₂₋₆ alkynyl, optionally substituted C₃₋₁₀ cycloalkyl, and optionally substituted 3- to 10-membered heterocyclyl, wherein the term "optionally substituted" means that hydrogen on the substituted group is not substituted, or one or more substitutable sites of the substituted group are independently substituted with one or more substituents selected from the group consisting of deuterium, halo, hydroxy, amino, nitro, mercapto, cyano, oxo, C₁₋₆ alkyl, C₁₋₆ alkoxy, C₁₋₆ alkylaminyl, C₂₋₆ alkenyl, C₂₋₆ alkynyl, C₃₋₁₀ cycloalkyl, and 3- to 10-membered heterocyclyl;
m, n, o, and p are each independently 0, 1, 2, or 3;
unless otherwise stated, the heteroatom(s) in the above-mentioned heterocyclyl and heteroaryl is(are) independently selected from the group consisting of O, N and S, and the number of the heteroatoms is 1, 2, 3 or 4.

In some embodiments, in Formula (I'),
ring A is independently selected from the group consisting of phenyl and 5- to 6-membered heteroaryl;
ring B is independently selected from the group consisting of 8- to 10-membered bicyclic heterocyclyl, phenyl, 5- to 6-membered monocyclic heteroaryl, and 8- to 10-membered bicyclic heteroaryl;
ring C is independently selected from the group consisting of C₈₋₁₀ bicyclic fused carbocyclyl, 8- to 10-membered bicyclic fused heterocyclyl, and 8- to 10-membered bicyclic heteroaryl; 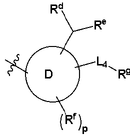 is independently selected from the group consisting of 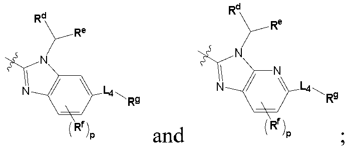
R^{a} is, at each occurrence, independently selected from the group consisting of deuterium, halo, hydroxy, amino, nitro, mercapto, cyano, oxo, -C(O)N(R^{a1})(R^{a2}), -N(R^{a1})C(O)(R^{a2}), C₁₋₆ alkyl, C₁₋₆ alkoxy, C₁₋₆ alkylaminyl, C₃₋₆ cycloalkyl, and 3- to 6-membered heterocyclyl, wherein the C₁₋₆ alkyl, the C₁₋₆ alkoxy, the C₁₋₆ alkylaminyl, the C₃₋₆ cycloalkyl, and the 3- to 6-membered heterocyclyl are optionally substituted with one or more R^{a3};
R^{a1} and R^{a2} are, at each occurrence, each independently selected from the group consisting of hydrogen, deuterium, C₁₋₆ alkyl, C₁₋₆ alkoxy, C₃₋₆ cycloalkyl, 3- to 6-membered heterocyclyl, phenyl, and 5- to 6-membered heteroaryl, wherein the C₁₋₆ alkyl, the C₁₋₆ alkoxy, the C₃₋₆ cycloalkyl, the 3- to 6-membered heterocyclyl, the phenyl, and the 5- to 6-membered heteroaryl are optionally substituted with one or more substituents selected from the group consisting of halo, hydroxy, amino, nitro, mercapto, cyano, oxo, C₁₋₆ alkyl, C₁₋₆ alkoxy, C₃₋₆ cycloalkyl, and 3- to 6-membered heterocyclyl;
R^{a3} is, at each occurrence, independently selected from the group consisting of deuterium, halo, hydroxy, amino, nitro, mercapto, cyano, oxo, and -R^{a4};
R^{a4} is independently selected from the group consisting of hydrogen, deuterium, C₁₋₆ alkyl, and C₁₋₆ alkoxy, wherein the C₁₋₆ alkyl and the C₁₋₆ alkoxy are optionally substituted with one or more substituents selected from the group consisting of halo, hydroxy, amino, nitro, mercapto, and cyano;
R^{b} is, at each occurrence, independently selected from the group consisting of deuterium, halo, hydroxy, amino, nitro, mercapto, cyano, oxo, optionally substituted C₁₋₆ alkyl, and optionally substituted C₁₋₆ alkoxy, wherein the term "optionally substituted" means that hydrogen on the substituted group is not substituted, or one or more substitutable sites of the substituted group are independently substituted with R^{b1};
R^{b1} is, at each occurrence, independently selected from the group consisting of deuterium, halo, hydroxy, amino, nitro, mercapto, cyano, oxo, and -R^{b2};
R^{b2} is independently selected from the group consisting of hydrogen, deuterium, C₁₋₆ alkyl, and C₁₋₆ alkoxy, each of which is optionally substituted with one or more substituents selected from the group consisting of halo, hydroxy, amino, nitro, mercapto, cyano, oxo, C₁₋₆ alkyl, and C₁₋₆ alkoxy;
R^{c} is, at each occurrence, independently selected from the group consisting of deuterium, halo, hydroxy, amino, cyano, oxo, optionally substituted C₁₋₆ alkyl, and optionally substituted C₁₋₆ alkoxy, wherein the term "optionally substituted" means that hydrogen on the substituted group is not substituted, or one or more substitutable sites of the substituted group are independently substituted with R^{c1}; or
two R^{c}s, together with the atom(s) to which they are attached, form 3- to 6-membered heterocyclyl or 5- to 6-membered heteroaryl, each of which is optionally substituted with one or more substituents selected from the group consisting of deuterium, halo, hydroxy, amino, cyano, oxo, methyl, ethyl, methoxy, and ethoxy;
R^{c1} is, at each occurrence, independently selected from the group consisting of deuterium, halo, hydroxy, amino, nitro, mercapto, cyano, oxo, and C₁₋₄ alkyl optionally substituted with one or more substituents selected from the group consisting of halo, hydroxy, amino, and cyano;
one of R^{d} and R^{e} is selected from the group consisting of hydrogen and deuterium, and the other is selected from the group consisting of optionally substituted C₃₋₆ cycloalkyl, optionally substituted 3- to 6-membered heterocyclyl, and optionally substituted 5- to 6-membered heteroaryl, wherein the term "optionally substituted" means that hydrogen on the substituted group is not substituted, or one or more substitutable sites of the substituted group are independently substituted with R^{d1};
R^{d1} is, at each occurrence, independently selected from the group consisting of deuterium, halo, hydroxy, amino, nitro, mercapto, cyano, oxo, -C(O)N(R^{d2})(R^{a3}), -N(R^{d2})C(O)(R^{d3}), C₁₋₆ alkyl, and C₁₋₆ alkoxy, wherein the C₁₋₆ alkyl and the C₁₋₆ alkoxy are optionally substituted with one or more substituents selected from the group consisting of deuterium, halo, hydroxy, amino, nitro, mercapto, cyano, oxo, C₁₋₆ alkyl, C₁₋₆ haloalkyl, and C₁₋₆ alkoxy;
R^{d2} and R^{d3} are, at each occurrence, each independently selected from the group consisting of hydrogen, deuterium, C₁₋₆ alkyl, and C₁₋₆ alkoxy, wherein the C₁₋₆ alkyl and the C₁₋₆ alkoxy are optionally substituted with one or more substituents selected from the group consisting of halo, hydroxy, amino, nitro, mercapto, cyano, oxo, C₁₋₆ alkyl, and C₁₋₆ alkoxy;
R^{f} is, at each occurrence, independently selected from the group consisting of deuterium, halo, hydroxy, amino, nitro, mercapto, cyano, oxo, -OR^{f4}, -C(O)N(R^{f4})(R^{f5}), -N(R^{f4})C(O)(R^{f5}), C₁₋₆ alkyl, C₁₋₆ alkoxy, C₁₋₆ alkylaminyl, C₃₋₆ cycloalkyl, 3- to 6-membered heterocyclyl, phenyl, and 5- to 6-membered heteroaryl, wherein the C₁₋₆ alkyl, the C₁₋₆ alkoxy, the C₁₋₆ alkylaminyl, the C₃₋₆ cycloalkyl, the 3- to 6-membered heterocyclyl, the phenyl, and the 5-to 6-membered heteroaryl are optionally substituted with one or more R^{f1};
R^{f1} is, at each occurrence, independently selected from the group consisting of deuterium, halo, hydroxy, amino, cyano, and -R^{f2};
R^{f2} is independently selected from the group consisting of hydrogen, deuterium, C₁₋₆ alkyl, C₁₋₆ alkoxy, C₃₋₆ cycloalkyl, 3- to 6-membered heterocyclyl, phenyl, and 5- to 6-membered heteroaryl, each of which is optionally substituted with one or more substituents selected from the group consisting of halo, hydroxy, amino, nitro, mercapto, cyano, oxo, C₁₋₆ alkyl, C₁₋₆ alkoxy, C₃₋₆ cycloalkyl, 3- to 6-membered heterocyclyl, phenyl, and 5- to 6-membered heteroaryl;
R^{f4} and R^{f5} are, at each occurrence, each independently selected from the group consisting of hydrogen, deuterium, C₁₋₆ alkyl, C₃₋₆ cycloalkyl, 3- to 6-membered heterocyclyl, phenyl, and 5- to 6-membered heteroaryl;
R^{g} is, at each occurrence, independently selected from -C(O)OR^{g1}, wherein R^{g1} is hydrogen;
L₁ is independently selected from the group consisting of a bond, -C(R^{L1})₂-, -O-, and -C(R^{L1})₂O-, wherein R^{L1} is independently selected from the group consisting of hydrogen, deuterium, and C₁₋₆ alkyl optionally substituted with one or more substituents selected from the group consisting of deuterium, halo, hydroxy, amino, nitro, mercapto, and cyano;
L₂ is independently selected from the group consisting of a bond, -C(R^{L2})₂-, -O-, and -C(R^{L1})₂O-, wherein R^{L2} is independently selected from the group consisting of hydrogen, deuterium, and C₁₋₆ alkyl optionally substituted with one or more substituents selected from the group consisting of deuterium, halo, hydroxy, amino, nitro, mercapto, cyano, and C₁₋₆ alkyl;
L³ is independently selected from the group consisting of a bond, -C(R^{L3})₂-, -O-, -S-, -C(O)-, -C(O)O-, and-OC(O)-, wherein R^{L3} is independently selected from the group consisting of hydrogen, deuterium, and C₁₋₆ alkyl optionally substituted with one or more substituents selected from the group consisting of deuterium, halo, hydroxy, amino, nitro, mercapto, cyano, and C₁₋₆ alkyl;
L₄ is a bond; m, n, o, and p are each independently 0, 1, 2, or 3;
unless otherwise stated, the heteroatom(s) in the above-mentioned heterocyclyl and heteroaryl is(are) independently selected from the group consisting of O, N and S, and the number of the heteroatoms is 1, 2, 3 or 4.

In some embodiments, ring A is independently selected from the group consisting of 3- to 6-membered heterocyclyl, phenyl, and 5- to 6-membered heteroaryl.

In some embodiments, ring A is independently selected from the group consisting of phenyl, 5-membered heteroaryl, and 6-membered heteroaryl, wherein the heteroatom(s) in the heteroaryl is(are) independently selected from the group consisting of N and S, and the number of the heteroatoms is 1 or 2.

In some embodiments, ring A is independently selected from the group consisting of phenyl and pyridyl.

In some embodiments, ring A is independently selected from phenyl.

In some embodiments, 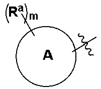 is independently selected from the group consisting of

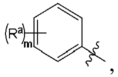

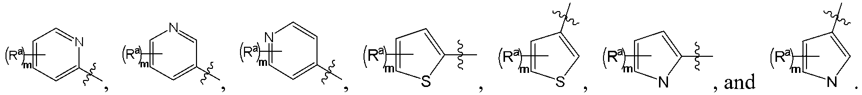

In some embodiments, 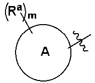 is independently selected from the group consisting of

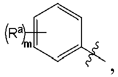

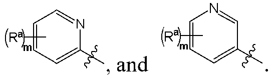

In some embodiments, 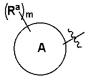 is independently selected from

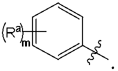

In some embodiments, R^{a} is independently selected from the group consisting of halo, hydroxy, amino, nitro, mercapto, cyano, oxo, -C(O)N(R^{a1})(R^{a2}), -N(R^{a1})C(O)(R^{a2}), C₁₋₆ alkyl, C₁₋₆ alkoxy, and 3- 6 membered heterocyclyl, wherein the C₁₋₆ alkyl, the C₁₋₆ alkoxy, and the 3- to 6-membered heterocyclyl are optionally substituted with one or more R^{a3}; and the heteroatom(s) in the 3- to 6-membered heterocyclyl is(are) selected from the group consisting of O and N, and the number of the heteroatom(s) is 1 or 2.

In some embodiments, R^{a} is independently selected from the group consisting of halo, hydroxy, amino, cyano, -C(O)N(C₁₋₄ alkyl)₂, -C(O)NH(C₁₋₄ alkyl), -C(O)NH₂, C₁₋₄ alkyl, C₁₋₄ alkoxy, and 3- to 6-membered heterocyclyl, wherein the C₁₋₄ alkyl, the C₁₋₄ alkoxy, and the 3- to 6-membered heterocyclyl are optionally substituted with one or more R^{a3}; and the heteroatom(s) in the 3- to 6-membered heterocyclyl is(are) selected from the group consisting of O and N, and the number of the heteroatom(s) is 1 or 2.

In some embodiments, R^{a} is independently selected from the group consisting of halo, hydroxy, amino, cyano, -C(O)NH₂, -C(O)N(CH₃)₂, -C(O)NH(CH₃), methyl, ethyl, n-propyl, isopropyl, n-butyl, methoxy, and ethoxy, wherein the methyl, the ethyl, the n-propyl, the isopropyl, the n-butyl, the methoxy, and the ethoxy are optionally substituted with one or more R^{a3}.

In some embodiments, R^{a} is independently selected from the group consisting of halo, cyano, -C(O)NH₂, C₁₋₃ alkyl, C₁₋₃ alkoxy, and 4- to 6-membered heterocyclyl, wherein the C₁₋₃ alkyl, the C₁₋₃ alkoxy, and the 4- to 6-membered heterocyclyl are optionally substituted with one or more substituents selected from halo; and the heteroatom in the 4- to 6-membered heterocyclyl is O, and the number of the heteroatom is 1.

**In** some embodiments, R^{a} is independently selected from the group consisting of halo, cyano, -C(O)NH₂, C₁₋₆ alkyl, C₁₋₆ haloalkyl, C₁₋₆ alkoxy, C₁₋₆ haloalkoxy, and 3- to 6-membered heterocyclyl, wherein the heteroatom in the 3- to 6-membered heterocyclyl is O, and the number of the heteroatom is 1.

In some embodiments, R^{a} is independently selected from the group consisting of halo, cyano, C₁₋₃ alkyl, C₁₋₃ haloalkyl, C₁₋₃ alkoxy, C₁₋₃ haloalkoxy, and 3- to 6-membered heterocyclyl, wherein the heteroatom in the 3- to 6-membered heterocyclyl is O, and the number of the heteroatom is 1.

In some embodiments, R^{a} is independently selected from the group consisting of halo, cyano, -C(O)NH₂, C₁₋₃ alkyl, C₁₋₃ haloalkyl, C₁₋₃ alkoxy, C₁₋₃ haloalkoxy, and oxetanyl.

In some embodiments, R^{a} is independently selected from the group consisting of halo, cyano, C₁₋₃ alkyl, C₁₋₃ haloalkyl, C₁₋₃ alkoxy, C₁₋₃ haloalkoxy, and oxetanyl.

In some embodiments, R^{a} is independently selected from the group consisting of halo, cyano, C₁₋₂ alkyl, C₁₋₂ haloalkyl (preferably C₁₋₂ fluoroalkyl), C₁₋₂ alkoxy, C₁₋₂ haloalkoxy (preferably C₁₋₂ fluoroalkoxy), and oxetanyl.

In some embodiments, R^{a} is independently selected from the group consisting of halo, cyano, -C(O)NH₂, C₁₋₃ haloalkyl, C₁₋₃ haloalkoxy, and oxetanyl.

In some embodiments, R^{a} is independently selected from the group consisting of fluoro, chloro, bromo, cyano, -C(O)NH₂, -CF₃, -OCHF₂, -OCF₃, methyl, ethyl, methoxy, ethoxy, and

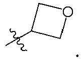

In some embodiments, R^{a} is independently selected from the group consisting of fluoro, chloro, cyano, - C(O)NH₂, -CF₃, -OCHF₂, methyl, methoxy, and

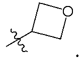

In some embodiments, R^{a} is independently selected from the group consisting of fluoro, chloro, cyano, -CF₃, - OCHF₂, -OCF₃, methyl, methoxy, and

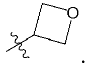

In some embodiments, R^{a} is independently selected from the group consisting of fluoro, chloro, bromo, cyano, -C(O)NH₂, -CF₃, -OCHF₂, methyl, ethyl, methoxy, ethoxy, and

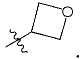

In some embodiments, R^{a} is independently selected from the group consisting of halo, cyano, and -C(O)NH₂.

In some embodiments, R^{a} is independently selected from the group consisting of fluoro, chloro, and cyano.

In some embodiments, R^{a1} and R^{a2} are each independently selected from the group consisting of hydrogen and C₁₋₆ alkyl, wherein the C₁₋₆ alkyl is optionally substituted with one or more substituents selected from the group consisting of halo, hydroxy, and amino.

In some embodiments, R^{a1} and R^{a2} are each independently selected from hydrogen.

In some embodiments, R^{a3} is independently selected from halo; preferably fluoro or chloro; more preferably fluoro.

In some embodiments, m is independently selected from the group consisting of 1 and 2; more preferably 2.

In some embodiments, ring A is a benzene ring, m is 2, one R^{a} is located at the para-position of the attachment point between ring A and L₁, and the other R^{a} is located at the ortho-position of the attachment point between ring A and L₁.

In some embodiments, 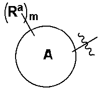 is independently selected from the group consisting of

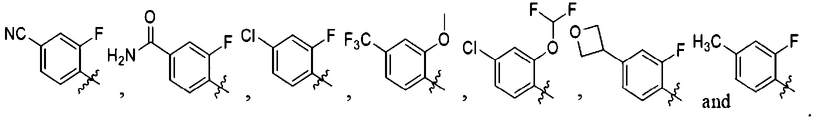

In some embodiments, L₁ is independently selected from the group consisting of a bond and -C (R^{L1})₂O-, wherein R^{L1} is selected from the group consisting of hydrogen, methyl and ethyl, and the methyl and the ethyl are each optionally substituted with one or more substituents selected from the group consisting of fluoro, chloro, bromo, hydroxy, amino, and cyano.

In some embodiments, L₁ is independently selected from the group consisting of a bond and -CH₂O-.

In some embodiments, L₁ is independently selected from the group consisting of a bond and -CH₂O-, wherein the CH₂ group is attached to an end of ring A and the O group is attached to an end of ring B.

In some embodiments, L₁ is independently selected from a bond.

In some embodiments, L₁ is independently selected from -CH₂O-.

In some embodiments, L₁ is independently selected from -CH₂O-, wherein the CH₂ group is attached to an end of ring A and the O group is attached to an end of ring B.

In some embodiments, L₁ is independently selected from -CH₂O-, wherein the CH₂ group is attached to an end of ring B and the O group is attached to an end of ring A.

In some embodiments, ring B is independently selected from the group consisting of 4- to 6-membered heterocycloalkyl, 8- to 10-membered bicyclic heterocyclyl, phenyl, 5- to 6-membered monocyclic heteroaryl, and 8- to 10-membered bicyclic heteroaryl.

In some embodiments, ring B is independently selected from the group consisting of 9-membered bicyclic heterocyclyl and 5- to 6-membered monocyclic heteroaryl, wherein the heteroatom(s) in the 9-membered bicyclic heterocyclyl or the 5- to 6-membered monocyclic heteroaryl is(are) independently selected from the group consisting of N and O, and the number of the heteroatoms is 1 or 2.

In some embodiments, ring B is independently selected from the group consisting of 5- to 6-membered monocyclic heteroaryl, 5-membered/6-membered fused heterocyclyl, and 6-membered/5-membered fused heterocyclyl, wherein the heteroatom(s) in the 5- to 6-membered monocyclic heteroaryl, the 5-membered/6-membered fused heterocyclyl, or the 6-membered/5-membered fused heterocyclyl is(are) independently selected from the group consisting of N and O, and the number of the heteroatoms is 1 or 2.

In some embodiments, ring B is independently selected from the group consisting of phenyl and 6-membered heteroaryl, wherein the heteroatom(s) in the heteroaryl is(are) N, and the number of the heteroatoms is 1 or 2.

In some embodiments, ring B is independently selected from the group consisting of phenyl, pyridyl, pyrimidinyl, and 9-membered bicyclic heterocyclyl, wherein the heteroatom(s) in the 9-membered bicyclic heterocyclyl is(are) O, and the number of the heteroatoms is 2.

In some embodiments, ring B is independently selected from the group consisting of phenyl, pyridyl, pyrimidinyl, and

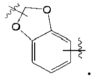

In some embodiments, ring B is independently selected from the group consisting of phenyl, pyridyl, and pyrimidinyl.

In some embodiments, ring B is independently selected from the group consisting of pyridyl and pyrimidinyl.

In some embodiments, ring B is independently selected from pyridyl.

In some embodiments, ring B is independently selected from the group consisting of 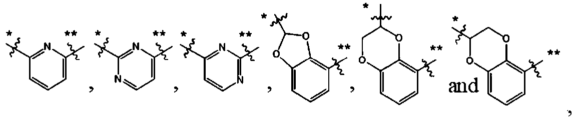 wherein the "*" end denotes the end attached to L₁, and the "**" end denotes the end attached to L₂.

In some embodiments, ring B is independently selected from the group consisting of 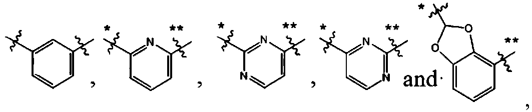 wherein the "*" end denotes the end attached to L₁, and the "**" end denotes the end attached to L₂.

In some embodiments, ring B is independently selected from the group consisting of 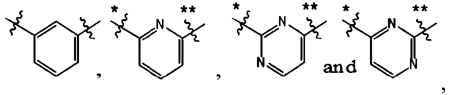 wherein the "*" end denotes the end attached to L₁, and the "**" end denotes the end attached to L₂.

In some embodiments, ring B is independently selected from the group consisting of 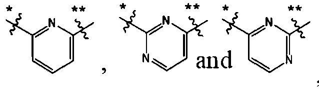 , wherein the "*" end denotes the end attached to L₁, and the "**" end denotes the end attached to L₂.

In some embodiments, ring B is independently selected from 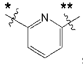 , wherein the "*" end denotes the end attached to L₁, and the "**" end denotes the end attached to L₂.

In some embodiments, R^{b} is independently selected from the group consisting of halo, hydroxy, amino, cyano, optionally substituted C₁₋₄ alkyl, and optionally substituted C₁₋₄ alkoxy, wherein the term "optionally substituted" means that hydrogen on the substituted group is not substituted, or one or more substitutable sites of the substituted group are independently substituted with R^{b1}.

In some embodiments, R^{b} is independently selected from the group consisting of halo, C₁₋₃ alkyl, and C₁₋₃ haloalkyl.

In some embodiments, R^{b} is independently selected from the group consisting of halo and C₁₋₃ alkyl.

In some embodiments, R^{b} is independently selected from C₁₋₃ alkyl.

In some embodiments, R^{b} is independently selected from the group consisting of halo and methyl; preferably halo.

In some embodiments, R^{b} is independently selected from the group consisting of fluoro, chloro, bromo, and methyl; preferably fluoro, chloro, and bromo; more preferably fluoro.

In some embodiments, R^{b} is independently selected from methyl.

In some embodiments, R^{b1} is independently selected from halo (e.g., fluoro, chloro, bromo).

In some embodiments, n is independently selected from the group consisting of 0, 1, and 2; preferably 0 or 1; more preferably 0.

In some embodiments, n is 1 and R^{b} is selected from the group consisting of halo and C₁₋₃ alkyl; or n is 1 and R^{b} is selected from the group consisting of fluoro, chloro, bromo, and methyl.

In some embodiments, n is 1 and R^{b} is fluoro.

In some embodiments, 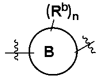 is selected from the group consisting of 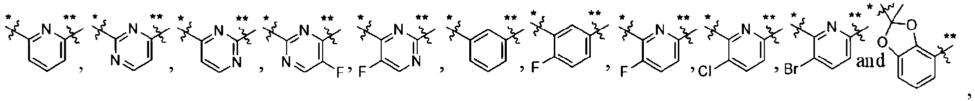 where the "*" end denotes the end attached to L₁, and the "**" end denotes the end attached to L₂.

In some embodiments, 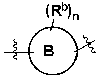 is selected from the group consisting of 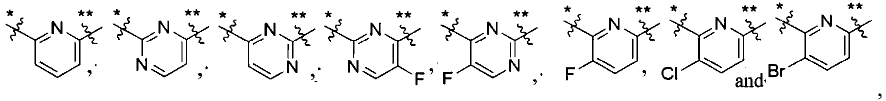 or is selected from the group consisting of 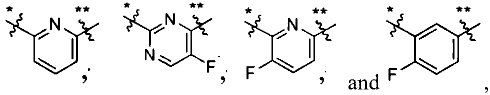 or is selected from the group consisting of 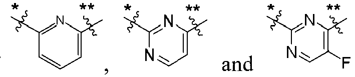 , wherein the "*" end denotes the end attached to L₁, and the "**" end denotes the end attached to L₂.

In some embodiments, L₂ is a bond.

In some embodiments, ring C is independently selected from the group consisting of C₃₋₁₀ carbocyclyl, 3- to 10-membered heterocyclyl, C₆₋₁₀ aryl, and 5- to 10-membered heteroaryl.

In some embodiments, ring C is independently selected from C₉ bicyclic fused carbocyclyl.

In some embodiments, ring C is independently selected from 8- to 10-membered bicyclic fused heterocyclyl.

In some embodiments, ring C is independently selected from 9-membered bicyclic fused heterocyclyl, wherein the heteroatom(s) in the 9-membered bicyclic fused heterocyclyl is(are) O, and the number of the heteroatoms is 1 or 2.

In some embodiments, ring C is independently selected from 9-membered bicyclic heteroaryl, wherein the heteroatom(s) in the 9-membered bicyclic heteroaryl is(are) N, and the number of the heteroatoms is 1 or 2.

In some embodiments, ring C is independently selected from the group consisting of C₈₋₁₀ bicyclic fused carbocyclyl, 8- to 10-membered bicyclic fused heterocyclyl, and 8- to 10-membered bicyclic heteroaryl.

In some embodiments, ring C is independently selected from the group consisting of C₉ bicyclic fused carbocyclyl, 9-membered bicyclic fused heterocyclyl, 10-membered bicyclic fused heterocyclyl, and 9-membered bicyclic heteroaryl, wherein the heteroatom(s) in the C₉ bicyclic fused carbocyclyl, the 9-membered bicyclic fused heterocyclyl, the 10-membered bicyclic fused heterocyclyl, or the 9-membered bicyclic heteroaryl is(are) independently selected from the group consisting of N and O, and the number of the heteroatoms is 1 or 2.

In some embodiments, ring C is independently selected from the group consisting of C₉ bicyclic fused carbocyclyl, 9-membered bicyclic fused heterocyclyl, and 9-membered bicyclic heteroaryl, wherein the heteroatom(s) in the C₉ bicyclic fused carbocyclyl, the 9-membered bicyclic fused heterocyclyl, or the 9-membered bicyclic heteroaryl is(are) independently selected from the group consisting of N and O, and the number of the heteroatoms is 1 or 2.

In some embodiments, ring C is independently selected from the group consisting of 5-membered/6-membered fused heterocyclyl, 6-membered/5-membered fused heterocyclyl, 6-membered/6-membered fused heterocyclyl, 5-membered/6-membered fused carbocyclyl, 6-membered/5-membered fused carbocyclyl, 6-membered/6-membered fused carbocyclyl, 5-membered/6-membered fused heteroaryl, 6-membered/5-membered fused heteroaryl, and 6-membered/6-membered fused heteroaryl.

In some embodiments, ring C is independently selected from the group consisting of 5-membered/6-membered fused heterocyclyl, and 6-membered/5-membered fused heterocyclyl, wherein the heteroatom(s) in the 5-membered/6-membered fused heterocyclyl or the 6-membered/5-membered fused heterocyclyl is(are) O, and the number of the heteroatoms is 1 or 2.

In some embodiments, ring C is independently selected from the group consisting of

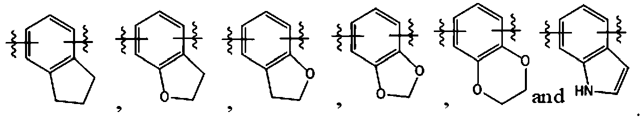

In some embodiments, ring C is independently selected from the group consisting of 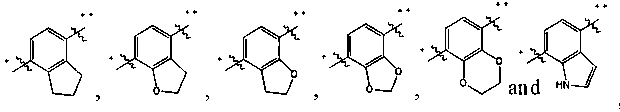 , wherein the "+" end denotes the end attached to L₂, and the "++" end denotes the end attached to L₃.

In some embodiments, ring C is independently selected from the group consisting of 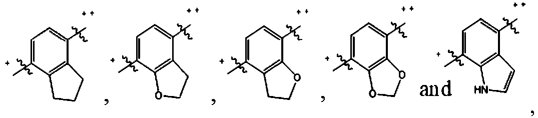 wherein the "+" end denotes the end attached to L₂, and the "++" end denotes the end attached to L₃.

In some embodiments, ring C is independently selected from 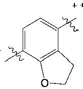 , wherein the "+" end denotes the end attached to L₂, and the "++" end denotes the end attached to L₃.

In some embodiments, when ring C is 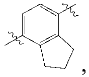 R^{c} is selected from the group consisting of hydroxy, oxo, and halo (preferably fluoro), and o is 1.

In some embodiments, when ring C is 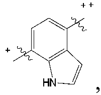 R^{c} is methyl, and o is 0 or 1, wherein the "+" end denotes the end attached to L₂, and the "++" end denotes the end attached to L₃.

In some embodiments, R^{c} is independently selected from the group consisting of halo, hydroxy, amino, cyano, oxo, and optionally substituted C₁₋₆ alkyl, wherein the term "optionally substituted" means that hydrogen on the substituted group is not substituted, or one or more substitutable sites of the substituted group are independently substituted with R^{c1}, wherein R^{c1} is independently selected from the group consisting of halo, hydroxy, amino, and cyano.

In some embodiments, R^{c} is independently selected from the group consisting of halo, hydroxy, amino, cyano, oxo, and C₁₋₄ alkyl.

In some embodiments, R^{c} is independently selected from the group consisting of halo, hydroxy, oxo, and C₁₋₃ alkyl.

In some embodiments, R^{c} is independently selected from the group consisting of fluoro, chloro, hydroxy, oxo, methyl, and ethyl.

In some embodiments, R^{c} is independently selected from the group consisting of fluoro, chloro, oxo, methyl, ethyl, n-propyl, isopropyl, and n-butyl.

In some embodiments, R^{c} is independently selected from the group consisting of fluoro, chloro, oxo, methyl, and -OH.

In some embodiments, R^{c} is independently selected from the group consisting of fluoro, chloro, and methyl.

In some embodiments, R^{c} is independently selected from halo; preferably fluoro or chloro; more preferably fluoro.

In some embodiments, R^{c1} is independently selected from halo (e.g., fluoro, chloro, and bromo).

In some embodiments, o is independently selected from the group consisting of 0, 1, and 2; preferably 0 or 1; more preferably 0.

In some embodiments, 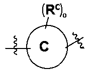 is selected from the group consisting of 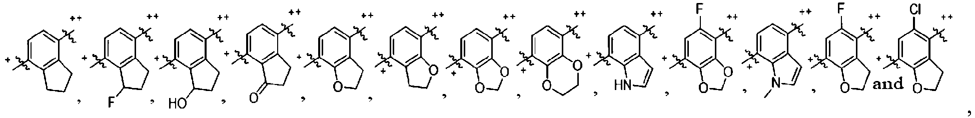 or is selected from the group consisting of 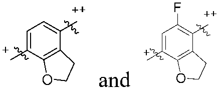 , or is selected from 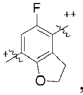 **,** wherein the "+" end denotes the end attached to L₂, and the "++" end denotes the end attached to L₃.

In some embodiments, L³ is independently selected from the group consisting of -C(R^{L3})₂- and -C(O)-; R^{L3} is selected from the group consisting of hydrogen, methyl, ethyl, n-propyl, isopropyl, and n-butyl.

In some embodiments, L₃ is independently selected from the group consisting of -CH₂-, -C(O)-, and -CH(CH₃)-; preferably -CH₂- or -CH(CH₃)-; more preferably -CH₂-.

In some embodiments, ring D is independently selected from the group consisting of 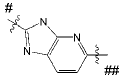 and 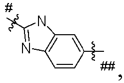 preferably 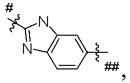 wherein the "#" end denotes the end attached to L₃, and the "##" end denotes the end attached to L₄.

In some embodiments, 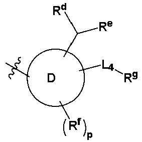 is independently selected from the group consisting of 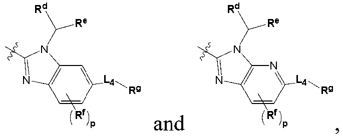 preferably

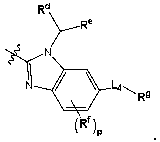

In some embodiments, 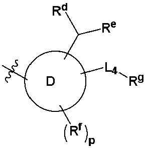 is independently selected from the group consisting of 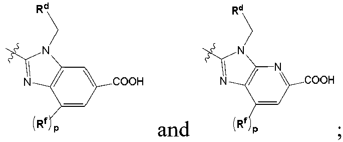 preferably 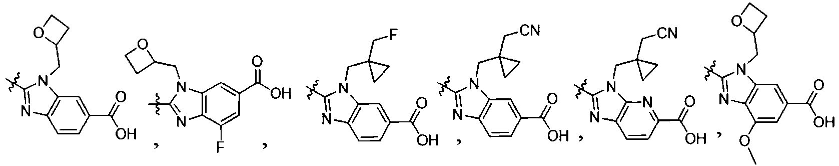 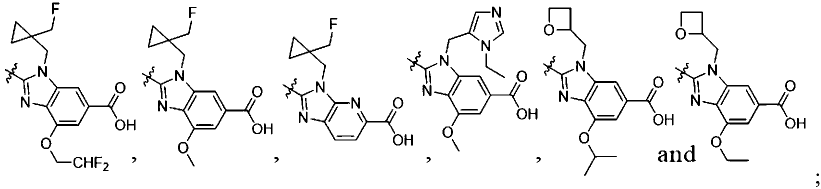 more preferably

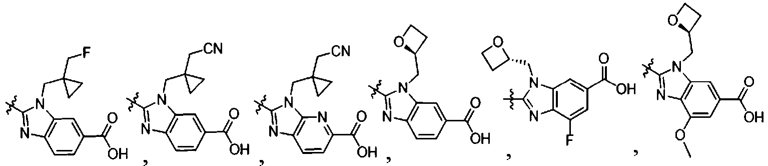

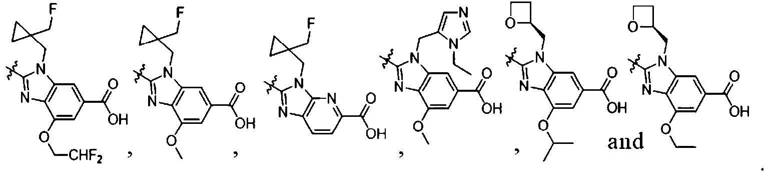

In some embodiments, one of R^{d} and R^{c} is selected from hydrogen and the other is selected from the group consisting of optionally substituted C₃₋₆ cycloalkyl, optionally substituted 3- to 6-membered heterocyclyl, and optionally substituted 5- to 6-membered heteroaryl, wherein the term "optionally substituted" means that hydrogen on the substituted group is not substituted, or one or more substitutable sites of the substituted group are independently substituted with R^{d1}.

In some embodiments, one of R^{d} and R^{e} is selected from hydrogen and the other is selected from the group consisting of optionally substituted C₃₋₄ cycloalkyl, optionally substituted 3- to 4-membered heterocyclyl, and optionally substituted 5- to 6-membered heteroaryl, wherein the heteroatom in the heterocyclyl is O and the number of the heteroatom is 1, and the heteroatom(s) in the heteroaryl is(are) N, and the number of the heteroatoms is 1 or 2 , wherein the term "optionally substituted" means that hydrogen on the substituted group is not substituted, or one or more substitutable sites of the substituted group are independently substituted with R^{d1}.

In some embodiments, R^{d} and R^{e} are each independently selected from the group consisting of hydrogen, and optionally substituted 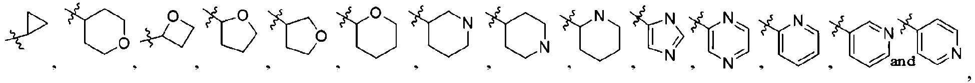 wherein the term "optionally substituted" means that hydrogen on the substituted group is not substituted, or one or more substitutable sites of the substituted group are independently substituted with R^{d1}.

In some embodiments, one of R^{d} and R^{e} is selected from hydrogen and the other is selected from the group consisting of optionally substituted 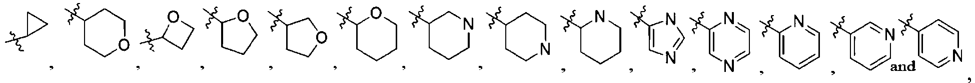 wherein the term "optionally substituted" means that hydrogen on the substituted group is not substituted, or one or more substitutable sites of the substituted group are independently substituted with R^{d1}.

In some embodiments, R^{d} and R^{e} are each independently selected from the group consisting of hydrogen, optionally substituted 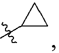 , optionally substituted 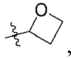 , and optionally substituted 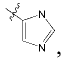 wherein the term "optionally substituted" means that hydrogen on the substituted group is not substituted, or one or more substitutable sites of the substituted group are independently substituted with R^{d1}.

In some embodiments, R^{d} and R^{e} are each independently selected from the group consisting of hydrogen, optionally substituted 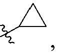 and optionally substituted 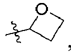 , wherein the term "optionally substituted" means that hydrogen on the substituted group is not substituted, or one or more substitutable sites of the substituted group are independently substituted with R^{d1}.

In some embodiments, R^{e} is hydrogen, and R^{d} is selected from the group consisting of optionally substituted 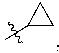 , optionally substituted 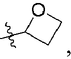 , and optionally substituted 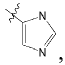 wherein the term "optionally substituted" means that hydrogen on the substituted group is not substituted, or one or more substitutable sites of the substituted group are independently substituted with R^{d1}, wherein R^{d1} is independently selected from the group consisting of - N(C₁₋₂ alkyl)C(O)(C₁₋₂ alkyl), and C₁₋₃ alkyl optionally substituted with one or more substituents selected from the group consisting of fluoro, chloro, and cyano.

In some embodiments, one of R^{d} and R^{e} is selected from hydrogen and the other is selected from the group consisting of optionally substituted 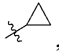 , and optionally substituted 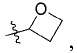 wherein the term "optionally substituted" means that hydrogen on the substituted group is not substituted, or one or more substitutable sites of the substituted group are independently substituted with R^{d1}, wherein R^{d1} is independently selected from the group consisting of C(O)N(R^{d2})(R^{a3}), -N(R^{d2})C(O)(R^{d3}), methyl, ethyl, n-propyl, isopropyl, and n-butyl, and the methyl, ethyl, n-propyl, isopropyl, or n-butyl is optionally substituted with one or more substituents selected from the group consisting of fluoro, chloro, and cyano; R^{d2} and R^{d3} are, at each occurrence, each independently selected from the group consisting of hydrogen, methyl, and ethyl.

In some embodiments, 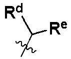 is independently selected from the group consisting of

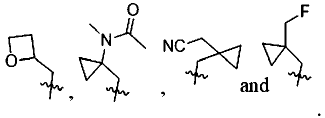

In some embodiments, 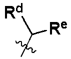 is independently selected from the group consisting of

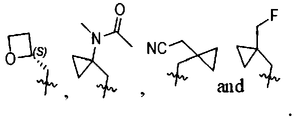

In some embodiments, 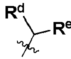 is independently selected from the group consisting of

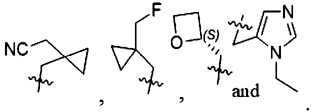

In some embodiments, 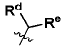 is independently selected from the group consisting of 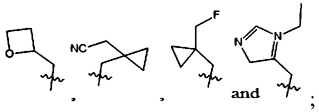 preferably 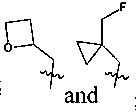 ; more preferably 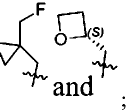 more preferably 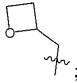 ; more preferably

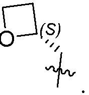

In some embodiments,

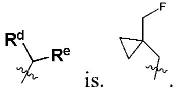

In some embodiments, R^{d1} is independently selected from C₁₋₆ alkyl optionally substituted with one or more substituents selected from the group consisting of halo, hydroxy, amino, and cyano.

In some embodiments, R^{d1} is independently selected from C₁₋₄ alkyl (e.g., methyl, ethyl, n-propyl, isopropyl, n-butyl) optionally substituted with one or more substituents selected from the group consisting of fluoro, chloro, and cyano.

In some embodiments, R^{d1} is independently selected from the group consisting of fluoro, chloro, bromo, hydroxy, amino, cyano, trifluoromethyl, difluoromethyl, monofluoromethyl, trichloromethyl, dichloromethyl, monochloromethyl, methyl, ethyl, n-propyl, isopropyl, n-butyl, methoxy, ethoxy, -CH₂CN, and -CH₂CH₂CN.

In some embodiments, R^{d1} is independently selected from the group consisting of monofluoromethyl, -CH₂CN, methyl, and ethyl.

In some embodiments, R^{d1} is independently selected from the group consisting of monofluoromethyl, and-CH₂CN.

In some embodiments, R^{f} is independently selected from the group consisting of halo, hydroxy, amino, cyano, -OR^{f4}, -C(O)N(R^{f4})(R^{f5}), -N(R^{f4})C(O)(R^{f5}), C₁₋₆ alkyl, and C₁₋₆ alkoxy, wherein the C₁₋₆ alkyl and the C₁₋₆ alkoxy are optionally substituted with one or more R^{f1}.

In some embodiments, R^{f} is independently selected from the group consisting of halo, hydroxy, amino, cyano, -OR^{f4}, -C(O)N(R^{f4})(R^{f5}), -N(R^{f4})C(O)(R^{f5}), C₁₋₄ alkyl, and C₁₋₄ alkoxy, wherein the C₁₋₄ alkyl and the C₁₋₄ alkoxy are optionally substituted with one or more R^{f1}.

In some embodiments, R^{f} is independently selected from the group consisting of halo, hydroxy, amino, cyano, -O(C₃₋₆ cycloalkyl), -C(O)NH(C₁₋₃ alkyl), -NHC(O)(C₁₋₃ alkyl), C₁₋₃ alkyl, and C₁₋₃ alkoxy, wherein the C₁₋₃ alkyl and the C₁₋₃ alkoxy are optionally substituted with one or more substituents selected from the group consisting of fluoro, chloro and phenyl.

In some embodiments, R^{f} is independently selected from the group consisting of halo, and C₁₋₃ alkoxy, wherein the C₁₋₃ alkoxy is optionally substituted with one or more fluoro.

In some embodiments, R^{f} is independently selected from the group consisting of fluoro, chloro, hydroxy, amino, -O-cyclopropyl, -C(O)NHCH₃, -NHC(O)CH₃, -CH₃, -CH₂CH₃, -CH(CH₃)₂, -OCH₃, -OCH₂CH₃, -OCH(CH₃)₂,-OCHF₂, -OCF₃, -OCH₂-phenyl, -OCH₂CF₃, and -OCH₂CHF₂.

In some embodiments, R^{f} is independently selected from halo.

In some embodiments, R^{f} is independently selected from the group consisting of fluoro and chloro.

In some embodiments, R^{f} is independently selected from the group consisting of halo, C₁₋₆ alkoxy, and C₁₋₆ haloalkoxy.

In some embodiments, R^{f} is independently selected from the group consisting of fluoro, chloro, bromo, C₁₋₄ alkoxy, and C₁₋₃ haloalkoxy.

In some embodiments, R^{f} is independently selected from the group consisting of fluoro, chloro, -OCH₃,-OCH₂CH₃, -OCH(CH₃)₂, -OCHF₂, -OCF₃, -OCH₂CF₃, and -OCH₂CHF₂.

In some embodiments, R^{f} is independently selected from the group consisting of fluoro, -OCH₃, -OCH₂CH₃,-OCH(CH₃)₂, and -OCH₂CHF₂.

In some embodiments, R^{f} is -OCH₃.

In some embodiments, R^{f1} is independently selected from the group consisting of halo, hydroxy, amino, cyano, and -R^{f2}; R^{f2} and R^{f3} are each independently selected from the group consisting of hydrogen, C₁₋₄ alkyl and phenyl, and the C₁₋₄ alkyl or phenyl is optionally substituted with one or more substituents selected from the group consisting of halo, hydroxy, amino, and cyano.

In some embodiments, R^{f1} is independently selected from the group consisting of fluoro, chloro, methyl, and phenyl.

In some embodiments, R^{f1} is halo; preferably fluoro.

In some embodiments, p is independently selected from the group consisting of 0, 1, and 2; preferably 0 or 1; preferably 1.

In some embodiments, p is 0.

In some embodiments, L₄ is a bond.

In some embodiments, R^{g} is independently selected from -COOH.

In some embodiments, 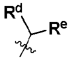 is independently selected from the group consisting of 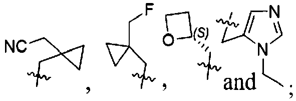 L₄ is a bond; R^{g} is -COOH; p is 0 or 1; R^{f} is selected from the group consisting of halo and C₁₋₃ alkoxy; and the C₁₋₃ alkoxy is optionally substituted with one or more substituents selected from fluoro; or R^{f} is independently selected from the group consisting of fluoro, -OCH₃, -OCH₂CH₃, -OCH(CH₃)₂, and -OCH₂CHF₂.

In some embodiments, m, n, and o are each independently 0, 1, or 2; and p is 1.

In some embodiments, m is 1 or 2, and n, o, and p are all 0; in some embodiments, m is 1 or 2, n and o are both 0, and p is 1; in some embodiments, m is 2, n is 0 or 1, o is 1, and p is 1.

In some embodiments, ring A is phenyl, m is 2, and two R^{a}s are located at the ortho-position and para-position of the attachment point between ring A and L₁, respectively.

In some embodiments, ring A is phenyl, m is 2, two R^{a}s are located at the ortho-position and para-position of the attachment point between ring A and L₁, respectively, and R^{a} is independently selected from the group consisting of halo, cyano, -C(O)NH₂, C₁₋₃ alkyl, C₁₋₃ haloalkyl, C₁₋₃ alkoxy, C₁₋₃ haloalkoxy, and oxetanyl.

In some embodiments, ring A is phenyl, m is 2, two R^{a}s are located at the ortho-position and para-position of the attachment point between ring A and L₁, respectively, and R^{a} is independently selected from the group consisting of fluoro, chloro, cyano, -C(O)NH₂, -CF₃, -OCHF₂, methyl, methoxy, and

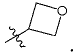

In some embodiments, ring A is phenyl; ring B is phenyl, pyridyl, or pyrimidinyl; ring C is 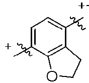 , wherein the "+" end denotes the end attached to L₂, and the "++" end denotes the end attached to L₃; R^{a} is halo (preferably fluoro or chloro), cyano, C₁₋₂ alkyl, C₁₋₂ haloalkyl (preferably C₁₋₂ fluoroalkyl), C₁₋₂ alkoxy, C₁₋₂ haloalkoxy (preferably C₁₋₂ fluoroalkoxy), or oxetanyl; R^{b} is halo (preferably fluoro); R^{c} is fluoro; 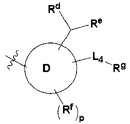 is 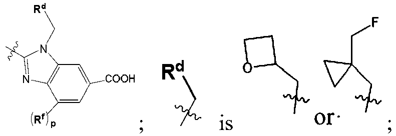 R^{f} is hydrogen, halo (preferably fluoro), C₁₋₃ alkoxy (preferably - OCH₃), or C₁₋₃ haloalkoxy (preferably -OCH₂CHF₂); m is 2, n is 0 or 1, o is 1, p is 0 or 1; and L₁ is -CH₂O-, wherein the CH₂ group is attached to an end of ring A and the O group is attached to an end of ring B; L₂ is a bond; L₃ is - CH₂-.

In some embodiments, 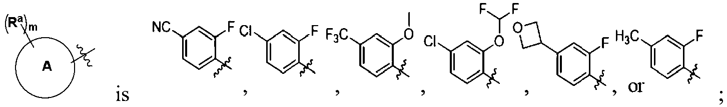 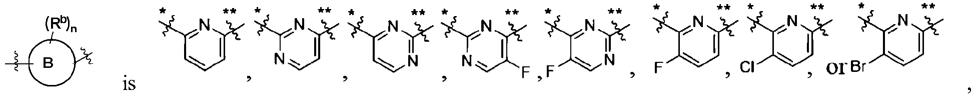 wherein the "*" end denotes the end attached to L₁, and the "**" end denotes the end attached to L₂; 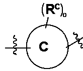 is 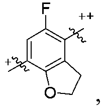 wherein the " +" end denotes the end attached to L₂, and the "++"end denotes the end attached to L₃; 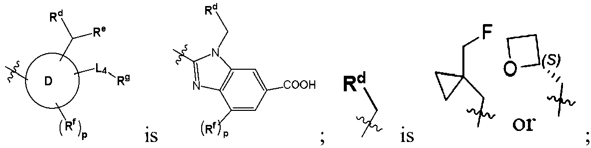 R^{f} is -OCH₃, -OCH₂CH₃, -OCH(CH₃)₂, or - OCH₂CHF₂(R^{f} is preferably -OCH₃); p is 1; L₁ is -CH₂O-, wherein the CH₂ group is attached to an end of ring A and the O group is attached to an end of ring B; L₂ is a bond; L₃ is -CH₂-.

In some embodiments, ring A is independently selected from the group consisting of phenyl and pyridyl; ring B is independently selected from the group consisting of 9-membered bicyclic fused heterocyclyl and 5- to 6-membered monocyclic heteroaryl; ring C is independently selected from the group consisting of C₉ bicyclic fused carbocyclyl, 9-membered bicyclic fused heterocyclyl, 10-membered bicyclic fused heterocyclyl, 9-membered bicyclic heteroaryl, piperidinyl, and piperazinyl; 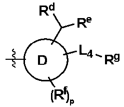 is independently selected from 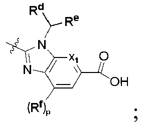 X₁ is CH or N;
R^{a} is independently selected from the group consisting of halo, hydroxy, amino, cyano, -C(O)N(C₁₋₄ alkyl)₂, - C(O)NH(C₁₋₄ alkyl), -C(O)NH₂, C₁₋₄ alkyl, C₁₋₄ alkoxy, and 4- to 6-membered heterocyclyl, wherein the C₁₋₄ alkyl, the C₁₋₄ alkoxy, and the 4- to 6-membered heterocyclyl is optionally substituted with one or more substituents selected from the group consisting of halo, hydroxy, and amino; and the heteroatom in the 4- to 6-membered heterocyclyl is O, and the number of the heteroatom is 1;
R^{b} is independently selected from C₁₋₄ alkyl;
R^{c} is independently selected from the group consisting of halo, hydroxy, amino, cyano, oxo, C₁₋₄ alkyl, and C₁₋₄ haloalkyl;
R^{f} is independently selected from the group consisting of halo, hydroxy, amino, cyano, -O(C₃₋₆ cycloalkyl), - C(O)NH(C₁₋₃ alkyl), -NHC(O)(C₁₋₃ alkyl), C₁₋₃ alkyl, and C₁₋₃ alkoxy, wherein the C₁₋₃ alkyl and the C₁₋₃ alkoxy are optionally substituted with one or more substituents selected from the group consisting of fluoro, chloro and phenyl;
L₁ is independently selected from the group consisting of a bond and -CH₂O-; L₂ is independently selected from the group consisting of a bond and -CH₂-; L₃ is independently selected from the group consisting of -CH₂-, - C(O)-, and -CH(C₁₋₄ alkyl)-; L₄ is independently selected from a bond;
m, n, o, and p are each independently 0, 1, 2, or 3; and
the compound is not the following compounds:

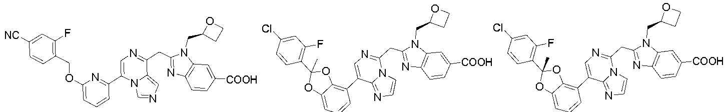

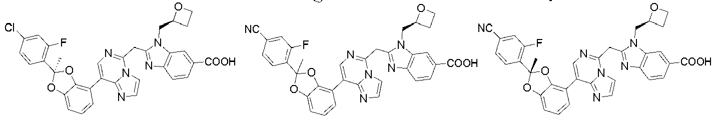

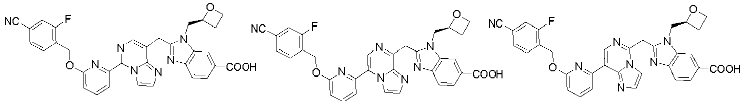

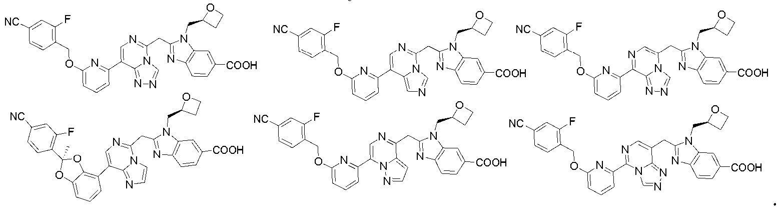

In some embodiments, ring A is independently selected from the group consisting of phenyl and 5- to 6-membered heteroaryl;
ring B is independently selected from the group consisting of 9-membered bicyclic heterocyclyl and 5- to 6-membered monocyclic heteroaryl, wherein the heteroatom(s) in the 9-membered bicyclic heterocyclyl or the 5- to 6-membered monocyclic heteroaryl is(are) independently selected from the group consisting of N and O, and the number of the heteroatoms is 1 or 2;
ring C is independently selected from the group consisting of C₉ bicyclic fused carbocyclyl, 9-membered bicyclic fused heterocyclyl, and 9-membered bicyclic heteroaryl, wherein the heteroatom(s) in the C₉ bicyclic fused carbocyclyl, the 9-membered bicyclic fused heterocyclyl, or the 9-membered bicyclic heteroaryl is(are) independently selected from the group consisting of N and O, and the number of the heteroatoms is 1 or 2;
ring D is independently selected from the group consisting of 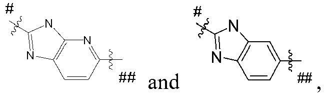 wherein the "#" end denotes the end attached to L₃ and the "##" end denotes the end attached to L₄;
R^{a} is independently selected from the group consisting of fluoro, chloro, cyano, -C(O)NH₂, -C(O)N(CH₃)₂, - C(O)NH(CH₃), methyl, ethyl, methoxy, and ethoxy;
R^{b} is independently selected from methyl;
R^{c} is independently selected from the group consisting of fluoro, chloro, oxo, methyl, ethyl, n-propyl, isopropyl, and n-butyl;
one of R^{d} and R^{e} is selected from hydrogen, and the other is selected from the group consisting of optionally substituted C₃₋₆ cycloalkyl and optionally substituted 3- to 6-membered heterocyclyl, wherein the term "optionally substituted" means that hydrogen on the substituted group is not substituted, or one or more substitutable sites of the substituted group are independently substituted with R^{d1}, wherein R^{d1} is independently selected from C₁₋₄ alkyl optionally substituted with one or more substituents selected from the group consisting of fluoro, chloro, hydroxy, amino, and cyano;
R^{f} is independently selected from the group consisting of fluoro and chloro; R^{g} is independently selected from -COOH;
L₁ is independently selected from the group consisting of a bond and -CH₂O-; L₂ is independently selected from a bond; L₃ is independently selected from the group consisting of -CH₂-, -C(O)-, and -CH(CH₃)-; L₄ is independently selected from a bond; and
m, n, o, and p are each independently 0, 1, or 2.

In some embodiments, ring A is independently selected from the group consisting of phenyl and pyridyl; ring B is independently selected from pyridyl; ring C is independently selected from the group consisting of C₉ bicyclic fused carbocyclyl, 9-membered bicyclic fused heterocyclyl, and 9-membered bicyclic heteroaryl, wherein the heteroatom(s) in the C₉ bicyclic fused carbocyclyl, the 9-membered bicyclic fused heterocyclyl, or the 9-membered bicyclic heteroaryl is(are) independently selected from the group consisting of N and O, and the number of the heteroatoms is 1 or 2;
ring D is independently selected from the group consisting of 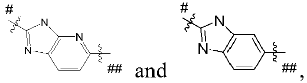 wherein the "#" end denotes the end attached to L₃ and the "##" end denotes the end attached to L₄;
R^{a} is independently selected from the group consisting of fluoro, chloro, cyano, -C(O)NH₂, -C(O)N(CH₃)₂, - C(O)NH(CH₃), methyl, ethyl, methoxy, and ethoxy;
R^{b} is independently selected from methyl;
R^{c} is independently selected from the group consisting of fluoro, chloro, oxo, methyl, ethyl, n-propyl, isopropyl, and n-butyl;
one of R^{d} and R^{e} is selected from hydrogen, and the other is selected from the group consisting of optionally substituted C₃₋₆ cycloalkyl and optionally substituted 3- to 6-membered heterocyclyl, wherein the term "optionally substituted" means that hydrogen on the substituted group is not substituted, or one or more substitutable sites of the substituted group are independently substituted with R^{d1}, wherein R^{d1} is independently selected from C₁₋₄ alkyl optionally substituted with one or more substituents selected from the group consisting of fluoro, chloro, hydroxy, amino, and cyano;
R^{f} is independently selected from the group consisting of fluoro and chloro; R^{g} is independently selected from -COOH;
L₁ is independently selected from the group consisting of a bond and -CH₂O-; L₂ is independently selected from a bond; L₃ is independently selected from the group consisting of -CH₂-, -C(O)-, and -CH(CH₃)-; L₄ is independently selected from a bond; and
m, n, o, and p are each independently 0, 1, or 2.

In some embodiments, ring A is independently selected from the group consisting of phenyl and 5- to 6-membered heteroaryl; ring B is independently selected from the group consisting of 9-membered bicyclic fused heterocyclyl and 5- to 6-membered monocyclic heteroaryl; ring C is independently selected from 9-membered bicyclic fused heterocyclyl;
ring D is independently selected from the group consisting of 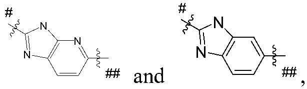 wherein the "#" end denotes the end attached to L₃ and the "##" end denotes the end attached to L₄;
R^{a} is independently selected from the group consisting of halo, hydroxy, amino, cyano, -C(O)N(C₁₋₄ alkyl)₂, - C(O)NH(C₁₋₄ alkyl), -C(O)NH₂, C₁₋₄ alkyl, and C₁₋₄ alkoxy, wherein the C₁₋₄ alkyl and the C₁₋₄ alkoxy are optionally substituted with one or more substituents selected from the group consisting of halo, hydroxy, and amino;
R^{b} is independently selected from C₁₋₆ alkyl;
R^{c} is independently selected from the group consisting of halo, hydroxy, amino, cyano, oxo, C₁₋₄ alkyl, and C₁₋₄ haloalkyl;
one of R^{d} and R^{e} is selected from hydrogen, and the other is selected from the group consisting of optionally substituted C₃₋₆ cycloalkyl and optionally substituted 3- to 6-membered heterocyclyl, wherein the term "optionally substituted" means that hydrogen on the substituted group is not substituted, or one or more substitutable sites of the substituted group are independently substituted with R^{d1}, wherein R^{d1} is independently selected from C₁₋₆ alkyl optionally substituted with one or more substituents selected from the group consisting of halo, hydroxy, amino, and cyano;
R^{f} is independently selected from halo; R^{g} is selected from -COOH;
L₁ is independently selected from the group consisting of a bond and -CH₂O-; L₂ is independently selected from the group consisting of a bond and -CH₂-; L₃ is independently selected from the group consisting of -CH₂-, - C(O)-, and -CH(C₁₋₄ alkyl)-; L₄ is independently selected from a bond; and
m, n, o, and p are each independently 0, 1, or 2.

In some embodiments, ring A is independently selected from the group consisting of phenyl and 5- to 6-membered heteroaryl; ring B is independently selected from the group consisting of 9-membered bicyclic fused heterocyclyl and 5- to 6-membered monocyclic heteroaryl; ring C is independently selected from the group consisting of C₉ bicyclic fused carbocyclyl, 9-membered bicyclic fused heterocyclyl, 10-membered bicyclic fused heterocyclyl, 9-membered bicyclic heteroaryl, piperidinyl, and piperazinyl; 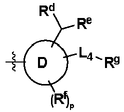 is independently selected from 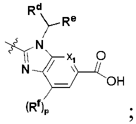 X₁ is CH or N;
R^{a} is independently selected from the group consisting of halo, hydroxy, amino, cyano, -C(O)N(C₁₋₄ alkyl)₂, - C(O)NH(C₁₋₄ alkyl), -C(O)NH₂, C₁₋₄ alkyl, C₁₋₄ alkoxy, and 4- to 6-membered heterocyclyl, wherein the C₁₋₄ alkyl, the C₁₋₄ alkoxy, and the 4- to 6-membered heterocyclyl are optionally substituted with one or more substituents selected from the group consisting of halo, hydroxy, and amino; and the heteroatom in the 4- to 6-membered heterocyclyl is O, and the number of the heteroatom is 1;
R^{b} is independently selected from C₁₋₄ alkyl;
R^{c} is independently selected from the group consisting of halo, hydroxy, amino, cyano, oxo, C₁₋₄ alkyl, and C₁₋₄ haloalkyl;
R^{f} is independently selected from the group consisting of fluoro, chloro, bromo, hydroxy, amino, cyano, -O(C₃₋₆ cycloalkyl), -C(O)NH(C₁₋₃ alkyl), -NHC(O)(C₁₋₃ alkyl), C₁₋₃ alkyl, and C₁₋₃ alkoxy, wherein the C₁₋₃ alkyl and the C₁₋₃ alkoxy are optionally substituted with one or more substituents selected from the group consisting of fluoro, chloro and phenyl;
L₁ is independently selected from the group consisting of a bond and -CH₂O-; L₂ is independently selected from the group consisting of a bond and -CH₂-; L₃ is independently selected from the group consisting of -CH₂-, - C(O)-, and -CH(C₁₋₄ alkyl)-; L₄ is independently selected from a bond;
m, n, and o are each independently 0, 1, or 2; and p is 1; and
the compound is not the following compounds:

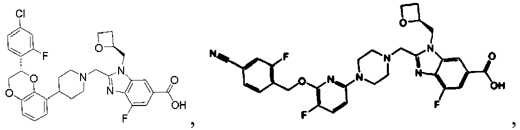

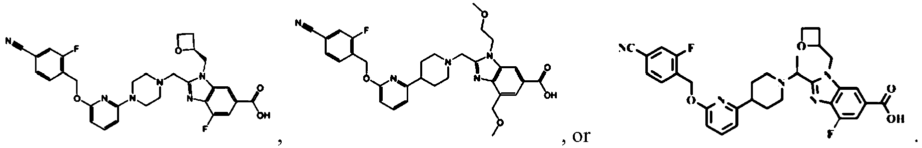

In another aspect, the present application provides a compound represented by Formula (II) or Formula (III), or a tautomer, stereoisomer, or pharmaceutically acceptable salt thereof: 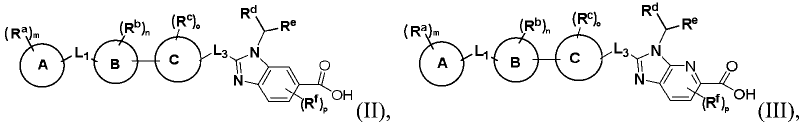 wherein ring A, ring B, ring C, L₁, L₃, R^{a}, R^{b}, R^{c}, R^{d}, R^{e}, R^{f}, m, n, o, and p are as defined in the compound represented by Formula (I') of the present application.

In some embodiments, ring A is independently selected from the group consisting of phenyl and pyridyl;
ring B is independently selected from the group consisting of 9-membered bicyclic fused heterocyclyl, pyridyl, phenyl, and pyrimidinyl, wherein the heteroatom(s) in the 9-membered bicyclic fused heterocyclyl is(are) O, and the number of the heteroatoms is 1 or 2; and
ring C is independently selected from the group consisting of C₉ bicyclic fused carbocyclyl, 9-membered bicyclic fused heterocyclyl, 10-membered bicyclic fused heterocyclyl, and 9-membered bicyclic heteroaryl, wherein the heteroatom(s) in the C₉ bicyclic fused carbocyclyl, the 9-membered bicyclic fused heterocyclyl, the 10-membered bicyclic fused heterocyclyl, or the 9-membered bicyclic heteroaryl is(are) independently selected from the group consisting of N and O, and the number of the heteroatoms is 1 or 2.

In some embodiments, ring A is independently selected from the group consisting of phenyl and pyridyl;
ring B is independently selected from the group consisting of 9-membered bicyclic fused heterocyclyl, 10-membered bicyclic fused heterocyclyl, and pyridyl, wherein the heteroatom(s) in the 9-membered bicyclic fused heterocyclyl or the 10-membered bicyclic fused heterocyclyl is(are) O, and the number of the heteroatoms is 1 or 2;
ring C is independently selected from the group consisting of C₉ bicyclic fused carbocyclyl, 9-membered bicyclic fused heterocyclyl, 10-membered bicyclic fused heterocyclyl, and 9-membered bicyclic heteroaryl, wherein the heteroatom(s) in the C₉ bicyclic fused carbocyclyl, the 9-membered bicyclic fused heterocyclyl, the 10-membered bicyclic fused heterocyclyl, or the 9-membered bicyclic heteroaryl is(are) independently selected from the group consisting of N and O, and the number of the heteroatoms is 1 or 2.

In some embodiments, ring C is independently selected from the group consisting of 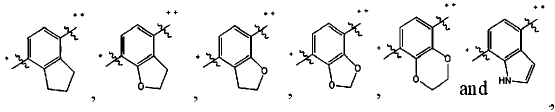 wherein the "+" end denotes the end attached to L₂, and the "++" end denotes the end attached to L₃.

In some embodiments, ring A is independently selected from the group consisting of phenyl and pyridyl;
ring B is independently selected from the group consisting of 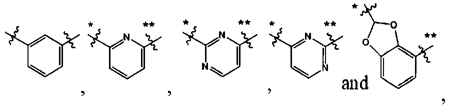 wherein the "*" end denotes the end attached to L₁, and the "**" end denotes the end attached to L₂.

In some embodiments, ring A is independently selected from phenyl.

In yet another aspect, the present application provides a compound represented by Formula (II-1) or Formula (III-1), or a tautomer, stereoisomer, or pharmaceutically acceptable salt thereof: 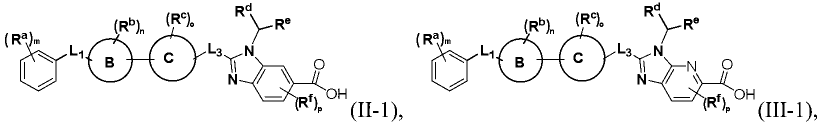 wherein ring B, ring C, L₁, L₃, R^{a}, R^{b}, R^{c}, R^{d}, R^{e}, R^{f}, m, n, o, and p are as defined in the compound represented by Formula (I'), Formula (II), or Formula (III) of the present application.

In yet another aspect, the present application provides a compound represented by Formula (II-2), Formula (II-3), Formula (III-2), or Formula (III-3), or a tautomer, stereoisomer, or pharmaceutically acceptable salt thereof: 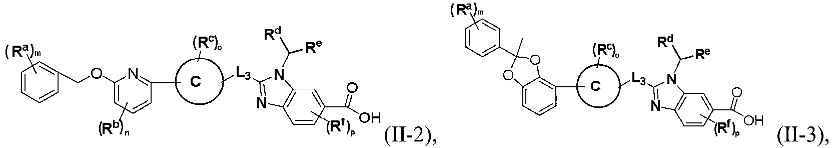 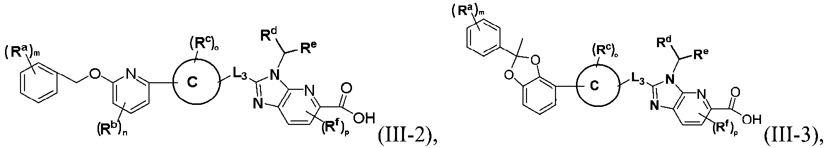 wherein ring C, L₁, L₃, R^{a}, R^{b}, R^{c}, R^{d}, R^{e}, R^{f}, m, n, o, and p are as defined in the compound represented by Formula (I'), Formula (II), Formula (III), Formula (II-1), or Formula (III-1) of the present application.

In some embodiments, ring C is independently selected from the group consisting of C₉ bicyclic fused carbocyclyl, 9-membered bicyclic fused heterocyclyl, 10-membered bicyclic fused heterocyclyl, and 9-membered bicyclic heteroaryl, wherein the heteroatom(s) in the C₉ bicyclic fused carbocyclyl, the 9-membered bicyclic fused heterocyclyl, the 10-membered bicyclic fused heterocyclyl, or the 9-membered bicyclic heteroaryl is(are) independently selected from the group consisting of N and O, and the number of the heteroatoms is 1 or 2.

In some embodiments, ring C is independently selected from the group consisting of: 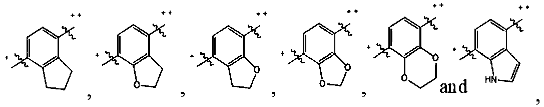 or is selected from 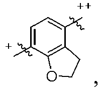 **wherein** the "+" end denotes the end attached to L₂, and the "++" end denotes the end attached to L₃.

In some embodiments, 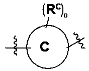 is selected from the group consisting of 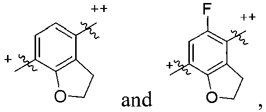 or is selected from 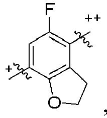 , wherein the "+" end denotes the end attached to L₂, and the "++" end denotes the end attached to L₃.

In a further aspect, the present application provides a compound represented by Formula (IV), or a tautomer, a stereoisomer, or a pharmaceutically acceptable salt thereof: 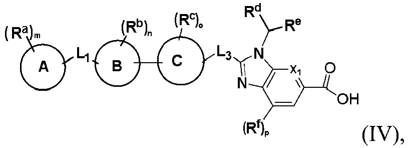 wherein X₁ is CH or N;
ring A, ring B, ring C, L₁, L₃, R^{a}, R^{b}, R^{c}, R^{d}, R^{e}, R^{f}, m, n, o, and p are as defined in the compound represented by Formula (I'), Formula (II), Formula (III), Formula (II-1), Formula (II-2), Formula (II-3), Formula (III-1), Formula (III-2), or Formula (III-3) of the present application.

In some embodiments, ring A is phenyl or pyridyl.

In some embodiments, ring B is independently selected from the group consisting of 9-membered bicyclic fused heterocyclyl, pyridyl, phenyl, and pyrimidinyl, wherein the heteroatoms in the 9-membered bicyclic fused heterocyclyl are O, and the number of the heteroatoms is 2.

In some embodiments, ring C is independently selected from the group consisting of C₉ bicyclic fused carbocyclyl, 9-membered bicyclic fused heterocyclyl, 10-membered bicyclic fused heterocyclyl, and 9-membered bicyclic heteroaryl, wherein the heteroatom(s) in the 9-membered bicyclic fused heterocyclyl, the 10-membered bicyclic fused heterocyclyl, or the 9-membered bicyclic heteroaryl is(are) independently selected from the group consisting of N and O, and the number of the heteroatoms is 1 or 2.

In some embodiments, ring C is independently selected from the group consisting of 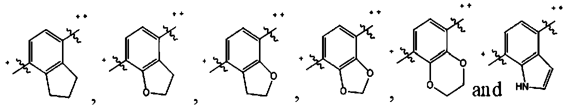 , or is selected from 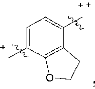 , wherein the "+" end denotes the end attached to L₂, and the "++" end denotes the end attached to L₃.

In some embodiments, R^{f} is independently selected from halo (more preferably fluoro or chloro).

In some embodiments, R^{f} is independently selected from the group consisting of halo, C₁₋₆ alkoxy, and C₁₋₆ haloalkoxy.

In some embodiments, R^{f} is independently selected from the group consisting of fluoro, chloro, bromo, C₁₋₄ alkoxy, and C₁₋₃ haloalkoxy.

In some embodiments, R^{f} is independently selected from the group consisting of fluoro, chloro, -OCH₃, - OCH₂CH₃, -OCH(CH₃)₂, -OCHF₂, -OCF₃, -OCH₂CF₃, and -OCH₂CHF₂.

In some embodiments, p is 0. In some embodiments, p is 1.

In some embodiments, X₁ is CH.

In some embodiments, p is 0 or 1, and ring C is independently selected from the group consisting of 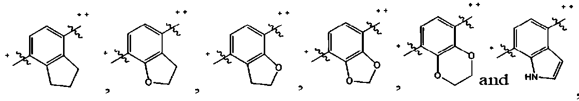 , wherein the "+" end denotes the end attached to L₂, and the "++" end denotes the end attached to L₃.

In some embodiments, p is 1, ring C is independently selected from the group consisting of C₉ bicyclic fused carbocyclyl, 9-membered bicyclic fused heterocyclyl, 10-membered bicyclic fused heterocyclyl, and 9-membered bicyclic heteroaryl, wherein the heteroatom(s) in the 9-membered bicyclic fused heterocyclyl, the 10-membered bicyclic fused heterocyclyl, or the 9-membered bicyclic heteroaryl is(are) independently selected from the group consisting of N and O, and the number of the heteroatoms is 1 or 2.

In some embodiments, p is 0, 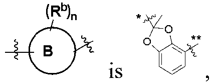 wherein the "*" end denotes the end attached to L₁, and the "**" end denotes the end attached to L₂, and ring C is selected from the group consisting of 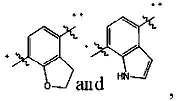 wherein the "+" end denotes the end attached to L₂, and the "++" end denotes the end attached to L₃.

In still another aspect, the present application provides a compound represented by Formula (IV-1), or a tautomer, a stereoisomer, or a pharmaceutically acceptable salt thereof: 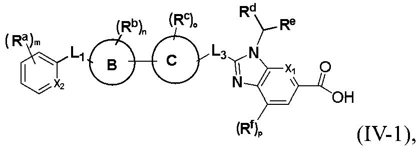 wherein X₁ is CH or N; X₂ is CH or N;
ring B, ring C, L₁, L₃, R^{a}, R^{b}, R^{c}, R^{d}, R^{e}, R^{f}, m, n, o, and p are as defined in the compound represented by Formula (I'), Formula (II), Formula (III), Formula (II-1), Formula (II-2), Formula (II-3), Formula (III-1), Formula (III-2), Formula (III-3), or Formula (IV) of the present application.

In some embodiments, X₁ is CH. In some embodiments, X₂ is CH.

In some embodiments, p is 0. In some embodiments, p is 1.

In still another aspect, the present application provides a compound represented by Formula (IV-3), or a tautomer, stereoisomer, or pharmaceutically acceptable salt thereof: 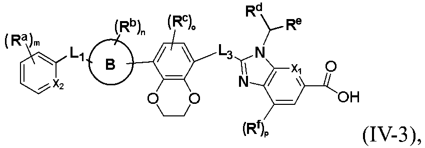 wherein X₁ is CH or N; X₂ is CH or N;
ring B, L₁, L₃, R^{a}, R^{b}, R^{c}, R^{d}, R^{e}, R^{f}, m, n, o, and p are as defined in the compound represented by Formula (I'), Formula (II), Formula (III), Formula (II-1), Formula (II-2), Formula (II-3), Formula (III-1), Formula (III-2), Formula (III-3), Formula (IV), or Formula (IV-1) of the present application.

In still another aspect, the present application provides a compound represented by Formula (IV-4) or Formula (IV-6), or a tautomer, stereoisomer, or pharmaceutically acceptable salt thereof: 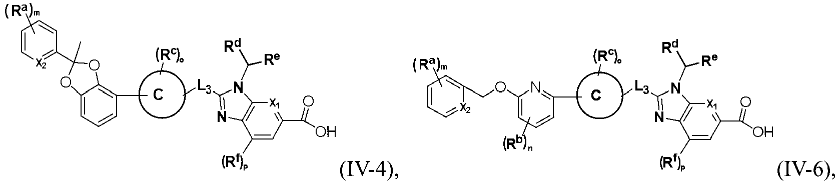 wherein X₁ is CH or N; X₂ is CH or N;
ring C, L₃, R^{a}, R^{b}, R^{c}, R^{d}, R^{e}, R^{f}, m, n, o, and p are as defined in the compound represented by Formula (I'), Formula (II), Formula (III), Formula (II-1), Formula (II-2), Formula (II-3), Formula (III-1), Formula (III-2), Formula (III-3), Formula (IV), Formula (IV-1), or Formula (IV-3) of the present application.

In some embodiments, p is 0. In some embodiments, p is 1.

In some embodiments of Formula (IV-4), p is 0 and ring C is selected from the group consisting of 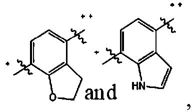 wherein the "+" end denotes the end attached to L₂, and the "++" end denotes the end attached to L₃;
In some embodiments, X₁ is CH. In some embodiments, X₂ is CH.

In some embodiments, X₂ is CH, and m is 2.

In some embodiments, n is 0. In some embodiments, n is 1.

In some embodiments, o is 0. In some embodiments, o is 1.

In still another aspect, the present application provides a compound represented by Formula (IV-10), Formula (IV-11), Formula (IV-12), Formula (IV-13), Formula (IV-14), Formula (IV-15), Formula (IV-16), or Formula (IV-17), or a tautomer, stereoisomer, or pharmaceutically acceptable salt thereof: 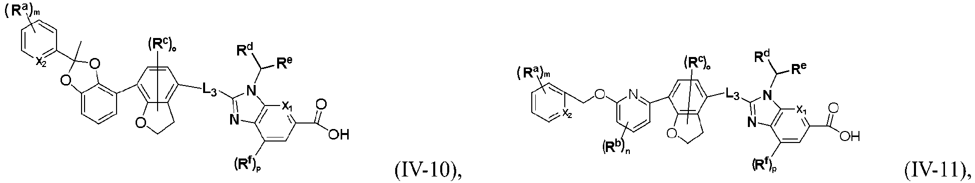 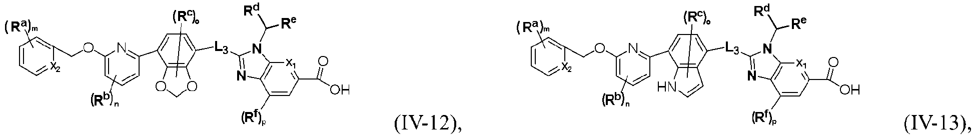 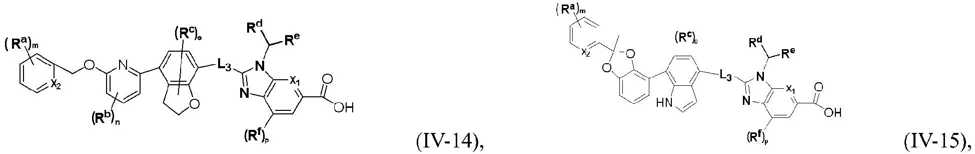 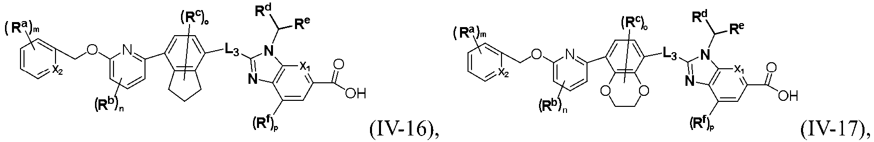 wherein X₁ is CH or N; X₂ is CH or N; X₃ is CH or N; X₄ is N; L₃, R^{a}, R^{b}, R^{c}, R^{d}, R^{e}, R^{f}, m, n, o, and p are as defined in the compound represented by Formula (I'), Formula (II), Formula (III), Formula (II-1), Formula (II-2), Formula (II-3), Formula (III-1), Formula (III-2), Formula (III-3), Formula (IV), Formula (IV-1), Formula (IV-3), Formula (IV-4), or Formula (IV-6) of the present application.

In a further aspect, the present application provides a compound represented by Formula (V), or a tautomer, a stereoisomer, or a pharmaceutically acceptable salt thereof: 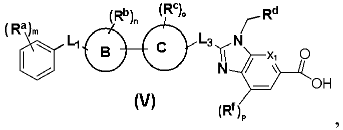 wherein X₁ is CH or N; ring B, ring C, L₁, L₃, R^{a}, R^{b}, R^{c}, R^{d}, R^{f}, m, n, o, and p are as defined in the compound represented by Formulae (I'), Formula (II), Formula (III), Formula (II-1), Formula (III-1), Formula (IV), or Formula (IV-1) of the present application.

In yet another aspect, the present application provides a compound represented by Formula (V-1), or a tautomer, a stereoisomer, or a pharmaceutically acceptable salt thereof: 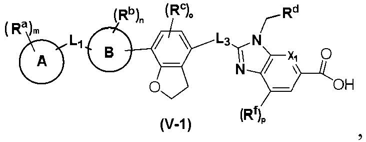 wherein X₁ is CH or N; ring A, ring B, L₁, L₃, R^{a}, R^{b}, R^{c}, R^{d}, R^{f}, m, n, o, and p are as defined in the compound represented by Formulae (I'), Formula (II), Formula (III), Formula (II-1), Formula (III-1), Formula (IV), or Formula (IV-1) of the present application.

In some embodiments, ring A is independently selected from the group consisting of phenyl and 5- to 6-membered heteroaryl, wherein the heteroatom(s) in the heteroaryl is(are) N, and the number of the heteroatoms is 1 or 2.

In some embodiments, ring A is independently selected from the group consisting of phenyl and pyridyl, preferably phenyl.

In some embodiments, ring B is independently selected from the group consisting of phenyl and 6-membered heteroaryl, wherein the heteroatom(s) in the heteroaryl is(are) N, and the number of the heteroatoms is 1 or 2.

In some embodiments, ring B is independently selected from the group consisting of phenyl, pyridyl, and pyrimidinyl; preferably pyridyl or pyrimidinyl.

In some embodiments, ring B is independently selected from the group consisting of 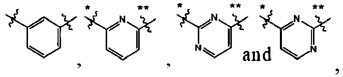 preferably 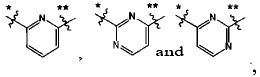 wherein the "*" end denotes the end attached to L₁, and the "**" end denotes the end attached to L₂.

In some embodiments, R^{a} is independently selected from the group consisting of halo, cyano, -C(O)NH₂, C₁₋₆ alkyl, C₁₋₆ haloalkyl, C₁₋₆ alkoxy, C₁₋₆ haloalkoxy, and 3- to 6-membered heterocyclyl, wherein the heteroatom in the 3- to 6-membered heterocyclyl is O, and the number of the heteroatom is 1.

In some embodiments, R^{a} is independently selected from the group consisting of halo, cyano, C₁₋₃ alkyl, C₁₋₃ haloalkyl, C₁₋₃ alkoxy, C₁₋₃ haloalkoxy, and oxetanyl.

In some embodiments, R^{a} is independently selected from the group consisting of fluoro, chloro, cyano, -CF₃, - OCHF₂, -OCF₃, methyl, methoxy, and

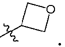

In some embodiments, R^{b} is independently selected from halo; preferably fluoro, chloro, or bromo; preferably fluoro.

In some embodiments, R^{c} is independently selected from halo; preferably fluoro, chloro or bromo; preferably fluoro.

In some embodiments, R^{d} is selected from the group consisting of optionally substituted 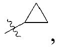 optionally substituted 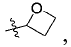 and optionally substituted 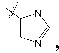 wherein the term "optionally substituted" means that hydrogen on the substituted group is not substituted, or one or more substitutable sites of the substituted group are independently substituted with R^{d1}, wherein R^{d1} is independently selected from C₁₋₃ alkyl optionally substituted with one or more substituents selected from the group consisting of fluoro, chloro, and cyano.

In some embodiments, 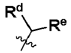 is independently selected from the group consisting of 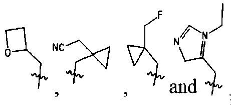 ; preferably 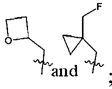 more preferably

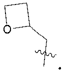

In some embodiments, R^{f} is independently selected from the group consisting of halo, C₁₋₆ alkoxy, and C₁₋₆ haloalkoxy.

In some embodiments, R^{f} is independently selected from the group consisting of fluoro, chloro, bromo, C₁₋₄ alkoxy, and C₁₋₃ haloalkoxy.

In some embodiments, R^{f} is independently selected from the group consisting of fluoro, -OCH₃, -OCH₂CH₃, - OCH(CH₃)₂, and -OCH₂CHF₂.

In some embodiments, L₁ is independently selected from the group consisting of a bond and -CH₂O-; preferably -CH₂O-; more preferably -CH₂O-, wherein the CH₂ group is attached to an end of ring A and the O group is attached to an end of ring B.

In some embodiments, L₃ is independently selected from the group consisting of -CH₂-, -C(O)-, and -CH(CH₃)-; preferably -CH₂- or -CH(CH₃)-; more preferably -CH₂-.

In some embodiments, m is 2.

In some embodiments, n is independently selected from the group consisting of 0 and 1; preferably 0.

In some embodiments, o is independently selected from the group consisting of 0 and 1; preferably 0.

In some embodiments, p is independently selected from the group consisting of 0 and 1; preferably 1.

In some embodiments, X₁ is CH.

In yet another aspect, the present application provides a compound represented by Formula (V-2), or a tautomer, a stereoisomer, or a pharmaceutically acceptable salt thereof: 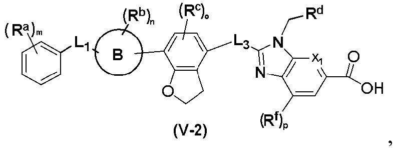 wherein X₁ is CH or N; ring B, L₁, L₃, R^{a}, R^{b}, R^{c}, R^{d}, R^{f}, m, n, o, and p are as defined in the compound represented by Formula (I'), Formula (II), Formula (III), Formula (II-1), Formula (III-1), Formula (IV), Formula (IV-1), Formula (V), or Formula (V-1) of the present application.

In still another aspect, the present application provides a compound represented by Formula (V-3), or a tautomer, a stereoisomer, or a pharmaceutically acceptable salt thereof: 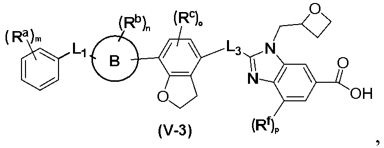 wherein ring B, L₁, L₃, R^{a}, R^{b}, R^{c}, R^{f}, m, n, o, and p are as defined in the compound represented by Formula (I'), Formula (II), Formula (III), Formula (II-1), Formula (III-1), Formula (IV), Formula (IV-1), Formula (V), or Formula (V-1) of the present application.

In still another aspect, the present application provides a compound represented by Formula (V-4), Formula (V-5), Formula (V-6), Formula (V-7), or Formula (V-8), or a tautomer, stereoisomer, or pharmaceutically acceptable salt thereof: 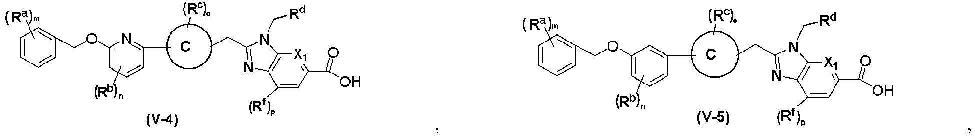 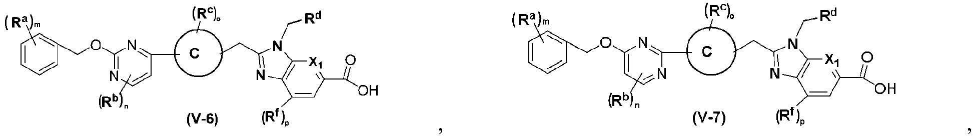 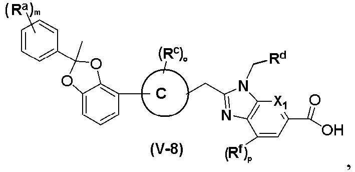 wherein X₁ is CH or N; ring C, R^{a}, R^{b}, R^{c}, R^{d}, R^{f}, m, n, o, and p are as defined in the compound represented by Formula (I'), Formula (II), Formula (III), Formula (II-1), Formula (III-1), Formula (IV), Formula (IV-1), Formula (V), or Formula (V-1) of the present application.

In yet another aspect, the present application provides a compound represented by Formula (V-9), Formula (V-10), Formula (V-11), Formula (V-12), Formula (V-13), Formula (V-14), Formula (V-15), Formula (V-16), or Formula (V-17), or a tautomer, stereoisomer, or pharmaceutically acceptable salt thereof: 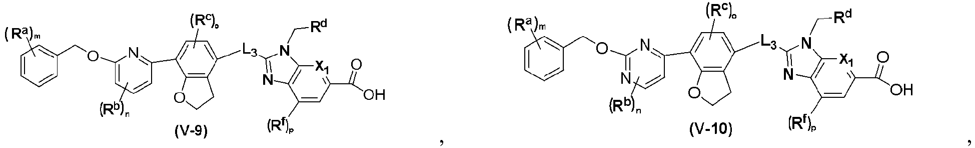 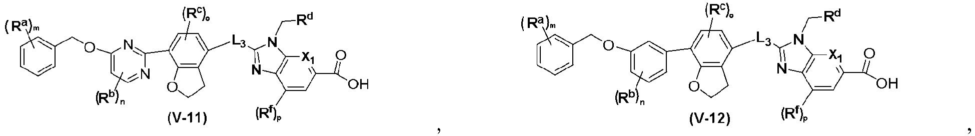 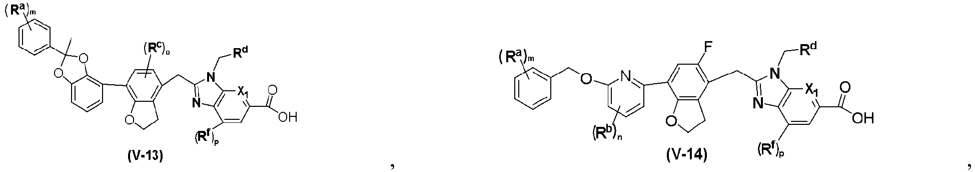 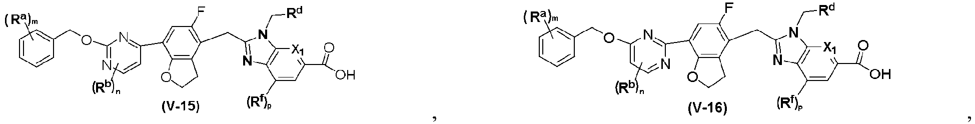 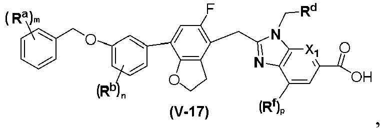 wherein X₁ is CH or N; R^{a}, R^{b}, R^{c}, R^{d}, R^{f}, m, n, o, and p are as defined in the compound represented by Formula (I'), Formula (II), Formula (III), Formula (II-1), Formula (III-1), Formula (IV), Formula (IV-1), Formula (V), or Formula (V-1) of the present application.

In some embodiments, m is 2, and two R^{a}s are located at the ortho-position and para-position of the attachment point between ring A and L₁, respectively, R^{a} is independently selected from the group consisting of halo, cyano, - C(O)NH₂, C₁₋₃ alkyl, C₁₋₃ haloalkyl, C₁₋₃ alkoxy, and oxetanyl; or R^{a} is independently selected from the group consisting of fluoro, chloro, cyano, -C(O)NH₂, -CF₃, -OCHF₂, methyl, methoxy, and

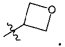

In some embodiments, n is 0. In some embodiments, n is 1 and R^{b} is independently selected from halo; or R^{b} is independently selected from the group consisting of fluoro, chloro, and bromo; or R^{b} is fluoro.

In some embodiments, o is 0. In some embodiments, o is 1, and R^{c} is independently selected from halo, or independently selected from the group consisting of fluoro and chloro.

In some embodiments, L₃ is independently selected from the group consisting of -CH₂-, -C(O)-, and - CH(CH₃)-; or L₃ is -CH₂-.

In some embodiments, 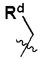 is independently selected from the group consisting of

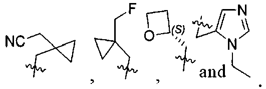

In some embodiments, 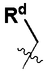 is independently 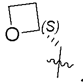 , In some embodiments, 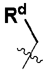 is independently

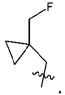

In some embodiments, X₁ is CH. In some embodiments, X₁ is N.

In some embodiments, p is 0.

In some embodiments, p is 1, R^{f} is independently selected from the group consisting of halo and C₁₋₃ alkoxy optionally substituted with one or more fluoro; or R^{f} is independently selected from C₁₋₃ alkoxy; or R^{f} is independently selected from the group consisting of fluoro, -OCH₃, -OCH₂CH₃, -OCH(CH₃)₂, and -OCH₂CHF₂.

On the basis of the routine knowledge in the art, the above-mentioned preferred conditions may be arbitrarily combined to obtain preferred embodiments of the present application.

Preferably, among the compounds, or the tautomers, stereoisomers, or pharmaceutically acceptable salts thereof provided in the present application, the compounds have the following structures:

| **Compound Structure and Numbering** | **Compound Structure and Numbering** |
|---|---|
| 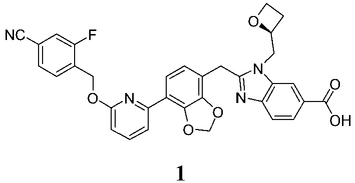 | 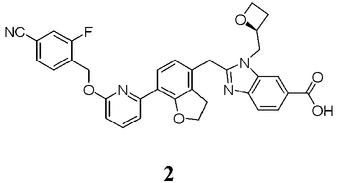 |
| 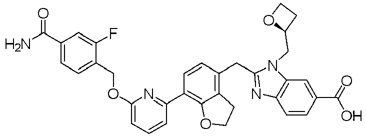 **3** | 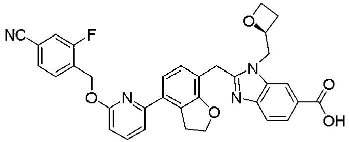 **4** |
| 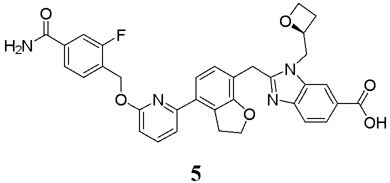 | 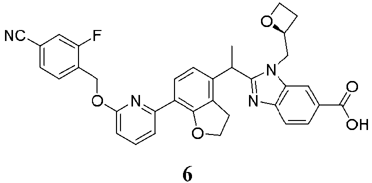 |
| 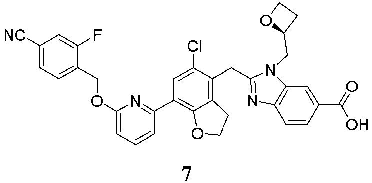 | 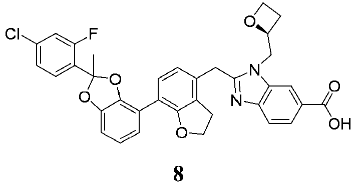 |
| 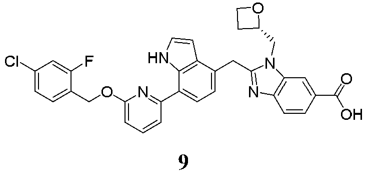 | 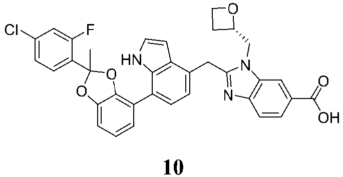 |
| 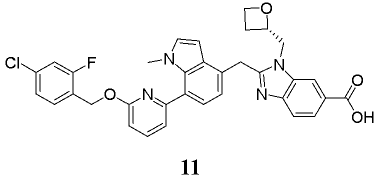 | 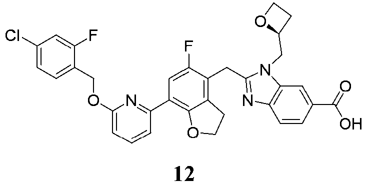 |
| 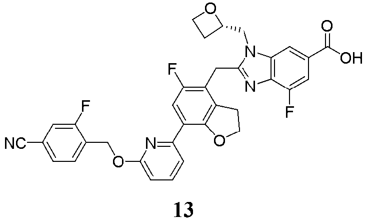 | 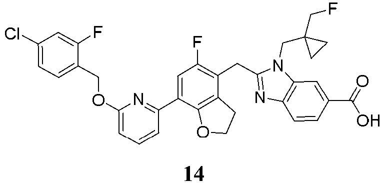 |
| 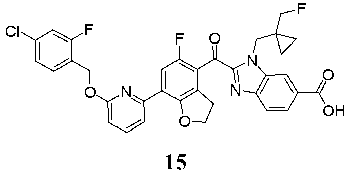 | 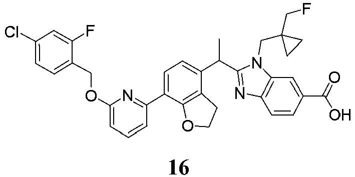 |
| 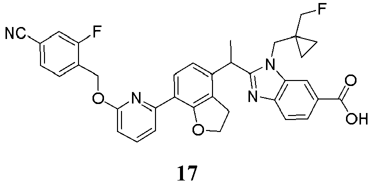 | 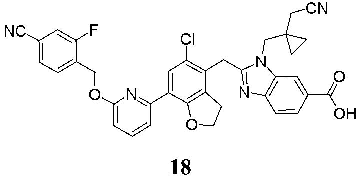 |
| 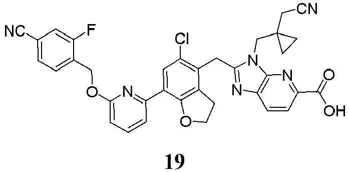 | 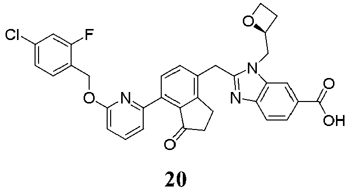 |
| 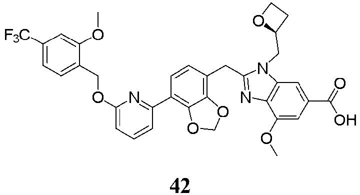 | 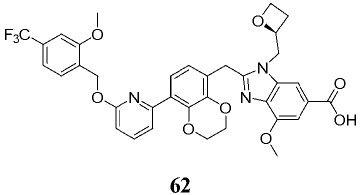 |
| 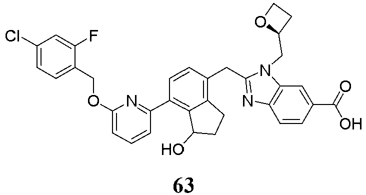 | 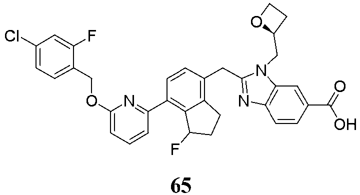 |
| 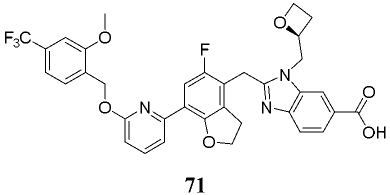 | 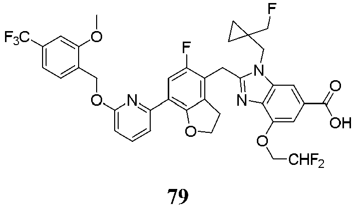 |
| 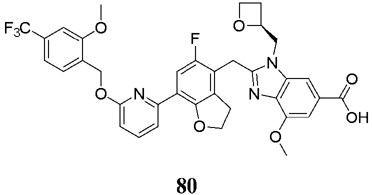 | 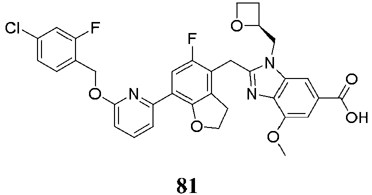 |
| 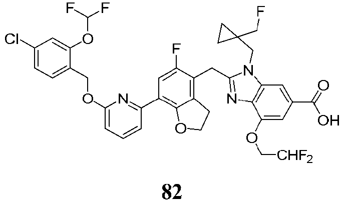 | 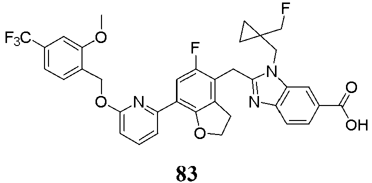 |
| 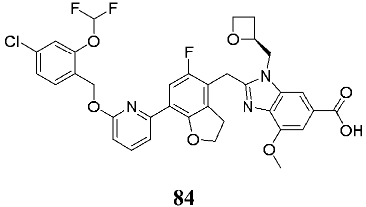 | 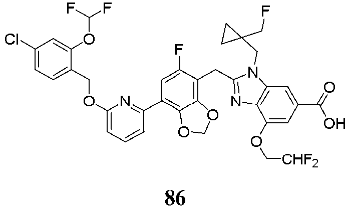 |
| 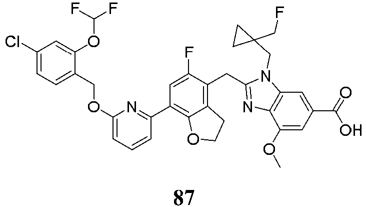 | 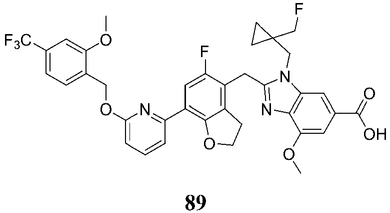 |
| 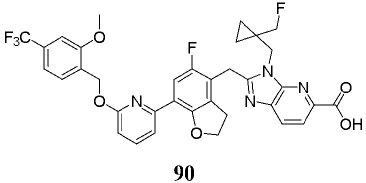 | 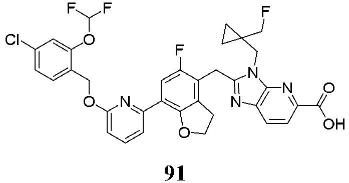 |
| 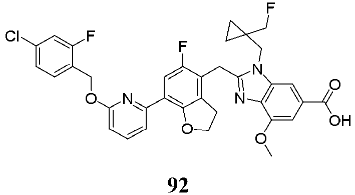 | 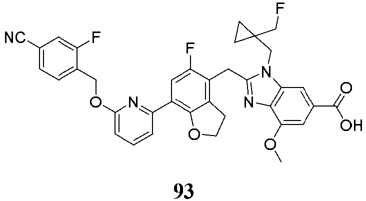 |
| 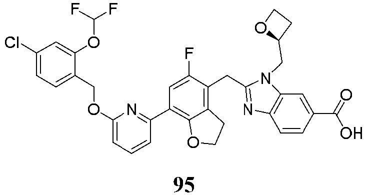 | 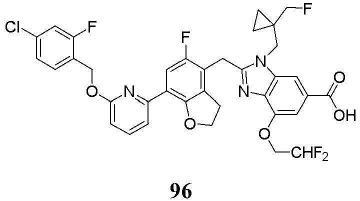 |
| 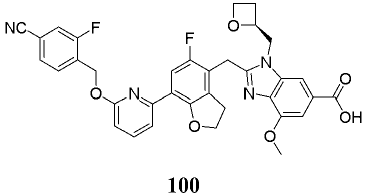 | 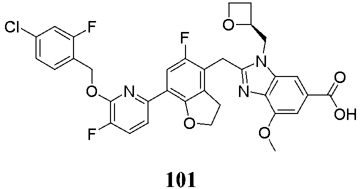 |
| 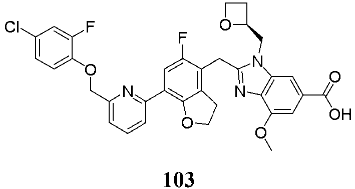 | 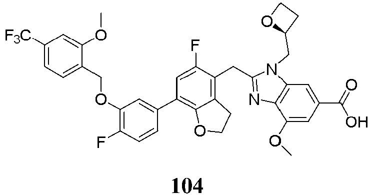 |
| 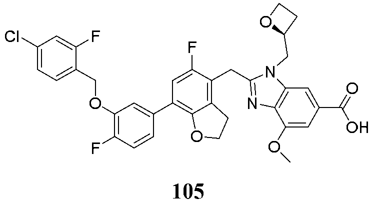 | 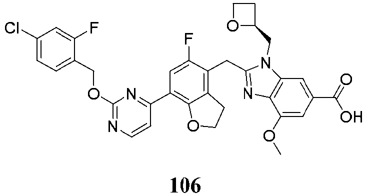 |
| 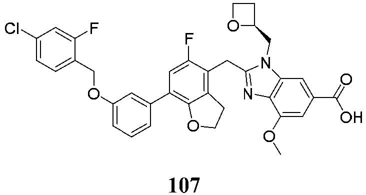 | 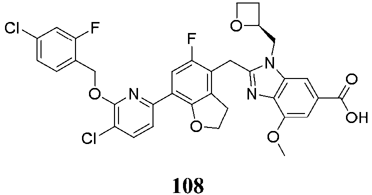 |
| 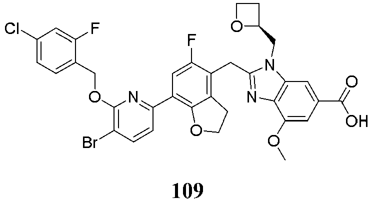 | 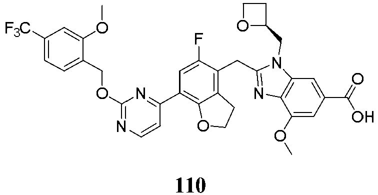 |
| 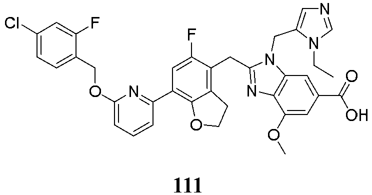 | 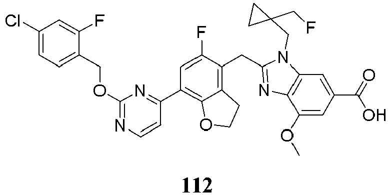 |
| 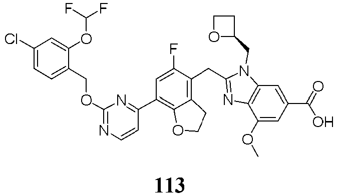 | 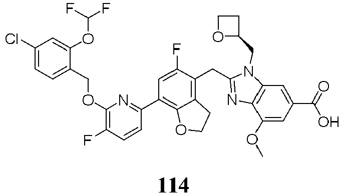 |
| 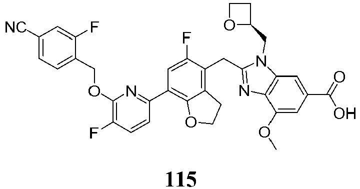 | 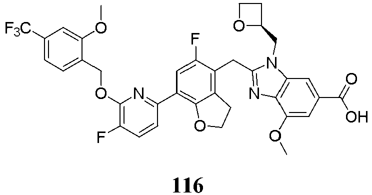 |
| 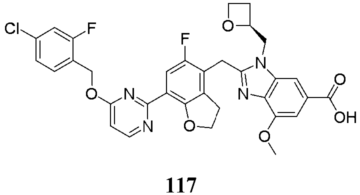 | 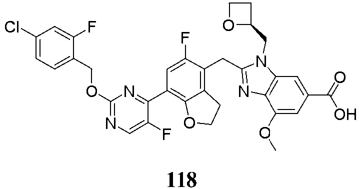 |
| 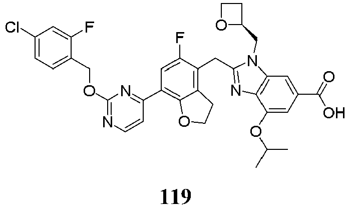 | 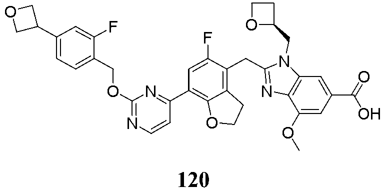 |
| 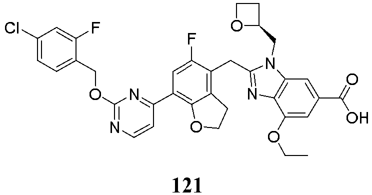 | 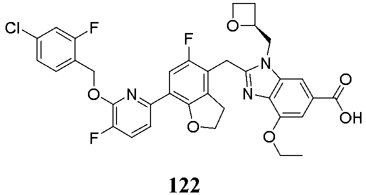 |
| 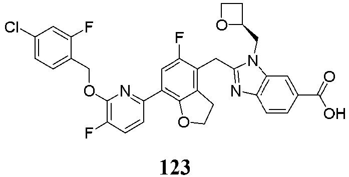 | 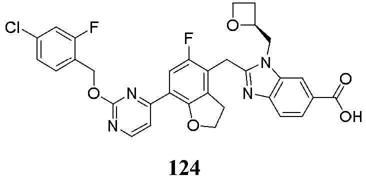 |
| 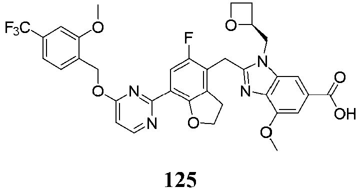 | 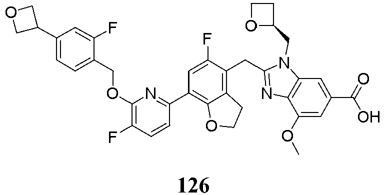 |
| 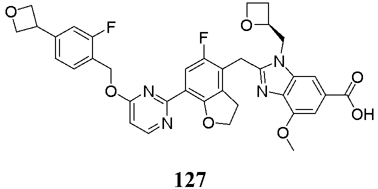 | 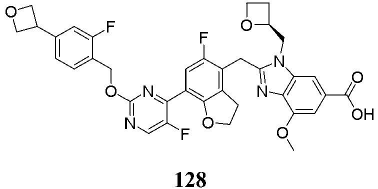 |
| 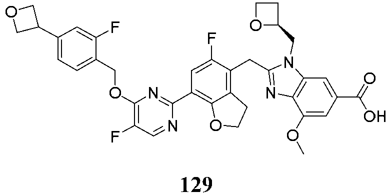 | 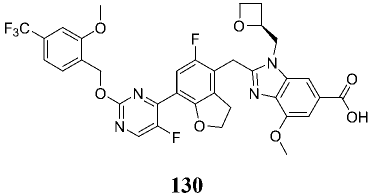 |
| 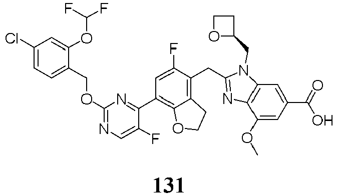 | 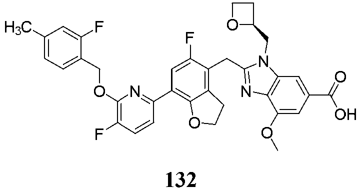 |
| 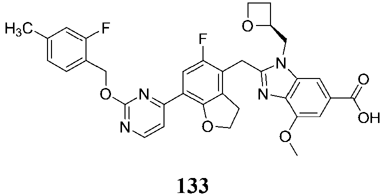 | 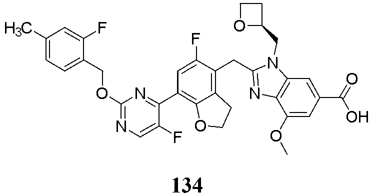 |

The object of the present application also includes the provision of a method for preparing the compound represented by Formula (I'), or the tautomer, stereoisomer, or pharmaceutically acceptable salt thereof.

The compound can be prepared, for example, using the methods shown in the following schemes.

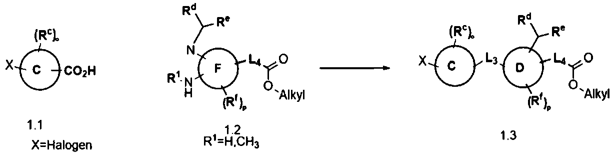

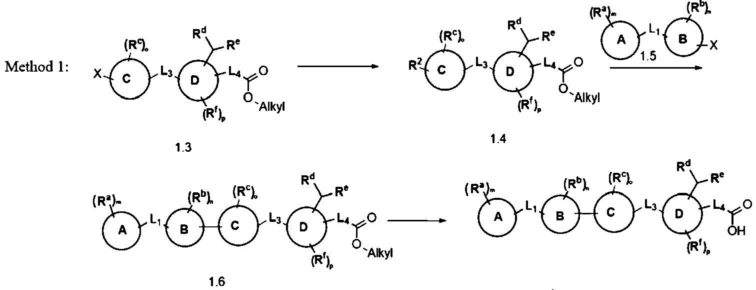

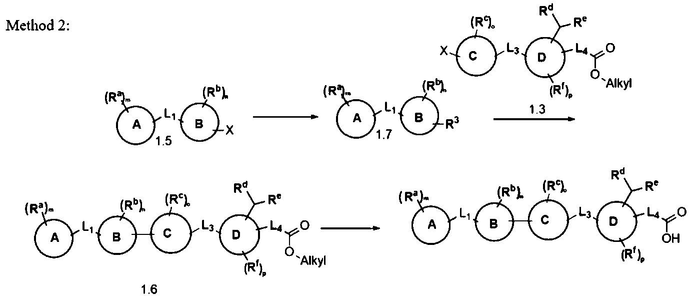

The carboxyl group of Intermediate 1.1 is reacted with two amino groups of Compound 1.2 to afford Intermediate 1.3, which is converted to the target compound through two methods.
Method 1: The halo group of Intermediate 1.1 is converted to boronic acid or borate ester, followed by a Suzuki coupling reaction with Compound 1.5 to afford Intermediate 1.6, which then undergoes an ester hydrolysis reaction to obtain the target compound;
Method 2: The halo group of Compound 1.5 is converted to a substituted tin moiety 1.7, followed by a stille coupling reaction with Intermediate 1.3 to afford Intermediate 1.6, which is finally converted to the target compound.
Method 3: Intermediate 1.8 is reacted with Intermediate 1.9 to form Intermediate 1.10, followed by hydrolysis of the ester group under a basic condition to afford the target compound 1.11.

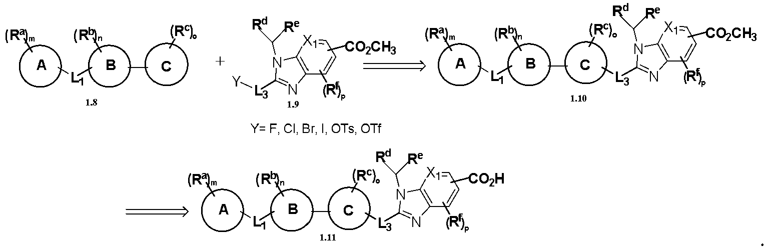

Note: Reaction steps of protection and deprotection involved in this scheme have been omitted.

Among them, ring F is monocyclic aryl or monocyclic heteroaryl, preferably phenyl or 5- to 6-membered heteroaryl, more preferably phenyl or pyridyl; ring A, ring B, ring C, ring D, L₁, L₂, L₃, L₄, R^{a}, R^{b}, R^{c}, R^{d}, R^{e}, R^{f}, R^{g}, m, n, o, p, and the like are as defined in the compounds of the present application.

In another aspect, the present application further provides a pharmaceutical composition comprising the compound of the present application, or the tautomer, stereoisomer, or pharmaceutically acceptable salt thereof.

Further, the pharmaceutical composition of the present application comprises the compound of the present application, or the tautomer, stereoisomer, or pharmaceutically acceptable salt thereof, and a pharmaceutically acceptable excipient.

The compound of the present application, or the tautomer, stereoisomer, or pharmaceutically acceptable salt thereof, may be administrated in a pure form or in the form of a suitable pharmaceutical composition via any acceptable administration routes for drugs with similar uses. The pharmaceutical composition of the present application may be prepared by combining the compound of the present application with a suitable pharmaceutically acceptable excipient. The pharmaceutical composition of the present application may be formulated into solid, semisolid, liquid, or gaseous formulations. Generally, the above-mentioned pharmaceutical composition may be prepared by a conventional preparation method using a conventional excipient in the field of formulation.

In still another aspect, the present application provides the use of the compound, or the tautomer, stereoisomer, or pharmaceutically acceptable salt thereof of the present application, or the pharmaceutical composition of the present application, in the preparation of a medicament for the prevention and/or treatment of diseases, disorders, and conditions through the activation of GLP-1R-mediated cascade signaling.

Further, according to the use provided in the present application, the diseases, disorders, and conditions are GLP-1R-related, including diabetes, obesity, overweight, metabolic-related fatty liver diseases, Alzheimer's disease, etc.

In still another aspect, the present application further provides the use of the compound, or the tautomer, stereoisomer, or pharmaceutically acceptable salt thereof of the present application, or the pharmaceutical composition of the present application, in the preparation of a medicament for the prevention and/or treatment of diabetes, overweight, metabolic-related fatty liver diseases, Alzheimer's disease, etc.

In yet another aspect, the present application provides a method for preventing and/or treating diseases, disorders, and conditions through the activation of GLP-1R-mediated cascade signaling, comprising administering the compound, or the tautomer, stereoisomer, or pharmaceutically acceptable salt thereof of the present application, or the pharmaceutical composition of the present application to a subject in need thereof; preferably, the diseases, disorders, and conditions are GLP-1R-related, including diabetes, obesity, overweight, metabolic-related fatty liver diseases, Alzheimer's disease, etc.

In yet another aspect, the present application provides the compound, or the tautomer, stereoisomer, or pharmaceutically acceptable salt thereof of the present application, or the pharmaceutical composition of the present application for use in the prevention and/or treatment of diseases, disorders, and conditions through the activation of GLP-1R-mediated cascade signaling; preferably, the diseases, disorders, and conditions are GLP-1R-related, including diabetes, obesity, overweight, metabolic-related fatty liver diseases, Alzheimer's disease, etc.

In yet another aspect, the present application provides the use of the compound, or the tautomer, stereoisomer, or pharmaceutically acceptable salt thereof of the present application, or the pharmaceutical composition of the present application, for the prevention and/or treatment of diseases, disorders, and conditions through the activation of GLP-1R-mediated cascade signaling; preferably, the diseases, disorders and conditions are GLP-1R-related, including diabetes, obesity, overweight, metabolic-related fatty liver diseases, Alzheimer's disease, etc.

Further, according to the uses or methods provided in the present application, the compound, or the tautomer, stereoisomer, or pharmaceutically acceptable salt thereof of the present application, or the pharmaceutical composition of the present application is used in combination with additional one, two or more compounds useful for the same or similar indications.

The present application also provides a pharmaceutical composition comprising the compound, or the tautomer, stereoisomer, or pharmaceutically acceptable salt thereof of the present application, or the pharmaceutical composition of the present application, and additional one, two or more compounds useful for the same or similar indications.

### Definition

Cₘ₋ₙ, as used herein, means that the moiety has an integer number of carbon atoms in a given range. For example, "C₁₋₆" means that the group may have 1 carbon atom, 2 carbon atoms, 3 carbon atoms, 4 carbon atoms, 5 carbon atoms, or 6 carbon atoms.

When any variable (e.g., R^{a}) occurs more than once in the composition or structure of a compound, it is independently defined at each occurrence. Thus, for example, if a group, a site, or an atom is substituted with two R^{a}s, then each R^{a} has an independent option.

The term "more" refers to 2 to 10, for example 2, 3, 4, 5, 6, 7, 8, 9 or 10, preferably 2, 3, 4, 5, 6, 7, or 8; preferably 2, 3, 4, 5, or 6; more preferably, 2 or 3.

"R^{b} is independently selected from the group consisting of hydrogen, deuterium, halo, hydroxy, amino, nitro, mercapto, cyano, oxo, optionally substituted C₁₋₆ alkyl, C₁₋₆ alkoxy, C₁₋₆ alkylaminyl, C₂₋₆ alkenyl, C₂₋₆ alkynyl, C₃₋₁₀ cycloalkyl, 3- to 10-membered heterocyclyl, C₆₋₁₄ aryl, and 5- to 12-membered heteroaryl" should be understood as "R^{b} is independently selected from the group consisting of hydrogen, deuterium, halo, hydroxy, amino, nitro, mercapto, cyano, oxo, optionally substituted C₁₋₆ alkyl, optionally substituted C₁₋₆ alkoxy, optionally substituted C₁₋₆ alkylaminyl, optionally substituted C₂₋₆ alkenyl, optionally substituted C₂₋₆ alkynyl, optionally substituted C₃₋₁₀ cycloalkyl, optionally substituted 3- to 10-membered heterocyclyl, optionally substituted C₆₋₁₄ aryl, and optionally substituted 5- to 12-membered heteroaryl". Other similar expressions in the present application should be understood with reference to the above-described manner of understanding.

The terms "optional" and "optionally" mean that the subsequently described event or circumstance may but does not certainly occur, and that the description includes instances where said event or circumstance occurs and instances wherein said event or circumstance does not occur.

The term "oxo" means that two hydrogen atoms at the same substitution site are replaced by the same oxygen atom to form a double bond (i.e., =O).

Unless otherwise specified, the term "alkyl" refers to a monovalent saturated aliphatic hydrocarbon group, including a linear or branched group containing 1-20 carbon atoms, preferably 1-10 carbon atoms (i.e., C₁₋₁₀ alkyl), further preferably 1-8 carbon atoms (C₁₋₈ alkyl), more preferably 1 to 6 carbon atoms (i.e., C₁₋₆ alkyl). For example, "C₁₋₆ alkyl" means that the group is an alkyl containing 1-6 (specifically, 1, 2, 3, 4, 5, or 6) carbon atoms in the carbon chain. Examples include, but are not limited to, methyl, ethyl, n-propyl, isopropyl, n-butyl, isobutyl, tert-butyl, sec-butyl, n-pentyl, neopentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, n-octyl, etc.

Unless otherwise specified, the term "alkenyl" refers to a linear or branched unsaturated aliphatic hydrocarbon group composed of carbon and hydrogen atoms and containing at least one double bond. The alkenyl may contain 2-20 carbon atoms, preferably 2-10 carbon atoms (i.e., C₂₋₁₀ alkenyl), further preferably 2-8 carbon atoms (C₂₋₈ alkenyl), more preferably 2-6 carbon atoms (i.e., C₂₋₆ alkenyl), 2-5 carbon atoms (i.e., C₂₋₅ alkenyl), 2-4 carbon atoms (i.e., C₂₋₄ alkenyl), 2-3 carbon atoms (i.e., C₂₋₃ alkenyl), or 2 carbon atoms (i.e., C₂ alkenyl). For example, "C₂₋₆ alkenyl" means that the group is an alkenyl containing 2-6 (specifically 2, 3, 4, 5, or 6) carbon atoms in the carbon chain. Non-limiting examples of the alkenyl include vinyl, 1-propenyl, 2-propenyl, 1-butenyl, isobutenyl, 1,3-butadienyl, etc.

Unless otherwise specified, the term "alkynyl" refers to a linear or branched unsaturated aliphatic hydrocarbon group composed of carbon and hydrogen atoms and containing at least one triple bond. The alkynyl may contain 2-20 carbon atoms, preferably 2-10 carbon atoms (i.e., C₂₋₁₀ alkynyl), further preferably 2-8 carbon atoms (C₂₋₈ alkynyl), more preferably 2-6 carbon atoms (i.e., C₂₋₆ alkynyl), 2-5 carbon atoms (i.e., C₂₋₅ alkynyl), 2-4 carbon atoms (i.e., C₂₋₄ alkynyl), 2-3 carbon atoms (i.e., C₂₋₃ alkynyl), or 2 carbon atoms (i.e., C₂ alkynyl). For example, "C₂₋₆ alkynyl" means that the group is an alkynyl containing 2-6 (specifically 2, 3, 4, 5, or 6) carbon atoms in the carbon chain. Non-limiting examples of the alkynyl include ethynyl, 1-propynyl, 2-propynyl, 1-butynyl, etc.

Unless otherwise specified, the term "alkoxy" refers to -O-alkyl, wherein the alkyl is as defined above, containing 1-20 carbon atoms, preferably 1-10 carbon atoms, more preferably 1-8 carbon atoms, and even more preferably 1-6 carbon atoms (specifically 1, 2, 3, 4, 5, or 6 carbon atoms). Representative examples include, but are not limited to, methoxy, ethoxy, propoxy, isopropoxy, butoxy, 1-methylpropoxy, 2-methylpropoxy, tert-butoxy, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, etc.

Unless otherwise specified, the term "alkylaminyl" refers to -NR'R", wherein R' and R" are the same or different, and may be H or alkyl as defined above, i.e., the alkyl as defined above containing 1 to 20 carbon atoms, preferably 1 to 10 carbon atoms, more preferably 1 to 8 carbon atoms, even more preferably 1 to 6 carbon atoms (specifically 1, 2, 3, 4, 5 or 6 carbon atoms). Representative examples include, but are not limited to, -NH(CH₃), - N(CH₃)(CH₃), -N(CH₂CH₃)(CH₃), -N(CH₂CH₃)[CH(CH₃)₂], etc.

Unless otherwise specified, the term "halogen" or "halo" refers to F, Cl, Br, or I. The term "haloalkyl" refers to an alkyl group as defined above, wherein one, two, more, or all hydrogen atoms are replaced by halogen. Representative examples of the haloalkyl include CCl₃, CF₃, CHCl₂, CH₂Cl, CH₂Br, CH₂I, CH₂CF₃, CF₂CF₃, etc.

Unless otherwise specified, the term "carbocyclyl" or "carbocycle" refers to a non-aromatic cyclic hydrocarbon group with 3 to 14 ring carbon atoms ("C₃₋₁₄ carbocyclyl"), and with no heteroatoms in the non-aromatic ring system. In some examples, the carbocyclyl group has 3-12 ring carbon atoms ("C₃₋₁₂ carbocyclyl"), 4-12 ring carbon atoms ("C₄₋₁₂ carbocyclyl"), or 3-10 ring carbon atoms ("C₃₋₁₀ carbocyclyl"). In some examples, the carbocyclyl group has 3-8 ring carbon atoms ("C₃₋₈ carbocyclyl"). In some examples, the carbocyclyl group has 3-7 ring carbon atoms ("C₃₋₇ carbocyclyl"). In some examples, the carbocyclyl group has 4-6 ring carbon atoms ("C₄₋₆ carbocyclyl"). In some examples, the carbocyclyl group has 5-10 ring carbon atoms ("C₅₋₁₀ carbocyclyl") or 5-7 ring carbon atoms ("C₅₋₇ carbocyclyl"). Exemplary C₃₋₆ carbocyclyl groups include, but are not limited to, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), etc. Exemplary C₃₋₈ carbocyclyl groups include, but are not limited to, the aforementioned C₃₋₆ carbocyclyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptyl (C₇), bicyclo[2.2.2]octyl (C₈), etc. Exemplary C₃₋₁₀ carbocyclyl groups include, but are not limited to, the aforementioned C₃₋₈ carbocyclyl groups as well as cyclononyl (C₉), cyclononenyl (C₉), cyclodecyl (C₁₀), cyclodecenyl (C₁₀), octahydro-1H-indenyl (C₉), decahydronaphthyl (C₁₀), spiro[4.5]decyl (C₁₀), etc. As illustrated in the above examples, in certain examples, the carbocyclyl is monocyclic ("monocyclic carbocyclyl") or a fused (fused carbocyclyl), bridged (bridged cyclyl), or spiro-fused (spirocyclyl) ring system, such as a bicyclic system ("bicyclic carbocyclyl"), and may be saturated or partially unsaturated. "Carbocyclyl" also includes ring systems wherein the carbocyclyl ring as defined above is fused with one or more aryl groups, where the point of attachment is on the carbocyclyl ring or aryl ring, and in such cases, the number of members of the carbocyclyl ring system is the number of carbons in the resulting carbocyclic system after fusion. In certain examples, each example of the carbocyclyl groups is independently optionally substituted, e.g., unsubstituted ("unsubstituted carbocyclyl") or substituted with one or more substituents ("substituted carbocyclyl"). In certain examples, the carbocyclyl group is unsubstituted C₃₋₁₀ carbocyclyl. In certain examples, the carbocyclyl group is substituted C₃₋₁₀ carbocyclyl.

Unless otherwise specified, the term "cycloalkyl" refers to a monocyclic saturated aliphatic hydrocarbon group with a specific number of carbon atoms, preferably 3-12 carbon atoms (i.e., C₃₋₁₂ cycloalkyl), more preferably 3-10 carbon atoms (C₃₋₁₀ cycloalkyl), and further preferably 3-7 carbon atoms (C₃₋₇ cycloalkyl), 4-6 carbon atoms (C₄₋₆ cycloalkyl), or 5-6 carbon atoms (C₅₋₆ cycloalkyl). Examples include, but are not limited to, cyclopropyl, cyclobutyl, cyclopentyl, cyclohexyl, methylcyclopropyl, 2-ethyl-cyclopentyl, dimethylcyclobutyl, etc.

Unless otherwise specified, the term "heterocyclyl" or "heterocycle" refers to a saturated or partially unsaturated monocyclic or polycyclic non-aromatic substituent having ring carbon atom(s) and 1 to 4 ring heteroatoms, and containing 3-20 ring atoms, wherein 1, 2, 3 or more ring atoms are selected from the group consisting of N, O, and S, and the remaining ring atoms are C. Preferably, heterocyclyl contains 3-12 ring atoms (3-12 membered heterocyclyl), more preferably 3-10 ring atoms (3-10 membered heterocyclyl), or 3-8 ring atoms (3-8 membered heterocyclyl), or 3-6 ring atoms (3-6 membered heterocyclyl), or 4-6 ring atoms (4-6 membered heterocyclyl), or 5-6 ring atoms (5-6 membered heterocyclyl). The number of the heteroatoms is preferably 1-4, more preferably 1-3 (i.e., 1, 2 or 3). Examples of monocyclic heterocyclyl include pyrrolidinyl, imidazolidinyl, tetrahydrofuranyl, dihydropyrrolyl, piperidinyl, piperazinyl, pyranyl, and the like. The polycyclic heterocyclyl includes fused-ring, spiro-ring, and bridged-ring heterocyclyl. "Heterocyclyl" may be monocyclic ("monocyclic heterocyclyl") or fused (fused heterocyclyl), bridged ("bridged heterocyclyl" or "bridged-ring heterocyclyl"), or spiro ("hetero-spirocyclyl", "spiro-heterocyclyl", or "spirocyclic heterocyclyl") ring systems, such as a bicyclic system ("bicyclic heterocyclyl"), and may be saturated or partially unsaturated. The heterocyclyl bicyclic system may contain one or more heteroatoms in one or two rings. "Heterocyclyl" also includes ring systems wherein the heterocyclyl ring as defined above is fused with one or more carbocyclyl groups with the point of attachment on the carbocyclyl or heterocyclyl ring, or ring systems wherein the heterocyclyl ring as defined above is fused with one or more aryl or heteroaryl groups, or ring systems wherein the cycloalkyl ring as defined above is fused with one or more heteroaryl groups with the point of attachment on one of the heterocyclyl ring, cycloalkyl ring, aryl ring, and heteroaryl ring. In such cases, the number of members of the heterocyclyl ring system is the number of atoms in the resulting ring system after fusion. In certain examples, each example of the heterocyclyl groups is independently optionally substituted, e.g., unsubstituted ("unsubstituted heterocyclyl") or substituted with one or more substituents ("substituted heterocyclyl"). Exemplary 3-membered heterocyclyl groups containing 1 heteroatom include, but are not limited to, aziridinyl, oxiranyl, and thiiranyl. Exemplary 4-membered heterocyclyl groups containing 1 heteroatom include, but are not limited to, azetidinyl, oxetanyl, and thietanyl. Exemplary 5-membered heterocyclyl groups containing 1 heteroatom include, but are not limited to, tetrahydrofuranyl, dihydrofuranyl, tetrahydrothiophenyl, dihydrothiophenyl, pyrrolidinyl, dihydropyrrolyl, and pyrrolyl-2,5-dione. Exemplary 5-membered heterocyclyl groups containing 2 heteroatoms include, but are not limited to, dioxolanyl, oxathiolanyl, dithiolanyl, and oxazolidin-2-one. Exemplary 5-membered heterocyclyl groups containing 3 heteroatoms include, but are not limited to, triazolinyl, oxadiazolinyl, and thiadiazolinyl. Exemplary 6-membered heterocyclyl groups containing 1 heteroatom include, but are not limited to, piperidinyl, tetrahydropyranyl, dihydropyridyl, and thianyl. Exemplary 6-membered heterocyclyl groups containing 2 heteroatoms include, but are not limited to, piperazinyl, morpholinyl, dithianyl, and dioxanyl. Exemplary 6-membered heterocyclyl groups containing 3 heteroatoms include, but are not limited to, triazinanyl, oxadiazinanyl, thiadiazinanyl, oxathiazinanyl, and dioxazinanyl. Exemplary 7-membered heterocyclyl groups containing 1 heteroatom include, but are not limited to, azepanyl, oxepanyl, and thiepanyl. Exemplary 8-membered heterocyclyl groups containing 1 heteroatom include, but are not limited to, azocanyl, oxocanyl, and thiocanyl. An exemplary 5-membered heterocyclyl group fused with one C₆ aryl ring (also referred to herein as a 5,6-bicyclic heterocycle) includes, but is not limited to, indolinyl, isoindolinyl, dihydrobenzofuranyl, dihydrobenzothiophenyl, benzoxazolinonyl, and the like. An exemplary 6-membered heterocyclyl group fused with one aryl ring (also referred to herein as a 6,6-bicyclic heterocycle) includes, but is not limited to, tetrahydroquinolinyl, tetrahydroisoquinolinyl, and the like.

Unless otherwise specified, the term "fused-cycle" refers to a non-aromatic, saturated or partially unsaturated ring system formed by two or more cyclic structures sharing two adjacent atoms with each other, including fused carbocyclyl and fused heterocyclyl. Said "non-aromatic" means that the ring system as a whole is non-aromatic.

The fused carbocyclyl may contain 5-14 ring atoms, preferably 6-12 ring atoms, and more preferably 7-10 ring atoms. The fused carbocyclyl includes bicyclic, tricyclic, tetracyclic, or polycyclic fused carbocyclyl, preferably bicyclic, tricyclic, or tetracyclic fused carbocyclyl, and more preferably bicyclic or tricyclic fused carbocyclyl. Exemplary examples of the fused carbocyclyl include (but are not limited to) 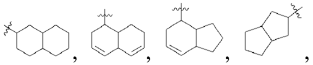 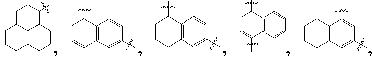 etc.

The fused heterocyclyl may contain 5-14 ring atoms, preferably 6-12 ring atoms, and more preferably 7-10 ring atoms, including 1-4 ring heteroatoms, preferably 1-3 (i.e., 1, 2, or 3) ring heteroatoms, where the heteroatoms are independently selected from the group consisting of N, O, and S. The fused heterocyclyl includes bicyclic, tricyclic, tetracyclic, or polycyclic fused heterocyclyl, preferably bicyclic, tricyclic, or tetracyclic fused heterocyclyl, and more preferably bicyclic or tricyclic fused heterocyclyl. Exemplary examples of the fused heterocyclyl include (but are not limited to) 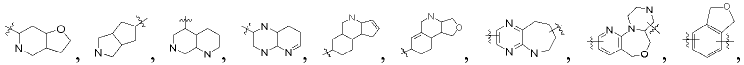 etc. Illustrative examples of the bicyclic heterocyclyl or bicyclic fused heterocyclyl include (but are not limited to) 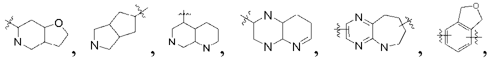 etc.

Unless otherwise specified, the term "aryl" or "aromatic cyclyl" refers to monocyclic, bicyclic and tricyclic aromatic carbocyclic ring systems containing 6-16 carbon atoms, or 6-14 carbon atoms, or 6-12 carbon atoms, or 6-10 carbon atoms, preferably 6-10 carbon atoms. The term "aryl" can be used interchangeably with the term "aromatic ring". Examples of aryl groups may include, but are not limited to, phenyl, naphthyl, anthracenyl, phenanthrenyl, pyrenyl, and the like.

Unless otherwise specified, the term "heteroaryl" or "heteroaromatic cyclyl" refers to an aromatic monocyclic or polycyclic ring system containing a 5-14 membered structure, or preferably a 5-10 membered structure, or preferably a 5-8 membered structure, more preferably a 5-6 membered structure, wherein 1, 2, 3 or more ring atoms are heteroatoms and the remaining ring atom(s) is/are carbon, the heteroatom(s) is/are independently selected from the group consisting of O, N, and S, and the number of heteroatom is preferably 1, 2 or 3. The polycyclic heteroaryl is a fused heteroaryl. Examples of heteroaryl include, but are not limited to, furanyl, thienyl, oxazolyl, thiazolyl, isoxazolyl, oxadiazolyl, thiadiazolyl, pyrrolyl, pyrazolyl, imidazolyl, triazolyl, tetrazolyl, pyridyl, pyrimidinyl, pyrazinyl, pyridazinyl, thiadiazolyl, triazinyl, phthalazinyl, quinolinyl, isoquinolinyl, pteridinyl, purinyl, indolyl, isoindolyl, indazolyl, benzofuranyl, benzothienyl, benzopyridyl, benzopyrimidinyl, benzopyrazinyl, benzimidazolyl, benzophthalazinyl, pyrrolo[2,3-b]pyridyl, imidazo[1,2-a]pyridyl, pyrazolo[1,5-a]pyridyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidinyl, [1,2,4]triazolo[1,5-a]pyridyl, and the like.

Unless otherwise specified, the term "fused heteroaryl" refers to an aromatic ring system formed by two or more cyclic structures sharing two adjacent atoms with each other, each ring in a fused heteroaryl being an unsaturated aromatic ring, which may contain 5-20 ring atoms, preferably 6-14 ring atoms, more preferably 7-10 ring atoms, and 1-4 ring heteroatoms, preferably 1-3 (i.e., 1, 2 or 3) ring heteroatoms independently selected from the group consisting of N, O and S. The fused heteroaryl includes bicyclic, tricyclic, tetracyclic, or polycyclic fused heteroaryl, preferably bicyclic, tricyclic, or tetracyclic fused heteroaryl, more preferably bicyclic or tricyclic fused heteroaryl. Illustrative examples of the fused heteroaryl include, but are not limited to 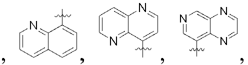 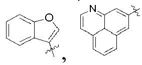 , etc.

Unless otherwise specified, the term "pharmaceutically acceptable salt" or "pharmaceutical salt" refers to those salts that are, within the scope of sound medical judgment, suitable for use in contact with mammalian tissues, particularly human tissues, without excessive toxicity, irritation, allergic responses, and the like, and are commensurate with a reasonable benefit/risk ratio. For example, medically acceptable salts of amines, carboxylic acids, and other types of compounds are well-known in the art. The salts may be prepared in situ during the final isolation and purification of the compounds of the present application, or may be prepared separately by reacting the free base or free acid with an appropriate reagent.

Unless otherwise specified, the compound of the present application also includes an "isotopic derivative" thereof. The term "isotopic derivative" means that the compounds of the present application may exist in isotopically labelled or enriched forms, containing one or more atoms whose atomic weight or mass number is different from that of the most abundant atom found in the nature. Isotopes may be radioactive or nonradioactive isotopes. Isotopes commonly used as isotopic labelling are: hydrogen isotopes, ²H and ³H; carbon isotopes: ¹³C and ¹⁴C; chlorine isotopes: ³⁵Cl and ³⁷Cl; fluorine isotope: ¹⁸F; iodine isotopes: ¹²³I and ¹²⁵I; nitrogen isotopes: ¹³N and ¹⁵N; oxygen isotopes: ¹⁵O, ¹⁷O and ¹⁸O; and sulfur isotope: ³⁵S. These isotopically labeled compounds can be used to study the distribution of pharmaceutical molecules in tissues. In particular, ³H and ¹³C are more widely used due to their ease of labeling and ease of detection. The substitution with certain heavy isotopes, such as heavy hydrogen (²H), can enhance metabolic stability, and prolong half-life, thereby achieving the purpose of reducing dosage and providing therapeutic advantages. The isotopically labeled compounds are generally synthesized starting from labeled starting materials in the same way as non-isotopically labeled compounds using known synthetic techniques.

Unless otherwise specified, the compounds of the present application also include a "solvate" thereof. The term "solvate" means a physical association of a compound of the present application with one or more solvent molecules, whether organic or inorganic. This physical association includes hydrogen bond. In certain instances, the solvate will be capable of isolation, for example, when one or more solvent molecules are incorporated into the crystal lattice of a crystalline solid. The solvent molecules in the solvate may be present in a regular arrangement and/or disordered arrangement. The solvate may comprise either a stoichiometric or non-stoichiometric number of solvent molecule(s). "Solvate" encompasses both solution phase and isolable solvates. Exemplary solvates include, but are not limited to, hydrates, ethanolates, methanolates, and isopropanolates. Methods of solvation are well known in the art. The term "hydrate" refers to a substance formed either by water molecules bound to cations or anions in a compound via coordinate bonds or covalent bonds, or by water molecules (not directly bound to cations or anions) existing in a certain proportion at specific positions in the solid crystal lattice.

Unless otherwise specified, the term "stereoisomer" refers to a compound that has the identical chemical constitution, but differs in the arrangement of atom(s) or group(s) in space. Stereoisomers include enantiomers, diastereomers, conformers (rotamers), geometric isomers (cis/trans), atropisomers, etc. A mixture of the resulting any stereoisomers can be separated into pure or substantially pure geometric isomers, enantiomers, or diastereomers, for example, by chromatography and/or fractional crystallization, on the basis of a difference in a physicochemical property of the components. Unless otherwise specified, the term "geometric (cis/trans) isomers" may include carbon-carbon double bonds or carbon-nitrogen double bonds with E or Z configuration, wherein the term "E" denotes the higher-order substituent on the opposite side of the carbon-carbon or carbon-nitrogen double bond, and the term "Z" denotes the higher-order substituent on the same side of the carbon-carbon or carbon-nitrogen double bond (determined using the Cahn-Ingold-Prelog priority rules). The compounds of the present application may also exist as a mixture of "E" and "Z" isomers.

Unless otherwise specified, the term "tautomer" refers to structural isomers with different energies which are interconvertible via a low energy barrier. If tautomerism is possible (such as, in solution), a chemical equilibrium of tautomers can be achieved. For example, proton tautomers (also known as prototropic tautomers) include interconversions via migration of a proton, such as keto-enol isomerization and imine-enamine isomerization. Valence tautomers include interconversions by reorganization of some bonding electrons.

Unless indicated otherwise, the structural formulae depicted in the present application include all isomeric (e.g., enantiomeric, diastereomeric, and geometric (or conformational)) forms: e.g., R and S configurations containing an asymmetric center, (Z) and (E) isomers of double bond, and (Z) and (E) conformational isomers. Therefore, individual stereochemical isomers of the compounds of the present application or mixtures of enantiomers, diastereomers, or geometrical isomers (or conformational isomers) thereof are within the scope of the present application.

Unless otherwise specified, the compounds of the present application also comprise a "prodrug" thereof. The term "prodrug" refers to a drug that is converted to its parent drug *in vivo.* Prodrugs are usually useful, and may improve some identified and undesirable physical or biological properties. Physical properties are generally relevant solubility (excessive or insufficient solubility in lipid or water) or stability, while problematic biological properties include too rapid metabolism or poor bioavailability, which may be related to physicochemical properties. For example, they may be bioavailable by oral administration, while the parent drug is not. The solubility of a prodrug in a pharmaceutical composition is also improved compared to a parent drug thereof. One example of a prodrug may be, but is not limited to, any one of the compounds of the present application administered as an ester ("prodrug") to facilitate delivery through cell membranes, where the water solubility thereof is detrimental to migration, but beneficial once the prodrug enters a cell, and the prodrug is subsequently metabolized and hydrolyzed to a carboxylic acid, i.e., the active entity. Another example of a prodrug may be a short peptide (polyamino acid) bound to an acid group, in which the peptide is metabolized to present an active moiety.

Unless otherwise specified, the term "treatment/treating" encompasses any treatment of a disease, disorder, or condition in a patient, including: (a) inhibiting the symptoms of the disease, disorder, or condition, i.e., preventing its progression; or (b) alleviating the symptoms of the disease, disorder, or condition, i.e., causing regression of the disease or symptoms; or (c) ameliorating or eliminating the disease, disorder, or condition, or one or more symptoms associated with the disease.

Abbreviations used in Preparation Examples, Examples, and elsewhere herein are:
AIBN: azobisisobutyronitrile; DCM: dichloromethane; DIAD: diisopropyl azodicarboxylate; DIPEA: N,N-diisopropylethylamine; DMF: N,N-dimethylformamide; EA: ethyl acetate; HATU: 2-(7-azabenzotriazol-1-yl)-N,N,N',N'-tetramethyluronium hexafluorophosphate; LDA: lithium diisopropylamide; NBS: N-bromosuccinimide; NCS: N-chlorosuccinimide; NMI: N-methylimidazole; NMP: N-methylpyrrolidone; Pd(dppf)Cl₂: (1,1'-bis(diphenylphosphino)ferrocene)dichloropalladium(II); TCFH: N,N,N',N'-tetramethylchloroformamidinium hexafluorophosphate; THF: tetrahydrofuran; BINAP: 1,1'-binaphthyl-2,2'-diphemyl phosphine; DMA: N,N-dimethylacetamide; DMSO: dimethyl sulfoxide; XantPhos: 4,5-bis(diphenylphosphino)-9,9-dimethylxanthene.

### Beneficial effects of the present application

The present application designs a class of compounds with novel structures. Enzymatic assays have shown that the compounds of the present application have a potent agonistic effect on GLP-1R, providing a new direction for the treatment of diseases such as diabetes, metabolic-related fatty liver disease, and Alzheimer's disease, as well as for weight-reduction. In vivo experiments have shown that the compounds of the present application have good pharmacokinetic properties. The hERG test has shown that the compounds of the present application have low risk of cardiotoxicity and good safety. The food intake inhibition rate has shown that the compounds of the present application have a good food intake inhibition effect, and the weight-reducing pharmacodynamic study in mice has shown that the compounds of the present application have a weight-reducing effect. In addition, the present application explores a specific synthesis method, which features simple processes and convenient operations, facilitating large-scale industrial production and application.

### EXAMPLE

The present application will be further described below in conjunction with specific examples. It should be understood that these examples are merely used to illustrate the present application, and are not intended to limit the scope of the present application. For the experimental methods in the following examples where specific conditions are not indicated, conventional conditions or conditions recommended by the manufacturer are generally followed. Unless otherwise defined, all professional and scientific terms used herein have the same meaning as commonly understood by one of ordinary skill in the art. In addition, any methods and materials similar or equivalent to those described herein can be applied to the methods of the present application. The preferred implementation methods and materials shown herein are illustrative only.

The following are preparation examples of exemplary compounds of the present application.

### Synthesis of 2-(7-bromobenzo[d] [1,3]dioxolan-4-yl)acetic acid (Intermediate 1.1-A)

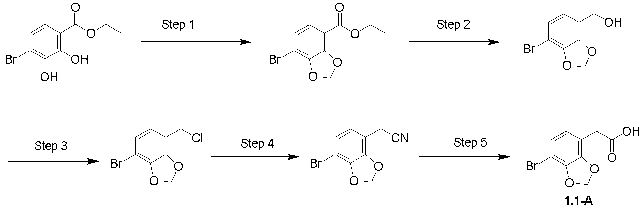

### Step 1: Synthesis of ethyl 7-bromobenzo[d][1,3]dioxolane-4-carboxylate

Ethyl 4-bromo-2,3-dihydroxybenzoate (2 g, 7.66 mmol) was dissolved in DMF (20 mL), then cesium carbonate (5.5 g, 16.86 mmol) was added, and the resulting mixture reacted under stirring at 20 °C for 1 hour. CH₂I₂ (328.45 mg, 1.24 mmol) was then added and the resulting mixture reacted under stirring at 70 °C for 12 hours. After the reaction was completed, the reaction mixture was quenched with water, extracted with ethyl acetate, washed with saturated brine, dried over anhydrous sodium sulfate, and concentrated under reduced pressure to obtain a crude product, which was purified by column chromatography (ethyl acetate: petroleum ether =1:1) to obtain ethyl 7-bromobenzo[d][1,3]dioxolane-4-carboxylate (1.05 g), ESI-MS (m/z): 273.2[M+H]⁺.

### Step 2: Synthesis of (7-bromobenzo[d][1,3]dioxolan-4-yl)methanol

The product obtained from the previous step (1.05 g, 1.94 mmol) was added to THF (11 mL), cooled to 0 °C, and added with LiAlH₄ (175.12 mg, 2.33 mmol) in batches. The resulting mixture further reacted under stirring at 0 °C for 1 hour. After the reaction was completed, the reaction mixture was quenched with water, extracted with ethyl acetate, washed with saturated brine, dried over anhydrous sodium sulfate, and concentrated under reduced pressure to obtain a crude product of (7-bromobenzo[d][1,3]dioxolan-4-yl)methanol (1.05 g), ESI-MS (m/z): 231.2[M+H]⁺.

### Step 3: Synthesis of 4-bromo-7-(chloromethyl)benzo[d] [1,3]dioxolane

The product obtained from the previous step (1.05g, 4.54 mmol) was dissolved in DCM (10 mL), SOCl₂ (2.70g, 22.72 mmol) was added, and the resulting mixture reacted under stirring at 20 °C for 2 hours. TLC detected that the starting materials reacted completely, and concentration under reduced pressure was performed to obtain a crude product, which was purified by column chromatography (ethyl acetate: petroleum ether =1:2) to obtain 4-bromo-7-(chloromethyl)benzo[d][1,3]dioxolane (830 mg), ESI-MS (m/z): 249.0[M+H]⁺.

### Step 4: Synthesis of 2-(7-bromobenzo[d][1,3]dioxolan-4-yl)acetonitrile

The product obtained from the previous step (830 mg, 3.33 mmol) was added to DMF (8.3 mL), then NaCN (1.63 g, 33.3 mmol) was added, and the resulting mixture reacted under stirring at 90 °C for 5 hours. After the reaction was completed, the reaction mixture was quenched with water, then extracted with ethyl acetate, washed with saturated brine, dried over anhydrous sodium sulfate, and concentrated under reduced pressure to obtain a crude product, which was purified by column chromatography (ethyl acetate: petroleum ether =1:2) to obtain 2-(7-bromobenzo[d][1,3]dioxolan-4-yl)acetonitrile (570 mg), ESI-MS (m/z): 240.1[M+H]⁺.

### Step 5: Synthesis of 2- (7-bromobenzo[d][1,3]dioxolan-4-yl)acetic acid

The product obtained from the previous step (570 mg, 2.37 mmol) was added to EtOH (5.7 mL), then KOH (483.6 mg, 8.62 mmol) dissolved in H₂O (5.7 mL) was added, and the resulting mixture reacted under stirring at 80 °C for 5 hours. After the reaction was completed, the reaction mixture was added with water and then extracted with trichloromethane, and the resulting aqueous phase was adjusted to pH = 5 with 1 M hydrochloric acid, then extracted with dichloromethane, dried over anhydrous sodium sulfate and concentrated under reduced pressure to obtain a crude product of 2-(7-bromobenzo[d][1,3]dioxolan-4-yl)acetic acid (500 mg), ESI-MS (m/z): 257.0[M-H]⁻.

### Synthesis of 2-(7-bromo-2,3-dihydrobenzofuran-4-yl)acetic acid (Intermediate 1.1-B)

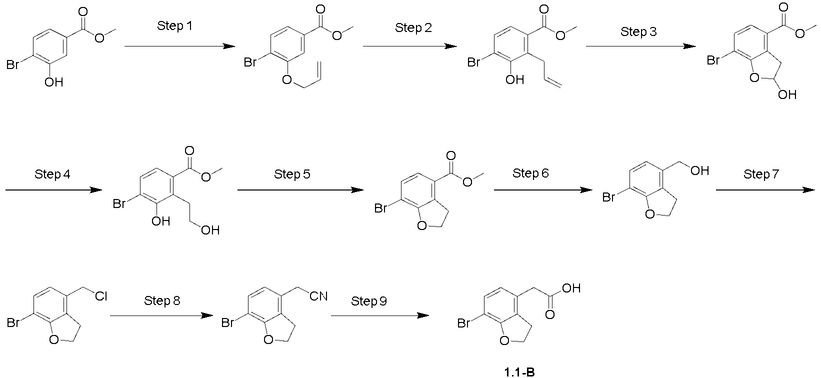

### Step 1: Synthesis of methyl 3-(allyloxy)-4-bromobenzoate

Methyl 4-bromo-3-hydroxybenzoate (30 g, 129.84 mmol) was dissolved in DMF (300 mL), then K₂CO₃ (26.93 g, 194.81 mmol) was added, and the resulting mixture reacted under stirring at 60 °C for 3 hours. After the reaction was completed, the reaction mixture was added with water, extracted with ethyl acetate, washed with saturated brine, dried over anhydrous sodium sulfate, and concentrated under reduced pressure to obtain a crude product of methyl 3-(allyloxy)-4-bromobenzoate (31 g), ESI-MS (m/z): 271.1[M+H]⁺.

### Step 2: Synthesis of methyl 2-allyl-4-bromo-3-hydroxybenzoate

The product obtained from the previous step (10 g, 7.37 mmol) was added to NMP (58 mL). The resulting mixture was stirred and reacted under microwave at 200 °C for 5 hours. After the reaction was completed, the reaction mixture was added with water, extracted with ethyl acetate, washed with saturated brine, dried over anhydrous sodium sulfate, and concentrated under reduced pressure to obtain a crude product, which was purified by column chromatography (ethyl acetate: petroleum ether =1:20) to obtain methyl 2-allyl-4-bromo-3-hydroxybenzoate (6.4 g), ESI-MS (m/z): 271.1 [M+H]⁺.

### Step 3: Synthesis of methyl 2-hydroxy-7-bromo-2,3-dihydrobenzofuran-4-carboxylate

The product obtained from the previous step (4.7 g, 18.44 mmol) was dissolved in THF (90 mL) and H₂O (90 mL), OsO₄ (3.34 g, 13.15 mmol) and NaIO₄ (7.89 g, 36.89 mmol) were added, and the resulting mixture reacted under stirring at 20 °C for 6 hours. After the reaction was completed, the reaction mixture was added with water, extracted with ethyl acetate, washed with saturated brine, dried over anhydrous sodium sulfate and concentrated under reduced pressure to obtain a crude product, which was purified by column chromatography (ethyl acetate: petroleum ether =1:5) to obtain methyl 2-hydroxy-7-bromo-2,3-dihydrobenzofuran-4-carboxylate (4.2 g), ESI-MS (m/z): 273.1[M+H]⁺.

### Step 4: Synthesis of methyl 4-bromo-3-hydroxy-2-(2-hydroxyethyl) benzoate

The product obtained from the previous step (4.2 g, 14.65 mmol) was added to THF (56 mL) and MeOH (56 mL), then NaBH₄ (554.16 mg, 14.65 mmol) was added, and the resulting mixture reacted under stirring at 0 °C for 1 hour. After the reaction was completed, the reaction mixture was added with water, then extracted with ethyl acetate, washed with saturated brine, dried over anhydrous sodium sulfate, and concentrated under reduced pressure to obtain a crude product of methyl 4-bromo-3-hydroxy-2-(2-hydroxyethyl)benzoate (3.5 g), ESI-MS (m/z): 275.0[M+H]⁺.

### Step 5: Synthesis of methyl 7-bromo-2,3-dihydrobenzofuran-4-carboxylate

The product obtained from the previous step (3.5 g, 12.00 mmol) was dissolved in THF (103 mL), then PPh₃ (3.78 g, 14.41 mmol) and DIAD (2.91 g, 14.40 mmol) were added, and the resulting mixture reacted under stirring at 50 °C for 5 hours. TLC detected that the starting materials reacted completely, and then concentration under reduced pressure was performed to obtain a crude product, which was purified by column chromatography (ethyl acetate: petroleum ether =1:5) to obtain methyl 7-bromo-2,3-dihydrobenzofuran-4-carboxylate (2.5 g), ESI-MS (m/z): 257.2[M+H]⁺.

### Step 6: Synthesis of (7-bromo-2,3-dihydrobenzofuran-4-yl)methanol

The product obtained from the previous step (2.5 g, 9.72 mmol) was added to THF (38 mL), cooled to 0 °C, and added with LiAlH₄ (443 mg, 11.67 mmol) in batches. The resulting mixture was further stirred and reacted at 0 °C for 1 hour. After the reaction was completed, the reaction mixture was quenched with water, extracted with ethyl acetate, washed with saturated brine, dried over anhydrous sodium sulfate, and concentrated under reduced pressure to obtain a crude product of (7-bromo-2,3-dihydrobenzofuran-4-yl)methanol (1.9 g), ESI-MS (m/z): 229.0[M+H]⁺.

### Step 7: Synthesis of 7-bromo-4-(chloromethyl)-2,3-dihydrobenzofuran

The product obtained from the previous step (1.9 g, 8.16 mmol) was dissolved in dichloromethane (18.7 mL), SOCl₂ (4.86 g, 40.82 mmol) was added, and the resulting mixture reacted under stirring at 20 °C for 2 hours. TLC detected that the starting materials reacted completely, and the reaction mixture was quenched with water, extracted with ethyl acetate, washed with saturated brine, dried over anhydrous sodium sulfate and concentrated under reduced pressure to obtain a crude product, which was purified by column chromatography (ethyl acetate: petroleum ether =1:5) to obtain 7-bromo-4-(chloromethyl)-2,3-dihydrobenzofuran (1.6 g), ESI-MS (m/z): 247.1[M+H]⁺.

### Step 8: Synthesis of 2-(7-bromo-2,3-dihydrobenzofuran-4-yl)acetonitrile

The product obtained from the previous step (2.0 g, 8.07 mmol) was added to DMF (16 mL), then NaCN (3.17 g, 80.7 mmol) was added, and the resulting mixture reacted under stirring at 90 °C for 4 hours. After the reaction was completed, the reaction mixture was added with water, then extracted with ethyl acetate, washed with saturated brine, dried over anhydrous sodium sulfate and concentrated under reduced pressure to obtain a crude product, which was subjected to column chromatography (ethyl acetate: petroleum ether =1:3) to obtain 2-(7-bromo-2,3-dihydrobenzofuran-4-yl)acetonitrile (1 g), ESI-MS (m/z): 238.1[M+H]⁺.

### Step 9: Synthesis of 2-(7-bromo-2,3-dihydrobenzofuran-4-yl)acetic acid

The product obtained from the previous step (1 g, 4.20 mmol) was dissolved in EtOH (10 mL) and H₂O (10 mL), then KOH (855 mg, 15.25 mmol) was added, and the resulting mixture reacted under stirring at 80 °C for 3 hours. After the reaction was completed, the reaction mixture was quenched with water, extracted with trichloromethane, adjusted to pH=5 with 1 M HCl, extracted with dichloromethane, dried over anhydrous sodium sulfate, and concentrated under reduced pressure to obtain a crude product of 2-(7-bromo-2,3-dihydrobenzofuran-4-yl)acetic acid (900 mg), ESI-MS (m/z): 255.1[M-H]⁻.

### Synthesis of 2-(4-bromo-2,3-dihydrobenzofuran-7-yl)acetic acid (Intermediate 1.1-C)

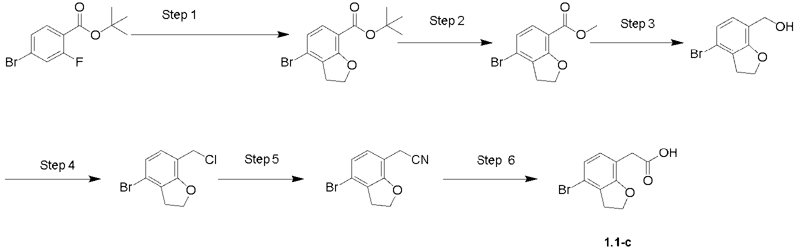

### Step 1: Synthesis of tert-butyl 4-bromo-2,3-dihydrobenzofuran-7-carboxylate

Tert-butyl 4-bromo-2-fluoro-benzoate (20 g, 72.7 mmol) was added to THF (400 mL), and air replacement with nitrogen was performed. The temperature in the flask was lowered to -78 °C with a dry ice acetone bath, LDA (1.5 M, 48.46 mL) was slowly added dropwise to the resulting mixture using a constant-pressure dropping funnel, and the dropwise addition was completed within 60 minutes. The reaction mixture was stirred at -78 °C for 4 hours, and the solution presented a yellow color. Ethylene oxide was added to the reaction mixture, and the reaction mixture was stirred at -78 °C for 6 hours. After the reaction was completed, the reaction was quenched with aqueous ammonium chloride solution, and extracted with ethyl acetate. The resulting organic phase was collected, washed with brine, dried over anhydrous sodium sulfate, and concentrated under reduced pressure to obtain tert-butyl 4-bromo-2,3-dihydrobenzofuran-7-carboxylate (3 g), ESI-MS (m/z): 299.2[M+H]⁺.

### Step 2: Synthesis of methyl 4-bromo-2,3-dihydrobenzofuran-7-carboxylate

The product obtained from the previous step (2.3 g, 7.69 mmol) was dissolved in a methanol solution (46 mL) of hydrochloric acid, and the resulting mixture reacted under stirring at 20 °C for 3 hours. After the reaction was completed, the reaction mixture was concentrated under reduced pressure and the resulting crude product was purified by column chromatography (ethyl acetate: petroleum ether =1:10) to obtain methyl 4-bromo-2,3-dihydrobenzofuran-7-carboxylate (1.7 g), ESI-MS (m/z): 257.2[M+H]⁺.

### Step 3: Synthesis of (4-bromo-2,3-dihydrobenzofuran-7-yl) methanol

The product obtained from the previous step (1.7 g, 6.61 mmol) was added to THF (17 mL), then lithium aluminum hydride (301 mg, 7.94 mmol) was added at 0 °C, and the resulting mixture reacted under stirring at 0 °C for 2 h. After the reaction was completed, the reaction mixture was quenched with water, then extracted with ethyl acetate, washed with saturated brine, dried over anhydrous sodium sulfate, and concentrated under reduced pressure to obtain a crude product of (4-bromo-2,3-dihydrobenzofuran-7-yl)methanol (1.4 g), ESI-MS (m/z): 229.1[M+H]⁺.

### Step 4: Synthesis of 4-bromo-7-(chloromethyl)-2,3-dihydrobenzofuran

The product obtained from the previous step (1.4 g, 6.11 mmol) was added to DCM (14 mL), cooled to 0 °C, and then added with SOCl₂ (3.64 g, 30.56 mmol). The resulting mixture reacted under stirring at 0 °C for 2 hours. TLC detected that the starting materials reacted completely. The reaction mixture was quenched with water, then extracted with ethyl acetate, washed with saturated brine, dried over anhydrous sodium sulfate and concentrated under reduced pressure to obtain a crude product, which was purified by column chromatography (ethyl acetate: petroleum ether =1:5) to obtain 4-bromo-7-(chloromethyl)-2,3-dihydrobenzofuran (1.3 g), ESI-MS (m/z): 247.2[M+H]⁺.

### Step 5: Synthesis of 2-(4-bromo-2,3-dihydrobenzofuran-7-yl)acetonitrile

The product obtained from the previous step (1.3 g, 5.25 mmol) was added to DMF (13 mL), then NaCN (2.57 g, 52.52 mmol) was added, and the resulting mixture reacted under stirring at 90 °C for 4 hours. After the reaction was completed, the reaction mixture was quenched with water, then extracted with ethyl acetate, washed with saturated brine, dried over anhydrous sodium sulfate and concentrated under reduced pressure to obtain a crude product, which was subjected to column chromatography (ethyl acetate: petroleum ether =1:5) to obtain 2-(4-bromo-2,3-dihydrobenzofuran-7-yl)acetonitrile (1 g), ESI-MS (m/z): 238.0[M+H]⁺.

### Step 6: Synthesis of 2-(4-bromo-2,3-dihydrobenzofuran-7-yl)acetic acid

The product obtained from the previous step (800 mg, 3.36 mmol) was dissolved in EtOH (8 mL), then KOH (684 mg, 12.20 mmol) was added, and the resulting mixture reacted under stirring at 80 °C for 3 hours. TLC detected that the starting materials reacted completely, and the reaction mixture was added with water, extracted with chloroform, adjusted to pH=5 with 1 M HCl, extracted with dichloromethane, dried over anhydrous sodium sulfate, and concentrated under reduced pressure to obtain a crude product of 2-(4-bromo-2,3-dihydrobenzofuran-7-yl)acetic acid (760 mg), ESI-MS (m/z): 255.1[M-H]⁻.

### Synthesis of 2-(7-bromo-1H-indol-4-yl)acetic acid (Intermediate 1.1-D)

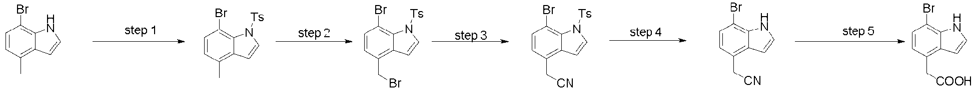

### Step 1: Synthesis of 7-bromo-4-methyl-1-(p-toluenesulfonyl)-1H-indole

7-Bromo-4-methyl-1H-indole (1.4 g, 6.7 mmol) and N,N-dimethylformamide (20 mL) were added to a 100 mL flask, sodium hydride (536 mg, 13.4 mmol) was added in batches at 0 °C, and the resulting mixture was further stirred for 10 minutes. p-toluenesulfonyl chloride (1.91 g, 10 mmol) was added, the temperature was slowly raised to room temperature, and the resulting mixture further reacted under stirring. After the reaction was completed, the reaction mixture was poured into water, extracted with ethyl acetate, dried, and concentrated. The resulting crude product was purified by column chromatography (ethyl acetate: petroleum ether=1:10) to obtain 7-bromo-4-methyl-1-(p-toluenesulfonyl)-1H-indole (2 g), ESI-MS (m/z): 364.0[M+H]⁺.

### Step 2: Synthesis of 7-bromo-4-(bromomethyl)-1-(p-toluenesulfonyl)-1H-indole

7-Bromo-4-methyl-1-(p-toluenesulfonyl)-1H-indole (2 g, 5.51 mmol), NBS (1.17 g, 6.61 mmol), AIBN (181 mg, 1.1 mmol) and carbon tetrachloride (30 mL) were added into a 100 mL flask, and the resulting mixture was heated to 80 °C and reacted under stirring. After the reaction was completed, the solvent was removed by concentration and the resulting crude product was isolated by column chromatography (ethyl acetate: petroleum ether =1:10) to obtain 7-bromo-4-(bromomethyl)-1-(p-toluenesulfonyl)-1H-indole (1.6 g), ESI-MS (m/z): 441.9[M+H]⁺.

### Step 3: Synthesis of 2-(7-bromo-1-(p-toluenesulfonyl)-1H-indol-4-yl)acetonitrile

7-Bromo-4-(bromomethyl)-1-(p-toluenesulfonyl)-1H-indole (1.6 g, 3.62 mmol), trimethylsilyl cyanide (538 mg, 5.44 mmol), potassium carbonate (1.5 g, 11.2 mmol) and acetonitrile (50 mL) were added into a 100 mL flask, and the resulting mixture was heated to 80 ° and reacted under stirring. After the reaction was completed, the reaction mixture was poured into water, extracted with ethyl acetate, dried, and then concentrated, and the resulting crude product was isolated and purified by column chromatography (ethyl acetate: petroleum ether =1:10) to obtain 2-(7-bromo-1-(p-toluenesulfonyl)-1H-indol-4-yl)acetonitrile (1.2 g), ESI-MS (m/z): 389.0[M+H]⁺.

### Step 4: Synthesis of 2-(7-bromo-1H-indol-4-yl)acetonitrile

2-(7-Bromo-1-(p-toluenesulfonyl)-1H-indol-4-yl)acetonitrile (1.4 g, 3.61 mmol) and methanol (40 mL) were added to a 100 mL flask. Sodium hydroxide (1.44 g, 36.1 mmol) was added. The resulting mixture was heated to 65 °C and reacted under stirring. After the reaction was completed, the reaction mixture was concentrated and the resulting crude product was purified by column chromatography (ethyl acetate: petroleum ether =1:5) to obtain 2-(7-bromo-1H-indol-4-yl)acetonitrile (500 mg), ESI-MS (m/z): 235.0[M+H]⁺.

### Step 5: Synthesis of 2-(7-bromo-1H-indol-4-yl)acetic acid

2-(7-Bromo-1H-indol-4-yl)acetonitrile (200 mg, 0.85 mmol), potassium hydroxide (476 mg, 8.5 mmol), ethanol (5 mL) and water (10 mL) were added to a 25 mL round bottom flask, heated to 100 °C and reacted under stirring. After the reaction was completed, the reaction mixture was concentrated, adjusted to pH=3 with acetic acid, and further concentrated, and the resulting crude product was purified by column chromatography (methanol: dichloromethane =1:15) to obtain 2-(7-bromo-1H-indol-4-yl)acetic acid (180 mg), ESI-MS (m/z): 254.0[M+H]⁺.

### Synthesis of 2-(7-bromo-5-chloro-2,3-dihydrobenzofuran-4-yl)acetic acid (Intermediate 1.1-E)

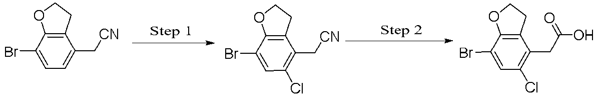

### Step 1: 2-(7-bromo-5-chloro-2,3-dihydrobenzofuran-4-yl)acetonitrile

2-(7-Bromo-2,3-dihydrobenzofuran-4-yl)acetonitrile (500 mg, 2.11 mmol) and N, N-dimethylformamide (10 mL) were added to a 100 mL flask, and then N-chlorosuccinimide (337 mg, 2.5 mmol) was added. The reaction mixture reacted under stirring at 60 °C. After the reaction was completed, the temperature was lowered, the resulting mixture was added into ethyl acetate and washed with water, and the organic phase was dried over anhydrous sodium sulfate, then filtered and concentrated. The resulting crude product was isolated and purified by column chromatography (ethyl acetate: petroleum ether =1:10) to obtain 2-(7-bromo-5-chloro-2,3-dihydrobenzofuran-4-yl)acetonitrile (480 mg).

### Step 2: 2-(7-bromo-5-chloro-2,3-dihydrobenzofuran-4-yl)acetic acid

2-(7-Bromo-5-chloro-2,3-dihydrobenzofuran-4-yl)acetonitrile (480 mg, 1.77 mmol) and ethanol (20 mL) were added to a 100 mL flask, and then potassium hydroxide (992 mg, 17.7 mmol) and water (25 mL) were added. The reaction mixture reacted under stirring at 100 °C. After the reaction was completed, the temperature was lowered, the solution was adjusted to be acidic with 1 N hydrochloric acid solution, and extracted with ethyl acetate, and the resulting organic phase was dried over anhydrous sodium sulfate, then filtered and concentrated. The resulting crude product was isolated and purified by thin layer chromatography (methanol: dichloromethane =1:15) to obtain 2-(7-bromo-5-chloro-2,3-dihydrobenzofuran-4-yl)acetic acid (480 mg), ESI-MS (m/z): 289.0[M-H]⁻.

### Synthesis of 2-(7-bromo-5-fluoro-2,3-dihydrobenzofuran-4-yl)acetic acid (Intermediate 1.1-F)

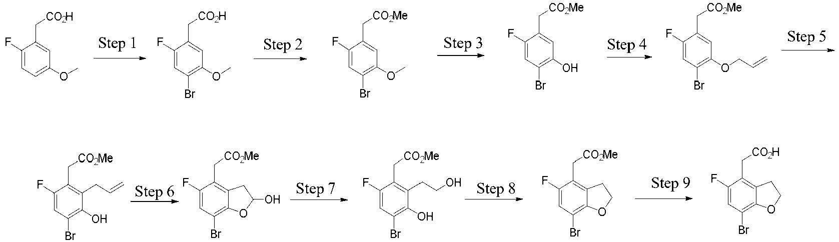

### Step 1: 2-(4-Bromo-2-fluoro-5-methoxyphenyl)acetic acid

2-(4-Bromo-5-methoxyphenyl)acetic acid (4.5 g, 24.43 mmol) was dissolved in acetonitrile (120 mL), Br₂ (3.51g, 21.99 mmol, 1.13 mL) was added at 0 °C, and the reaction mixture reacted under stirring at 20 °C. After the reaction was completed, the reaction mixture was added with water, adjusted to pH= about 5 with acetic acid, and then extracted with ethyl acetate. The resulting organic phase was washed with NaCl solution, dried over anhydrous sodium sulfate, and distilled under reduced pressure to obtain 2-(4-bromo-2-fluoro-5-methoxyphenyl)acetic acid (8.6g), ESI-MS (m/z): 261.0[M-H]⁻.

### Step 2: Methyl 2-(4-bromo-2-fluoro-5-methoxyphenyl)acetate

The product obtained from the previous step (7.9 g, 17.72 mmol) was dissolved in DMF (80 mL), K₂CO₃ (4.9 g, 35.44 mmol) and MeI (3.77 g, 26.58 mmol) were added, and the reaction mixture reacted under stirring at 20 °C. After the reaction was completed, the reaction mixture was added with water and extracted with ethyl acetate, and the resulting organic phase was washed with a half-saturated NaCl solution, dried over anhydrous sodium sulfate and distilled under reduced pressure to obtain a crude product, which was purified by column chromatography (ethyl acetate: petroleum ether =1:1) to obtain methyl 2-(4-bromo-2-fluoro-5-methoxyphenyl)acetate (4.2 g), ESI-MS (m/z): 277.0[M+H]⁺.

### Step 3: Methyl 2-(4-bromo-2-fluoro-5-hydroxyphenyl)acetate

The product obtained from the previous step (4.2 g, 15.16 mmol) was dissolved in dichloromethane (72 mL), then BBr₃ (11.39 g, 45.47 mmol) was added, and the reaction mixture reacted under stirring at 0 °C. After the reaction was completed, the reaction mixture was quenched with a saturated NaHCO₃ solution (20 mL) and extracted with ethyl acetate, and the resulting organic phase was washed with a saturated NaCl solution, dried over anhydrous sodium sulfate, and distilled under reduced pressure to obtain methyl 2-(4-bromo-2-fluoro-5-hydroxyphenyl)acetate (4 g), ESI-MS (m/z): 263.0 [M+H]⁺.

### Step 4: Methyl 2-(5-allyloxy-4-bromo-2-fluorophenyl)acetate

The product obtained from the previous step (4 g, 15.21 mmol) and allyl bromide (2.76 g, 22.81 mmol) were dissolved in DMF (40 mL), then potassium carbonate (3.15 g, 22.81 mmol) was added, and the reaction mixture reacted under stirring at 60 °C. After the reaction was completed, the reaction mixture was added with water and extracted with ethyl acetate. The resulting organic phase was washed with a half-saturated NaCl solution, dried over anhydrous sodium sulfate and distilled under reduced pressure to obtain a crude product, which was purified by column chromatography (ethyl acetate: petroleum ether =1:2) to obtain methyl 2-(5-allyloxy-4-bromo-2-fluorophenyl)acetate (3.7 g), ESI-MS (m/z): 303.1[M+H]⁺.

### Step 5: Methyl 2-(2-allyl-4-bromo-6-fluoro-3-hydroxyphenyl)acetate

The product obtained from the previous step (3.7 g, 12.21 mmol) was dissolved in NMP (15 mL) and reacted under microwave at 200 °C for 4 hours. After the reaction was completed, the reaction mixture was added with water and extracted with ethyl acetate, and the resulting organic phase was washed with a half-saturated NaCl solution, dried over anhydrous sodium sulfate and distilled under reduced pressure to obtain a crude product, which was purified by column chromatography (ethyl acetate: petroleum ether =1:3) to obtain methyl 2-(2-allyl-4-bromo-6-fluoro-3-hydroxyphenyl)acetate (2.45 g), ESI-MS (m/z): 303.1[M+H]⁺.

### Step 6: Methyl 2-(7-bromo-5-fluoro-2-hydroxy-2,3-dihydrobenzofuran-4-yl)acetate

The product obtained from the previous step (2.35 g, 7.75 mmol) was dissolved in THF (40 mL) and H₂O (40 mL), NaIO₄ (3.32 g, 15.51 mmol) and OsO₄ (1.41 g, 5.53 mmol) were added, and the reaction mixture reacted under stirring at 20 °C. After the reaction was completed, the reaction mixture was added with water and extracted with ethyl acetate, and the resulting organic phase was washed with NaCl solution, dried over anhydrous sodium sulfate and distilled under reduced pressure to obtain a crude product, which was purified by column chromatography (ethyl acetate: petroleum ether =1:2) to obtain methyl 2-(7-bromo-5-fluoro-2-hydroxy-2,3-dihydrobenzofuran-4-yl)acetate (1.3 g), ESI-MS (m/z): 305.1[M+H]⁺.

### Step 7: Methyl 2-(4-bromo-6-fluoro-3-hydroxy-2-(2-hydroxyethyl) phenyl)acetate

The product obtained from the previous step (1.2 g, 3.93 mmol) was dissolved in THF (15 mL) and MeOH (15 mL), NaBH₄ (148.80 mg, 3.93 mmol) was added, and the reaction mixture reacted under stirring at 0 °C. After the reaction was completed, the reaction mixture was quenched with NH₄Cl solution and extracted with ethyl acetate, and the resulting organic phase was washed with half-saturated NaCl, dried over anhydrous sodium sulfate, and distilled under reduced pressure to obtain a crude product of methyl 2-(4-bromo-6-fluoro-3-hydroxy-2-(2-hydroxyethyl)phenyl)acetate (1.1 g), ESI-MS (m/z): 307.1[M+H]⁺.

### Step 8: Methyl 2-(7-bromo-5-fluoro-2,3-dihydrobenzofuran-4-yl)acetate

The product obtained from the previous step (1.1 g, 3.58 mmol) was dissolved in THF (22 mL), PPh₃ (1.13 g, 4.3 mmol) and DIAD (1.9 M, 2.26 mL) were added at 20 °C, and the reaction mixture reacted under stirring at 50 °C. LCMS detected the presence of the desired product, the reaction mixture was quenched with H₂O and extracted with ethyl acetate, and the resulting organic phase was washed with half-saturated NaCl (20 mL), dried over anhydrous sodium sulfate and distilled under reduced pressure to obtain a crude product, which was purified by column chromatography (PE:EA=2:1) to obtain methyl 2-(7-bromo-5-fluoro-2,3-dihydrobenzofuran-4-yl)acetate (834 mg), ESI-MS (m/z): 289.1[M+H]⁺.

### Step 9: 2-(7-bromo-5-fluoro-2,3-dihydrobenzofuran-4-yl)acetic acid

The product obtained from the previous step (200 mg, 0. 7 mmol) was dissolved in MeOH (2 mL), THF (2 mL) and H₂O (2 mL), LiOH (66 mg, 2.7 mmol) was added, and the resulting mixture reacted under stirring at 20 °C. After the reaction was completed, the reaction mixture was concentrated under reduced pressure, adjusted to be acidic with dilute hydrochloric acid, extracted with ethyl acetate, dried over anhydrous sodium sulfate, filtered, concentrated, and purified by column chromatography (ethyl acetate: petroleum ether =1:2) to obtain 2-(7-bromo-5-fluoro-2,3-dihydrobenzofuran-4-yl)acetic acid (120 mg), ESI-MS (m/z): 273.1[M-H]⁻.

### Synthesis of 2-(8-bromo-2,3-dihydrobenzo[b][1,4]dioxan-5-yl)acetic acid (Intermediate 1.1-G)

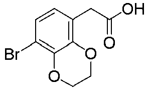

The target compound 2-(8-bromo-2,3-dihydrobenzo[b][1,4]dioxan-5-yl)acetic acid was obtained by referring to the synthetic method of Intermediate 1.1-A using ethyl 4-bromo-2,3-dihydroxybenzoate and 1,2-dibromoethane as the starting materials. ESI-MS (m/z): 271.0[M-H]⁻.

### Synthesis of 2-(1-oxo-7-(((trifluoromethyl)sulfonyl)oxy)-2,3-dihydro-1H-inden-4-yl)acetic acid (Intermediate 1.1-H)

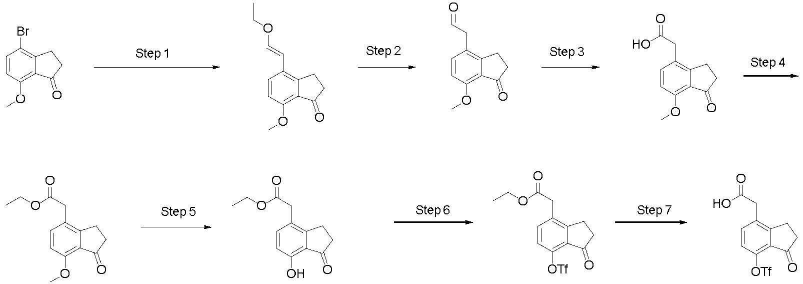

### Step 1: (E)-4-(2-ethoxyvinyl)-7-methoxy-2,3-dihydro-1H-inden-1-one

4-Bromo-7-methoxy-1-indanone (5 g, 20.74 mmol), (E)-1-ethoxyvinyl-2-boronic acid pinacol ester (4.5 g, 22.85 mmol), [1,1'-Bis(diphenylphosphino)ferrocene]dichloropalladium(II) (1.5 g, 2.05 mmol) and cesium carbonate (20 g, 60.79 mmol) were added to dioxane (50 mL) and water (10 mL), and under the protection of argon, the resulting mixture was heated to 100 °C and reacted under stirring. After the reaction was completed, the reaction mixture was added with saturated brine and extracted with dichloromethane, and the resulting organic phase was dried over anhydrous sodium sulfate and concentrated under reduced pressure to obtain a crude product, which was purified by column chromatography (ethyl acetate: petroleum ether =1:10) to obtain (E)-4-(2-ethoxyvinyl)-7-methoxy-2,3-dihydro-1H-inden-1-one (4.6 g), ESI-MS (m/z): 233.1[M+H]⁺.

### Step 2: 2-(7-methoxy-1-oxo-2,3-dihydro-1H-inden-4-yl)acetaldehyde

The product obtained from the previous step (4.6 g, 19.82 mmol) was dissolved in anhydrous formic acid (40 mL) and reacted under stirring at room temperature. After the reaction was completed, distillation under reduced pressure was performed to obtain a crude product of 2-(7-methoxy-1-oxo-2,3-dihydro-1H-inden-4-yl)acetaldehyde.

### Step 3: 2-(7-methoxy-1-oxo-2,3-dihydro-1H-inden-4-yl)acetic acid

The product obtained from the previous step was dissolved in a mixed solution of dimethyl sulfoxide (25 mL) and tetrahydrofuran (25 mL). Sodium chlorite (9 g, 99.51 mmol) and sodium dihydrogen phosphate (16.6 g, 138.36 mmol) were dissolved in water (25 mL), and added dropwise into the above reaction mixture in an ice-water bath. The reaction mixture reacted under stirring for 30 min in an ice-water bath, and was further stirred at room temperature. After the reaction was completed, the reaction mixture was added with saturated brine, adjusted to pH =2-3 by dropwise addition of 1 N hydrochloric acid, and extracted with ethyl acetate, and the resulting organic phase was washed with saturated brine, dried over anhydrous sodium sulfate, and concentrated under reduced pressure to obtain a crude product of 2-(7-methoxy-1-oxo-2,3-dihydro-1H-inden-4-yl)acetic acid.

### Step 4: Ethyl 2-(7-methoxy-1-oxo-2,3-dihydro-1H-inden-4-yl)acetate

The product obtained from the previous step was dissolved in anhydrous *N, N*-dimethylformamide (30 mL), then potassium carbonate (5.5 g, 39.57 mmol) and iodoethane (3.7 g, 23.72 mmol) were added, and the resulting mixture reacted under stirring at room temperature. After the reaction was completed, saturated brine was added, extraction was performed with ethyl acetate, and the resulting organic phase was washed with saturated brine, dried over anhydrous sodium sulfate and distilled under reduced pressure to obtain a crude product, which was purified by column chromatography (ethyl acetate: petroleum ether=1:10) to obtain ethyl 2-(7-methoxy-1-oxo-2,3-dihydro-1H-inden-4-yl)acetate (4.1 g), ESI-MS (m/z): 249.1[M+H]⁺.

### Step 5: Ethyl 2-(7-hydroxy-1-oxo-2,3-dihydro-1H-inden-4-yl)acetate

The product obtained from the previous step was dissolved in dichloromethane (10 mL), then boron tribromide (1 g, 3.99 mmol) was added dropwise under an ice-water bath, and the reaction mixture reacted under stirring in an ice bath. After the reaction was completed, the reaction was quenched with anhydrous ethanol, a saturated sodium bicarbonate solution was added, extraction was performed with dichloromethane, and the resulting organic phase was washed with saturated brine, dried over anhydrous sodium sulfate and distilled under reduced pressure to obtain a crude product of ethyl 2-(7-hydroxy-1-oxo-2,3-dihydro-1H-inden-4-yl)acetate.

### Step 6: Ethyl 2-(1-oxo-7-(((trifluoromethyl)sulfonyl)oxy)-2,3-dihydro-1H-inden-4-yl)acetate

The product obtained from the previous step was dissolved in dichloromethane (20 mL), then diisopropylethylamine (331 mg, 2.57 mmol) was added, then trifluoromethanesulfonic anhydride (360 mg, 1.28 mmol) was added dropwise, and the resulting mixture reacted under stirring at room temperature. After the reaction was completed, saturated brine was added, extraction was performed with dichloromethane, and the resulting organic phase was washed with saturated brine, dried over anhydrous sodium sulfate and distilled under reduced pressure to obtain a crude product, which was purified by column chromatography (ethyl acetate: petroleum ether =1:8) to obtain ethyl 2-(1-oxo-7-(((trifluoromethyl)sulfonyl)oxy)-2,3-dihydro-1H-inden-4-yl)acetate (190 mg), ESI-MS (m/z): 367.0[M+H]⁺.

### Step 7: 2-(1-oxo-7-(((trifluoromethyl)sulfonyl)oxy)-2,3-dihydro-1H-inden-4-yl)acetic acid

The product obtained from the previous step (110 mg, 0.31 mmol) was dissolved in dioxane (8 mL), then water (1.5 mL) and lithium hydroxide (24 mg, 1.00 mmol) were added, and the resulting mixture reacted under stirring at room temperature. After the reaction was completed, the pH of the reaction mixture was adjusted to about 2 with dilute hydrochloric acid, and then extraction was performed with ethyl acetate. The resulting organic phase was washed with saturated sodium chloride solution, dried over anhydrous sodium sulfate, filtered, concentrated, and subjected to column chromatography (ethyl acetate: petroleum ether =1:2) to obtain 2-(1-oxo-7-(((trifluoromethyl)sulfonyl)oxy)-2,3-dihydro-1H-inden-4-yl)acetic acid (75 mg), ESI-MS (m/z): 337.0[M-H]⁻.

### Synthesis of methyl (S)-4-amino-3-((oxetan-2-ylmethyl)amino)benzoate (Intermediate 1.2-A)

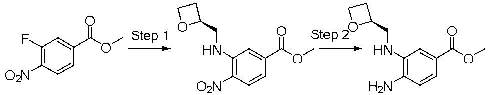

### Step 1: Synthesis of methyl (S)-4-nitro-3-((oxetan-2-ylmethyl)amino)benzoate

Methyl 3-fluoro-4-nitrobenzoate (10 g, 50.20 mmol), (S)-oxetane-2-methanamine (5.25 g, 60.20 mmol), DIPEA (12.90 g, 100.4 mmol) and DMF (50 mL) were added to a 200 mL round bottom flask, heated to 70 °C and stirred for 3 h. After the reaction was completed, the temperature was lowered to room temperature, and extraction was performed with water and ethyl acetate, followed by washing with water and washing with saturated sodium chloride. The resulting solution was dried over anhydrous sodium sulfate, filtered and concentrated to obtain the target product methyl (S)-4-nitro-3-((oxetan-2-ylmethyl)amino)benzoate (13.1 g), ESI-MS (m/z): 267.1[M+H]⁺.

### Step 2: Synthesis of methyl (S)-4-amino-3-((oxetan-2-ylmethyl)amino)benzoate

The product obtained from the previous step methyl (S)-4-nitro-3-((oxetan-2-ylmethyl)amino)benzoate (5 g, 18.79 mmol), iron powder (5.24 g, 93.98 mmol), ammonium chloride (9.96 g, 187.9 mmol) and ethanol (50 mL), and water (10 mL) were added to a 100 mL round bottom flask and reacted at room temperature for 3 hours. After the reaction was completed, filtration was performed, followed by removal of ethanol by rotary evaporation. The resulting mixture was extracted with water and ethyl acetate, washed with saturated sodium chloride, dried over anhydrous sodium sulfate, filtered and spin-dried to obtain the target product methyl (S)-4-amino-3-((oxetan-2-ylmethyl)amino)benzoate (3.6 g), ESI-MS (m/z): 237.1[M+H]⁺.

### Synthesis of methyl (S)-4-amino-5-fluoro-3-((oxetan-2-ylmethyl) amino)benzoate (Intermediate 1.2-B)

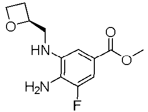

The compound methyl (S)-4-amino-5-fluoro-3-((oxetan-2-ylmethyl) amino)benzoate (3.8 g) was obtained by referring to the synthetic method of Intermediate 1.1-A using methyl 3,5-difluoro-4-nitrobenzoate as the starting material for synthesis. ESI-MS (m/z): 255.1 [M+H]⁺.

### Synthesis of methyl 4-amino-3-(((1-(cyanomethyl)cyclopropyl)methyl)amino)benzoate (Intermediate 1.2-C)

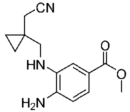

Methyl 4-amino-3-(((1-(cyanomethyl)cyclopropyl)methyl)amino)benzoate (520 mg) was obtained by referring to the synthetic method of Intermediate 1.2-A using 2-(1-(aminomethyl)cyclopropyl)acetonitrile as the starting material. ESI-MS (m/z): 260.2[M+H]⁺.

### Synthesis of methyl 4-amino-3-(((1-(fluoromethyl)cyclopropyl)methyl)amino)benzoate (Intermediate 1.2-D)

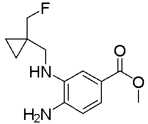

Methyl 4-amino-3-(((1-(fluoromethyl)cyclopropyl)methyl)amino)benzoate (1543 mg) was obtained by referring to the synthetic method of Intermediate 1.2-C using (1-(fluoromethyl)cyclopropyl)methanamine as the starting material. ESI-MS (m/z): 253.1[M+H]⁺.

Among them, the synthesis of (1-(fluoromethyl)cyclopropyl)methanamine was as follows:

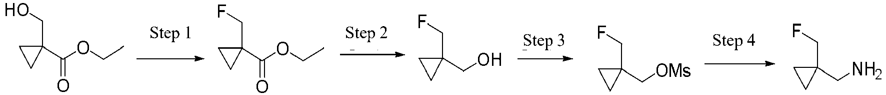

### Step 1: Ethyl 1-(fluoromethyl)cyclopropane-1-carboxylate

Ethyl 1-(hydroxymethyl)cyclopropane-1-carboxylate (3 g, 26.3 mmol) and dichloromethane (50 mL) were added into a 100 mL flask, and cooled to -78 °C. Diethylaminosulfur trifluoride (5.1 g, 31.1 mmol) was then added, and the reaction mixture reacted under stirring at room temperature. After the reaction was completed, the reaction mixture was poured into water, and extracted with dichloromethane, and the resulting organic phase was dried over anhydrous magnesium sulfate, then filtered and concentrated to obtain ethyl 1-(fluoromethyl)cyclopropane-1-carboxylate (4.7 g, crude).

### Step 2: (1-(fluoromethyl)cyclopropyl)methanol

The product obtained from the previous step (4.7 g, crude) and tetrahydrofuran (30 mL) were added into a 250 mL flask, lithium aluminum hydride (1.5 g, 39.45 mmol) was added in batches under an ice bath, and the resulting mixture reacted under stirring at room temperature. After the reaction was completed, the reaction mixture was slowly added dropwise into water and extracted with dichloromethane, and the resulting organic phase was dried over anhydrous magnesium sulfate, then filtered and concentrated to obtain (1-(fluoromethyl)cyclopropyl)methanol (4.0 g, crude).

### Step 3: (1-(fluoromethyl)cyclopropyl)methyl methanesulfonate

(1-(Fluoromethyl)cyclopropyl)methanol (4.0 g, crude) and dichloromethane (50 mL) were added into a 250 mL flask, then triethylamine (11.6 g, 0.11 mol) and methylsulfonyl chloride (5.3 g, 46.1 mmol) were added, and the resulting mixture reacted under stirring at room temperature. After the reaction was completed, the reaction mixture was poured into water and extracted with dichloromethane, and the resulting organic phase was dried over anhydrous magnesium sulfate, then filtered and concentrated to obtain (1-(fluoromethyl)cyclopropyl)methyl methanesulfonate (6 g, crude).

### Step 4: (1-(fluoromethyl)cyclopropyl)methanamine

(1-(Fluoromethyl)cyclopropyl)methyl methanesulfonate (6 g, crude) and methanol (50 mL) were added into a 250 mL flask, an ammonia/methanol solution (7 M, 10 mL) was added, and the resulting mixture reacted under stirring at room temperature. After the reaction was completed, concentration was performed to obtain (1-(fluoromethyl)cyclopropyl)methanamine (4 g), ESI-MS (m/z): 104.1 [M+H]⁺.

### Synthesis of methyl 5-amino-6-(((1-(cyanomethyl)cyclopropyl)methyl)amino)picolinate (Intermediate 1.2-E)

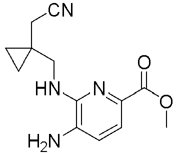

Methyl 5-amino-6-(((1-(cyanomethyl)cyclopropyl)methyl)amino)picolinate (640 mg) was synthesized by referring to the synthetic method of Intermediate 1.2-C using methyl 6-chloro-5-nitropicolinate as the starting material. ESI-MS (m/z): 261.1 [M+H]⁺.

### Methyl (S)-4-amino-3-methoxy-5-((oxetan-2-yl-methyl)amino)benzoate (Intermediate 1.2-F)

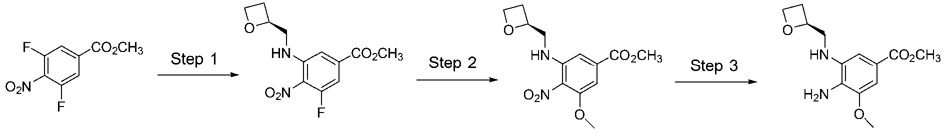

### Step 1: methyl (S)-3-fluoro-4-nitro-5-((oxetan-2-yl-methyl)amino)benzoate

Methyl 3,5-difluoro-4-nitro-benzoate (1.9 g, 8.75 mmol) was added to DMF (20 mL), K₂CO₃ (3.63 g, 26.25 mmol) and (S)-oxetan-2-ylmethanamine (915 mg, 10.50 mmol) were successively added to the reaction mixture, and the reaction mixture reacted under stirring at 20 °C for 2 hours. After the reaction was completed, the reaction mixture was extracted with water and ethyl acetate, and the resulting organic phase was washed with brine, dried over anhydrous sodium sulfate, filtered and concentrated under reduced pressure to obtain methyl (S)-3-fluoro-4-nitro-5-((oxetan-2-yl-methyl)amino)benzoate (2.8 g), ESI-MS (m/z): 285.1[M+H]⁺.

### Step 2: methyl (S)-3-methoxy-4-nitro-5-((oxetan-2-yl-methyl)amino)benzoate

The product obtained from the previous step (1.2 g, 4.22 mmol) was dissolved in MeOH (24 mL), sodium methoxide (1.52 g, 8.44 mmol, 30% content) was added to the reaction mixture, and the resulting mixture reacted under stirring at 20 °C for 36 hours. After the reaction was completed, the reaction mixture was added with a saturated ammonium chloride solution and extracted with ethyl acetate, and the resulting organic phase was washed with saturated brine, dried over anhydrous sodium sulfate, filtered and concentrated under reduced pressure to obtain a crude product. The crude product was purified by column chromatography (ethyl acetate: petroleum ether =1:2) to obtain methyl (S)-3-methoxy-4-nitro-5-((oxetan-2-yl-methyl)amino)benzoate (850 mg), ESI-MS (m/z): 297.1[M+H]⁺.

### Step 3: methyl (S)-4-amino-3-methoxy-5-((oxetan-2-yl-methyl)amino)benzoate

The product obtained from the previous step (650 mg, 2.19 mmol) was dissolved in EtOH (13 mL) and H₂O (2.6 mL), then NH₄Cl (587 mg, 10.97 mmol) and iron powder (368mg, 6.59 mmol) were added to the reaction mixture, and the resulting mixture reacted under stirring at 80 °C for 1 hour. After the reaction was completed, the iron powder was filtered off, and the filtrate was concentrated under reduced pressure, then dissolved in dichloromethane and filtered. The resulting filtrate was concentrated under reduced pressure to obtain methyl (S)-4-amino-3-methoxy-5-((oxetan-2-yl-methyl)amino)benzoate (650 mg), ESI-MS (m/z): 267.0[M+H]⁺.

### Intermediates 1.2-G to 1.2-O were synthesized by referring to the synthetic methods of other intermediates:

| **Intermediate numberings** | **Structures and chemical names** | **Reference intermediates** | **Starting materials used** | **Quality and characterization parameters of intermediates obtained** |
|---|---|---|---|---|
| **Intermediate 1.2-G** | 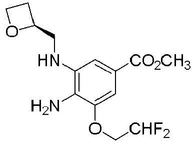 Methyl (S)-4-amino-3-(2,2-difluoroethoxy)-5-((oxetan-2-yl-methyl)amino)benzoate | Intermediate 1.2-L | Methyl 3,5-difluoro-4-nitro-benzoate, (S)-oxetan-2-ylmethanamine and 2,2-difluoroethanol | 840 mg. |
| | | | | ESI-MS (m/z): 317.1 [M+H]⁺ |
| **Intermediate 1.2-H** | 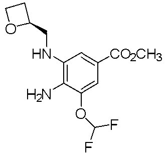 Methyl (S)-4-amino-3-difluoromethoxy-5-((oxetan-2-yl-methyl)amino)benzoate | Intermediate 1.9-C | Consistent with Intermediate 1.9-C | 720 mg. |
| | | | | ESI-MS (m/z): 303.1 [M+H]⁺ |
| **Intermediate 1.2-I** | 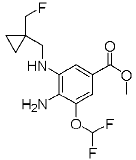 Methyl 4-amino-5-difluoromethoxy-3-(((1-(fluoromethyl)cyclopropyl) methyl)amino)benzoate | Intermediate 1.9-I | Consistent with Intermediate 1.9-I | 930 mg. |
| | | | | ESI-MS (m/z): 319.1[M+H]⁺ |
| **Intermediate 1.2-J** | 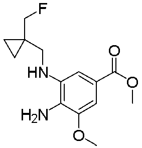 Methyl 4-amino-5-methoxy-3-(((1-(fluoromethyl)cyclopropyl) methyl)amino)benzoate | Intermediate 1.2-0 | Methyl 3,5-difluoro-4-nitro-benzoate and (1-(fluoromethyl) cyclopropyl) methanamine | 1010 mg. |
| | | | | ESI-MS (m/z): 283.2[M+H]⁺ |
| **Intermediate 1.2-K** | 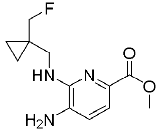 Methyl 5-amino-6-(((1-(fluoromethyl)cyclopropyl) methyl)amino)picolinate | Intermediate 1.2-E | (1-(fluoromethyl) cyclopropyl) methanamine | 720 mg. |
| | | | | ESI-MS (m/z): 253.2 [M+H]⁺ |
| **Intermediate 1.2-L** | 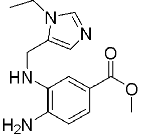 Methyl 4-amino-3-((((1-ethyl-1H-imidazol-4-yl)methyl)amino)-benzoate | Intermediate 1.2-A | (1-ethyl-1H-imidazol-4-yl)methanamine | 504 mg. |
| | | | | ESI-MS (m/z): 275.1 [M+H]⁺ |
| **Intermediate 1.2-M** | 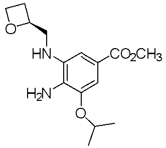 Methyl (S)-4-amino-3-isopropoxy-5-((oxetan-2-yl-methyl)amino)benzoate | Intermediate 1.9-B | Consistent with Intermediate 1.9-B | 790 mg. |
| | | | | ESI-MS (m/z): 295.1 [M+H]⁺ |
| **Intermediate 1.2-N** | 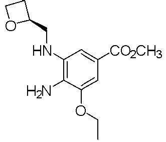 Methyl (S)-4-amino-3-ethoxy-5-((oxetan-2-yl-methyl)amino) benzoate | Intermediate 1.9-B | Ethanol | 790 mg. |
| | | | | ESI-MS (m/z): 281.1[M+H]⁺ |
| **Intermediate 1.2-0** | 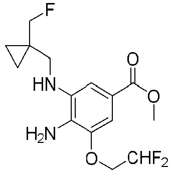 Methyl 4-amino-5-(2,2-difluoroethoxy)-3-(((1-(fluoromethyl)cyclopropyl) methyl)amino)benzoate | Intermediate | 2,2-difluoroethanol | 853 mg. |
| | | 1.9-O | | ESI-MS (m/z): 333.2[M+H]⁺ |

### Synthesis of 4-bromo-2-(4-chloro-2-fluorophenyl)-2-methylbenzo[d][1,3]dioxolane (Intermediate 1.5-A)

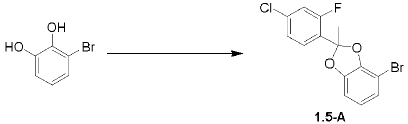

3-Bromocatechol (5 g, 26.45 mmol) was added to toluene (100 mL), and then 4'-chloro-2'-fluoroacetophenone (5.02 g, 29.10 mmol) and p-toluenesulfonic acid monohydrate (5.03 g, 26.45 mmol) were added. The resulting mixture reacted under stirring at 140 °C for 60 hours. Concentration under reduced pressure was performed to obtain a crude product, which was purified by column chromatography (ethyl acetate: petroleum ether =1:10) to obtain 4-bromo-2-(4-chloro-2-fluorophenyl)-2-methylbenzo[d][1,3]dioxolane (600 mg), ESI-MS (m/z): 343.1 [M+H]⁺.

### Synthesis of 2-bromo-6-((4-chloro-2-fluorobenzyl)oxy)pyridine (Intermediate 1.5-B)

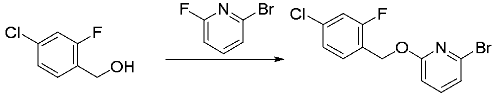

2-Fluoro-4-chlorobenzyl alcohol (3.2 g, 20 mmol) and N, N-dimethylformamide (50 mL) were added into a 100 mL round bottom flask, sodium hydride (1.2 g, 30 mmol) was added in batches under an ice bath, and the reaction mixture was further stirred for 20 minutes under an ice bath. Then 2-bromo-6-fluoropyridine (3.5 g, 20 mmol) was added, and the resulting mixture was subsequently warmed to room temperature and stirred. After the reaction was completed, the reaction mixture was added into ice water (250 mL) and extracted with ethyl acetate, and the resulting organic phase was dried over anhydrous sodium sulfate, filtered, and concentrated, and the resulting crude product was purified by column chromatography (ethyl acetate: n-hexane = 1: 100) to obtain 2-bromo-6-((4-chloro-2-fluorobenzyl)oxy)pyridine (5 g, 79% yield). ESI-MS (m/z): 316.0[M+H]⁺.

### Intermediates 1.5-C to 1.5-AH were synthesized by referring to the synthetic methods of the intermediates described above:

| **Intermediate numberings** | **Structures and chemical names** | **Reference intermediates** | **Starting materials used** | **Quality and characterization parameters of intermediates obtained** |
|---|---|---|---|---|
| **Intermediate 1.5-C** | 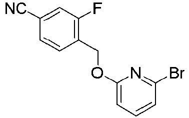 2-Bromo-6-((4-cyano-2-fluorobenzyl)oxy)pyridine | Intermediate 1.5-B | 2-Fluoro-4-cyanobenzyl alcohol | 1030 mg. |
| | | | | ESI-MS (m/z): 307.1[M+H]⁺ |
| **Intermediate 1.5-D** | 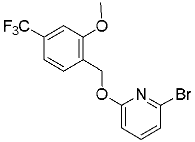 2-Bromo-6-((4-trifluoromethyl-2-methoxybenzyl)oxy)pyridine | Intermediate 1.5-B | 2-Methoxy-4-trifluoromethylbenzyl alcohol | 725 mg. |
| | | | | ESI-MS (m/z): 362.1 [M+H]⁺ |
| **Intermediate 1.5-E** | 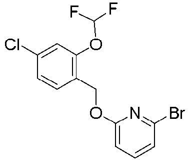 2-Bromo-6-((4-chloro-2-difluoromethoxybenzyl)oxy) pyridine | Intermediate 1.5-B | 2-Difluoromethoxy-4-chlorobenzyl alcohol | 720 mg. |
| | | | | ESI-MS (m/z): 364.1 [M+H]⁺ |
| **Intermediate 1.5-F** | 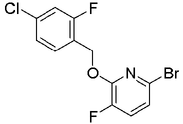 6-Bromo-3-fluoro-2-((4-chloro-2-fluorobenzyl)oxy)pyridine | Intermediate 1.5-B | 2-Chloro-3-fluoro-6-bromopyridine | 820 mg. |
| | | | | ESI-MS (m/z): 334.1 [M+H]⁺ |
| **Intermediate 1.5-G** | 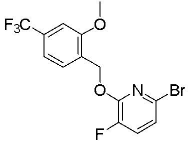 6-Bromo-3-fluoro-2-((4-trifluoromethyl-2-methoxybenzyl)oxy)pyridine | Intermediate 1.5-B | 2-Methoxy-4-trifluoromethylbenzyl alcohol | 700 mg. |
| | | | | ESI-MS (m/z): 380.1[M+H]⁺ |
| **Intermediate 1.5-H** | 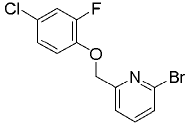 2-Bromo-6-((4-chloro-2-fluorophenoxy)methyl)pyridine | Intermediate 1.5-B | 2-Fluoro-4-chlorophenol and 2-bromo-6-(chloromethyl) pyridine | 180 mg. |
| | | | | ESI-MS (m/z): 316.1[M+H]⁺ |
| **Intermediate 1.5-I** | 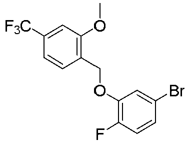 4-Bromo-1-fluoro-2-((4-trifluoromethyl-2-methoxybenzyl)oxy)benzene | Intermediate 1.5-B | 2-Methoxy-4-trifluoromethylbenzyl alcohol and 5-bromo-2-fluorophenol | 340 mg. |
| | | | | ESI-MS (m/z): 379.1 [M+H]⁺ |
| **Intermediate 1.5-J** | 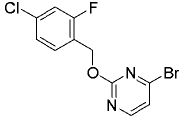 6-Bromo-2-((4-chloro-2-fluoro)benzyloxy)pyrimidine | Intermediate 1.5-B | 2-Chloro-4-fluorobenzyl alcohol and 2-(methylsulfonyl)-4-bromopyrimidine | 830 mg. |
| | | | | ESI-MS (m/z): 317.0.1[M+H]⁺ |
| **Intermediate 1.5-K** | 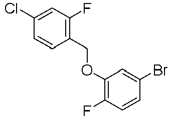 4-Bromo-1-fluoro-2-((4-chloro-2-fluorobenzyl)oxy)benzene | Intermediate 1.5-I | 2-Fluoro-4-chlorobenzyl alcohol | 1500 mg. |
| | | | | ESI-MS (m/z): 333.0[M+H]⁺ |
| **Intermediate 1.5-L** | 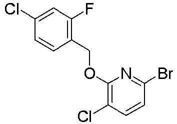 6-Bromo-3-chloro-2-((4-chloro-2-fluorobenzyl)oxy)pyridine | Intermediate 1.5-F | 2,3-Dichloro-6-bromopyridine | 1390 mg. |
| | | | | ESI-MS (m/z): 350.1[M+H]⁺ |
| **Intermediate 1.5-M** | 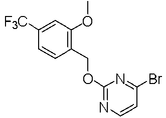 6-Bromo-2-((4-trifluoromethyl-2-methoxybenzyl)oxy) pyrimidine | Intermediate 1.5-J | 2-Methoxy-4-trifluoromethylbenzyl alcohol and 2-(methylsulfonyl)-4-bromopyrimidine | 1010 mg. |
| | | | | ESI-MS (m/z): 363.1[M+H]⁺ |
| **Intermediate 1.5-N** | 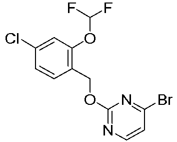 6-Bromo-2-((4-chloro-2-difluoromethoxybenzyl)oxy) pyrimidine | Intermediate 1.5-J | 2-Difluoromethoxy-4-chlorobenzyl alcohol and 2-(methylsulfonyl)-4-bromopyrimidine | 648 mg. |
| | | | | ESI-MS (m/z): 365.1 [M+H]⁺ |
| **Intermediate 1.5-0** | 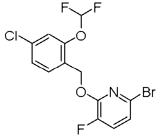 6-Bromo-3-fluoro-2-((4-chloro-2-difluoromethoxybenzyl)oxy) pyridine | Intermediate 1.5-F | 4-Chloro-2-difluoromethoxybenzyl alcohol | 790 mg. |
| | | | | ESI-MS (m/z): 382.1[M+H]⁺ |
| **Intermediate 1.5-P** | 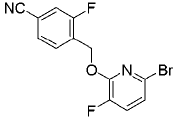 6-Bromo-3-fluoro-2-((4-cyano-2-fluorobenzyl)oxy)pyridine | Intermediate 1.5-F | 2-Fluoro-4-cyanobenzyl alcohol | 640 mg. |
| | | | | ESI-MS (m/z): 325.1 [M+H]⁺ |
| **Intermediate 1.5-Q** | 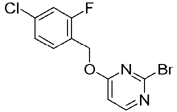 2-Bromo-4-((4-chloro-2-fluorobenzyl)oxy)pyrimidine | Intermediate 1.5-B | 2-Chloro-4-bromopyrimidine | 730 mg. |
| | | | | ESI-MS (m/z): 317.1[M+H]⁺ |
| **Intermediate 1.5-R** | 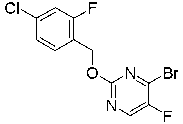 4-Bromo-5-fluoro-2-((4-chloro-2-fluorobenzyl)oxy)pyrimidine | Intermediate 1.5-B | 2-Chloro-4-bromo-5-fluoropyrimidine | 240 mg. |
| | | | | ESI-MS (m/z): 335.1[M+H]⁺ |
| **Intermediate 1.5-S** | 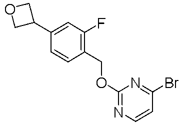 6-Bromo-2-((4-oxetan-3-yl-2-fluorobenzyl)oxy)pyrimidine | Intermediate 1.5-J | 2-Fluoro-4-oxetan-3-ylbenzyl alcohol | 734 mg. |
| | | | | ESI-MS (m/z): 339.1[M+H]⁺ |
| **Intermediate 1.5-T** | 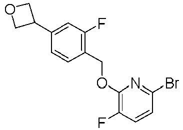 6-Bromo-3-fluoro-2-((4-oxetan-3-yl-2-fluorobenzyl)oxy) pyridine | Intermediate 1.5-F | 2-Fluoro-4-oxetan-3-ylbenzyl alcohol | 640 mg. |
| | | | | ESI-MS (m/z): 356.1 [M+H]⁺ |
| **Intermediate 1.5-U** | 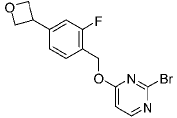 2-Bromo-4-((4-oxetan-3-yl-2-fluorobenzyl)oxy)pyrimidine | Intermediate 1.5-Q | 2-Fluoro-4-oxetan-3-ylbenzyl alcohol | 580 mg. |
| | | | | ESI-MS (m/z): 339.0[M+H]⁺ |
| **Intermediate 1.5-V** | 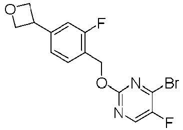 4-Bromo-5-fluoro-2-((4-oxetan-3-yl-2-fluorobenzyl)oxy) pyrimidine | Intermediate 1.5-R | 2-Fluoro-4-oxetan-3-ylbenzyl alcohol | 310 mg. |
| | | | | ESI-MS (m/z): 357.1[M+H]⁺ |
| **Intermediate 1.5-W** | 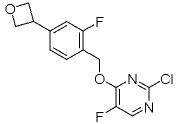 2-Chloro-5-fluoro-4-((4-oxetan-3-yl-2-fluorobenzyl)oxy) pyrimidine | Intermediate 1.5-F | 2-Fluoro-4-oxetan-3-ylbenzyl alcohol and 2,4-dichloro-5-fluoropyrimidine | 185 mg. |
| | | | | ESI-MS (m/z): 313.1[M+H]⁺ |
| **Intermediate 1.5-X** | 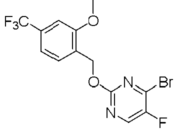 4-Bromo-5-fluoro-2-((4-trifluoromethyl-2-methoxybenzyl)oxy)pyrimidine | Intermediate 1.5-B | 4-Trifluoromethyl-2-methoxybenzyl alcohol | 320 mg. |
| | | | | ESI-MS (m/z): 381.1[M+H]⁺ |
| **Intermediate 1.5-Y** | 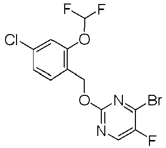 4-Bromo-5-fluoro-2-((4-chloro-2-difluoromethoxybenzyl)oxy) pyrimidine | Intermediate 1.5-B | 4-Chloro-2-difluoromethoxybenzyl alcohol | 322 mg. |
| | | | | ESI-MS (m/z): 383.1[M+H]⁺ |
| **Intermediate 1.5-Z** | 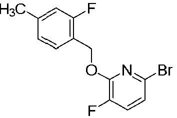 6-Bromo-3-fluoro-2-((4-methyl-2-fluorobenzyl)oxy)pyridine | Intermediate 1.5-F | 2-Fluoro-4-methylbenzyl alcohol | 990 mg. |
| | | | | ESI-MS (m/z): 314.1 [M+H]⁺ |
| **Intermediate 1.5-AA** | 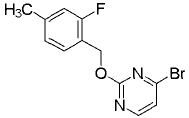 6-Bromo-2-((4-methyl-2-fluorobenzyl)oxy)pyrimidine | Intermediate 1.5-J | 2-Fluoro-4-methylbenzyl alcohol | 838 mg. |
| | | | | ESI-MS (m/z): 297.1[M+H]⁺ |
| **Intermediate 1.5-AB** | 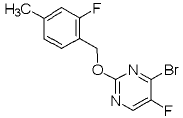 4-Bromo-5-fluoro-2-((4-methyl-2-fluorobenzyl)oxy)pyrimidine | Intermediate 1.5-B | 4-Methyl-2-fluorobenzyl alcohol | 320 mg. |
| | | | | ESI-MS (m/z): 315.1[M+H]⁺ |
| **Intermediate 1.5-AC** | 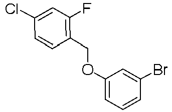 2-Bromo-6-(((4-chloro-2-fluorobenzyl)oxy)benzene | Intermediate 1.5-B | 3-Bromophenol | 800 mg. |
| | | | | ESI-MS (m/z): 315.1 [M+H]⁺ |
| **Intermediate 1.5-AD** | 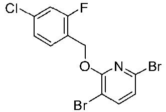 3,6-Dibromo-2-((4-chloro-2-fluorobenzyl)oxy)pyridine | Intermediate 1.5-B | 3,6-Dibromo-2-fluoropyridine | 1250 mg. |
| | | | | ESI-MS (m/z): 394.0[M+H]⁺ |
| **Intermediate 1.5-AE** | 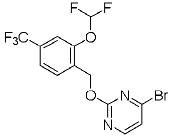 6-Bromo-2-((4-trifluoromethyl-2-difluoromethoxybenzyl)oxy) pyrimidine | Intermediate 1.5-J | 2-Difluoromethoxy-4-trifluoromethylbenzyl alcohol | 648 mg. |
| | | | | ESI-MS (m/z): 399.1 [M+H]⁺ |
| **Intermediate 1.5-AH** | 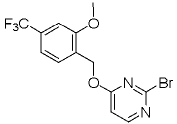 2-Bromo-4-((4-trifluoromethyl-2-methoxybenzyl)oxy) pyrimidine | Intermediate 1.5-Q | 4-Trifluoromethyl-2-methoxybenzyl alcohol | 510 mg. |
| | | | | ESI-MS (m/z): 363.0[M+H]⁺ |

### Synthesis of 3-fluoro-4-(((6-(tri-n-butyltin)pyridin-2-yl)oxy)methyl)benzonitrile (Intermediate 1.7-A)

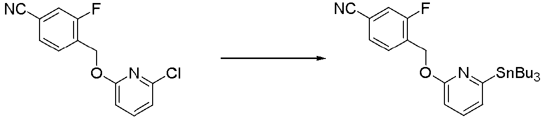

4-(((6-Chloropyridin-2-yl)oxy)methyl)-3-fluorobenzonitrile (1 g, 3.81 mmol) was added to 1,4-dioxane (10 mL), and then hexa-n-butylditin (2.65 g, 4.57 mmol), Pd(PPh₃)₄ (220 mg, 0.19 mmol), tricyclohexylphosphine (128 mg, 0.46 mmol) and LiCl (968 mg, 22.84 mmol) were added. The resulting mixture was heated to 120 ° C and reacted under stirring for 12 h. After the reaction was completed, the reaction mixture was quenched with water, then extracted with ethyl acetate, washed with saturated brine, dried over anhydrous sodium sulfate, and concentrated under reduced pressure to obtain a crude product, which was purified by column chromatography (ethyl acetate: petroleum ether =1:5) to obtain 3-fluoro-4-(((6-(tri-n-butyltin) pyridin-2-yl)oxy)methyl)benzonitrile (700 mg), ESI-MS (m/z): 519.2[M+H]⁺.

### Synthesis of tri-n-butyl (2-(4-chloro-2-fluorophenyl)-2-methylbenzo[d][1,3]dioxolan-4-yl) tin (Intermediate 1.7-B)

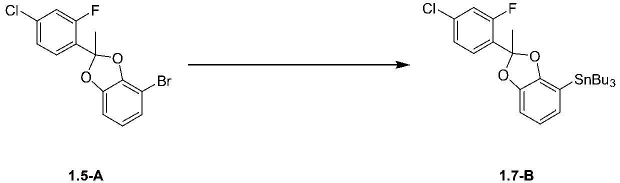

Tri-n-butyl (2-(4-chloro-2-fluorophenyl)-2-methylbenzo[d][1,3]dioxolan-4-yl) tin (320 mg) was obtain by referring to the synthetic method of Compound 1.7-A using 4-bromo-2-(4-chloro-2-fluorophenyl)-2-methylbenzo[d][1,3]dioxolane (1.5-A) as the staring material. ESI-MS (m/z): 555.1 [M+H]⁺.

### Methyl (S)-2-(chloromethyl)-4-methoxy-1-((oxetan-2-yl)methyl)-1H-benzo[d]imidazole-6-carboxylate (Intermediate 1.9-A)

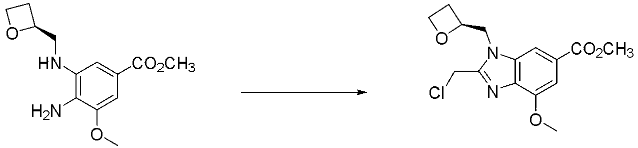

Intermediate 1.2-F (850 mg, 3.19 mmol) and 2-chloro-1,1,1-trimethoxyethane (592 mg, 3.83 mmol) were dissolved in MeCN (17 mL). The resulting mixture was stirred at room temperature for 20 minutes, and then p-toluenesulfonic acid monohydrate (61 mg, 319.20 µmol) was added. The reaction mixture reacted under stirring at 50 °C for 2 hours. After the reaction was completed, the reaction mixture was concentrated and purified by column chromatography (ethyl acetate: petroleum ether= 1:2) to obtain methyl (S)-2-(chloromethyl)-4-methoxy-1-(oxetan-2-ylmethyl)-1H-benzo[d]imidazole-6-carboxylate (601 mg), ESI-MS (m/z): 325.0 [M+H]⁺, ¹H NMR (400 MHz, CDCl₃) δ 7.76 (s, 1H), 7.42 (s, 1H), 5.22 (td, J = 4.9, 7.2 Hz, 1H), 5.10 - 4.99 (m, 2H), 4.65 - 4.50 (m, 3H), 4.31 (td, J = 6.0, 9.2 Hz, 1H), 4.09 (s, 3H), 3.96 (s, 3H), 2.80 - 2.69 (m, 1H), 2.45 - 2.36 (m, 1H).

### Synthesis of isopropyl (S)-2-(chloromethyl)-4-isopropoxy-1-((oxetan-2-yl)methyl)-1H-benzo[d]imidazole-6-carboxylate (Intermediate 1.9-B)

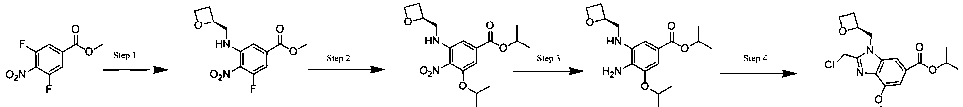

### Step 1: methyl (S)-3-fluoro-4-nitro-5-(oxetan-2-ylmethyl)amino)benzoate

Methyl 3,5-difluoro-4-nitrobenzoate (1.00 g, 4.61 mmol), (S)-oxetan-2-ylmethanamine (0.48 g, 5.53 mmol), potassium carbonate (1.27 g, 9.22 mmol) and acetonitrile (15 mL) were added to a reaction flask and reacted at 60 °C for 2 hours. After the reaction was completed, the reaction mixture was cooled, concentrated and purified by column chromatography (ethyl acetate: petroleum ether =1:2) to obtain methyl (S)-3-fluoro-4-nitro-5-(oxetan-2-ylmethyl)amino)benzoate (770 mg), ESI-MS (m/z): 285.18[M+H]⁺.

### Step 2: isopropyl (S)-3-isopropoxy-4-nitro-5-((oxetan-2-ylmethyl)amino)benzoate

The product obtained from the previous step (700 mg, 2.46 mmol), cesium carbonate (1.6 g, 4.92 mmol) and isopropanol (15 mL) were added into a reaction flask and reacted at room temperature for 20 hours. After the reaction was completed, the solvent was removed by vacuum evaporation, followed by purification by column chromatography (ethyl acetate: petroleum ether =1:2) to obtain isopropyl (S)-3-isopropoxy-4-nitro-5-((oxetan-2-ylmethyl)amino)benzoate (400 mg), ESI-MS (m/z): 353.27[M+H]⁺.

### Step 3: isopropyl (S)-4-amino-3-isopropoxy-5-((oxetan-2-ylmethyl)amino)benzoate

The product obtained from the previous step (400 mg, 1.14 mmol), iron powder (319 mg, 5.70 mmol), ammonium chloride (302 mg, 5.70 mmol), ethanol (15 mL) and water (3 mL) were added to a reaction flask and reacted at 80 °C for 1 hour. After the reaction was completed, the reaction mixture was filtered, concentrated under reduced pressure, and purified by column chromatography (ethyl acetate: petroleum ether =1:2) to obtain isopropyl (S)-4-amino-3-isopropoxy-5-((oxetan-2-ylmethyl)amino)benzoate (320 mg), ESI-MS (m/z): 323.31[M+H]⁺.

### Step 4: isopropyl (S)-2-(chloromethyl)-4-isopropoxy-1-((oxetan-2-yl)methyl)-1H-benzo[d]imidazole-6-carboxylate

The product obtained from the previous step (320 mg, 0.99 mmol), 2-chloro-1,1,1-trimethoxyethane (184 mg, 1.19 mmol), p-toluenesulfonic acid (19 mg, 0.10 mmol) and acetonitrile (15 mL) were added to a reaction flask and reacted at 50 °C for 2 h. After the reaction was completed, the solvent was removed by vacuum evaporation, followed by purification by column chromatography (ethyl acetate: petroleum ether =1:2) to obtain isopropyl (S)-2-(chloromethyl)-4-isopropoxy-1-((oxetan-2-yl)methyl)-1H-benzo[d]imidazole-6-carboxylate (260 mg), ESI-MS (m/z): 381.21[M+H]⁺.

### Methyl (S)-2-(chloromethyl)-4-difluoromethoxy-1-((oxetan-2-yl)methyl)-1H-benzo[d]imidazole-6-carboxylate (Intermediate 1.9-C)

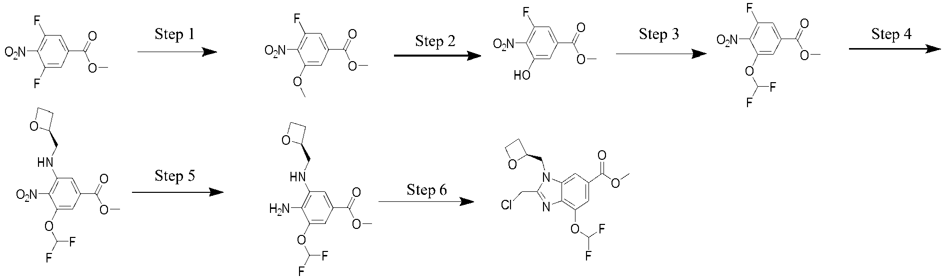

### Step 1: Methyl 3-fluoro-5-methoxy-4-nitrobenzoate

Methyl 3,5-difluoro-4-nitrobenzoate (4.5 g, 20.73 mmol) was dissolved in methanol (90 mL), a methanol solution containing sodium methoxide (3.92 g, 21.76 mmol) was added dropwise to the reaction mixture, and the reaction mixture reacted at 65 °C for 2.5 hours. After the reaction was completed, water was added, extraction was performed with ethyl acetate, and the resulting organic phases were combined, dried over anhydrous sodium sulfate, filtered, concentrated, and purified by column chromatography (ethyl acetate: petroleum ether =1:3) to obtain methyl 3-fluoro-5-methoxy-4-nitrobenzoate (4.5 g), ESI-MS (m/z): 230.0[M+H]⁺.

### Step 2: Methyl 3-fluoro-5-hydroxy-4-nitrobenzoate

The product obtained from the previous step (4.9 g, 21.30 mmol) was dissolved in dichloromethane (50 mL) and boron tribromide (5.33 g, 21.30 mmol) was added dropwise to the reaction mixture in an ice-water bath. The resulting mixture reacted under stirring at room temperature overnight. After the reaction was completed, water was added, extraction was performed with dichloromethane, and the resulting organic phases were combined, dried over anhydrous sodium sulfate, filtered, concentrated, and isolated by column chromatography (ethyl acetate: petroleum ether =1:3) to obtain methyl 3-fluoro-5-hydroxy-4-nitrobenzoate (2.5 g), ESI-MS (m/z): 216.0[M+H]⁺.

### Step 3: Methyl 3-difluoromethoxy-5-fluoro-4-nitrobenzoate

The product obtained from the previous step (2.0 g, 9.3 mmol) and sodium difluorochloroacetate were dissolved in DMF (70 mL) and water (70 mL), then cesium carbonate (6.06 g, 18.59 mmol) was added, and the resulting mixture was stirred at 100 °C for 4 hours. After completion of reaction, the reaction mixture was filtered and lyophilized to obtain methyl 3-difluoromethoxy-5-fluoro-4-nitrobenzoate (2.0 g), ESI-MS (m/z): 252.0[M+H]⁺.

### Step 4: methyl (S)-3-difluoromethoxy-4-nitro-5-(oxetan-2-ylmethyl)amino)benzoate

The product obtained from the previous step (1.5 g, 5.66 mmol) was dissolved in DMF (20 mL), potassium carbonate (2.35 g, 16.97 mmol) and (S)-oxetan-2-ylmethanamine (0.74 g, 8.49 mmol) were added, and the resulting mixture reacted under stirring at room temperature overnight. After the reaction was completed, water was added, extraction was performed with ethyl acetate, and the resulting organic phase was dried over anhydrous sodium sulfate, filtered, concentrated, and isolated by column chromatography (ethyl acetate: petroleum ether =1:3) to obtain methyl (S)-3-difluoromethoxy-4-nitro-5-(oxetan-2-ylmethyl)amino)benzoate (1.6 g), ESI-MS (m/z): 333.1[M+H]⁺.

### Step 5: methyl (S)-4-amino-3-difluoromethoxy-5-(oxetan-2-ylmethyl)amino)benzoate

The product obtained from the previous step (1.6 g, 4.82 mmol) was dissolved in ethanol (16 mL) and water (4 mL), then ammonium chloride (1.29 g, 24.08 mmol) and iron powder (0.87 g, 14.45 mmol) were added, and the resulting mixture reacted under stirring at 100 °C for 2 hours. After the reaction was completed, water was added, extraction was performed with ethyl acetate, and the resulting organic phase was dried over anhydrous sodium sulfate, filtered, concentrated, and isolated by column chromatography (ethyl acetate: petroleum ether =1:3) to obtain methyl (S)-4-amino-3-difluoromethoxy-5-(oxetan-2-ylmethyl)amino)benzoate (1.4 g), ESI-MS (m/z): 303.1[M+H]⁺.

### Step 6: methyl (S)-2-(chloromethyl)-4-difluoromethoxy-1-((oxetan-2-yl)methyl)-1H-benzo[d]imidazole-6-carboxylate

The product obtained from the previous step (500 mg, 1.65 mmol) was dissolved in acetonitrile (20 mL), 2-chloro-1,1,1-trimethoxyethane (640 mg, 4.13 mmol) and a catalytic amount of p-toluenesulfonic acid were added, and the resulting mixture reacted under stirring at 60°C for 4 hours. After the reaction was completed, water was added, extraction was performed with ethyl acetate, and the resulting organic phase was dried over anhydrous sodium sulfate, filtered, concentrated, and isolated by thin layer chromatography (ethyl acetate: petroleum ether =1:3) to obtain methyl (S)-2-(chloromethyl)-4-difluoromethoxy-1-((oxetan-2-yl)methyl)-1H-benzo[d]imidazole-6-carboxylate (460 mg), ESI-MS(m/z): 361.1[M+H]⁺, ¹H NMR (400 MHz, CDCl₃) δ 7.99 (d, *J* = 1.4 Hz, 1H), 7.75 (s, 1H), 7.54 - 7.32 (m, 1H), 5.24-5.12 (m, 1H), 5.04 (s, 2H), 4.69 - 4.59 (m, 2H), 4.58 - 4.50 (m, 1H), 4.39-4.34 (m, 1H), 3.97 (s, 3H), 2.82-2.71m, 1H), 2.50 - 2.36 (m, 1H).

### Methyl (S)-2-(chloromethyl)-7-methoxy-3-((oxetan-2-yl)methyl)-3H-imidazo[4,5-b]pyridine-5-carboxylate (Intermediate 1.9-D)

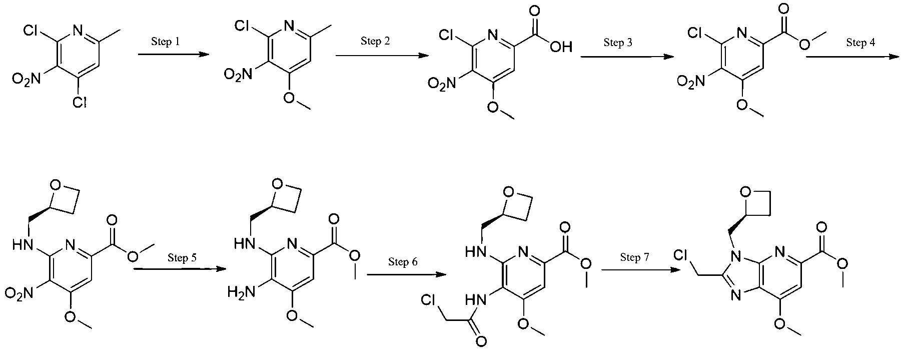

### Step 1: 2-chloro-4-methoxy-6-methyl-3-nitropyridine

2,4-Dichloro-6-methyl-3-nitropyridine (3 g, 14.5 mmol) and anhydrous methanol (25 mL) were added into a 100 mL round bottom flask, then sodium methoxide (1.17g, 21.7 mmol) was added, and the resulting mixture reacted under stirring at 60 °C for 1.5 hours. After the reaction was completed, concentration was performed to obtain 2-chloro-4-methoxy-6-methyl-3-nitropyridine (2.7 g), ESI-MS (m/z): 203.1[M+H]⁺.

### Step 2: 6-chloro-4-methoxy-5-nitropicolinic acid

The product obtained from the previous step (2.7g, 13.4 mmol) and concentrated sulfuric acid (25 mL) were added into a 100 mL round bottom flask, chromium oxide (2.67g, 26.7 mmol) was added, and the resulting mixture reacted under stirring at 60 °C for 3 hours. After the reaction was completed, the reaction mixture was poured into ice water, extracted with ethyl acetate, and concentrated to obtain 6-chloro-4-methoxy-5-nitropicolinic acid (ESI-MS (m/z): 233.3[M+H]⁺), which was directly subjected to the next reaction.

### Step 3: methyl 6-chloro-4-methoxy-5-nitropicolinate

The product obtained from the previous step (2.6 g, 11.2 mmol) and anhydrous methanol (25 mL) were added into a 100 mL round bottom flask, thionyl chloride (2 g, 16.8 mmol) was added, and the reaction mixture was heated to 60 °C and reacted under stirring for 2 hours. After the reaction was completed, the solvent was removed under reduced pressure, the residue was extracted with ethyl acetate, and a saturated aqueous sodium bicarbonate solution was added. The resulting organic phase was concentrated, and the resulting crude product was isolated and purified by column chromatography (ethyl acetate: petroleum ether =1:20) to obtain methyl 6-chloro-4-methoxy-5-nitropicolinate (2.5g), ESI-MS (m/z): 247.3[M+H]⁺.

### Step 4: methyl (S)-4-methoxy-5-nitro-6-((oxetan-2-ylmethyl)amino)picolinate

The product obtained from the previous step (2.5g, 10.1 mmol), (S)-oxetane-2-ylmethanamine (1.32g, 15.2 mmol), 1,1'-binaphthyl-2,2'-bis(diphenylphosphine) (628 mg, 1.01 mmol), tris(dibenzylideneacetone)dipalladium (916 mg, 1.01 mmol), and dioxane (30 mL) were added into a 100 mL round bottom flask, and the reaction mixture reacted under stirring at 90 °C for 5 hours under the protection of nitrogen. After the reaction was completed, the solvent was removed under reduced pressure, and the resulting crude product was isolated and purified by column chromatography (ethyl acetate: petroleum ether =1:5) to obtain methyl (S)-4-methoxy-5-nitro-6-((oxetan-2-ylmethyl)amino)picolinate (1.3g), ESI-MS (m/z): 298.3[M+H]⁺.

### Step 5: methyl (S)-5-amino-4-methoxy-6-((oxetan-2-ylmethyl)amino)picolinate

The product obtained from the previous step (1.3g, 4.3 mmol), iron powder (613 mg, 10.9 mmol), aqueous ammonium chloride solution (924 mg, 21.5 mmol), and ethanol (30mL) were added into a 100 mL round bottom flask and the resulting mixture reacted under stirring at 80 °C for 3 hours. After the reaction was completed, the solvent was removed under reduced pressure and the resulting crude product was isolated and purified by column chromatography (methanol: dichloromethane =1:20) to obtain methyl (S)-5-amino-4-methoxy-6-((oxetan-2-ylmethyl)amino)picolinate (800 mg), ESI-MS (m/z): 268.3[M+H]⁺.

### Step 6: methyl (S)-5-(2-chloroacetylamino)-4-methoxy-6-((oxetan-2-ylmethyl)amino)picolinate

The product obtained from the previous step (800 mg, 3.0 mmol), triethylamine (605 mg, 6.0 mmol) and dichloromethane (20 mL) were added into a 50 mL round bottom flask, chloroacetyl chloride (504 mg, 4.5 mmol) was added dropwise at 0 °C, and the resulting mixture reacted under stirring at room temperature for 2 hours. After the reaction was completed, the solvent was removed under reduced pressure and the resulting crude product was isolated and purified by column chromatography (methanol: dichloromethane =1:30) to obtain methyl (S)-5-(2-chloroacetylamino)-4-methoxy-6-((oxetan-2-ylmethyl)amino)picolinate (800 mg), ESI-MS (m/z): 344.3[M+H]⁺.

### Step 7: methyl (S)-2-(chloromethyl)-7-methoxy-3-((oxetan-2-yl)methyl)-3H-imidazo[4,5-b]pyridine-5-carboxylate

The product obtained from the previous step (800 mg, 2.33 mmol), acetic acid (0.5 mL), and tetrahydrofuran (20 mL) were added into a 50 mL round bottom flask and the resulting mixture reacted under stirring at 80 °C for 2 hours. After the reaction was completed, the solvent was removed under reduced pressure and the resulting crude product was isolated and purified by column chromatography (methanol: dichloromethane =1:30) to obtain methyl (S)-2-(chloromethyl)-7-methoxy-3-((oxetan-2-yl)methyl)-3H-imidazo[4,5-b]pyridine-5-carboxylate (600 mg), ESI-MS (m/z): 326.3[M+H]⁺.

### Intermediates 1.9-I to 1.9-O were synthesized by referring to the synthetic methods of the intermediates described above:

| **Intermediate numberings** | **Structures and chemical names** | **Reference intermediates** | **Starting materials used** | **Quality and characterization parameters of intermediates obtained** |
|---|---|---|---|---|
| **Intermediate 1.9-I** | 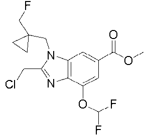 Methyl 2-(chloromethyl)-4-(difluoromethoxy)-1-((1-(fluoromethyl)cyclopropyl) methyl)-1H-benzo[d]imidazole-6-carboxylate | Intermediate 1.9-C | Methyl 3-fluoro-5-methoxy-4-nitrobenzoate and (1-(fluoromethyl) cyclopropyl) methanamine | 410 mg. |
| | | | | ESI-MS (m/z): 377.1[M+H]⁺ |
| **Intermediate 1.9-L** | 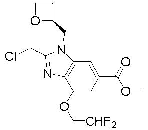 Methyl (S)-2-(chloromethyl)-4-(2,2-difluoroethoxy)-1-((oxetan-2-yl)methyl)-1H-benzo[d]imidazole-6-carboxylate | Intermediate 1.9-A | Methyl 3,5-difluoro-4-nitro-benzoate, (S)-oxetan-2-ylmethanamine and 2,2-difluoroethanol | 305 mg. |
| | | | | ESI-MS (m/z): 375.1[M+H]⁺ |
| **Intermediate 1.9-N** | 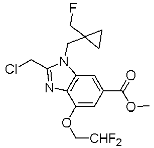 Methyl (S)-2-(chloromethyl)-4-(2,2-difluoroethoxy)-1-((1-(fluoromethyl)cyclopropyl)methyl)-1H-benzo[d]imidazole-6-carboxylate | Intermediate 1.9-L | Methyl 3,5-difluoro-4-nitro-benzoate, (1-(fluoromethyl)cyclopropyl) methanamine and 2,2-difluoroethanol | 305 mg. |
| | | | | ESI-MS (m/z): 391.1[M+H]⁺ |
| **Intermediate 1.9-0** | 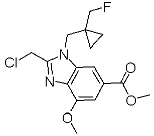 Methyl 2-(chloromethyl)-1-((1-(fluoromethyl)cyclopropyl) methyl)-4-methoxy-1H-benzo[d]imidazole-6-carboxylate | Intermediate 1.9-J | (1-(fluoromethyl) cyclopropyl) methanamine | 330 mg. |
| | | | | ESI-MS (m/z): 341.1[M+H]⁺ |

### Example 1: Synthesis of (S)-2-((7-(6-((4-cyano-2-fluorobenzyl)oxy)pyridin-2-yl)benzo[d][1,3]dioxolan-4-yl)methyl)-1-(oxetan-2-ylmethyl)-1H-benzo[d]imidazole-6-carboxylic acid (Compound 1)

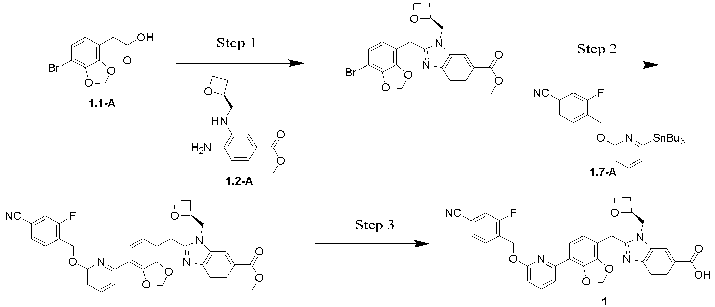

### Step 1: Synthesis of methyl (S)-2-((7-bromobenzo[d][1,3]dioxolan-4-yl)methyl)-1-(oxetan-2-ylmethyl)-1H-benzo[d]imidazole-6-carboxylate

Intermediate 1.1-A (500 mg, 1.93 mmol) and Intermediate 1.2-A (547 mg, 2.31 mmol) were added to acetonitrile (10 mL), then TCFH (649 mg, 2.31 mmol) and NMI (554 mg, 6.75 mmol, 485 µL) were added to the reaction mixture, and the reaction mixture reacted under stirring at 25 °C for 16 hours. After the reaction was completed, the reaction mixture was quenched with water, then extracted with ethyl acetate, washed with saturated brine, dried over anhydrous sodium sulfate, and concentrated under reduced pressure to obtain a crude product, which was purified by column chromatography (ethyl acetate: petroleum ether =1:5). The purified intermediate was then added to glacial acetic acid (10 mL), heated to 50 °C, and reacted under stirring for 3 hours. After the reaction was completed, the reaction mixture was added with water, then extracted with ethyl acetate, washed with NaHCO₃ solution and saturated brine, dried over anhydrous sodium sulfate, and concentrated under reduced pressure to obtain a crude product of methyl (S)-2-((7-bromobenzo[d][1,3]dioxolan-4-yl)methyl)-1-(oxetan-2-ylmethyl)-1H-benzo[d]imidazole-6-carboxylate (430 mg), ESI-MS (m/z): 459.1[M+H]⁺.

### Step 2: Synthesis of methyl (S)-2-((7-(6-((4-cyano-2-fluorobenzyl)oxy)pyridin-2-yl)benzo[d][1,3]dioxolan-4-yl) methyl)-1-(oxetan-2-ylmethyl)-1H-benzo[d]imidazole-6-carboxylate

The product obtained from the previous step (119 mg, 259.05 µmol) and Intermediate 1.7-A (200 mg, 386.65 µmol) were added to 1,4-dioxane (4 mL), then LiCl (87 mg, 2.06 mmol, 42 µL) and Pd(PPh₃)₄ (4.5 mg, 3.87 µmol) were added to the resulting mixture, and the reaction mixture reacted under stirring at 100 °C for 16 h. The reaction mixture was quenched with water, then extracted with ethyl acetate, washed with saturated brine, dried over anhydrous sodium sulfate and concentrated under reduced pressure to obtain a crude product, which was purified by column chromatography (methanol: dichloromethane =1:15) to obtain methyl (S)-2-((7-(6-((4-cyano-2-fluorobenzyl)oxy)pyridin-2-yl)benzo[d][1,3]dioxolan-4-yl)methyl)-1-(oxetan-2-ylmethyl)-1H-benzo[d]imidazole-6-carboxylate (90 mg), ESI-MS (m/z): 607.2 [M+H]⁺.

### Step 3: Synthesis of (S)-2-((7-(6-((4-cyano-2-fluorobenzyl)oxy)pyridin-2-yl)benzo[d][1,3]dioxolan-4-yl)methyl)-1-(oxetan-2-ylmethyl)-1H-benzo[d]imidazole-6-carboxylic acid

The product obtained from the previous step (130 mg, 214.31 µmol) was added to THF (0.2 mL) and isopropanol (0.2 mL), and then LiOH (20.5 mg, 854.60 µmol) was dissolved in H₂O (0.2 mL) and added to the reaction mixture at 0 °C. The resulting mixture was slowly warmed to room temperature and reacted under stirring for 4 hours. After the reaction was completed, the reaction mixture was added with water and then extracted with trichloromethane. The resulting aqueous phase was adjusted to pH=5 with 1 M acetic acid, then extracted with ethyl acetate, dried over anhydrous sodium sulfate and concentrated under reduced pressure to obtain a crude product, which was purified by column chromatography (methanol: dichloromethane =1:15) to obtain (S)-2-((7-(6-((4-cyano-2-fluorobenzyl)oxy)pyridin-2-yl)benzo[d][1,3]dioxolan-4-yl)methyl)-1-(oxetan-2-ylmethyl)-1H-benzo[d]imidazole-6-carboxylic acid (8.2 mg), ESI-MS (m/z): 593.1 [M+H]⁺, ¹H NMR (400 MHz, CDCl₃) δ 8.25 - 8.18 (m, 1H), 8.12 - 8.04 (m, 1H), 7.93 - 7.84 (m, 1H), 7.71 - 7.62 (m, 4H), 7.44 (dd, J = 0.9, 7.8 Hz, 1H), 7.38 (dd, J = 1.2, 9.3 Hz, 1H), 7.02 - 6.91 (m, 1H), 6.77 (dd, J = 2.0, 6.9 Hz, 1H), 6.11 (s, 2H), 5.59 (s, 2H), 5.11 (dq, J = 3.0, 6.9 Hz, 1H), 4.67 - 4.61 (m, 1H), 4.60 - 4.46 (m, 3H), 4.46 - 4.39 (m, 2H), 2.78 - 2.66 (m, 1H), 2.43 - 2.34 (m, 1H).

### Example 2: Synthesis of (S)-2-((7-(6-((4-cyano-2-fluorobenzyl)oxy)pyridin-2-yl)-2,3-dihydrobenzofuran-4-yl)methyl)-1-(oxetan-2-ylmethyl)-1H-benzo[d]imidazole-6-carboxylic acid (Compound 2)

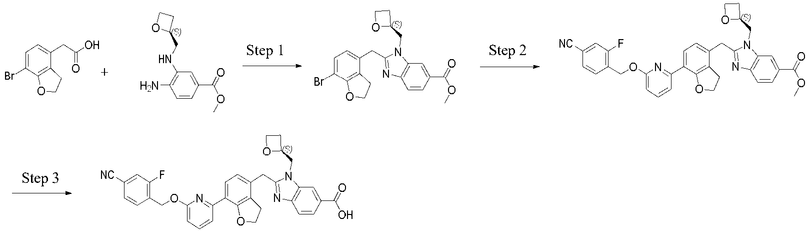

### Step 1: Synthesis of methyl (S)-2-((7-bromo-2,3-dihydrobenzofuran-4-yl)methyl)-1-(oxetan-2-ylmethyl)-1H-benzo[d]imidazole-6-carboxylate

Intermediate 1.1-B (760 mg, 2.96 mmol) was added to acetonitrile (15.2 mL), and then Intermediate 1.2-A (838.16 mg, 3.55 mmol), TCFH (995.37 mg, 3.55 mmol) and NMI (849.49 mg, 10.35 mmol) were added. The reaction mixture reacted under stirring at 20 °C for 2.5 hours. After the reaction was completed, the reaction mixture was quenched with 20 mL of water and filtered. The filter cake was dissolved in acetic acid (17 mL) and the resulting mixture reacted under stirring at 50 °C for 3 hours. After the reaction was completed, the reaction mixture was added with water, extracted with ethyl acetate, washed with saturated brine, dried over anhydrous sodium sulfate and concentrated under reduced pressure to obtain a crude product of methyl (S)-2-((7-bromo-2,3-dihydrobenzofuran-4-yl) methyl)-1-(oxetan-2-ylmethyl)-1H-benzo[d]imidazole-6-carboxylate (760 mg), ESI-MS (m/z): 457.1[M+H]⁺.

### Step 2: Synthesis of methyl (S)-2-((7-(6-((4-cyano-2-fluorobenzyl)oxy)pyridin-2-yl)-2,3-dihydrobenzofuran-4-yl)methyl)-1-(oxetan-2-ylmethyl)-1H-benzo[d]imidazole-6-carboxylate

The product obtained from the previous step (118.5 mg, 0.26 mmol) was added to 1,4-dioxane (4 mL), then Intermediate 1.7-A (200 mg, 0.39 mmol), LiCl (87.4 mg, 2.06 mmol) and Pd (PPh₃)₄ (4.5 mg, 0.00387mmol) were added, and the resulting mixture was heated to 100 °C and reacted under stirring for 16 hours. After the reaction was completed, the reaction mixture was quenched with water, then extracted with ethyl acetate, washed with saturated brine, dried over anhydrous sodium sulfate and concentrated under reduced pressure to obtain a crude product, which was purified by column chromatography (ethyl acetate: petroleum ether =1:10) to obtain methyl (S)-2-((7-(6-((4-cyano-2-fluorobenzyl)oxy)pyridin-2-yl)-2,3-dihydrobenzofuran-4-yl)methyl)-1-(oxetan-2-ylmethyl)-1H-benzo[d]imidazole-6-carboxylate (54 mg), ESI-MS (m/z): 605.1 [M+H]⁺.

### Step 3: Synthesis of (S)-2-((7-(6-((4-cyano-2-fluorobenzyl)oxy)pyridin-2-yl)-2,3-dihydrobenzofuran-4-yl)methyl)-1-(oxetan-2-ylmethyl)-1H-benzo[d]imidazole-6-carboxylic acid (Compound 2)

The product obtained from the previous step (54 mg, 0.09 mmol) was dissolved in isopropanol (0.5 mL), THF (0.5 mL) and H₂O (0.5 mL). LiOH (8.6 mg, 0.36 mmol) was added, and the reaction mixture reacted under stirring at 20 °C for 12 hours. After the reaction was completed, the reaction mixture was added with water and extracted with methyl tert-butyl ether. The resulting aqueous phase was adjusted to pH=5 with acetic acid, extracted with ethyl acetate, dried over anhydrous sodium sulfate and concentrated under reduced pressure to obtain a crude product, which was purified by column chromatography (ethyl acetate: petroleum ether =1:5) to obtain two components, one of which was (S)-2-((7-(6-((4-cyano-2-fluorobenzyl)oxy)pyridin-2-yl)-2,3-dihydrobenzofuran-4-yl)methyl)-1-(oxetan-2-ylmethyl)-1H-benzo[d]imidazole-6-carboxylic acid (6.4 mg), ESI-MS (m/z): 589.1 [M-H]⁻. ¹H NMR (400 MHz, DMSO-d₆) δ 8.22 (s, 1H), 7.92 - 7.88 (m, 1H), 7.86 (d, *J =* 8.1 Hz, 1H), 7.82 - 7.76 (m, 3H), 7.76 - 7.68 (m, 2H), 7.61 (d, *J =* 8.4 Hz, 1H), 6.85 - 6.77 (m, 2H), 5.57 (s, 2H), 4.98 (dq, *J =* 2.3, 7.1 Hz, 1H), 4.70 - 4.59 (m, 3H), 4.54 (d, *J* = 2.5 Hz, 1H), 4.50 - 4.45 (m, 1H), 4.41 - 4.31 (m, 3H), 3.16 (dt, *J* = 3.4, 8.6 Hz, 2H), 2.68 - 2.65 (m, 1H), 2.35 - 2.30 (m, 1H).

### Example 3: Synthesis of (S)-2-((7-(6-((4-aminocarbonyl-2-fluorobenzyl)oxy)pyridin-2-yl)-2,3-dihydrobenzofuran-4-yl)methyl)-1-(oxetan-2-ylmethyl)-1H-benzo[d]imidazole-6-carboxylic acid (Compound 3)

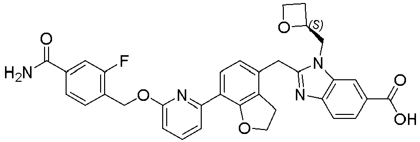

The synthetic method was the same as that for Compound 2, and the other component was finally collected to obtain (S)-2-((7-(6-((4-aminocarbonyl-2-fluorobenzyl)oxy)pyridin-2-yl)-2,3-dihydrobenzofuran-4-yl)methyl)-1-(oxetan-2-ylmethyl)-1H-benzo[d]imidazole-6-carboxylic acid (1 mg), ESI-MS (m/z): 607.1 [M-H]⁻, ¹H NMR (400 MHz, DMSO-*d₆*) δ 8.22 (s, 1H), 8.02 (br s, 1H), 7.92 (d, *J* = 8.1 Hz, 1H), 7.81 - 7.79 (m, 1H), 7.79 - 7.76 (m, 2H), 7.72 - 7.66 (m, 2H), 7.66 - 7.58 (m, 2H), 7.49 (br s, 1H), 6.83 - 6.78 (m, 2H), 5.54 (s, 2H), 5.02 - 4.94 (m, 1H), 4.70 - 4.62 (m, 3H), 4.54 (d, *J* = 2.5 Hz, 1H), 4.51 - 4.47 (m, 1H), 4.41 - 4.37 (m, 2H), 4.35 (dd, *J* = 3.0, 5.9 Hz, 1H), 3.16 (dt, *J =* 3.2, 8.7 Hz, 3H), 2.69 - 2.64 (m, 1H), 2.33 (br dd, *J =* 1.9, 3.6 Hz, 1H).

### Example 4: Synthesis of (S)-2-((4-(6-((4-cyano-2-fluorobenzyl)oxy)pyridin-2-yl)-2,3-dihydrobenzofuran-7-yl)methyl)-1-(oxetan-2-ylmethyl)-1H-benzo[d]imidazole-6-carboxylic acid (Compound 4)

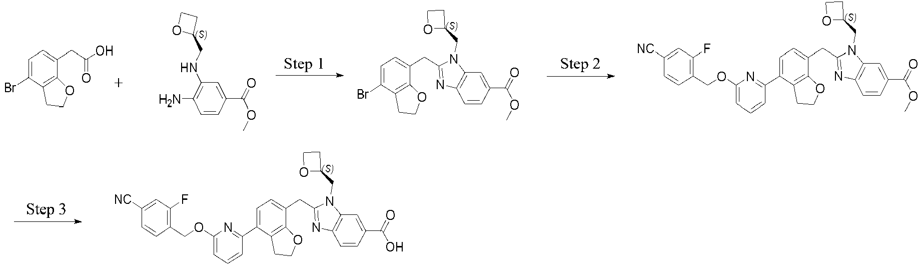

### Step 1: Synthesis of methyl (S)-2-((4-bromo-2,3-dihydrobenzofuran-7-yl)methyl)-1-(oxetan-2-ylmethyl)-1H-benzo[d]imidazole-6-carboxylate

Intermediate 1.1-C (760 mg, 2.96 mmol) was added to acetonitrile (15.2 mL), and then Intermediate 1.2-A (698.47 mg, 3.55 mmol), TCFH (995.37 mg, 3.55 mmol), and NMI (849.49 mg, 10.35 mmol) were added. The reaction mixture reacted under stirring at 20 °C for 2.5 hours. After the reaction was completed, the reaction mixture was quenched with 20 mL of water and filtered, the filter cake was dissolved in acetic acid (20 mL), and the resulting mixture reacted under stirring at 50 °C for 3 hours. After the reaction was completed, the reaction mixture was added with water, extracted with ethyl acetate, washed with saturated brine, dried over anhydrous sodium sulfate and concentrated under reduced pressure to obtain a crude product of methyl (S)-2-((4-bromo-2,3-dihydrobenzofuran-7-yl)methyl)-1-(oxetan-2-ylmethyl)-1H-benzo[d]imidazole-6-carboxylate (880 mg), ESI-MS (m/z): 457.1 [M+H]⁺.

### Step 2: Synthesis of methyl (S)-2-((4-(6-((4-cyano-2-fluorobenzyl)oxy)pyridin-2-yl)-2,3-dihydrobenzofuran-7-yl)methyl)-1-(oxetan-2-ylmethyl)-1H-benzo[d]imidazole-6-carboxylate

The product obtained from the previous step (119 mg, 0.26 mmol) was added to 1,4-dioxane (4 mL), then Intermediate 1.7-A (200 mg, 0.39 mmol), LiCl (87 mg, 2.05 mmol) and Pd(PPh₃)₄ (4.5 mg, 3.87 µmol) were added, and the resulting mixture reacted under stirring at 100 °C for 16 hours. After the reaction was completed, the reaction mixture was added with water, then extracted with ethyl acetate, washed with saturated brine, dried over anhydrous sodium sulfate and concentrated under reduced pressure to obtain a crude product, which was purified by column chromatography (ethyl acetate: petroleum ether =1:10) to obtain methyl (S)-2-((4-(6-((4-cyano-2-fluorobenzyl)oxy)pyridin-2-yl)-2,3-dihydrobenzofuran-7-yl)methyl)-1-(oxetan-2-ylmethyl)-1H-benzo[d]imidazole-6-carboxylate (90 mg), ESI-MS (m/z): 605.1 [M+H]⁺.

### Step 3: Synthesis of (S)-2-((4-(6-((4-cyano-2-fluorobenzyl)oxy)pyridin-2-yl)-2,3-dihydrobenzofuran-7-yl)methyl)-1-(oxetan-2-ylmethyl)-1H-benzo[d]imidazole-6-carboxylic acid (Compound 4)

The product obtained from the previous step (80 mg, 0.13 mmol) was added to isopropanol (0.8 mL), THF (0.8 mL) and H₂O (0.8 mL). LiOH (12.7 mg, 0.53 mmol) was added, and the reaction mixture reacted under stirring at 20 °C for 12 hours. After the reaction was completed, the reaction mixture was added with 5 mL of water and extracted with methyl tert-butyl ether. The resulting aqueous phase was adjusted to pH=5 with acetic acid, extracted with ethyl acetate, dried over anhydrous sodium sulfate and concentrated under reduced pressure to obtain a crude product, which was purified by column chromatography (methanol: dichloromethane =1:15) to obtain two components, one of which was (S)-2-((4-(6-((4-cyano-2-fluorobenzyl)oxy)pyridin-2-yl)-2,3-dihydrobenzofuran-7-yl)methyl)-1-(oxetan-2-ylmethyl)-1H-benzo[d]imidazole-6-carboxylic acid (24.5 mg), ESI-MS (m/z): 589.1 [M-H]⁻. ¹H NMR (400 MHz, DMSO-*d₆*) δ 8.21 (s, 1H), 7.91 (d, *J* = 10.3 Hz, 1H), 7.84 (t, *J =* 7.9 Hz, 1H), 7.78 (d, *J* = 8.5 Hz, 1H), 7.72 (d, *J =* 3.9 Hz, 2H), 7.59 (br d, *J* = 8.4 Hz, 1H), 7.44 (d, *J=* 7.5 Hz, 1H), 7.28 (d, *J =* 8.0 Hz, 1H), 7.05 (d, *J* = 8.0 Hz, 1H), 6.89 (d, *J* = 8.1 Hz, 1H), 5.55 (s, 2H), 4.95 (dq, *J* = 2.8, 7.2 Hz, 1H), 4.70 - 4.61 (m, 1H), 4.59 - 4.52 (m, 3H), 4.52 - 4.42 (m, 2H), 4.39 - 4.32 (m, 2H), 4.28 - 4.22 (m, 1H), 3.49 (t, *J* = 8.7 Hz, 2H), 2.66 - 2.60 (m, 1H), 2.38 - 2.33 (m, 1H).

### Example 5: Synthesis of (S)-2-((4-(6-((4-aminocarbonyl-2-fluorobenzyl)oxy)pyridin-2-yl)-2,3-dihydrobenzofuran-7-yl)methyl)-1-(oxetan-2-ylmethyl)-1H-benzo[d]imidazole-6-carboxylic acid (Compound 5)

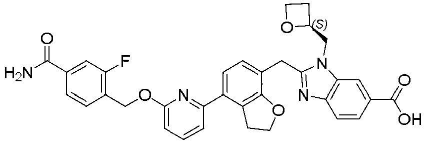

The synthetic method was the same as that for Compound 4, and the other component was finally collected to obtain (S)-2-((4-(6-((4-aminocarbonyl-2-fluorobenzyl)oxy)pyridin-2-yl)-2,3-dihydrobenzofuran-7-yl)methyl)-1-(oxetan-2-ylmethyl)-1H-benzo[d]imidazole-6-carboxylic acid (2.6 mg), ESI-MS (m/z): 607.1[M-H]⁻, ¹H NMR (400 MHz, DMSO-*d₆*) δ 8.19 (s, 1H), 8.04 (s, 1H), 7.85 - 7.80 (m, 1H), 7.80 - 7.77 (m, 1H), 7.74 - 7.68 (m, 2H), 7.64 - 7.59 (m, 1H), 7.58 - 7.54 (m, 1H), 7.52 - 7.49 (m, 1H), 7.43 (d, *J =* 7.4 Hz, 1H), 7.30 (d, *J* = 8.1 Hz, 1H), 7.05 (d, *J* = 8.0 Hz, 1H), 6.87 (d, *J* = 8.1 Hz, 1H), 5.51 (s, 2H), 4.99 - 4.91 (m, 1H), 4.69 - 4.60 (m, 1H), 4.59 - 4.53 (m, 2H), 4.53 - 4.43 (m, 2H), 4.39 - 4.32 (m, 2H), 4.28 - 4.21 (m, 1H), 3.52 (t, *J* = 8.6 Hz, 2H), 2.69 - 2.64 (m, 1H), 2.34 - 2.31 (m, 1H).

### Example 6: Synthesis of 2-(1-(7-(6-((4-cyano-2-fluorobenzyl)oxy)pyridin-2-yl)-2,3-dihydrobenzofuran-4-yl)ethyl)-1-(((S)-oxetan-2-yl)methyl)-1H-benzo[d]imidazole-6-carboxylic acid (Compound 6)

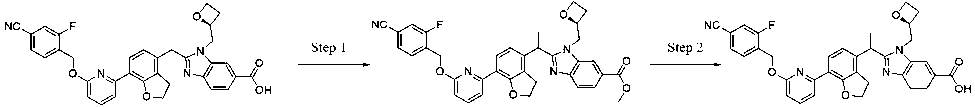

### Step 1: Synthesis of methyl 2-(1-(7-(6-((4-cyano-2-fluorobenzyl)oxy)pyridin-2-yl)-2,3-dihydrobenzofuran-4-yl)ethyl)-1-(((S)-oxetan-2-yl)methyl)-1H-benzo[d]imidazole-6-carboxylate

(*S*)-2-((7-(6-((4-cyano-2-fluorobenzyl)oxy)pyridin-2-yl)-2,3-dihydrobenzofuran-4-yl)methyl)-1-(oxetan-2-ylmethyl)-1*H*-benzo[*d*]imidazole-6-carboxylic acid (34 mg, 0.058 mmol), iodomethane (17 mg, 0.12 mmol), potassium carbonate (17 mg, 0.12 mmol) and DMF (1 mL) were stirred at room temperature for half an hour, then iodomethane (17 mg, 0.12 mmol) and sodium hydride (5 mg, 0.12 mmol) were added, and the resulting mixture was further stirred at room temperature. After the reaction was completed, the reaction mixture was added with water and extracted with ethyl acetate. The resulting organic phases were combined, dried over anhydrous sodium sulfate, filtered, concentrated, and purified by column chromatography (methanol: dichloromethane =1:30) to obtain methyl 2-(1-(7-(6-((4-cyano-2-fluorobenzyl)oxy)pyridin-2-yl)-2,3-dihydrobenzofuran-4-yl)ethyl)-1-(((S)-oxetan-2-yl)methyl)-1*H*-benzo[*d*]imidazole-6-carboxylate (23 mg), ESI-MS (m/z): 619.5[M+H]⁺.

### Step 2: Synthesis of 2-(1-(7-(6-((4-cyano-2-fluorobenzyl)oxy)pyridin-2-yl)-2,3-dihydrobenzofuran-4-yl)ethyl)-1-(((S)-oxetan-2-yl)methyl)-1H-benzo[d]imidazole-6-carboxylic acid

Methyl 2-(1-(7-(6-((4-cyano-2-fluorobenzyl)oxy)pyridin-2-yl)-2,3-dihydrobenzofuran-4-yl)ethyl)-1-(((*S*)-oxetan-2-yl)methyl)-1*H*-benzo[*d*]imidazole-6-carboxylate (23 mg, 0.037 mmol), lithium hydroxide (5 mg, 0.19 mmol), isopropanol (0.5 mL), tetrahydrofuran (0.5 mL) and water (0.5 mL) were added into a 25 mL round bottom flask and the resulting mixture reacted under stirring at 50 °C. After the reaction was completed, the pH was adjusted to 6 with acetic acid, followed by concentration, and isolation by thin layer chromatography (methanol: dichloromethane =1:15) to obtain 2-(1-(7-(6-((4-cyano-2-fluorobenzyl)oxy)pyridin-2-yl)-2,3-dihydrobenzofuran-4-yl)ethyl)-1-(((S)-oxetan-2-yl)methyl)-1*H*-benzo[*d*]imidazole-6-carboxylic acid (13 mg), ESI-MS (m/z): 605.2[M+H]⁺, ¹H NMR (600 MHz, CDCl₃) δ 8.15 - 8.02 (m, 2H), 7.97 - 7.77 (m, 3H), 7.70 - 7.59 (m, 2H), 7.47 - 7.33 (m, 2H), 6.77 - 6.64 (m, 2H), 5.64 - 5.54 (m, 2H), 4.94 - 4.83 (m, 1H), 4.74 - 4.58 (m, 3H), 4.57 - 4.49 (m, 1H), 4.36 - 4.25 (m, 1H), 4.20 - 4.08 (m, 2H), 3.06 - 2.85 (m, 2H), 2.72 - 2.51 (m, 2H), 1.91 - 1.84 (m, 3H).

### Example 7: Synthesis of (S)-2-((5-chloro-7-(6-((4-cyano-2-fluorobenzyl)oxy)pyridin-2-yl)-2,3-dihydrobenzofuran-4-yl)methyl)-1-(oxetan-2-ylmethyl)-1H-benzo[d]imidazole-6-carboxylic acid (Compound 7)

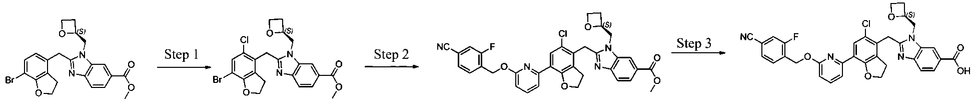

### Step 1: Synthesis of methyl (S)-2-((7-bromo-5-chloro-2,3-dihydrobenzofuran-4-yl)methyl)-1-(oxetan-2-ylmethyl)-1H-benzo[d]imidazole-6-carboxylate

Methyl (S)-2-((7-bromo-2,3-dihydrobenzofuran-4-yl)methyl)-1-(oxetan-2-ylmethyl)-1H-benzo[d]imidazole-6-carboxylate (300 mg, 0.65 mmol) was dissolved in DMF (6 mL), then NCS (70 mg, 0.52 mmol) was added, and the resulting mixture reacted under stirring at 60 °C for 2 hours. After the reaction was completed, the reaction mixture was concentrated under reduced pressure and the resulting crude product was purified by column chromatography (methanol: dichloromethane =1:50) to obtain methyl (S)-2-((7-bromo-5-chloro-2,3-dihydrobenzofuran-4-yl)methyl)-1-(oxetan-2-ylmethyl)-1H-benzo[d]imidazole-6-carboxylate (138 mg), ESI-MS (m/z): 491.1 [M+H]⁺.

### Step 2: Synthesis of methyl (S)-2-((5-chloro-7-(6-((4-cyano-2-fluorobenzyl)oxy)pyridin-2-yl)-2,3-dihydrobenzofuran-4-yl)methyl)-1-(oxetan-2-ylmethyl)-1H-benzo[d]imidazole-6-carboxylate

The product obtained from the previous step (100 mg, 0.20 mmol) was added to 1,4-dioxane (2 mL), and then Intermediate 1.7-A (105 mg, 0.20 mmol), Pd(PPh₃)₄ (7.0 mg, 0.06 mmol) and LiCl (46 mg, 1.08 mmol) were added sequentially. The reaction mixture was heated to 100 °C and reacted under stirring for 48 h. After the reaction was completed, the reaction mixture was added with water, then extracted with ethyl acetate, washed with saturated brine, dried over anhydrous sodium sulfate, and concentrated under reduced pressure to obtain a crude product of methyl (S)-2-((5-chloro-7-(6-((4-cyano-2-fluorobenzyl)oxy)pyridin-2-yl)-2,3-dihydrobenzofuran-4-yl)methyl)-1-(oxetan-2-ylmethyl)-1H-benzo[d]imidazole-6-carboxylate (150 mg), ESI-MS (m/z): 639.2[M+H]⁺.

### Step 3: Synthesis of (S)-2-((5-chloro-7-(6-((4-cyano-2-fluorobenzyl)oxy)pyridin-2-yl)-2,3-dihydrobenzofuran-4-yl)methyl)-1-(oxetan-2-ylmethyl)-1H-benzo[d]imidazole-6-carboxylic acid

The product obtained from the previous step (7 mg, 0.10 mmol) was added to isopropanol (0.7 mL), THF (0.7 mL) and H₂O (0.7 mL). LiOH (10.5 mg, 0.43 mmol) was added, and the reaction mixture reacted under stirring at 20 °C for 12 hours. After the reaction was completed, the reaction mixture was added with water and extracted with an organic solvent methyl tert-butyl ether, and the resulting aqueous phase was adjusted to pH=5 with acetic acid, extracted with ethyl acetate, dried over anhydrous sodium sulfate and concentrated under reduced pressure to obtain a crude product, which was purified by column chromatography (methanol: dichloromethane=1:15) to obtain (S)-2-((5-chloro-7-(6-((4-cyano-2-fluorobenzyl)oxy)pyridin-2-yl)-2,3-dihydrobenzofuran-4-yl)methyl)-1-(oxetan-2-ylmethyl)-1H-benzo[d]imidazole-6-carboxylic acid (5.34 mg), ESI-MS (m/z): 623.1 [M-H]⁻, ¹H NMR (400 MHz, DMSO-*d₆*) δ 12.75 (br s, 1H), 8.24 (d, *J =* 1.0 Hz, 1H), 7.92 (d, *J =* 10.4 Hz, 1H), 7.86 - 7.81 (m, 2H), 7.81 - 7.70 (m, 4H), 7.55 (d, *J* = 8.4 Hz, 1H), 6.92 - 6.89 (m, 1H), 5.59 (s, 2H), 5.17 - 5.04 (m, 1H), 4.81 - 4.73 (m, 1H), 4.70 (br t, *J =* 8.7 Hz, 2H), 4.52 - 4.45 (m, 2H), 4.37 (td, *J =* 6.1, 9.1 Hz, 1H), 3.79 - 3.61 (m, 2H), 3.13 (dt, *J =* 3.6, 8.2 Hz, 2H), 2.73 (ddd, *J* = 3.0, 5.8, 8.3 Hz, 1H), 2.44 - 2.36 (m, 1H).

### Example 8: Synthesis of 2-((7-(2-(4-chloro-2-fluorophenyl)-2-methylbenzo[d][1,3]dioxolan-4-yl)-2,3-dihydrobenzofuran-4-yl)methyl)-1-(((S)-oxetan-2-yl)methyl)-1H-benzo[d]imidazole-6-carboxylic acid (Compound 8)

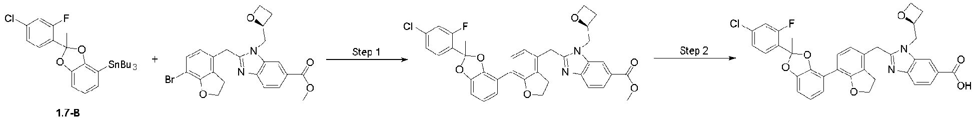

### Step 1: Synthesis of methyl 2-((7-(2-(4-chloro-2-fluorophenyl)-2-methylbenzo[d][1,3]dioxolan-4-yl)-2,3-dihydrobenzofuran-4-yl)methyl)-1-(((S)-oxetan-2-yl)methyl)-1H-benzo[d]imidazole-6-carboxylate

Intermediate 1.7-B (320 mg, 577.9 µmol) was dissolved in 1,4-dioxane (3.2 mL), then the intermediate methyl (S)-2-((7-bromobenzo[d][1,3]dioxolan-4-yl)methyl)-1-(oxetan-2-ylmethyl)-1H-benzo[d]imidazole-6-carboxylate (132 mg, 288.95 µmol), LiCl (131 mg, 3.08 mmol) and Pd(PPh₃)₄ (33.4 mg, 28.90 µmol) were added, and the reaction mixture reacted under stirring at 120 °C for 16 hours. After the reaction was completed, the reaction mixture was filtered, then diluted with water, then extracted with ethyl acetate, washed with saturated brine, dried over anhydrous sodium sulfate, concentrated under reduced pressure and purified by column chromatography (methanol: dichloromethane =1:30) to obtain methyl 2-((7-(2-(4-chloro-2-fluorophenyl)-2-methylbenzo[d][1,3]dioxolan-4-yl)-2,3-dihydrobenzofuran-4-yl)methyl)-1-(((S)-oxetan-2-yl)methyl)-1H-benzo[d]imidazole-6-carboxylate (140 mg), ESI-MS (m/z): 641.1 [M+H]⁺.

### Step 2: Synthesis of 2-((7-(2-(4-chloro-2-fluorophenyl)-2-methylbenzo[d][1,3]dioxolan-4-yl)-2,3-dihydrobenzofuran-4-yl)methyl)-1-(((S)-oxetan-2-yl)methyl)-1H-benzo[d]imidazole-6-carboxylic acid

The product obtained from the previous step (120 mg, 187.18 µmol) was added to THF (0.12 mL), isopropanol (0.12 mL), and H₂O (0.12 mL). Then LiOH (18 mg, 748.73 µmol) was added, and the reaction mixture reacted under stirring at 20 °C for 16 hours. After the reaction was completed, the reaction mixture was diluted with water, then extracted with methyl tert-butyl ether, adjusted to pH=5 with acetic acid, and extracted with ethyl acetate. The resulting organic phase was dried over anhydrous sodium sulfate and concentrated under reduced pressure to obtain a crude product, which was purified by column chromatography (methanol: dichloromethane=1:15) to obtain 2-((7-(2-(4-chloro-2-fluorophenyl)-2-methylbenzo[d][1,3]dioxolan-4-yl)-2,3-dihydrobenzofuran-4-yl)methyl)-1-(((S)-oxetan-2-yl)methyl)-1H-benzo[d]imidazole-6-carboxylic acid (36.3 mg), ESI-MS (m/z): 625.1 [M-H]⁻, ¹H NMR (400 MHz, DMSO-*d₆*) δ 12.76 (br s, 1H), 8.24 (s, 1H), 7.83 - 7.77 (m, 1H), 7.69 (t*, J =* 8.4 Hz, 1H), 7.63 (d, *J =* 8.4 Hz, 1H), 7.56 (br d, *J* = 12.2 Hz, 1H), 7.39 - 7.34 (m, 1H), 7.26 (d, *J* = 7.8 Hz, 1H), 7.07 (dd, *J* = 2.0, 7.1 Hz, 1H), 6.94 - 6.86 (m, 2H), 6.76 (d, *J =* 8.1 Hz, 1H), 5.03 - 4.93 (m, 1H), 4.70 - 4.44 (m, 5H), 4.40 - 4.32 (m, 3H), 3.22 - 3.12 (m, 2H), 2.67 (br t, *J =* 8.6 Hz, 1H), 2.42 - 2.34 (m, 1H), 2.02 (s, 3H).

### Example 9: Synthesis of (S)-2-((7-(6-((4-chloro-2-fluorobenzyl)oxy)pyridin-2-yl)-1H-indol-4-yl)methyl)-1-(oxetan-2-ylmethyl)-1H-benzo[d]imidazole-6-carboxylic acid (Compound 9)

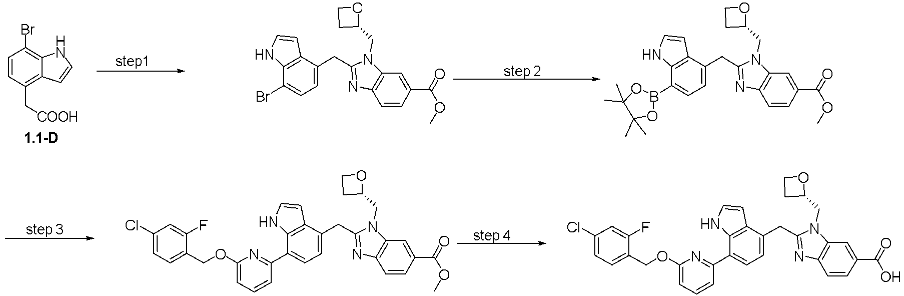

### Step 1: Synthesis of methyl (S)-2-((7-bromo-1H-indol-4-yl)methyl-1-(oxetan-2-ylmethyl)-1H-benzo[d]imidazole-6-carboxylate

2-(7-Bromo-1H-indol-4-yl)acetic acid (1.1-D) (250 mg, 1 mmol) and N, N-dimethylformamide (25 mL) were added into a 100 mL flask. HATU (57 mg, 1.5 mmol) and triethylamine (305 mg, 3 mmol) were added, and then methyl (S)-4-amino-3-((oxetan-2-ylmethyl)amino)benzoate (305 mg, 1.3 mmol) was added under stirring. The resulting mixture reacted under stirring at room temperature. After the reaction was completed, the reaction mixture was added to water, extracted with ethyl acetate, dried over anhydrous sodium sulfate, and concentrated, and the resulting crude product was by column chromatography (ethyl acetate: petroleum ether =1:10). The purified intermediate (300 mg) and glacial acetic acid (5 mL) were added into a 100 mL flask, heated to 120 °C and reacted under stirring. After the reaction was completed, the reaction mixture was added to water, extracted with ethyl acetate, washed with aqueous sodium bicarbonate solution, and concentrated, and the resulting crude product was isolated and purified by column chromatography (methanol: dichloromethane =1:50) to obtain methyl (S)-2-((7-bromo-1H-indol-4-yl)methyl-1-(oxetan-2-ylmethyl)-1H-benzo[d]imidazole-6-carboxylate (220 mg), ESI-MS (m/z): 454.1[M+H]⁺.

### Step 2: Synthesis of methyl (S)-1-(oxetan-2-ylmethyl)-2-((7-(4,4,5,5-tetramethyl-1,3,2-dioxaborolan-2-yl)-1H-indol-4-yl)methyl)-1H-benzo[d]imidazole-6-carboxylate

Methyl (S)-2-((7-bromo-1H-indol-4-yl)methyl-1-(oxetan-2-ylmethyl)-1H-benzo[d]imidazole-6-carboxylate (220 mg, 0.49 mmol), bis(pinacolato)diboron (247 mg, 0.97 mmol), Pd(dppf)Cl₂ (71 mg, 0.097mmol), potassium acetate (95 mg, 0.97 mmol) and dioxane (10 mL) were added into a 50 mL round bottom flask. The resulting mixture was placed in a nitrogen atmosphere and reacted under stirring at 100 °C. After the reaction was completed, concentration was performed, and the resulting crude product was purified by column chromatography (methanol: dichloromethane =1:30) to obtain methyl (S)-1-(oxetan-2-ylmethyl)-2-((7-(4,4,5,5-tetramethyl-1,3,2-dioxaborolan-2-yl)-1H-indol-4-yl)methyl)-1H-benzo[d]imidazole-6-carboxylate (180 mg), ESI-MS (m/z): 502.2[M+H]⁺.

### Step 3: Synthesis of methyl (S)-2-((7-(6-((4-chloro-2-fluorobenzyl)oxy)pyridin-2-yl)-1H-indol-4-yl)methyl)-1-(oxetan-2-ylmethyl)-1H-benzo[d]imidazole-6-carboxylate

Methyl (S)-1-(oxetan-2-ylmethyl)-2-((7-(4,4,5,5-tetramethyl-1,3,2-dioxaborolan-2-yl)-1H-indol-4-yl)methyl)-1H-benzo[d]imidazole-6-carboxylate (80 mg, 0.16 mmol), 2-bromo-6-((4-chloro-2-fluorobenzyl)oxy)pyridine (50 mg, 0.16 mmol), Pd(dppf)Cl₂ (23 mg, 0.032 mmol), potassium carbonate (66 mg, 0.48 mmol), dioxane (5 mL) and water (2 mL) were added into a 100 mL flask. The resulting mixture was placed in a nitrogen atmosphere and reacted under stirring at 80 °C. After the reaction was completed, the reaction mixture was added to water, then extracted with ethyl acetate, dried and concentrated, and the resulting crude product was purified by thin layer chromatography (methanol: dichloromethane =1:30) to obtain methyl (S)-2-((7-(6-((4-chloro-2-fluorobenzyl)oxy)pyridin-2-yl)-1H-indol-4-yl)methyl)-1-(oxetan-2-ylmethyl)-1H-benzo[d]imidazole-6-carboxylate (20 mg), ESI-MS (m/z): 611.2[M+H]⁺.

### Step 4: Synthesis of (S)-2-((7-(6-((4-chloro-2-fluorobenzyl)oxy)pyridin-2-yl)-1H-indol-4-yl)methyl)-1-(oxetan-2-ylmethyl)-1H-benzo[d]imidazole-6-carboxylic acid

Methyl (S)-2-((7-(6-((4-chloro-2-fluorobenzyl)oxy)pyridin-2-yl)-1H-indol-4-yl)methyl)-1-(oxetan-2-ylmethyl)-1H-benzo[d]imidazole-6-carboxylate (50 mg, 0.082 mmol), sodium hydroxide (33 mg, 0.82 mmol), methanol (4 mL), tetrahydrofuran (4 mL) and water (2 mL) were added into a 25 mL flask. The resulting mixture reacted under stirring at room temperature. After the reaction was completed, the organic solvent was removed by concentration, glacial acetic acid was added to adjust the pH to 3, and concentration was performed. The resulting crude product was purified by column chromatography (methanol: dichloromethane=1:15) to obtain (S)-2-((7-(6-((4-chloro-2-fluorobenzyl)oxy)pyridin-2-yl)-1H-indol-4-yl)methyl)-1-(oxetan-2-ylmethyl)-1H-benzo[d]imidazole-6-carboxylic acid (21 mg), ESI-MS (m/z): 597.2[M+H]⁺, ¹H NMR (600 MHz, CDCl₃) δ 10.46 (s, 1H), 8.12 (s, 1H), 8.05 (d, *J* = 6.5 Hz, 1H), 7.86 (d, *J=* 7.1 Hz, 1H), 7.76 - 7.68 (m, 1H), 7.61-7.54 (m, 2H), 7.50 - 7.42 (m, 1H), 7.21-7.14 (m, 3H), 6.95 (d, *J* = 6.6 Hz, 1H), 6.80 (d, *J* = 7.2 Hz, 1H), 6.66 (s, 1H), 5.56 (s, 2H), 4.99 - 4.90 (m, 1H), 4.86-4.79 (m, 2H), 4.61 - 4.53 (m, 1H), 4.41 - 4.33 (m, 1H), 3.95-3.87 (m, 2H), 2.51-2.48 (m, 1H), 2.15-2.11 (m, 1H).

### Example 10: Synthesis of 2-((7-(2-(4-chloro-2-fluorophenyl)-2-methylbenzo[d][1,3]dioxolan-4-yl)-1H-indol-4-yl)methyl)-1-(((S)-oxetan-2-yl)methyl)-1H-benzo[d]imidazole-6-carboxylic acid (Compound 10)

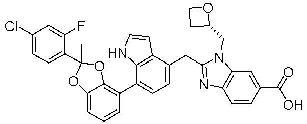

2-((7-(2-(4-Chloro-2-fluorophenyl)-2-methylbenzo[d][1,3]dioxolan-4-yl)-1H-indol-4-yl)methyl)-1-(((S)-oxetan-2-yl)methyl)-1H-benzo[d]imidazole-6-carboxylic acid (3 mg) was obtained by referring to the synthetic method of Compound 8. ESI-MS (m/z): 624.2[M+H]⁺, ¹H NMR (600 MHz, DMSO-*d₆*) δ 12.75 (s, 1H), 10.86 (s, 1H), 8.20 (s, 1H), 7.79 (d, *J* = 8.5 Hz, 1H), 7.63 (d, *J =* 8.4 Hz, 1H), 7.52-7.47 (m, 2H), 7.31-7.30 (m, 2H), 7.13 (d, *J* = 7.2 Hz, 1H), 7.06-7.05 (m, 1H), 7.02-6.99 (m, 2H), 6.93 (d, *J* = 7.4 Hz, 1H), 6.63 (s, 1H), 4.82-4.77 (m, 1H), 4.69-4.60 (m, 3H), 4.50-4.48 (m, 1H), 4.44-4.42 (m, 1H), 4.35-4.32 (m, 1H), 2.43-2.40 (m, 1H), 2.29-2.23 (m, 1H), 2.05 (s, 3H).

### Example 11: Synthesis of (S)-2-((7-(6-((4-chloro-2-fluorobenzyl)oxy)pyridin-2-yl)-1-methyl-1H-indol-4-yl)methyl-1-(oxetan-2-ylmethyl)-1H-benzo[d]imidazole-6-carboxylic acid (Compound 11)

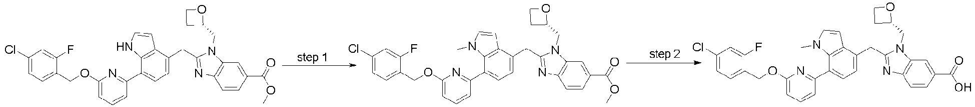

### Step 1: Synthesis of methyl (S)-2-((7-(6-((4-chloro-2-fluorobenzyl)oxy)pyridin-2-yl)-1-methyl-1H-indol-4-yl)methyl)-1-(oxetan-2-ylmethyl)-1H-benzo[d]imidazole-6-carboxylate

Methyl (S)-2-((7-(6-((4-chloro-2-fluorobenzyl)oxy)pyridin-2-yl)-1H-indol-4-yl)methyl)-1-(oxetan-2-ylmethyl)-1H-benzo[d]imidazole-6-carboxylate (70 mg, 0.11 mmol) and N, N-dimethylformamide (5 mL) were added into a 100 mL flask, and then cesium carbonate (375 mg, 1.15 mmol) and iodomethane (163 mg, 1.15 mmol) were added. The resulting mixture was stirred at room temperature. After the reaction was completed, the resulting mixture was added into water, extracted with ethyl acetate, dried and concentrated. The resulting crude product was purified by column chromatography (methanol: dichloromethane =1:30) to obtain methyl (S)-2-((7-(6-((4-chloro-2-fluorobenzyl)oxy)pyridin-2-yl)-1-methyl-1H-indol-4-yl)methyl)-1-(oxetan-2-ylmethyl)-1H-benzo[d]imidazole-6-carboxylate (60 mg), ESI-MS (m/z): 625.2[M+H]⁺.

### Step 2: Synthesis of (S)-2-((7-(6-((4-chloro-2-fluorobenzyl)oxy)pyridin-2-yl)-1-methyl-1H-indol-4-yl)methyl)-1-(oxetan-2-ylmethyl)-1H-benzo[d]imidazole-6-carboxylic acid

Methyl (S)-2-((7-(6-((4-chloro-2-fluorobenzyl)oxy)pyridin-2-yl)-1- methyl-1H-indol-4-yl)methyl)-1-(oxetan-2-ylmethyl)-1H-benzo[d]imidazole-6-carboxylate (60 mg, 0.096 mmol), sodium hydroxide (40 mg, 1 mmol), methanol (4 mL), tetrahydrofuran (4 mL) and water (2 mL) were added into a 100 mL flask. The resulting mixture was heated to 50 °C and reacted under stirring. After the reaction was completed, the organic solvent was removed under reduced pressure, the pH was adjusted to 3 with acetic acid, and further concentration was performed. The resulting crude product was purified by column chromatography (methanol: dichloromethane = 1:15) to obtain (S)-2-((7-(6-((4-chloro-2-fluorobenzyl)oxy)pyridin-2-yl)-1-methyl-1H-indol-4-yl)methyl)-1-(oxetan-2-ylmethyl)-1H-benzo[d]imidazole-6-carboxylic acid (23 mg), ESI-MS (m/z): 611.2[M+H]⁺, ¹H NMR (600 MHz, DMSO-*d₆*) δ 12.69 (s, 1H), 8.20 (s, 1H), 7.85 (t, *J =* 7.8 Hz, 1H), 7.78 (d, *J* = 8.4 Hz, 1H), 7.62 (d, *J* = 8.4 Hz, 1H), 7.56 (t, *J* = 7.9 Hz, 1H), 7.45 (d, *J =* 9.7 Hz, 1H), 7.34-7.25 (m, 2H), 7.20 (d, *J* = 7.2 Hz, 1H), 7.01 (d, *J* = 7.3 Hz, 1H), 6.92 (d, *J* = 6.2 Hz, 2H), 6.63 (s, 1H), 5.36 (s, 2H), 4.90-4.85 (m, 1H), 4.70-4.66 (m, 2H), 4.63-4.59 (m, 1H), 4.50-4.45 (m, 2H), 4.38-4.34 (m, 1H), 2.62-2.56 (m, 1H), 2.32-2.27 (m, 1H).

### Example 12: Synthesis of (S)-2-((7-(6-((4-chloro-2-fluorobenzyl)oxy)pyridin-2-yl)-5-fluoro-2,3-dihydrobenzofuran-4-yl)methyl)-1-(oxetan-2-ylmethyl)-1H-benzo[d]imidazole-6-carboxylic acid (Compound 12)

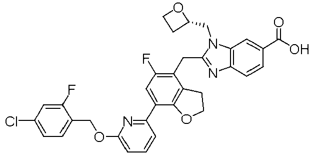

(S)-2-((7-(6-((4-Chloro-2-fluorobenzyl)oxy)pyridin-2-yl)-5-fluoro-2,3-dihydrobenzofuran-4-yl)methyl)-1-(oxetan-2-ylmethyl)-1*H*-benzo[d]imidazole-6-carboxylic acid (30 mg) was obtained by referring to the synthesis of Compound 7 using Intermediates 1.1-F and 1.2-A as the starting materials. ESI-MS (m/z): 618.2[M+H]⁺, ¹H NMR (600 MHz, DMSO-*d₆*) δ 8.22 (s, 1H), 7.83-7.79 (m, 2H), 7.78-7.73 (m, 2H), 7.60-7.58 (m, 1H), 7.56 (d, *J =* 8.4 Hz, 1H), 7.48 (dd, *J* = 9.9, 1.7 Hz, 1H), 7.31 (dd, *J* = 8.2, 1.5 Hz, 1H), 6.86-6.80 (m, 1H), 5.51 (s, 2H), 5.09-5.05 (m, 1H), 4.76-4.72 (m, 1H), 4.68 (t, *J* = 8.8 Hz, 2H), 4.62-4.59 (m, 1H), 4.52-4.45 (m, 2H), 4.39- 4.33 (m, 2H), 3.15-3.10 (m, 2H), 2.72-2.69 (m, 1H), 2.41-2.35 (m, 1H).

### Example 13: Synthesis of (S)-2-((7-(6-((4-cyano-2-fluorobenzyl)oxy)pyridin-2-yl)-5-fluoro-2,3-dihydrobenzofuran-4-yl)methyl)-4-fluoro-1-(oxetan-2-ylmethyl)-1H-benzo[d]imidazole-6-carboxylic acid (Compound 13)

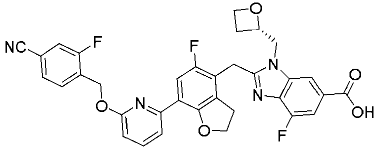

(S)-2-((7-(6-((4-cyano-2-fluorobenzyl)oxy)pyridin-2-yl)-5-fluoro-2,3-dihydrobenzofuran-4-yl)methyl)-4-fluoro-1-(oxetan-2-ylmethyl)-1*H*-benzo[*d*]imidazole-6-carboxylic acid (23 mg) was obtained by referring to the synthetic method of Compound 12 using Intermediates 1.1-F and 1.2-B as the starting materials. ESI-MS (m/z): 627.2[M+H]⁺, ¹H NMR (600 MHz, DMSO-*d₆*) δ 13.20 (s, 1H), 8.12 (s, 1H), 7.91 (d, *J* = 9.9 Hz, 1H), 7.84-7.81 (m, 2H), 7.76-7.70 (m, 2H), 7.68 (d, *J* = 11.6 Hz, 1H), 7.49 (d, *J* = 11.3 Hz, 1H), 6.89-6.87 (m, 1H), 5.60 (s, 2H), 5.08-5.04 (m, 1H), 4.79-4.75 (m, 1H), 4.69 (t,*J* = 9.0 Hz, 2H), 4.65-4.62 (m, 1H), 4.51-4.47 (m, 2H), 4.41-4.33 (m, 2H), 3.16-3.09 (m, 2H), 2.73-2.68 (m, 1H), 2.40-2.34 (m, 1H).

### Example 14: Synthesis of 2-((7-(6-((4-chloro-2-fluorobenzyl)oxy)pyridin-2-yl)-5-fluoro-2,3-dihydrobenzofuran-4-yl)methyl)-1-((1-(fluoromethyl)cyclopropyl)methyl)-1H-benzo[d]imidazole-6-carboxylic acid (Compound 14)

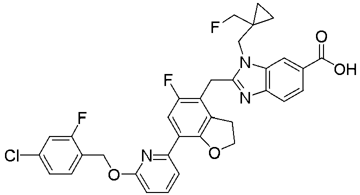

2-((7-(6-((4-Chloro-2-fluorobenzyl)oxy)pyridin-2-yl)-5-fluoro-2,3-dihydrobenzofuran-4-yl)methyl)-1-((1-(fluoromethyl)cyclopropyl)methyl)-1H-benzo[d]imidazole-6-carboxylic acid (32 mg) was obtained by referring to the synthetic method of Compound 12 using Intermediates 1.1-F and 1.2-D as the starting materials. ESI-MS (m/z): 634.2[M+H]⁺, ¹H NMR (600 MHz, CDCl₃) δ 8.20 (s, 1H), 8.01 (d, *J =* 8.2 Hz, 1H), 7.83 (d, *J =* 11.4 Hz, 1H), 7.79-7.75 (m, 2H), 7.61-7.59 (m, 1H), 7.47-7.44 (m, 1H), 7.13-7.12 (m, 2H), 6.70 (d, *J =* 8.1 Hz, 1H), 5.50 (s, 2H), 4.67 (t, *J =* 8.4 Hz, 2H), 4.47-4.35 (m, 4H), 4.18-4.13 (m, 1H), 4.10-4.05 (m, 1H), 3.20 (t, *J =* 8.5 Hz, 2H), 0.83-0.77 (m, 4H).

### Example 15: Synthesis of 2-(7-(6-((4-chloro-2-fluorobenzyl)oxy)pyridin-2-yl)-5-fluoro-2,3-dihydrobenzofuran-4-carbonyl)-1-((1-(fluoromethyl)cyclopropyl)methyl)-1H-benzo[d]imidazole-6-carboxylic acid (Compound 15)

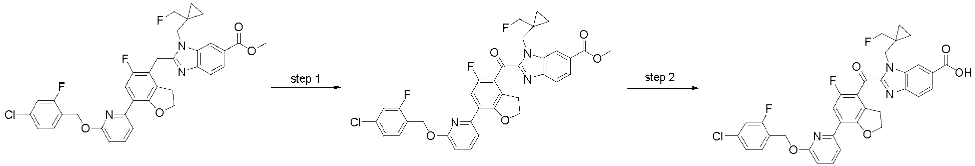

### Step 1: Synthesis of methyl 2-(7-(6-((4-chloro-2-fluorobenzyl)oxy)pyridin-2-yl)-5-fluoro-2,3-dihydrobenzofuran-4-carbonyl)-1-((1-(fluoromethyl)cyclopropyl)methyl)-1H-benzo[d]imidazole-6-carboxylate

Methyl 2-((7-(6-((4-chloro-2-fluorobenzyl)oxy)pyridin-2-yl)-5-fluoro-2,3-dihydrobenzofuran-4-yl)methyl)-1-((1-(fluoromethyl)cyclopropyl)methyl)-1*H*-benzo[*d*]imidazole-6-carboxylate (an intermediate of Compound 14, synthesized by referring to the synthetic methods of Compounds 12 and 14, 60 mg, 0.093 mmol) and N,N-dimethylformamide (5 mL) were added into a 100 mL flask, iodomethane (66 mg, 0.46 mmol) and cesium carbonate (91 mg, 0.28 mmol) were added, and the resulting mixture was stirred at room temperature. After the reaction was completed, the reaction mixture was added into water, and extracted with ethyl acetate. The resulting organic phase was dried over anhydrous sodium sulfate, filtered and concentrated. The resulting crude product was isolated and purified by thin layer chromatography (methanol: dichloromethane =1:50) to obtain methyl 2-(7-(6-((4-chloro-2-fluorobenzyl)oxy)pyridin-2-yl)-5-fluoro-2,3-dihydrobenzofuran-4-carbonyl)-1-((1-(fluoromethyl)cyclopropyl) methyl)-1*H*-benzo[*d*]imidazole-6-carboxylate (30 mg), ESI-MS (m/z): 662.2[M+H]⁺.

### Step 2: Synthesis of 2-(7-(6-((4-chloro-2-fluorobenzyl)oxy)pyridin-2-yl)-5-fluoro-2,3-dihydrobenzofuran-4-carbonyl)-1-((1-(fluoromethyl)cyclopropyl)methyl)-1H-benzo[d]imidazole-6-carboxylic acid

Methyl 2-(7-(6-((4-chloro-2-fluorobenzyl)oxy)pyridin-2-yl)-5-fluoro-2,3-dihydrobenzofuran-4-carbonyl)-1-((1-(fluoromethyl)cyclopropyl)methyl)-1*H*-benzo[*d*]imidazole-6-carboxylate (30 mg, 0.045mmol) and tetrahydrofuran (4 mL) were added into a 25 mL flask. Then methanol (4mL), sodium hydroxide (18 mg, 0.45 mmol) and water (4 mL) were added, and the resulting mixture reacted under stirring at 50 °C. After the reaction was completed, the temperature was lowered, 0.1 mL of glacial acetic acid was added, and the resulting mixture was stirred at room temperature for 10 minutes and then concentrated. The resulting crude product was isolated and purified by thin layer chromatography (methanol: dichloromethane=1:30) to obtain 2-(7-(6-((4-chloro-2-fluorobenzyl)oxy)pyridin-2-yl)-5-fluoro-2,3-dihydrobenzofuran-4-carbonyl)-1-((1-(fluoromethyl)cyclopropyl) methyl)-1*H*-benzo[d]imidazole-6-carboxylic acid (21 mg), ESI-MS (m/z): 648.1[M+H]⁺, ¹H NMR (600 MHz, CDCl₃) δ 8.45 (s, 1H), 8.11 (d, *J* = 6.0 Hz, 1H), 7.94-7.90 (m, 3H), 7.68-7.66 (m, 1H), 7.47-7.43 (m, 1H), 7.14-7.11 (m, 2H), 6.78 (d, *J* = 8.1 Hz, 1H), 5.50 (s, 2H), 4.90 (br, 2H), 4.79-4.76 (m, 2H), 4.09 (d, *J* = 47.8 Hz, 2H), 3.46-3.43 (m, 2H), 0.88-0.84 (m, 2H), 0.70 (br, 2H).

### Example 16: Synthesis of 2-(1-(7-(6-((4-chloro-2-fluorobenzyl)oxy)pyridin-2-yl)-2,3-dihydrobenzofuran-4-yl)ethyl)-1-((1-(fluoromethyl)cyclopropyl)methyl)-1H-benzo[d]imidazole-6-carboxylic acid (Compound 16)

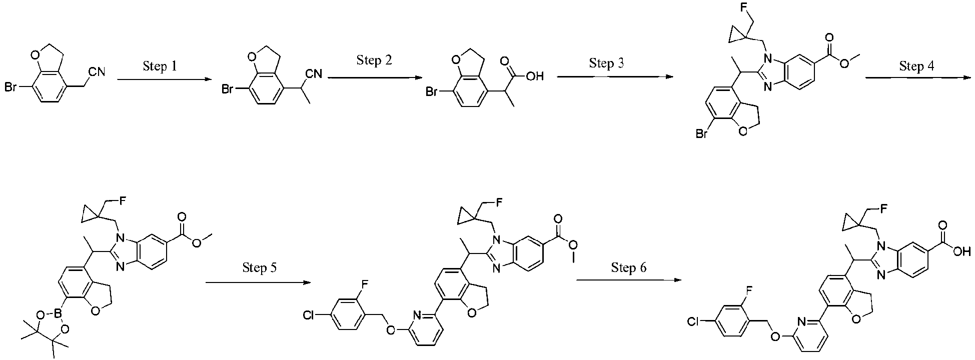

### Step 1: Synthesis of 2-(7-bromo-2,3-dihydrobenzofuran-4-yl)propionitrile

2-(7-Bromo-2,3-dihydrobenzofuran-4-yl)acetonitrile (300 mg, 1.27 mmol) and tetrahydrofuran (10 mL) were added into a 100 mL flask. Iodomethane (899 mg, 6.33 mmol) and cesium carbonate (2.1 g, 6.33 mmol) were added, and the resulting mixture reacted under stirring at room temperature. After the reaction was completed, the resulting mixture was added into water and extracted with ethyl acetate. The resulting organic phase was dried over anhydrous sodium sulfate, filtered and concentrated, and the resulting crude product was isolated and purified by thin layer chromatography (ethyl acetate: petroleum ether =1:15) to obtain 2-(7-bromo-2,3-dihydrobenzofuran-4-yl)propionitrile (200 mg).

### Step 2: Synthesis of 2-(7-bromo-2,3-dihydrobenzofuran-4-yl)propanoic acid

2-(7-Bromo-2,3-dihydrobenzofuran-4-yl)propionitrile (200 mg, 0.8 mmol) and ethanol (5 mL) were added into a 100 mL flask, potassium hydroxide (446 mg, 8 mmol) and water (10 mL) were added, and the resulting mixture was stirred at 100 °C. After the reaction was completed, the temperature was lowered, the resulting mixture solution was adjusted to be acidic with 1 N hydrochloric acid solution and extracted with ethyl acetate. The organic phase was dried over anhydrous sodium sulfate, filtered and concentrated, and the resulting crude product was isolated and purified by thin layer chromatography (ethyl acetate: petroleum ether =1:10) to obtain 2-(7-bromo-2,3-dihydrobenzofuran-4-yl)propionic acid (180 mg).

### Step 3: Synthesis of methyl 2-(1-(7-bromo-2,3-dihydrobenzofuran-4-yl)ethyl)-1-((1-(fluoromethyl)cyclopropyl)methyl)-1H-benzo[d]imidazole-6-carboxylate

2-(7-Bromo-2,3-dihydrobenzofuran-4-yl)propanoic acid (180 mg, 0.67 mmol) and N, N-dimethylformamide (10 mL) were added into a 100 mL flask. HATU (382 mg, 1 mmol) and triethylamine (202 mg, 2 mmol) were added, and the resulting mixture was stirred at room temperature for 10 minutes. Then methyl 4-amino-3-(((1-(fluoromethyl)cyclopropyl)methyl)amino)benzoate (1.2-D, 203 mg, 0.8 mmol) was added, and the resulting mixture reacted under stirring at room temperature. After the reaction was completed, the resulting mixture was added into water and extracted with ethyl acetate, and the resulting organic phase was dried over anhydrous sodium sulfate, filtered and concentrated. The resulting crude product and glacial acetic acid (10 mL) were added into a 100 mL flask, heated to 120 °C and reacted under stirring. After the reaction was completed, the temperature was lowered, and the resulting mixture was added to ethyl acetate and washed with water and saturated aqueous sodium bicarbonate solution. The resulting organic phase was dried over anhydrous sodium sulfate, filtered and then concentrated. The resulting crude product was isolated and purified by thin layer chromatography (methanol: dichloromethane =1:50) to obtain methyl 2-(1-(7-bromo-2,3-dihydrobenzofuran-4-yl)ethyl)-1-((1-(fluoromethyl)cyclopropyl)methyl)-1*H*-benzo[*d*]imidazole-6-carboxylate (240 mg), ESI-MS (m/z): 487.1[M+H]⁺.

### Step 4: Synthesis of methyl 1-((1-(fluoromethyl)cyclopropyl)methyl)-2-(1-(7-(4,4,5,5-tetramethyl-1,3,2-dioxaborolan-2-yl)-2,3-dihydrobenzofuran-4-yl)ethyl)-1H-benzo[d]imidazole-6-carboxylate

Methyl 2-(1-(7-bromo-2,3-dihydrobenzofuran-4-yl)ethyl)-1-((1-(fluoromethyl)cyclopropyl)methyl)-1*H-*benzo[d]imidazole-6-carboxylate (240 mg, 0.49 mmol), bis(pinacolato)diboron (752 mg, 2.96 mmol), Pd (dppf)Cl₂ (73 mg, 0.1 mmol), potassium acetate (115 mg, 1.47 mmol) and dioxane (10 mL) were added into a 100 mL flask and the resulting mixture was stirred under a nitrogen atmosphere at 100 °C. After the reaction was completed, the temperature was lowered and concentration was performed. The resulting crude product was isolated and purified by column chromatography (methanol: dichloromethane =1:30) to obtain methyl 1-((1-(fluoromethyl)cyclopropyl)methyl)-2-(1-(7-(4,4,5,5-tetramethyl-1,3,2-dioxaborolan-2-yl)-2,3-dihydrobenzofuran-4-yl)ethyl)-1*H*-benzo[*d*]imidazole-6-carboxylate (170 mg). ESI-MS (m/z): 535.3[M+H]⁺.

### Step 5: Synthesis of methyl 2-(1-(7-(6-((4-chloro-2-fluorobenzyl)oxy)pyridin-2-yl)-2,3-dihydrobenzofuran-4-yl)ethyl)-1-((1-(fluoromethyl)cyclopropyl)methyl)-1H-benzo[d]imidazole-6-carboxylate

Methyl 1-((1-(fluoromethyl)cyclopropyl)methyl)-2-(1-(7-(4,4,5,5-tetramethyl-1,3,2-dioxaborolan-2-yl)-2,3-dihydrobenzofuran-4-yl)ethyl)-1*H*-benzo[*d*]imidazole-6-carboxylate (70 mg, 0.13 mmol), 2-bromo-6-((4-chloro-2-fluorobenzyl)oxy)pyridine (41 mg, 0.13 mmol), Pd(dppf)Cl₂ (19 mg, 0.026mmol), potassium carbonate (54 mg, 0.39 mmol), dioxane (8 mL) and water (4 mL) were added into a 100 mL flask, and the resulting mixture was stirred under a nitrogen atmosphere at 80 °C. After the reaction was completed, the temperature was lowered, and the resulting mixture was added to water and extracted with ethyl acetate. The resulting organic phase was dried over anhydrous sodium sulfate, filtered and concentrated, and the resulting crude product was isolated and purified by thin layer chromatography (methanol: dichloromethane =1:50) to obtain methyl 2-(1-(7-(6-((4-chloro-2-fluorobenzyl)oxy)pyridin-2-yl)-2,3-dihydrobenzofuran-4-yl)ethyl)-1-((1-(fluoromethyl)cyclopropyl)methyl)-1*H-*benzo[d]imidazole-6-carboxylate (65 mg), ESI-MS (m/z): 644.2[M+H]⁺.

### Step 6: Synthesis of 2-(1-(7-(6-((4-chloro-2-fluorobenzyl)oxy)pyridin-2-yl)-2,3-dihydrobenzofuran-4-yl)ethyl)-1-((1-(fluoromethyl)cyclopropyl)methyl)-1H-benzo[d]imidazole-6-carboxylic acid

Methyl 2-(1-(7-(6-((4-chloro-2-fluorobenzyl)oxy)pyridin-2-yl)-2,3-dihydrobenzofuran-4-yl)ethyl)-1-((1-(fluoromethyl)cyclopropyl)methyl)-1*H*-benzo[*d*]imidazole-6-carboxylate (65 mg, 0.1 mmol) and tetrahydrofuran (4 mL) were added into a 100 mL flask. Methanol (4 mL), sodium hydroxide (40 mg, 1 mmol) and water (4 mL) were added, and the resulting mixture was heated to 50 °C and reacted under stirring. After the reaction was completed, the temperature was lowered, glacial acetic acid (0.1 mL) was added, and the resulting mixture was stirred for 10 minutes and then concentrated. The resulting crude product was isolated and purified by thin layer chromatography (methanol: dichloromethane=1:30) to obtain 2-(1-(7-(6-((4-chloro-2-fluorobenzyl)oxy)pyridin-2-yl)-2,3-dihydrobenzofuran-4-yl)ethyl)-1-((1-(fluoromethyl)cyclopropyl)methyl)-1*H*-benzo[d]imidazole-6-carboxylic acid (20 mg), ESI-MS (m/z): 630.2[M+H]⁺, ¹H NMR (600 MHz, DMSO-*d₆*) δ 12.65 (s, 1H), 8.19 (s, 1H), 7.89 (d, *J* = 8.2 Hz, 1H), 7.83 (d, *J* = 8.4 Hz, 1H), 7.77-7.73 (m, 3H), 7.57-7.54 (m, 1H), 7.46 (d, *J* = 9.9 Hz, 1H), 7.28 (d, *J* = 8.1 Hz, 1H), 6.76 (d, *J* = 7.4 Hz, 1H), 6.65 (d, *J* = 8.3 Hz, 1H), 5.43 (s, 2H), 4.71-4.67 (m, 2H), 4.57-4.53 (m, 1H), 4.30 (d, *J =* 15.4 Hz, 1H), 4.22 (d, *J* = 15.4 Hz, 1H), 4.18-4.03 (m, 2H), 3.29-3.25 (m, 1H), 3.20-3.16 (m, 1H), 1.73 (d, *J* = 6.6 Hz, 3H), 0.69-0.59 (m, 4H).

### Example 17: Synthesis of 2-(1-(7-(6-((4-cyano-2-fluorobenzyl)oxy)pyridin-2-yl)-2,3-dihydrobenzofuran-4-yl)ethyl)-1-((1-(fluoromethyl)cyclopropyl)methyl)-1H-benzo[d]imidazole-6-carboxylic acid (Compound 17)

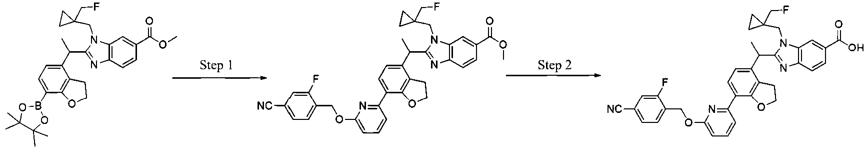

2-(1-(7-(6-((4-Cyano-2-fluorobenzyl)oxy)pyridin-2-yl)-2,3-dihydrobenzofuran-4-yl)ethyl)-1-((1-(fluoromethyl)cyclopropyl)methyl)-1*H*-benzo[d]imidazole-6-carboxylic acid (21 mg) was synthesized by referring to the synthetic method of Compound 16 using 4-(((6-bromopyridin-2-yl)oxy)methyl)-3-fluorobenzonitrile as the starting material. ESI-MS (m/z): 621.2[M+H]⁺, ¹H NMR (600 MHz, DMSO-*d₆*) δ 12.76 (s, 1H), 8.19 (d, *J* = 1.2 Hz, 1H), 7.88 (d, *J* = 9.0 Hz, 1H), 7.83-7.82 (m, 2H), 7.79-7.73 (m, 3H), 7.72-7.67 (m, 2H), 6.81 (dd, *J* = 7.9, 0.7 Hz, 1H), 6.64 (d, *J* = 8.3 Hz, 1H), 5.53 (s, 2H), 4.70-4.67 (m, 2H), 4.55 (q, *J* = 6.9 Hz, 1H), 4.30 (d, *J* = 15.4 Hz, 1H), 4.22 (d, *J* = 15.4 Hz, 1H), 4.18 (d, *J* = 10.2 Hz, 0.5H), 4.12-4.08 (m, 1H), 4.04 (d, *J* = 10.2 Hz, 0.5H), 3.28-3.24 (m, 1H), 3.19-3.15 (m, 1H), 1.73 (d, *J* = 7.2 Hz, 3H), 0.69-0.59 (m, 4H).

### Example 18: Synthesis of 2-((5-chloro-7-(6-((4-cyano-2-fluorobenzyl)oxy)pyridin-2-yl)-2,3-dihydrobenzofuran-4-yl)methyl)-1-((1-(cyanomethyl)cyclopropyl)methyl)-1H-benzo[d]imidazole-6-carboxylic acid (Compound 18)

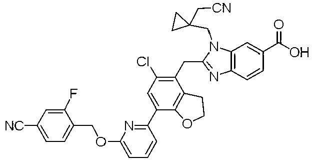

2-((5-Chloro-7-(6-((4-cyano-2-fluorobenzyl)oxy)pyridin-2-yl)-2, 3-dihydrobenzofuran-4-yl)methyl)-1-((1-(cyanomethyl)cyclopropyl)methyl)-1*H*-benzo[*d*]imidazole-6-carboxylic acid (23 mg) was obtained by referring to the synthetic method of Compound 14 using Intermediates 1.1-E and 1.2-C as the starting materials. ESI-MS (m/z): 648.2[M+H]⁺, ¹H NMR (600 MHz, DMSO-*d₆*) δ 12.78 (s, 1H), 8.26 (d, *J =* 1.2 Hz, 1H), 7.92-7.89 (m, 1H), 7.85-7.82 (m, 2H), 7.81-7.89 (m, 1H), 7.77 (dd, *J =* 8.4, 1.5 Hz, 1H), 7.72-7.70 (m, 2H), 7.56 *(d, J =* 8.4 Hz, 1H), 6.90 (dd, *J* = 8.1 Hz, 0.6 Hz, 1H), 5.59 (s, 2H), 4.73 (t, *J* = 8.8 Hz, 2H), 4.59 (s, 2H), 4.43 (s, 2H), 3.22 (t, *J* = 8.8 Hz, 2H), 2.69 (s, 2H), 0.78-0.77 (m, 2H), 0.75-0.73 (m, 2H).

### Example 19: Synthesis of 2-((5-chloro-7-(6-((4-cyano-2-fluorobenzyl)oxy)pyridin-2-yl)-2,3-dihydrobenzofuran-4-yl)methyl)-3-((1-(cyanomethyl)cyclopropyl)methyl)-3H-imidazo[4,5-b]pyridine-5-carboxylic acid (Compound 19)

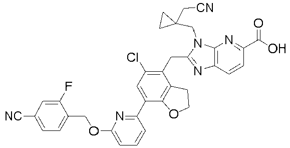

2-((5-Chloro-7-(6-((4-cyano-2-fluorobenzyl)oxy)pyridin-2-yl)-2,3-dihydrobenzofuran-4-yl)methyl)-3-((1-(cyanomethyl)cyclopropyl)methyl)-3*H*-imidazo[4,5-*b*]pyridine-5-carboxylic acid (42 mg) was obtained by referring to the synthetic method of Compound 14 using Intermediates 1.1-E and 1.2-E as the starting materials. ESI-MS (m/z): 649.2[M+H]⁺, ¹H NMR (600 MHz, DMSO-*d₆*) δ 12.97 (s, 1H), 8.04 (d, *J* = 8.2 Hz, 1H), 7.95 (d, *J* = 8.2 Hz, 1H), 7.91 (d, *J* = 9.4 Hz, 1H), 7.85-7.83 (m, 2H), 7.80 (d, *J* = 7.1 Hz, 1H), 7.74-7.70 (m, 2H), 6.92-6.89 (m, 1H), 5.59 (s, 2H), 4.74 (t, *J* = 8.8 Hz, 2H), 4.55 (s, 2H), 4.50 (s, 2H), 3.24 (t, *J* = 8.8 Hz, 2H), 2.80 (s, 2H), 1.10 (t, *J =* 5.3 Hz, 2H), 0.72 (t, *J* = 5.4 Hz, 2H).

### Example 20: Synthesis of (S)-2-((7-(6-((4-chloro-2-fluorobenzyl)oxy)pyridin-2-yl)-1-oxo-2,3-dihydro-1H-inden-4-yl)methyl)-1-(oxetan-2-ylmethyl)-1H-benzo[d]imidazole-6-carboxylic acid (Compound 20)

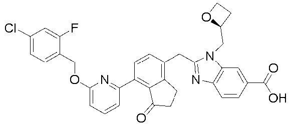

(S)-2-((7-(6-((4-chloro-2-fluorobenzyl)oxy)pyridin-2-yl)-1-oxo-2,3-dihydro-1H-inden-4-yl)methyl)-1-(oxetan-2-ylmethyl)-1H-benzo[d]imidazole-6-carboxylic acid (42 mg) was obtained by referring to the synthetic method of Compound 14 using 2-(7-bromo-1-oxo-2,3-dihydro-1H-inden-4-yl)acetic acid and Intermediate 1.2-A as the starting materials. ESI-MS (m/z): 610.2[M-H]⁻, ¹H NMR (600 MHz, CDCl₃) δ 8.11 - 8.05 (m, 2H), 7.88 (d, *J* = 8.5 Hz, 1H), 7.59 (t, *J* = 7.8 Hz, 1H), 7.52 (d, *J =* 7.7 Hz, 1H), 7.45 - 7.40 (m, 2H), 7.19 (d, *J* = 7.4 Hz, 1H), 7.11 (dd, *J =* 8.2, 2.3 Hz, 1H), 7.09 - 7.05 (m, 1H), 6.75 (d, *J* = 8.2 Hz, 1H), 5.35 (s, 2H), 5.21 - 5.14 (m, 1H), 4.74 - 4.60 (m, 3H), 4.43 - 4.35 (m, 2H), 4.31 (d, *J* = 15.3 Hz, 1H), 3.14 - 3.09 (m, 2H), 2.74 - 2.72 (m, 2H), 2.44 - 2.34 (m, 2H).

### Example 21: (S)-4-methoxy-2-((7-(6-((2-methoxy-4-(trifluoromethyl)benzyl)oxy)pyridin-2-yl)benzo[d][1,3]dioxolan-4-yl)methyl)-1-(oxetan-2-ylmethyl)-1H-benzo[d]imidazole-6-carboxylic acid (Compound 42)

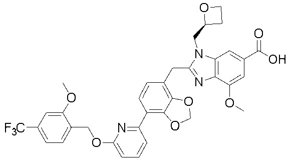

(S)-4-methoxy-2-((7-(6-((2-methoxy-4-(trifluoromethyl)benzyl)oxy)pyridin-2-yl)benzo[d][1,3]dioxolan-4-yl)methyl)-1-(oxetan-2-ylmethyl)-1*H*-benzo[*d*]imidazole-6-carboxylic acid (15 mg) was obtained by referring to the synthetic method of Compound 1 using Intermediates 1.1-A and 1.2-F as the starting materials. ESI-MS (m/z): 678.2[M+H]⁺, ¹H NMR (600 MHz, DMSO-*d₆*) δ 12.79 (s, 1H), 7.86 (s, 1H), 7.84-7.81 (m, 1H), 7.64 (d, *J =* 7.4 Hz, 1H), 7.61-7.58 (m, 2H), 7.33-7.30 (m, 2H), 7.25 (s, 1H), 6.85 (dd, *J* = 15.6, 7.1 Hz, 1H), 6.17-6.16 (m, 2H), 5.50 (s, 2H), 4.99-4.95 (m, 1H), 4.63 (dd, *J* = 19.6, 8.3 Hz 1H), 4.51-4.48 (m, 1H), 4.46-4.44 (m, 1H), 4.38 (d, *J* = 16.3 Hz, 1H), 4.34-4.32 (m, 1H), 4.30 (d, *J* = 16.5 Hz, 1H), 3.92 (s, 3H), 3.91 (s, 3H), 2.66-2.63 (m, 1H), 2.35-2.31 (m, 1H).

### Example 22: (S)-4-methoxy-2-((8-(6-((2-methoxy-4-(trifluoromethyl)benzyl)oxy)pyridin-2-yl)-2,3-dihydrobenzo[b][1,4]dioxan-5-yl)methyl)-1-(oxetan-2-ylmethyl)-1H-benzo[d]imidazole-6-carboxylic acid (Compound 62)

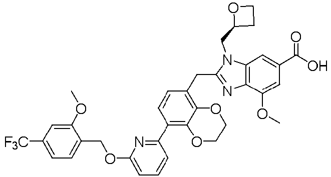

Compound (S)-4-methoxy-2-((8-(6-((2-methoxy-4-(trifluoromethyl)benzyl)oxy)pyridin-2-yl)-2,3-dihydrobenzo[b][1,4]dioxan-5-yl)methyl)-1-(oxetan-2-ylmethyl)-1*H*-benzo[*d*]imidazole-6-carboxylic acid (18 mg) was obtained by referring to the synthetic method of Compound 1 using Intermediates 1.1-G and 1.2-F as the starting materials. ESI-MS (m/z): 692.2[M+H]⁺, ¹H NMR (600 MHz, DMSO-*d₆*) δ 12.76 (s, 1H), 7.86 (s, 1H), 7.77-7.75 (m, 1H), 7.60 (d, *J* = 7.6 Hz, 1H), 7.52 (d, *J =* 7.5 Hz, 1H), 7.32-7.30 (m, 2H), 7.26-7.24 (m, 2H), 6.84 (d, *J =* 8.2 Hz, 1H), 6.72 (d, *J* = 8.1 Hz, 1H), 5.44 (s, 2H), 4.99-4.95 (m, 1H), 4.60 (dd, *J* = 15.4, 7.0 Hz, 1H), 4.49-4.45 (m, 2H), 4.36-4.31 (m, 6H), 4.22 (d, *J =* 16.2 Hz, 1H), 3.92 (s, 3H), 3.90 (s, 3H), 2.69-2.65 (m, 1H), 2.38-2.33 (m, 1H).

### Example 23: 2-((7-(6-((4-Chloro-2-fluorobenzyl)oxy)pyridin-2-yl)-1-hydroxy-2,3-dihydro-1H-inden-4-yl)methyl)-1-(((S)-oxetan-2-yl)methyl)-1H-benzo[d]imidazole-6-carboxylic acid (Compound 63)

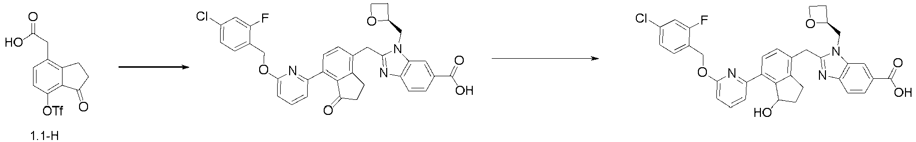

Intermediate 2-((7-(6-((4-chloro-2-fluorobenzyl)oxy)pyridin-2-yl)-1-oxo-2,3-dihydro-1H-inden-4-yl)methyl)-1-(((S)-oxetan-2-yl)methyl)-1H-benzo[d]imidazole-6-carboxylic acid was obtained by referring to Steps 2, 3 and 4 of Example 9 using Intermediate 1.1-H as the staring material. The product obtained from the previous step (13 mg, 0.02 mmol), methanol (10 mL), and sodium borohydride (3 mg, 0.08 mmol) were then added into a 50 mL eggplant flask and stirred at room temperature for 1 hour. After LC-MS detected the completion of reaction, ethyl acetate was added, and the resulting mixture was washed with saturated brine. The resulting organic phase was dried over anhydrous sodium sulfate, concentrated and purified by column chromatography (dichloromethane: methanol=15:1) to obtain 2-((7-(6-((4-chloro-2-fluorobenzyl)oxy)pyridin-2-yl)-1-hydroxy-2,3-dihydro-1H-inden-4-yl)methyl)-1-(((S)-oxetan-2-yl)methyl)-1H-benzo[d]imidazole-6-carboxylic acid (9 mg), ESI-MS (m/z): 614. 1 [M+H]⁺; ¹H NMR (600 MHz, CDCl₃) δ 8.13 *(d, J=* 6.0 Hz, 1H), 8.06 (d, *J* = 8.4 Hz, 1H), 7.87 (d, *J* = 8.5 Hz, 1H), 7.72 (t, *J* = 7.9 Hz, 1H), 7.41 (q, *J* = 8.1 Hz, 2H), 7.27 (s, 1H), 7.19 - 7.07 (m, 3H), 6.81 (d, *J* = 8.3 Hz, 1H), 5.39 - 5.36 (m, 2H), 5.30 (s, 1H), 5.15 - 5.06 (m, 1H), 4.70 - 4.54 (m, 3H), 4.42 - 4.33 (m, 2H), 4.30 - 4.24 (m, 1H), 3.25 - 3.13 (m, 1H), 3.08 - 2.94 (m, 1H), 2.73 - 2.66 (m, 1H), 2.41 - 2.31 (m, 2H), 2.29 - 2.23 (m, 1H).

### Example 24: 2-((7-(6-((4-Chloro-2-fluorobenzyl)oxy)pyridin-2-yl)-1-fluoro-2,3-dihydro-1H-inden-4-yl)methyl)-1-(((S)-oxetan-2-yl)methyl)-1H-benzo[d]imidazole-6-carboxylic acid (Compound 65)

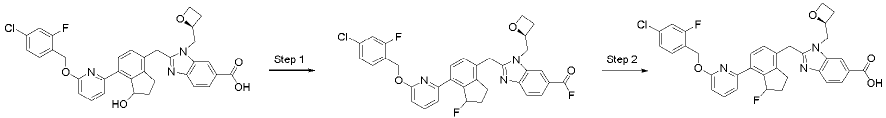

### Step 1: Synthesis of 2-((7-((6-((4-chloro-2-fluorobenzyl)oxy)pyridin-2-yl)-1-fluoro-2,3-dihydro-1H-inden-4-yl)methyl)-1-((S)-oxetan-2-yl)methyl)-1H-benzo[d]imidazole-6-carbonyl fluoride

2-((7-((6-((4-Chloro-2-fluorobenzyl)oxy)pyridin-2-yl)-1-hydroxy-2,3-dihydro-1*H*-inden-4-yl)methyl)-1-((*S*)-oxetan-2-yl)methyl)-1*H*-benzo[d]imidazole-6-carboxylic acid (2 mg, 3.25 µmol) was dissolved in anhydrous dichloromethane (1.5 mL) in a 10 mL two-necked flask, and cooled to -78 °C under the protection of nitrogen. Diethylaminosulfur trifluoride (100 µL) was added dropwise. The resulting mixture was stirred at -78 °C for 1 hour, then slowly warmed to room temperature, stirred for 1 hour, quenched with water, extracted with dichloromethane, washed with sodium bicarbonate, washed with water, washed with saturated sodium chloride, dried with anhydrous sodium sulfate, filtered and spin-dried to obtain the target product 2-((7-(((6-((4-chloro-2-fluorobenzyl)oxy)pyridin-2-yl)-1-fluoro-2,3-dihydro-1*H*-inden-4-yl)methyl)-1-((S)-oxetan-2-yl)methyl)-1*H*-benzo[*d*]imidazole-6-carbonyl fluoride (2.0 mg), ESI-MS (m/z): 618.2[M+H]⁺.

### Step 2: Synthesis of 2-((7-(6-((4-chloro-2-fluorobenzyl)oxy)pyridin-2-yl)-1-fluoro-2,3-dihydro-1H-inden-4-yl)methyl)-1-(((S)-oxetan-2-yl)methyl)-1H-benzo[d]imidazole-6-carboxylic acid

2-((7-((6-((4-Chloro-2-fluorobenzyl)oxy)pyridin-2-yl)-1-fluoro-2,3-dihydro-1*H*-inden-4-yl)methyl)-1-((S)-oxetan-2-yl)methyl)-1*H*-benzo[*d*]imidazole-6-carbonyl fluoride (2.01 mg, 3.25 µmol), tetrahydrofuran (2 mL) and methanol (2 mL) were added into a 25 mL round bottom flask, sodium hydroxide (65 mg, 1.63 mmol) dissolved in water (2 ml) was added, and the resulting mixture reacted at room temperature for 1 hour. After the reaction was completed, water was added, the resulting mixture was adjusted to pH=1-2 with dilute hydrochloric acid in an ice bath, extracted with ethyl acetate, and washed with saturated sodium chloride. The solvent was removed under reduced pressure, and the resulting crude product was subjected to thin layer preparative chromatography (dichloromethane: methanol =15:1) to obtain the target product 2-((7-(6-((4-chloro-2-fluorobenzyl)oxy)pyridin-2-yl)-1-fluoro-2,3-dihydro-1H-inden-4-yl)methyl)-1-(((S)-oxetan-2-yl)methyl)-1H-benzo[d]imidazole-6-carboxylic acid (1.5 mg), ESI-MS (m/z): 616.2[M+H]⁺.

### Example 25: (S)-2-((5-fluoro-7-(6-((2-methoxy-4-(trifluoromethyl)benzyl)oxy)pyridin-2-yl)-2,3-dihydrobenzofuran-4-yl)methyl)-1-(oxetan-2-ylmethyl)-1H-benzo[d]imidazole-6-carboxylic acid (Compound 71)

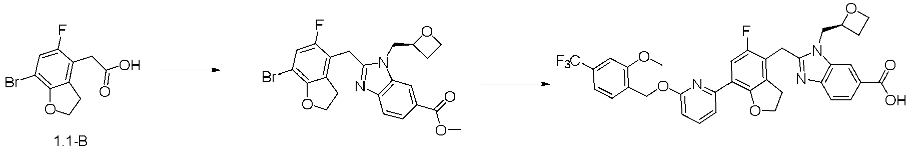

Intermediate methyl (S)-2-((7-bromo-5-fluoro-2,3-dihydrobenzofuran-4-yl)methyl)-1-(oxetan-2-ylmethyl)-1H-benzo[d]imidazole-6-carboxylate was synthesized by referring to Step 1 of Example 1 using Intermediate 1.1-B as the starting material, and then (S)-2-((5-fluoro-7-(6-((2-methoxy-4-(trifluoromethyl)benzyl)oxy)pyridin-2-yl)-2,3-dihydrobenzofuran-4-yl)methyl)-1-(oxetan-2-ylmethyl)-1*H*-benzo[*d*]imidazole-6-carboxylic acid (32 mg) was obtained by referring to Steps 2, 3, and 4 of Example 9. ESI-MS (m/z): 664.2[M+H]⁺, ¹H NMR (600 MHz, DMSO-*d₆*) δ 12.75 (s, 1H), 8.23 (d, *J* = 0.9 Hz, 1H), 7.83-7.79 (m, 2H), 7.77 (dd, *J* = 8.4, 1.5 Hz, 1H), 7.63 (d, *J =* 11.6 Hz, 1H), 7.59 (d, *J* = 7.8 Hz, 1H), 7.57 (d, *J* = 8.5 Hz, 1H), 7.33-7.30 (m, 2H), 6.87 (dd, *J* = 7.2, 1.8 Hz, 1H), 5.52 (s, 2H), 5.08-5.04 (m, 1H), 4.76-4.72 (m, 1H), 4.67 (t, *J* = 8.8 Hz, 2H), 4.61 (dd, *J* = 15.5, 2.3 Hz, 1H), 4.52-4.45 (m, 2H), 4.39-4.33 (m, 2H), 3.94 (s, 3H), 3.15-3.08 (m, 2H), 2.74-2.68 (m, 1H), 2.41-2.35 (m, 1H).

### Example 26: 4-(2,2-Difluoro-ethoxy)-2-((5-fluoro-7-(6-(2-methoxy-4-(trifluoromethyl)benzyl)oxy) pyridine-2-yl)-2,3-dihydrobenzofuran-4-yl)methyl)-1-((1-(fluoromethyl)cyclopropyl)methyl)-1H-benzo[d]imidazole-6-carboxylic acid (Compound 79)

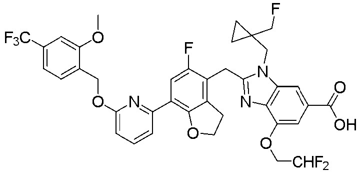

4-(2,2-Difluoro-ethoxy)-2-((5-fluoro-7-(6-(2-methoxy-4-(trifluoromethyl)benzyl)oxy)pyridin-2-yl)-2,3-dihydrobenzofuran-4-yl)methyl)-1-((1-(fluoromethyl)cyclopropyl)methyl)-1H-benzo[d]imidazole-6-carboxylic acid (23 mg) was obtained by referring to the synthetic method of Compound 71 using Intermediates 1.1-B, 1.5-D and 1.2-O as the staring materials. ESI-MS(m/z): 760.2[M+H]⁺, ¹H NMR (600 MHz, DMSO-*d₆*) δ 12.89 (brs, 1H), 7.99 (s, 1H), 7.84 - 7.78 (m, 2H), 7.65 (d, J = 11.5 Hz, 1H), 7.59 (d, J = 7.5 Hz, 1H), 7.35 - 7.28 (m, 3H), 6.87 (d, J = 4.2 Hz, 1H), 6.41 (t, J = 54.9 Hz, 1H), 5.52 (s, 2H), 4.83 (d, J = 24.1 Hz, 2H), 4.67 (t, J = 8.6 Hz, 2H), 4.53 (t, J = 13.3 Hz, 2H), 4.34 (s, 2H), 3.94 (s, 3H), 3.09 (t, J = 8.4 Hz, 2H), 2.29 - 2.19 (m, 4H), 1.93 - 1.83 (m, 2H).

### Example 27: (S)-2-((5-fluoro-7-(6-((2-methoxy-4-(trifluoromethyl)benzyl)oxy)pyridin-2-yl)-2,3-dihydrobenzofuran-4-yl)methyl)-4-methoxy-1-(oxetan-2-ylmethyl)-1H-benzo[d]imidazole-6-carboxylic acid (Compound 80)

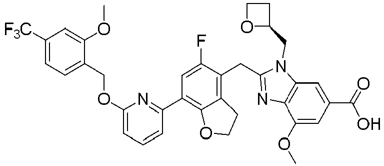

(S)-2-((5-fluoro-7-(6-((2-methoxy-4-(trifluoromethyl)benzyl)oxy)pyridin-2-yl)-2,3-dihydrobenzofuran-4-yl)methyl)-4-methoxy-1-(oxetan-2-ylmethyl)-1*H*-benzo[*d*]imidazole-6-carboxylic acid (30 mg) was obtained by referring to the synthetic method of Compound 71 using Intermediates 1.1-B, 1.5-D and 1.2-F as the staring materials. ESI-MS (m/z): 694.2[M+H]⁺, ¹H NMR (600 MHz, DMSO-*d₆*) δ 12.78 (s, 1H), 7.86 (s, 1H), 7.83-7.81 (m, 2H), 7.64 (d, *J =* 11.7 Hz, 1H), 7.60 (d, *J* = 7.7 Hz, 1H), 7.32-7.31 (m, 2H), 7.24 (s, 1H), 6.87 (dd, *J =* 6.6, 1.8 Hz, 1H), 5.53 (s, 2H), 5.06-5.02 (m, 1H), 4.71-4.66 (m, 3H), 4.58-4.55 (m, 1H), 4.51-4.47 (m, 1H), 4.43 (d, *J* = 17.0 Hz, 1H), 4.35-4.31 (m, 2H), 3.95 (s, 3H), 3.88 (s, 3H), 3.11-3.07 (m, 2H), 2.72-2.66 (m, 1H), 2.38-2.33 (m, 1H).

### Example 28: (S)-2-((7-(6-((4-chloro-2-fluorobenzyl)oxy)pyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 81)

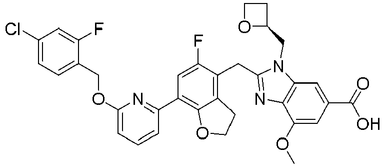

(S)-2-((7-(6-((4-chloro-2-fluorobenzyl)oxy)pyridin-2-yl)-5-fluoro-2,3-dihydrobenzofuran-4-yl)methyl)-4-methoxy-1-(oxetan-2-ylmethyl)-1*H*-benzo[*d*]imidazole-6-carboxylic acid (68 mg) was obtained by referring to the synthetic method of Compound 71 using Intermediates 1.1-B, 1.5-B and 1.2-F as the starting materials. ESI-MS (m/z): 648.2[M+H]⁺, ¹H NMR (600 MHz, DMSO-*d₆*) δ 12.80 (s, 1H), 7.87 (s, 1H), 7.83-7.79 (m, 2H), 7.75 (d, *J* = 11.5 Hz, 1H), 7.62-7.59 (m, 1H), 7.49 (d, *J* = 9.9 Hz, 1H), 7.32 (d, *J =* 8.3 Hz, 1H), 7.24 (s, 1H), 6.83 (dd, *J* = 6.2, 2.6 Hz, 1H), 5.51 (s, 2H), 5.07-5.04 (m, 1H), 4.72-4.67 (m, 3H), 4.59-4.56 (m, 1H), 4.51-4.48 (m, 1H), 4.45 (d, *J* = 16.9 Hz, 1H), 4.37-4.32 (m, 2H), 3.88 (s, 3H), 3.13-3.08 (m, 2H), 2.72-2.67 (m, 1H), 2.38-2.35 (m, 1H).

### Example 29: 2-((7-(6-((4-Chloro-2-(difluoromethoxy)benzyl)oxy)pyridin-2-yl)-5-fluoro-2,3-dihydrobenzofuran-4-yl)methyl)-4-(2,2-difluoroethoxy)-1-((1-(fluoromethyl)cyclopropyl)methyl)-1H-benzo[d]imidazole-6-carboxylic acid (Compound 82)

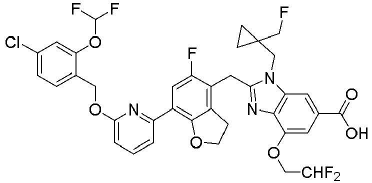

2-((7-(6-((4-Chloro-2-(difluoromethoxy)benzyl)oxy)pyridin-2-yl)-5-fluoro-2,3-dihydrobenzofuran-4-yl)methyl)-4-(2,2-diffuoroethoxy)-1-((1-(fluoromethyl)cyclopropyl)methyl)-1*H*-benzo[*d*]imidazole-6-carboxylic acid (26 mg) was obtained by referring to the synthetic method of Compound 71 using Intermediates 1.1-B, 1.5-E and 1.2-O as the starting materials. ESI-MS(m/z): 762.2[M+H]⁺, ¹H NMR (600 MHz, DMSO-*d₆*) δ 12.88 (brs, 1H), 7.99 (s, 1H), 7.83 - 7.80 (m, 2H), 7.72 (d, J = 11.6 Hz, 1H), 7.59 (d, J = 8.3 Hz, 1H), 7.39 (s, 1H), 7.36 (dd, J = 8.3, 1.8 Hz, 1H), 7.34 (s, 1H), 7.31 (s, 1H), 6.84 (dd, J = 6.3, 2.5 Hz, 1H), 6.42 (t, J = 54.6 Hz, 1H), 5.48 (s, 2H), 4.86 (s, 1H), 4.861(s, 1H), 4.68 (t, J = 8.8 Hz, 2H), 4.54 (td, J = 14.5, 3.4 Hz, 2H), 4.35 (s, 2H), 3.11 (t, J = 8.7 Hz, 2H), 2.29 - 2.19 (m, 4H), 1.92 - 1.83 (m, 2H).

### Example 30: 2-((5-Fluoro-7-(6-((2-methoxy-4-(trifluoromethyl)benzyl)oxy)pyridin-2-yl)-2,3-dihydrobenzofuran-4-yl)methyl)-1-((1-(fluoromethyl)cyclopropyl)methyl)-1H-benzo[d]imidazole-6-carboxylic acid (Compound 83)

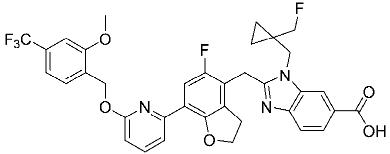

2-((5-Fluoro-7-(6-((2-methoxy-4-(trifluoromethyl)benzyl)oxy)pyridin-2-yl)-2,3-dihydrobenzofuran-4-yl)methyl)-1-((1-(fluoromethyl)cyclopropyl)methyl)-1*H*-benzo[*d*]imidazole-6-carboxylic acid (32 mg) was obtained by referring to the synthetic method of Compound 71 using Intermediates 1.1-B, 1.5-D and 1.2-D as the starting materials. ESI-MS (m/z): 680.2[M+H]⁺, ¹H NMR (600 MHz, DMSO-*d₆*) δ 12.78 (s, 1H), 8.28 (s, 1H), 7.82-7.75 (m, 3H), 7.63 (d, *J* = 11.5 Hz, 1H), 7.59 (d, *J* = 7.7 Hz, 1H), 7.56 (d, *J* = 8.5 Hz, 1H), 7.33-7.29 (m, 2H), 6.87 (d, *J* = 6.8 Hz, 1H), 5.52 (s, 2H), 5.21-5.19 (m, 2H), 4.88-4.84 (m, 2H), 4.68 (t, *J* = 8.6 Hz, 2H), 4.34 (s, 2H), 3.94 (s, 3H), 3.12 (t, *J* = 8.6 Hz, 2H), 2.02-1.93 (m, 2H), 1.92-1.84 (m, 2H).

### Example 31: (S)-2-((7-(6-((4-chloro-2-(difluoromethoxy)benzyl)oxy)pyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 84)

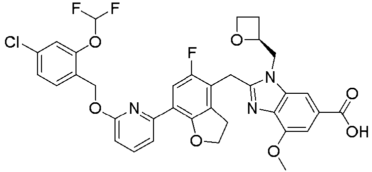

2-((7-(6-((4-Chloro-2-(difluoromethoxy)benzyl)oxy)pyridin-2-yl)-5-fluoro-benzo[*d*][1,3]dioxolan-4-yl)methyl)-4-(2,2-difluoroethoxy)-1-((1-(fluoromethyl)cyclopropyl)methyl)-1H-benzo[d]imidazole-6-carboxylic acid (19 mg) was obtained by referring to the synthetic method of Compound 71 using Intermediates 1.1-G, 1.5-E and 1.2-O as the starting materials. ESI-MS(m/z): 764.1[M+H]⁺, ¹H NMR (600 MHz, DMSO-*d₆*) δ 12.90 (brs, 1H), 7.99 (s, 1H), 7.86 (t, *J =* 7.9 Hz, 1H), 7.69 (d, *J* = 7.4 Hz, 1H), 7.60 (d, *J* = 8.3 Hz, 1H), 7.45 (d, *J* = 11.9 Hz, 1H), 7.39 (s, 1H), 7.36 (dd, *J* = 8.2, 1.9 Hz, 1H), 7.34 (t, *J =* 73 Hz, 1H), 7.30 (s, 1H), 6.89 (d, *J* = 8.2 Hz, 1H), 6.42 (tt, *J =* 54.6, 3.5 Hz, 1H), 6.20 (s, 2H), 5.49 (s, 2H), 4.85 (d, *J* = 24.3 Hz, 2H), 4.52 (td, *J* = 14.5, 3.5 Hz, 2H), 4.32 (s, 2H), 2.28-2.16 (m, 4H), 1.92 - 1.83 (m, 2H).

### Example 32: 2-((7-(6-((4-Chloro-2-(difluoromethoxy)benzyl)oxy)pyridin-2-yl)-5-fluoro-benzo[d][1,3]dioxolan-4-yl)methyl)-4-(2,2-difluoroethoxy)-1-((1-(fluoromethyl)cyclopropyl)methyl)-1H-benzo[d]imidazole-6-carboxylic acid (Compound 86)

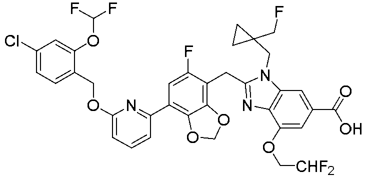

2-((7-(6-((4-Chloro-2-(difluoromethoxy)benzyl)oxy)pyridin-2-yl)-5-fluoro-benzo[*d*][1,3]dioxolan-4-yl)methyl)-4-(2,2-difluoroethoxy)-1-((1-(fluoromethyl)cyclopropyl)methyl)-1*H*-benzo[*d*]imidazole-6-carboxylic acid (19 mg) was obtained by referring to the synthetic method of Compound 71 using Intermediates 1.1-G, 1.5-E and 1.2-O as the starting materials. ESI-MS(m/z): 764.1[M+H]⁺, ¹H NMR (600 MHz, DMSO-*d₆*) δ 12.90 (brs, 1H), 7.99 (s, 1H), 7.86 (t, J = 7.9 Hz, 1H), 7.69 (d, J = 7.4 Hz, 1H), 7.60 (d, J = 8.3 Hz, 1H), 7.45 (d, J = 11.9 Hz, 1H), 7.39 (s, 1H), 7.36 (dd, J = 8.2, 1.9 Hz, 1H), 7.34 (t,J = 73 Hz, 1H), 7.30 (s, 1H), 6.89 (d, J = 8.2 Hz, 1H), 6.42 (tt, J = 54.6, 3.5 Hz, 1H), 6.20 (s, 2H), 5.49 (s, 2H), 4.85 (d, J = 24.3 Hz, 2H), 4.52 (td, J = 14.5, 3.5 Hz, 2H), 4.32 (s, 2H), 2.28-2.16 (m, 4H), 1.92 - 1.83 (m, 2H).

### Example 33: 2-((7-(6-((4-Chloro-2-(difluoromethoxy)benzyl)oxy)pyridin-2-yl)-5-fluoro-2,3-dihydrobenzofuran-4-yl)methyl)-1-((1-(fluoromethyl)cyclopropyl)methyl)-4-methoxy-1H-benzo[d]imidazole -6-carboxylic acid (Compound 87)

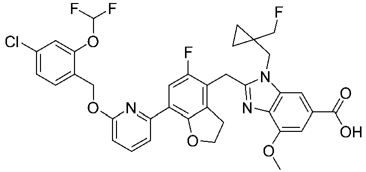

2-((7-(6-((4-Chloro-2-(difluoromethoxy)benzyl)oxy)pyridin-2-yl)-5-fluoro-2,3-dihydrobenzofuran-4-yl)methyl)-1-((1-(fluoromethyl)cyclopropyl)methyl)-4-methoxy-1*H*-benzo[*d*]imidazole-6-carboxylic acid (42 mg) was obtained by referring to the synthetic method of Compound 71 using Intermediates 1.1-B, 1.5-E and 1.2-J as the starting materials. ESI-MS (m/z): 712.2[M+H]⁺, ¹H NMR (600 MHz, DMSO-*d₆*) δ 12.82 (s, 1H), 7.92 (s, 1H), 7.82-7.80 (m, 2H), 7.71 (d, *J* = 11.7 Hz, 1H), 7.60 (d, *J* = 8.2 Hz, 1H), 7.39-7.35 (m, 2H), 7.34 (t, *J* = 73.5 Hz, 1H), 7.24 (s, 1H), 6.86-6.83 (m, 1H), 5.49 (s, 2H), 4.82 (d, *J* = 24.2 Hz, 2H), 4.69 (t, *J* = 8.8 Hz, 2H), 4.31 (s, 2H), 3.87 (s, 3H), 3.80-3.70 (m, 1H), 3.60-3.56 (m, 1H), 3.10 (t, *J* = 8.7 Hz, 2H), 2.26-2.21 (m, 4H).

### Example 34: 2-((5-Fluoro-7-(6-((2-methoxy-4-(trifluoromethyl)benzyl)oxy)pyridin-2-yl)-2,3-dihydrobenzofuran-4-yl)methyl)-1-((1-(fluoromethyl)cyclopropyl)methyl)-4-methoxy-1H-benzo[d]imidazole-6-carboxylic acid (Compound 89)

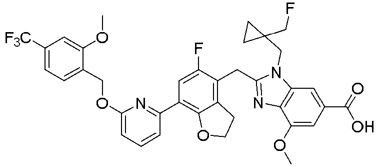

2-((5-Fluoro-7-(6-((2-methoxy-4-(trifluoromethyl)benzyl)oxy)pyridin-2-yl)-2,3-dihydrobenzofuran-4-yl)methyl)-1-((1-(fluoromethyl)cyclopropyl)methyl)-4-methoxy-1*H-*benzo[*d*]imidazole-6-carboxylic acid (33 mg) was obtained by referring to the synthetic method of Compound 71 using Intermediates 1.1-B, 1.5-D and 1.2-J as the starting materials. ESI-MS (m/z): 710.2[M+H]⁺, ¹H NMR (600 MHz, DMSO-*d₆*) δ 12.82 (s, 1H), 7.92 (s, 1H), 7.83-7.80 (m, 2H), 7.63 (d, *J =* 11.6 Hz, 1H), 7.60 (d, *J =* 7.7 Hz, 1H), 7.32-7.30 (m, 2H), 7.24 (s, 1H), 6.87 (dd, *J* = 6.7, 2.1 Hz, 1H), 5.52 (s, 2H), 4.82 (d, *J* = 24.2 Hz, 2H), 4.68 (t, *J* = 8.8 Hz, 2H), 4.31 (s, 2H), 3.94 (s, 3H), 3.87 (s, 3H), 3.780-3.72(m, 1H), 3.65-3.56 (m, 1H), 3.09 (t, *J =* 8.7 Hz, 2H), 2.26-2.21 (m, 4H).

### Example 35: 2-((5-Fluoro-7-(6-((2-methoxy-4-(trifluoromethyl)benzyl)oxy)pyridin-2-yl)-2,3-dihydrobenzofuran-4-yl)methyl)-3-((1-(fluoromethyl)cyclopropyl)methyl)-3H-imidazo [4,5-b]pyridine-5-carboxylic acid (Compound 90)

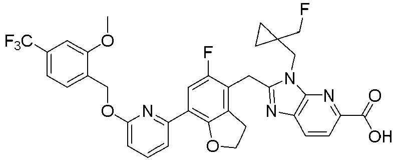

2-((5-Fluoro-7-(6-((2-methoxy-4-(trifluoromethyl)benzyl)oxy)pyridin-2-yl)-2,3-dihydrobenzofuran-4-yl)methyl)-3-((1-(fluoromethyl)cyclopropyl)methyl)-3*H*-imidazo[4,5-*b*]pyridine-5-carboxylic acid (22 mg) was obtained by referring to the synthetic method of Compound 71 using Intermediates 1.1-B, 1.5-D and 1.2-K as the starting materials. ESI-MS (m/z): 681.2[M+H]⁺, ¹H NMR (600 MHz, DMSO-*d₆*) δ 13.02 (s, 1H), 8.06 (d, *J* = 8.2 Hz, 1H), 7.97 (d, *J* = 8.2 Hz, 1H), 7.85-7.78 (m, 2H), 7.64-7.59 (m, 2H), 7.33-7.31 (m, 2H), 6.88 (dd, *J =* 7.4, 1.3 Hz, 1H), 5.52 (s, 2H), 4.85 (d, *J* = 23.2 Hz, 2H), 4.68 (t, *J* = 8.8 Hz, 2H), 4.39 (s, 2H), 3.94 (s, 3H), 3.79-3.71 (m, 1H), 3.61-3.56 (m, 1H), 3.11 (t, *J=* 8.7 Hz, 2H), 2.45-2.39 (m, 2H), 2.28-2.19 (m, 2H).

### Example 36: 2-((7-(6-((4-Chloro-2-(difluoromethoxy)benzyl)oxy)pyridin-2-yl)-5-fluoro-2,3-dihydrobenzofuran-4-yl)methyl)-3-((1-(fluoromethyl)cyclopropyl)methyl)-3H-imidazo[4,5-b]pyridine-5-carboxylic acid (Compound 91)

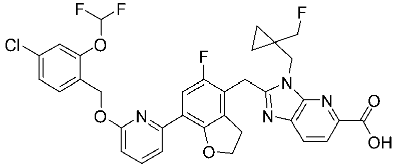

2-((7-(6-((4-Chloro-2-(difluoromethoxy)benzyl)oxy)pyridin-2-yl)-5-fluoro-2,3-dihydrobenzofuran-4-yl)methyl)-3-((1-(fluoromethyl)cyclopropyl)methyl)-3*H*-imidazo[4,5-b]pyridine-5-carboxylic acid (28 mg) was obtained by referring to the synthetic method of Compound 71 using Intermediates 1.1-B, 1.5-E and 1.2-J as the starting materials. ESI-MS (m/z): 683.2[M+H]⁺, ¹H NMR (600 MHz, DMSO-*d₆*) δ 13.01 (s, 1H), 8.07 (d, *J* = 8.2 Hz, 1H), 7.97 (d, *J* = 8.2 Hz, 1H), 7.82 (d, *J* = 4.2 Hz, 2H), 7.71 (d, *J =* 11.6 Hz, 1H), 7.59 (d, *J* = 8.2 Hz, 1H), 7.39 (s, 1H), 7.37-7.35 (m, 1H), 7.34 (t, *J* = 73.5 Hz,1H), 6.86-6.83 (m, 1H), 5.49 (s, 2H), 4.85 (d, *J =* 23.2 Hz, 2H), 4.69 (t, *J* = 8.8 Hz, 2H), 4.39 (s, 2H), 3.79-3.71 (m, 1H), 3.61-3.56 (m, 1H), 3.12 (t, *J =* 8.7 Hz, 2H), 2.45-2.38 (m, 2H), 2.28-2.19 (m, 2H).

### Example 37: 2-((7-(6-((4-Chloro-2-fluorobenzyl)oxy)pyridin-2-yl)-5-fluoro-2,3-dihydrobenzofuran-4-yl)methyl)-1-((1-(fluoromethyl)cyclopropyl)methyl)-4-methoxy-1H-benzo[d]imidazole-6-carboxylic acid (Compound 92)

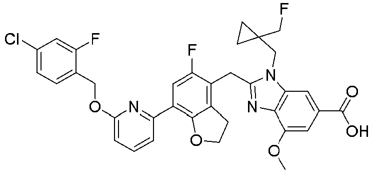

2-((7-(6-((4-Chloro-2-fluorobenzyl)oxy)pyridin-2-yl)-5-fluoro-2,3-dihydrobenzofuran-4-yl)methyl)-1-((1-(fluoromethyl)cyclopropyl)methyl)-4-methoxy-1H-benzo[d]imidazole-6-carboxylic acid (40 mg) was obtained by referring to the synthetic method of Compound 71 using Intermediates 1.1-B, 1.5-B and 1.2-J as the starting materials. ESI-MS (m/z): 664.2[M+H]⁺, ¹H NMR (600 MHz, DMSO-*d₆*) δ 7.92 (s, 1H), 7.82 (q, *J* = 4.6, 4.0 Hz, 2H), 7.74 (d, *J* = 11.5 Hz, 1H), 7.60 (t, *J* = 8.1 Hz, 1H), 7.48 (dd, *J* = 10.0, 2.1 Hz, 1H), 7.31 (dd, *J* = 8.3, 2.0 Hz, 1H), 7.24 (s, 1H), 6.83 (dd, *J* = 6.4, 2.5 Hz, 1H), 5.51 (s, 2H), 4.82 (d, *J* = 24.1 Hz, 2H), 4.69 (t, *J* = 8.7 Hz, 2H), 4.32 (s, 2H), 3.87 (s, 3H), 3.10 (t, *J* = 8.8 Hz, 2H), 2.30 - 2.21 (m, 4H), 1.93 - 1.78 (m, 2H).

### Example 38: 2-((7-(6-((4-Cyano-2-fluorobenzyl)oxy)pyridin-2-yl)-5-fluoro-2,3-dihydrobenzofuran-4-yl)methyl)-1-((1-(fluoromethyl)cyclopropyl)methyl)-4-methoxy-1H-benzo[d]imidazole-6-carboxylic acid (Compound 93)

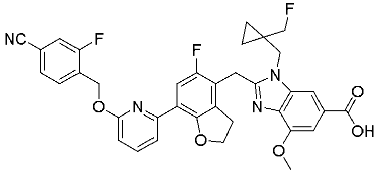

2-((7-(6-((4-Cyano-2-fluorobenzyl)oxy)pyridin-2-yl)-5-fluoro-2,3-dihydrobenzofuran-4-yl)methyl)-1-((1-(fluoromethyl)cyclopropyl)methyl)-4-methoxy-1*H*-benzo[*d*]imidazole-6-carboxylic acid (15 mg) was obtained by referring to the synthetic method of Compound 71 using Intermediates 1.1-B, 1.5-C and 1.2-J as the starting materials. ESI-MS (m/z): 655.2[M+H]⁺, ¹H NMR (600 MHz, DMSO-*d₆*) δ 12.82 (s, 1H), 7.96 - 7.88 (m, 2H), 7.82 (q, *J* = 4.2, 3.3 Hz, 2H), 7.76 - 7.70 (m, 2H), 7.66 (d, *J* = 11.6 Hz, 1H), 7.24 (d, *J* = 1.3 Hz, 1H), 6.87 (dt, *J* = 7.6, 3.8 Hz, 1H), 5.60 (s, 2H), 4.82 (d, *J* = 24.2 Hz, 2H), 4.68 (t, *J* = 8.8 Hz, 2H), 4.31 (s, 2H), 3.87 (s, 3H), 3.10 (t,*J=* 8.8 Hz, 2H), 2.31 - 2.15 (m, 4H), 2.03 - 1.75 (m, 2H).

### Example 39: (S)-2-((7-(6-((4-chloro-2-(difluoromethoxy)benzyl)oxy)pyridin-2-yl)-5-fluoro-2,3-dihydrobenzofuran-4-yl)methyl)-1-(oxetan-2-ylmethyl)-1H-benzimidazole-6-carboxylic acid (Compound 95)

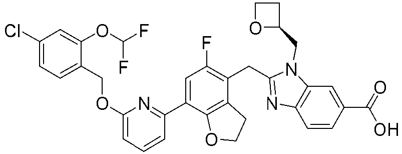

(S)-2-((7-(6-((4-chloro-2-(difluoromethoxy)benzyl)oxy)pyridin-2-yl)-5-fluoro-2,3-dihydrobenzofuran-4-yl)methyl)-1-(oxetan-2-ylmethyl)-1H-benzimidazole-6-carboxylic acid (7 mg) was obtained by referring to the synthetic method of Compound 71 using Intermediates 1.1-B, 1.5-E and 1.2-A as the starting materials. ESI-MS (m/z) 666.2[M+H]⁺.

### Example 40: (S)-2-((7-(6-((4-chloro-2-fluorobenzyl)oxy)pyridin-2-yl)-5-fluoro-2,3-dihydrobenzofuran-4-yl)methyl)-4-(2,2-difluoroethoxy)-1-((1-(fluoromethyl)cyclopropyl)methyl)-1H-benzo[d]imidazole-6-carboxylic acid (Compound 96)

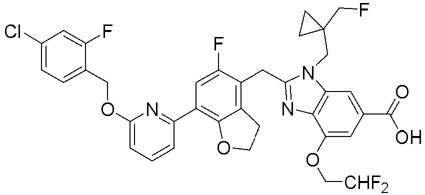

(S)-2-((7-(6-((4-chloro-2-fluorobenzyl)oxy)pyridin-2-yl)-5-fluoro-2,3-dihydrobenzofuran-4-yl)methyl)-4-(2,2-difluoroethoxy)-1-((1-(fluoromethyl)cyclopropyl)methyl)-1H-benzo[d]imidazole-6-carboxylic acid (30 mg) was obtained by referring to the synthetic method of Compound 71 using Intermediates 1.8-I and 1.9-O as the starting materials. ESI-MS (m/z): 714.2[M+H]⁺.

### Example 41: (S)-2-((7-(6-((4-cyano-2-fluorobenzyl)oxy)pyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 100)

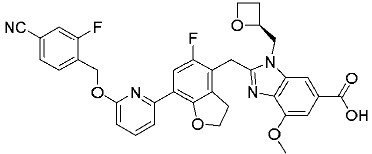

(S)-2-((7-(6-((4-cyano-2-fluorobenzyl)oxy)pyridin-2-yl)-5-fluoro-2,3-dihydrobenzofuran-4-yl)methyl)-4-methoxy-1-(oxetan-2-ylmethyl)-1*H*-benzo[*d*]imidazole-6-carboxylic acid (30 mg) was obtained by referring to the synthetic method of Compound 71 using Intermediates 1.1-B, 1.5-C and 1.2-F as the starting materials. ESI-MS (m/z): 639.2[M+H]⁺, ¹H NMR (600 MHz, DMSO-*d₆*) δ 12.80 (s, 1H), 7.91 (d, *J =* 9.7 Hz, 1H), 7.87 (s, 1H), 7.83-7.82 (m, 2H), 7.76-7.71 (m, 2H), 7.67 (d, *J =* 11.6 Hz, 1H), 7.24 (s, 1H), 6.89-6.87 (m, 1H), 5.61 (s, 2H), 5.07-5.03 (m, 1H), 4.72-4.66 (m, 3H), 4.59-4.57 (m, 1H), 4.51-4.47 (m, 1H), 4.44 (d, *J =* 16.8 Hz, 1H), 4.36-4.31 (m, 2H), 3.88 (s, 3H), 3.13-3.07 (m, 2H), 2.72-2.67 (m, 1H), 2.39-2.33 (m, 1H).

### Example 42: (S)-2-((7-(6-((4-chloro-2-fluorobenzyl)oxy)-5-fluoropyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 101)

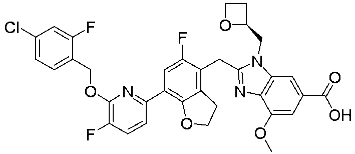

(S)-2-((7-(6-((4-chloro-2-fluorobenzyl)oxy)-5-fluoropyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (16 mg) was obtained by referring to the synthetic method of Compound 71 using Intermediates 1.1-B, 1.5-F and 1.2-F as the starting materials. ESI-MS (m/z): 666.2[M+H]⁺, ¹H NMR (600 MHz, DMSO-*d₆*) δ 12.79 (s, 1H), 7.87 (s, 1H), 7.83-7.81 (m, 1H), 7.80-7.76 (m, 1H), 7.69 (d, *J* = 11.5 Hz, 1H), 7.64-7.61 (m, 1H), 7.51 (dd, *J =* 10.0, 1.9 Hz, 1H), 7.34 (dd, *J =* 8.2, 1.7 Hz, 1H), 7.24 (s, 1H), 5.60 (s, 2H), 5.08-5.04 (m, 1H), 4.72-4.67 (m, 3H), 4.59-4.56 (m, 1H), 4.51-4.46 (m, 1H), 4.44 (d, *J* = 16.7 Hz, 1H), 4.36-4.31 (m, 2H), 3.88 (s, 3H), 3.14-3.08 (m, 2H), 2.73-2.67 (m, 1H), 2.39-2.34 (m, 1H).

### Example 43: (S)-2-((7-(6-((4-chloro-2-fluorophenoxy)methyl)pyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 103)

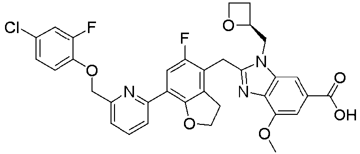

(S)-2-((7-(6-((4-chloro-2-fluorophenoxy)methyl)pyridin-2-yl)-5-fluoro-2,3-dihydrobenzofuran-4-yl)methyl)-4-methoxy-1-(oxetan-2-ylmethyl)-1*H*-benzo[*d*]imidazole-6-carboxylic acid (37 mg) was obtained by referring to the synthetic method of Compound 71 using Intermediates 1.1-B, 1.5-H and 1.2-F as the starting materials. ESI-MS (m/z): 648.2[M+H]⁺, ¹H NMR (600 MHz, DMSO-*d₆*) δ 12.78 (s, 1H), 8.12 (d, *J =* 7.8 Hz, 1H), 7.94 (dd, *J* = 7.8, 7.8 Hz, 1H), 7.88 (s, 1H), 7.71 (d, *J* = 12 Hz, 1H), 7.49-7.45 (m, 2H), 7.33 (dd, *J* = 9, 9 Hz, 1H), 7.25 (s, 1H), 7.21 (d, *J* = 8.4 Hz, 1H), 5.37 (s, 2H), 5.07-5.06 (m, 1H), 4.71-4.69 (m, 3H), 4.60-4.57 (m, 1H), 4.51-4.44 (m, 2H), 4.38-4.33 (m, 2H),3.89 (s, 3H), 3.13-3.12 (m, 2H), 2.73-2.69 (m, 1H), 2.38-2.35 (m, 1H).

### Example 44: (S)-2-((7-(3-((4-trifluoromethyl-2-methoxybenzyl)oxy)-4-fluorophenyl)-5-fluoro-2,3-dihydrobenzofuran-4-yl)methyl)-4-methoxy-1-(oxetan-2-ylmethyl)-1H-benzo[d]imidazole-6-carboxylic acid (Compound 104)

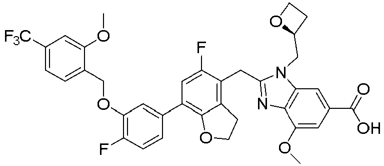

(S)-2-((7-(3-((4-trifluoromethyl-2-methoxybenzyl)oxy)-4-fluorophenyl)-5-fluoro-2,3-dihydrobenzofuran-4-yl)methyl)-4-methoxy-1-(oxetan-2-ylmethyl)-1*H*-benzo[*d*]imidazole-6-carboxylic acid (9 mg) was obtained by referring to the synthetic method of Compound 71 using Intermediates 1.1-B, 1.5-I and 1.2-F as the starting materials. ESI-MS (m/z): 711.2[M+H]⁺.

### Example 45: (S)-2-((7-(3-((4-chloro-2-fluorobenzyl)oxy)-4-fluorophenyl)-5-fluoro-2,3-dihydrobenzofuran-4-yl)methyl)-4-methoxy-1-(oxetan-2-ylmethyl)-1H-benzo[d]imidazole-6-carboxylic acid (Compound 105)

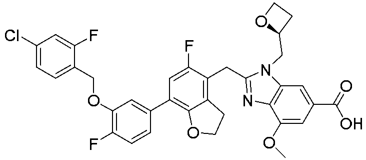

**(S)-2-((7-(3-((4-chloro-2-fluorobenzyl)oxy)-4-fluorophenyl)-5-fluoro-2,3-dihydrobenzofuran-4-yl)methyl)-4-methoxy-1-(oxetan-2-ylmethyl)-1*H*-benzo[*d*]imidazole-6-carboxylic** acid (55 mg) was obtained by referring to the synthetic method of Compound 71 using Intermediates 1.1-B, 1.5-K and 1.2-F as the starting materials. ESI-MS (m/z): 665.2[M+H]⁺, ¹H NMR (600 MHz, DMSO-*d₆*) δ 12.80 (s, 1H), 7.87 (s, 1H), 7.63-7.60 (m, 2H), 7.52 (dd, *J* = 9.9, 2.0 Hz, 1H), 7.38-7.36 (m, 2H), 7.30-7.26 (m, 2H), 7.24 (s, 1H), 5.29 (s, 2H), 5.07-5.04 (m, 1H), 4.72-4.68 (m, 1H), 4.60-4.57 (m, 3H), 4.51-4.48 (m, 1H), 4.42 (d, *J =* 16.6 Hz, 1H), 4.34-4.31 (m, 2H), 3.89 (s, 3H), 3.11-3.07 (m, 2H), 2.71-2.69 (m, 1H), 2.39-2.35 (m, 1H).

### Example 46: (S)-2-((7-(2-((4-chloro-2-fluorobenzyl)oxy)pyrimidin-4-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 106)

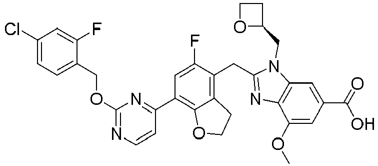

(S)-2-((7-(2-((4-chloro-2-fluorobenzyl)oxy)pyrimidin-4-yl)-5-fluoro-2,3-dihydrobenzofuran-4-yl)methyl)-4-methoxy-1-(oxetan-2-ylmethyl)-1H-benzo[d]imidazole-6-carboxylic acid (35 mg) was obtained by referring to the synthetic method of Compound 71 using Intermediates 1.1-B, 1.5-J and 1.2-F as the starting materials. ESI-MS (m/z): 649.2[M+H]⁺, ¹H NMR (600 MHz, DMSO-*d₆*) δ 12.80 (s, 1H), 8.68 (d, *J =* 4.8 Hz, 1H), 7.87-7.85 (m, 2H), 7.82 (d, *J* = 11.4 Hz, 1H), 7.62 (dd, *J* =8.4, 7.8 Hz, 1H), 7.50 (dd, *J* =10.2, 2.4 Hz, 1H), 7.33 (dd, *J* = 8.4, 1.8 Hz, 1H), 7.23 (d, *J* = 0.6Hz, 1H), 5.52 (s, 2H), 5.08-5.04 (m, 1H), 4.75-4.69 (m, 3H), 4.60-4.57 (m, 1H), 4.51-4.45 (m, 2H), 4.39-4.31 (m, 2H),3.87 (s, 3H), 3.18-3.08 (m, 2H), 2.73-2.67 (m, 1H), 2.38-2.33 (m, 1H).

### Example 47: (S)-2-((7-(3-((4-chloro-2-fluorobenzyl)oxy)phenyl)-5-fluoro-2,3-dihydrobenzofuran-4-yl)methyl)-4-methoxy-1-(oxetan-2-ylmethyl)-1H-benzo[d]imidazole-6-carboxylic acid (Compound 107)

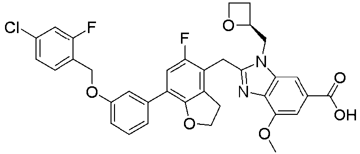

(S)-2-((7-(3-((4-chloro-2-fluorobenzyl)oxy)phenyl)-5-fluoro-2,3-dihydrobenzofuran-4-yl)methyl)-4-methoxy-1-(oxetan-2-ylmethyl)-1*H*-benzo[d*]*imidazole-6-carboxylic acid (20 mg) was obtained by referring to the synthetic method of Compound 71 using Intermediates 1.1-B, 1.5-AC and 1.2-F as the starting materials. ESI-MS (m/z): 647.2[M+H]⁺, ¹H NMR (600 MHz, DMSO-*d₆*) δ 12.79 (s, 1H), 7.87 (s, 1H), 7.63-7.61 (m, 1H), 7.51 (dd, *J* = 9.9, 1.9 Hz, 1H), 7.39-7.34 (m, 4H), 7.25-7.23 (m, 2H), 7.02-7.00 (m, 1H), 5.19 (s, 2H), 5.08-5.04 (m, 1H), 4.72-4.68 (m, 1H), 4.59-4.56 (m, 3H), 4.51-4.47 (m, 1H), 4.42 (d, *J* = 16.7 Hz, 1H), 4.34-4.31 (m, 2H), 3.89 (s, 3H), 3.79-3.72 (m, 1H), 3.62-3.51 (m, 1H), 3.12-3.06 (m, 2H), 2.73-2.67 (m, 1H), 2.39-2.33 (m, 1H).

### Example 48: (S)-2-((7-(5-chloro-6-((4-chloro-2-fluorobenzyl)oxy)pyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 108)

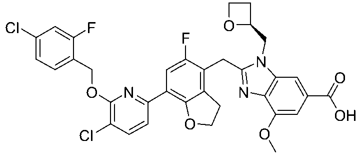

(S)-2-((7-(5-chloro-6-((4-chloro-2-fluorobenzyl)oxy)pyridin-2-yl)-5-fluoro-2,3-dihydrobenzofuran-4-yl)methyl)-4-methoxy-1-(oxetan-2-ylmethyl)-1*H*-benzo[*d*]imidazole-6-carboxylic acid (15 mg) was obtained by referring to the synthetic method of Compound 71 using Intermediates 1.1-B, 1.5-L and 1.2-F as the starting materials. ESI-MS(m/z): 682.1[M+H]⁺, ¹H NMR (600 MHz, DMSO-*d₆*) δ 12.80 (brs, 1H), 7.99 (d, *J* = 8.0 Hz, 1H), 7.87 (s, 1H), 7.83 (d, *J* = 8.0 Hz, 1H), 7.73 (d, *J =* 11.3 Hz, 1H), 7.61 (t, *J =* 7.8 Hz, 1H), 7.50 (d, *J=* 9.5 Hz, 1H), 7.34 (d, *J* = 7.8 Hz, 1H), 7.24 (s, 1H), 5.61 (s, 2H), 5.06 (d, *J* = 5.2 Hz, 1H), 4.73 - 4.67 (m, 3H), 4.58 (d, *J* = 14.8 Hz, 1H), 4.53 - 4.47 (m, 1H), 4.45 (d, *J* = 16.8 Hz, 1H), 4.38 - 4.31 (m, 2H), 3.88 (s, 3H), 3.14 - 3.07 (m, 2H), 2.73 - 2.66 (m, 1H), 2.39 - 2.32 (m, 1H).

### Example 49: (S)-2-((7-(5-bromo-6-((4-chloro-2-fluorobenzyl)oxy)pyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 109)

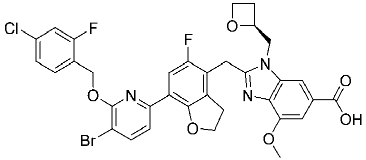

(S)-2-((7-(5-bromo-6-((4-chloro-2-fluorobenzyl)oxy)pyridin-2-yl)-5-fluoro-2,3-dihydrobenzofuran-4-yl)methyl)-4-methoxy-1-(oxetan-2-ylmethyl)-1H-benzimidazole-6-carboxylic acid (13 mg) was obtained by referring to the synthetic method of Compound 71 using Intermediates 1.1-B, 1.5-AD and 1.2-F as the starting materials. ESI-MS (m/z)726.1[M+H]⁺, ¹H NMR (600 MHz, DMSO-*d₆*) δ12.79 (s, 1H), 8.13 (d, *J* = 7.8 Hz, 1H), 7.87 (s, 1H), 7.75 (d, *J* = 8.4 Hz, 2H), 7.60 (t, *J* = 7.2 Hz, 1H), 7.50 (d, *J =* 9.6 Hz, 1H), 7.34 *(d, J=* 7.8 Hz, 1H), 7.24 (s, 1H), 5.60 (s, 2H), 5.10-5.01 (m, 1H), 4.76-4.65 (m, 3H), 4.61-4.54 (m, 1H), 4.53-4.41 (m, 2H), 4.39-4.30 (m, 2H), 3.88 (s, 3H), 3.16-3.06 (m, 2H), 2.73-2.66 (m, 1H), 2.41-2.34 (m, 1H).

### Example 50: (S)-2-((7-(2-((2-methoxy-4-(trifluoromethyl)benzyl)oxy)pyrimidin-4-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 110)

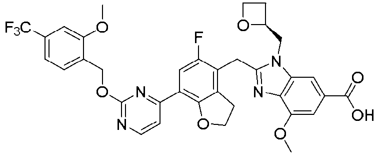

(S)-2-((7-(2-((2-methoxy-4-(trifluoromethyl)benzyl)oxy)pyrimidin-4-yl)-5-fluoro-2,3-dihydrobenzofuran-4-yl)methyl)-4-methoxy-1-(oxetan-2-ylmethyl)-1H-benzo[d]imidazole-6-carboxylic acid (29 mg) was obtained by referring to the synthetic method of Compound 71 using Intermediates 1.5-M, 1.5-E and 1.2-F as the starting materials. ESI-MS (m/z): 695.1[M+H]⁺, ¹H NMR (600 MHz, DMSO-*d₆*) δ 12.78 (s, 1H), 8.68 (d, *J* = 5.4 Hz, 1H), 7.87-7.85 (m, 2H), 7.77 (d, *J* = 11.4 Hz, 1H), 7.62 (d, J=8.4 Hz, 1H), 7.34-7.33(m, 2H), 7.24 (s, 1H), 5.53 (s, 2H), 5.08-5.04 (m, 1H), 4.75-4.69 (m, 3H), 4.60-4.57 (m, 1H), 4.51-4.45 (m, 2H), 4.39-4.31 (m, 2H), 3.94 (s, 3H), 3.87 (s, 3H), 3.18-3.08 (m, 2H), 2.73-2.67 (m, 1H), 2.38-2.32 (m, 1H).

### Example 51: 2-((7-((6-((4-Chloro-2-fluorobenzyl)oxy)pyridin-2-yl)-5-fluoro-2,3-dihydrobenzofuran-4-yl)methyl)-1-(1-ethyl-1H-imidazol-5-ylmethyl)-4-methoxy-1H-benzo[d]imidazole-6-carboxylic acid (Compound 111)

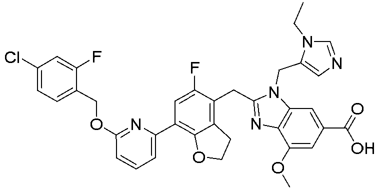

2-((7-((6-((4-Chloro-2-fluorobenzyl)oxy)pyridin-2-yl)-5-fluoro-2,3-dihydrobenzofuran-4-yl)methyl)-1-(1-ethyl-1H-imidazol-5-ylmethyl)-4-methoxy-1H-benzo[d]imidazole-6-carboxylic acid (11 mg) was obtained by referring to the synthetic method of Compound 71 using Intermediates 1.1-B, 1.5-B and 1.2-L as the starting materials. ESI-MS(m/z): 686.1[M+H]⁺.

### Example 52: 2-((7-(2-((4-Chloro-2-fluorobenzyl)oxy)pyrimidin-4-yl)-5-fluoro-2,3-dihydrobenzofuran-4-yl)methyl)-1-((1-(fluoromethyl)cyclopropyl)methyl)-4-methoxy-1H-benzo[d]imidazole-6-carboxylic acid (Compound 112)

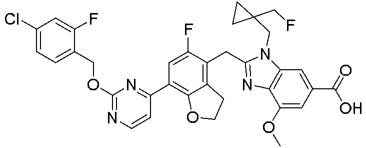

2-((7-(2-((4-Chloro-2-fluorobenzyl)oxy)pyrimidin-4-yl)-5-fluoro-2,3-dihydrobenzofuran-4-yl)methyl)-1-((1-(fluoromethyl)cyclopropyl)methyl)-4-methoxy-1H-benzo[d]imidazole-6-carboxylic acid (31 mg) was obtained by referring to the synthetic method of Compound 71 using Intermediates 1.1-B, 1.5-J and 1.2-J as the starting materials. ESI-MS (m/z): 665.2[M+H]⁺, ¹H NMR (600 MHz, DMSO-*d₆*) δ 12.83 (s, 1H), 8.69 (d, *J* = 5.4 Hz, 1H), 7.93 (s, 1H), 7.86 (d, *J =* 5.4 Hz, 1H), 7.82 (d, *J =* 11.4 Hz, 1H), 7.62 (dd, *J =* 7.8, 7.8 Hz, 1H), 7.50 (dd, *J* = 10.2, 1.8 Hz, 1H), 7.34 (dd, *J* = 7.8, 1.2 Hz, 1H), 7.24 (s, 1H), 5.52 (s, 2H), 4.83 (d, *J* = 24 Hz, 2H), 4.74 (t, *J* = 9 Hz, 2H), 4.34 (s, 2H), 3.87 (s, 3H), 3.13 (t, *J=* 9 Hz, 2H), 2.26-2.21 (m, 4H), 1.90-1.87 (m, 2H).

### Example 53: (S)-2-((7-(2-((2-difluoromethoxy-4-chlorobenzyl)oxy)pyrimidin-4-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 113)

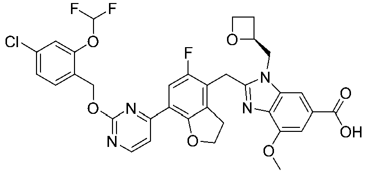

(S)-2-((7-(2-((2-difluoromethoxy-4-chlorobenzyl)oxy)pyrimidin-4-yl)-5-fluoro-2,3-dihydrobenzofuran-4-yl)methyl)-4-methoxy-1-(oxetan-2-ylmethyl)-1H-benzo[d]imidazole-6-carboxylic acid (22 mg) was obtained by referring to the synthetic method of Compound 71 using Intermediates 1.1-B, 1.5-N and 1.2-F as the starting materials. ESI-MS (m/z): 697.1[M+H]⁺, ¹H NMR (600 MHz, DMSO-*d₆*) δ 12.80 (s, 1H), 8.69 (d, *J* = 4.8 Hz, 1H), 7.87-7.85 (m, 2H), 7.80 (d, *J* = 11.4 Hz, 1H), 7.62 (d, J=8.4 Hz, 1H), 7.41-7.37 (m, 2H), 7.36 (t, *J* = 73.4 Hz, 1H), 7.24 (s, 1H), 5.50 (s, 2H), 5.08-5.04 (m, 1H), 4.75-4.69 (m, 3H), 4.59-4.57 (m, 1H), 4.51-4.45 (m, 2H), 4.39-4.31 (m, 2H), 3.87 (s, 3H), 3.15-3.11 (m, 2H), 2.74-2.67 (m, 1H), 2.38-2.33 (m, 1H).

### Example 54: (S)-2-((7-((6-((4-chloro-2-(difluoromethoxy)benzyl)oxy)-5-fluoropyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 114)

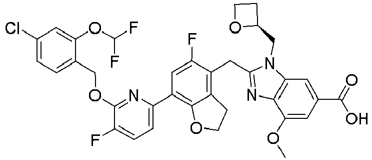

(S)-2-((7-((6-((4-chloro-2-(difluoromethoxy)benzyl)oxy)-5-fluoropyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (6 mg) was obtained by referring to the synthetic method of Compound 71 using Intermediates 1.1-B, 1.5-O and 1.2-F as the starting materials. ESI-MS (m/z): 714.2[M+H]⁺.

### Example 55: (S)-2-((7-((6-((4-cyano-2-fluorobenzyl)oxy)-5-fluoropyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 115)

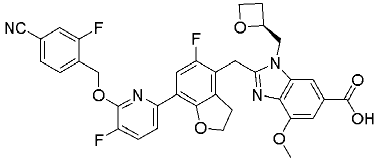

(S)-2-((7-((6-((4-cyano-2-fluorobenzyl)oxy)-5-fluoropyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (13 mg) was obtained by referring to the synthetic method of Compound 71 using Intermediates 1.1-B, 1.5-P and 1.2-F as the starting materials. ESI-MS(m/z): 657.1 [M+H]⁺, ¹H NMR (600 MHz, DMSO-*d₆*) δ 12.7 (brs, 1H) 7.91 (d, J = 9.6 Hz, 1H), 7.85 (s, 1H), 7.83 - 7.75 (m, 3H), 7.73 (d, J = 7.4 Hz, 1H), 7.60 (d, J = 11.4 Hz, 1H), 7.23 (s, 1H), 5.69 (s, 2H), 5.07 - 5.02 (m, 1H), 4.71 - 4.64 (m, 3H), 4.56 (d, J = 14.4 Hz, 1H), 4.50 - 4.46 (m, 1H), 4.43 (d, J = 16.8 Hz, 1H), 4.36 - 4.30 (m, 2H), 3.87 (s, 3H), 3.11 - 3.07 (m, 2H), 2.70 - 2.67 (m, 1H), 2.37 - 2.34 (m, 1H).

### Example 56: (S)-2-((7-(5-fluoro-6-((2-methoxy-4-(trifluoromethyl)benzyl)oxy)pyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 116)

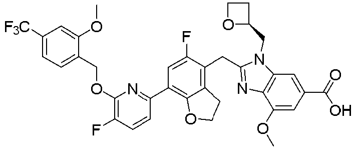

(S)-2-((7-(5-fluoro-6-((2-methoxy-4-(trifluoromethyl)benzyl)oxy)pyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (17 mg) was obtained by referring to the synthetic method of Compound 71 using Intermediates 1.1-B, 1.5-G and 1.2-F as the starting materials. ESI-MS (m/z): 712.2[M+H]⁺, ¹H NMR (600 MHz, DMSO-*d₆*) δ 7.87-7.75 (m, 3H), 7.62 (d, J = 7.6 Hz, 1H), 7.58 (d, J = 11.5 Hz, 1H), 7.34 (d, J = 9.0 Hz, 2H), 7.27 (s, 1H), 5.62 (s, 2H), 5.09-5.02 (m, 1H), 4.71-4.63 (m, 3H), 4.56-4.47 (m, 2H), 4.42 (d, J = 16.7 Hz, 1H), 4.36-4.31 (m, 2H), 3.96 (s, 3H), 3.86 (s, 3H), 3.12-3.06 (m, 2H), 2.72-2.65 (m, 1H), 2.41-2.33 (m, 1H).

### Example 57: (S)-2-((7-(4-((4-chloro-2-fluorobenzyl)oxy)pyrimidin-2-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 117)

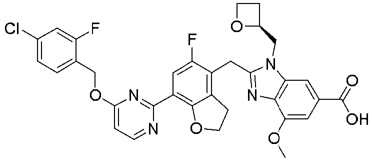

(S)-2-((7-(4-((4-chloro-2-fluorobenzyl)oxy)pyrimidin-2-yl)-5-fluoro-2,3-dihydrobenzofuran-4-yl)methyl)-4-methoxy-1-(oxetan-2-ylmethyl)-1*H*-benzo[*d*]imidazole-6-carboxylic acid (6 mg) was obtained by referring to the synthetic method of Compound 71 using Intermediates 1.1-B, 1.5-Q and 1.2-F as the starting materials. ESI-MS (m/z): 649.1 [M+H]⁺.

### Example 58: (S)-2-((7-(2-((4-chloro-2-fluorobenzyl)oxy)-5-fluoropyrimidin-4-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 118)

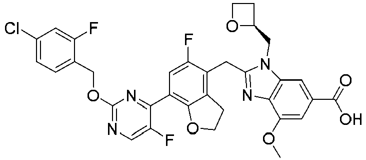

(S)-2-((7-(2-((4-chloro-2-fluorobenzyl)oxy)-5-fluoropyrimidin-4-yl)-5-fluoro-2,3-dihydrobenzofuran-4-yl)methyl)-4-methoxy-1-(oxetan-2-ylmethyl)-1*H*-benzo[*d*]imidazole-6-carboxylic acid (36 mg) was obtained by referring to the synthetic method of Compound 128 using 4-chloro-2-fluorobenzyl alcohol as the starting material. ESI-MS (m/z): 667.1[M+H]⁺, ¹H NMR (600 MHz, CDCl₃) δ 8.40 (s, 1H), 7.86 (s, 1H), 7.50-7.48 (m, 2H), 7.24 (d, *J* = 10.6 Hz, 1H), 7.15-7.11 (m, 2H), 5.46 (s, 2H), 5.07-5.03 (m, 1H), 4.65-4.60 (m, 5H), 4.51-4.47 (m, 1H), 4.45-4.41 (m, 1H), 4.35 (d, *J* = 13.9 Hz, 1H), 4.08 (s, 3H), 3.34 (br, 1H), 3.23-3.17 (m, 1H), 2.74-2.68 (m, 1H), 2.41-2.35 (m, 1H).

### Example 59: (S)-2-((7-(2-((4-chloro-2-fluorobenzyl)oxy)-pyrimidin-4-yl)-5-fluoro-2,3-dihydrobenzofuran-4-yl)methyl)-4-isopropoxy-1-(oxetan-2-ylmethyl)-1H-benzo[d]imidazole-6-carboxylic acid (Compound 119)

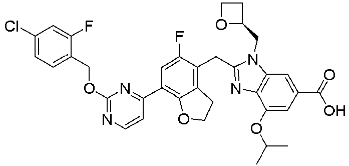

(S)-2-((7-(2-((4-chloro-2-fluorobenzyl)oxy)-pyrimidin-4-yl)-5-fluoro-2,3-dihydrobenzofuran-4-yl)methyl)-4-isopropoxy-1-(oxetan-2-ylmethyl)-1*H*-benzo[*d*]imidazole-6-carboxylic acid (28 mg) was obtained by referring to the synthetic method of Compound 71 using Intermediates 1.1-B, 1.5-J and 1.2-M as the starting materials. ESI-MS (m/z): 677.1 [M+H]⁺.

### Example 60: (S)-2-((7-(2-((2-fluoro-4-(oxetan-3-yl)benzyl)oxy)pyrimidin-4-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 120)

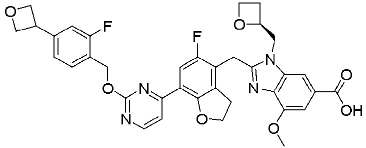

(S)-2-((7-(2-((2-fluoro-4-(oxetan-3-yl)benzyl)oxy)pyrimidin-4-yl)-5-fluoro-2,3-dihydrobenzofuran-4-yl)methyl)-4-methoxy-1-(oxetan-2-ylmethyl)-1H-benzo[d]imidazole-6-carboxylic acid (27 mg) was obtained by referring to the synthetic method of Compound 71 using Intermediates 1.1-B, 1.5-S and 1.2-F as the starting materials. ESI-MS (m/z): 671.1[M+H]⁺, ¹H NMR (600 MHz, DMSO-*d₆*) δ 12.80 (s, 1H), 8.68 (d, *J* =4.8 Hz, 1H), 7.89-7.81 (m, 3H), 7.58 (t, *J =* 7.8 Hz, 1H), 7.32 (d, *J* =11.4 Hz, 1H), 7.26 (dd, *J* =7.8, 1.1 Hz, 1H), 7.24 (s, 1H), 5.53 (s, 2H), 5.09-5.03 (m, 1H), 4.95-4.90 (m, 2H), 4.76-4.69 (m, 3H), 4.64-4.55 (m, 3H), 4.52-4.44 (m, 2H), 4.41-4.24 (m, 3H), 3.87 (s, 3H), 3.16-3.08 (m, 2H), 2.73-2.67 (m, 1H), 2.38-2.33 (m, 1H).

### Example 61: (S)-2-((7-(2-((4-chloro-2-fluorobenzyl)oxy)pyrimidin-4-yl)-5-fluoro-2,3-dihydrobenzofuran-4-yl)methyl)-4-ethoxy-1-(oxetan-2-ylmethyl)-1H-benzo[d]imidazole-6-carboxylic acid (Compound 121)

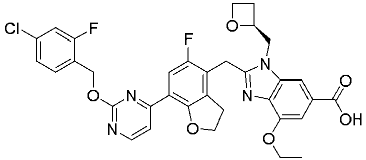

(S)-2-((7-(2-((4-chloro-2-fluorobenzyl)oxy)pyrimidin-4-yl)-5-fluoro-2,3-dihydrobenzofuran-4-yl)methyl)-4-ethoxy-1-(oxetan-2-ylmethyl)-1*H*-benzo[*d*]imidazole-6-carboxylic acid (32 mg) was obtained by referring to the synthetic method of Compound 71 using Intermediate 1.1-B, 1.5-J and 1.2-N as the starting materials. ESI-MS (m/z): 663.1[M+H]⁺.

### Example 62: (S)-2-((7-(6-((4-chloro-2-fluorobenzyl)oxy)-5-fluoropyridin-2-yl)-5-fluoro-2,3-dihydrobenzofuran-4-yl)methyl)-4-ethoxy-1-(oxetan-2-ylmethyl)-1H-benzo[d]imidazole-6-carboxylic acid (Compound 122)

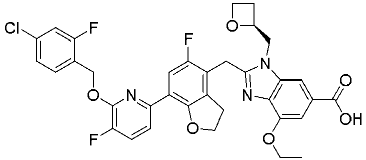

(S)-2-((7-(6-((4-chloro-2-fluorobenzyl)oxy)-5-fluoropyridin-2-yl)-5-fluoro-2,3-dihydrobenzofuran-4-yl)methyl)-4-ethoxy-1-(oxetan-2-ylmethyl)-1*H*-benzo[*d*]imidazole-6-carboxylic acid (24 mg) was obtained by referring to the synthetic method of Compound 71 using Intermediate 1.1-B, 1.5-F and 1.2-N as the starting materials. ESI-MS (m/z): 680.1[M+H]⁺, ¹H NMR (600 MHz, DMSO-*d₆*) δ 12.81 (s, 1H), 7.87 (s, 1H), 7.84-7.82 (m, 1H), 7.80-7.77 (m, 1H), 7.70 (d, *J* =11.4 Hz, 1H), 7.62 (dd, *J* =8.4, 7.8 Hz, 1H), 7.51 (dd, *J* =10.2, 1.8 Hz, 1H), 7.34 (dd, *J* =8.4, 1.8 Hz, 1H), 7.25 (s, 1H), 5.61 (s, 2H), 5.07-5.03 (m, 1H), 4.73-4.69 (m, 3H), 4.60-4.57 (m, 1H), 4.51-4.45 (m, 2H), 4.39-4.31 (m, 2H), 4.22 (q, *J* =6.6 Hz, 2H), 3.14-3.08 (m, 2H), 2.71-2.67 (m, 1H), 2.39-2.33 (m, 1H), 1.37 (t, *J =* 7.2 Hz, 3H).

### Example 63: (S)-2-((7-(6-((4-chloro-2-fluorobenzyl)oxy)-5-fluoropyridin-2-yl)-5-fluoro-2,3-dihydrobenzofuran-4-yl)methyl)-1-(oxetan-2-ylmethyl)-1H-benzo[d]imidazole-6-carboxylic acid (Compound 123)

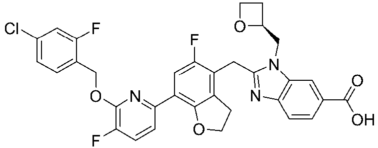

(S)-2-((7-(6-((4-chloro-2-fluorobenzyl)oxy)-5-fluoropyridin-2-yl)-5-fluoro-2,3-dihydrobenzofuran-4-yl)methyl)-1-(oxetan-2-ylmethyl)-1H-benzo[d]imidazole-6-carboxylic acid (21 mg) was obtained by referring to the synthetic method of Compound 71 using Intermediates 1.1-B, 1.5-F and 1.2-A as the starting materials. ESI-MS (m/z): 636.2[M+H]⁺, ¹H NMR (600 MHz, DMSO-*d₆*) δ 12.76 (s, 1H), 8.23 (s, 1H), 7.84-7.75 (m, 3H), 7.69 (d, *J* = 11.5 Hz, 1H), 7.65-7.60 (m, 1H), 7.56 (d, *J* = 8.4 Hz, 1H), 7.51 (dd, *J* = 9.9, 1.6 Hz, 1H), 7.34 (d, *J=* 8.2 Hz, 1H), 5.60 (s, 2H), 5.10-5.05 (m, 1H), 4.77-4.72 (m, 1H), 4.69 (t, *J =* 8.8 Hz, 2H), 4.64-4.60 (m, 1H), 4.52-4.45 (m, 2H), 4.40-4.33 (m, 2H), 3.15-3.10 (m, 2H), 2.75-2.68 (m, 1H), 2.40-2.35 (m, 1H).

### Example 64: (S)-2-((7-(2-((4-chloro-2-fluorobenzyl)oxy)pyrimidin-4-yl)-5-fluoro-2,3-dihydrobenzofuran-4-yl)methyl)-1-(oxetan-2-ylmethyl)-1H-benzo[d]imidazole-6-carboxylic acid (Compound 124)

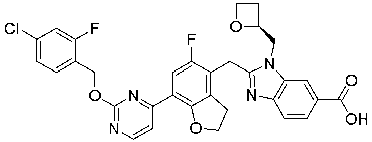

(S)-2-((7-(2-((4-chloro-2-fluorobenzyl)oxy)pyrimidin-4-yl)-5-fluoro-2,3-dihydrobenzofuran-4-yl)methyl)-1-(oxetan-2-ylmethyl)-1H-benzo[d]imidazole-6-carboxylic acid (33 mg) was obtained by referring to the synthetic method of Compound 71 using Intermediates 1.1-B, 1.5-J and 1.2-A as the starting materials. ESI-MS (m/z): 619.2[M+H]⁺, ¹H NMR (600 MHz, DMSO-*d₆*) δ 8.69 (s, 1H), 8.23 (s, 1H), 7.90-7.74 (m, 3H), 7.66-7.49 (m, 3H), 7.41-7.29 (m, 1H), 5.52 (s, 2H), 5.15-5.03 (m, 1H), 4.80-4.70 (m, 3H), 4.65-4.58 (m, 1H), 4.54-4.48 (m, 2H), 4.43-4.33 (m, 2H), 3.20-3.10 (m, 2H), 2.74-2.66 (m, 1H), 2.40-2.35 (m, 1H).

### Example 65: (S)-2-((7-(4-((2-methoxy-4-(trifluoromethyl)benzyl)oxy)pyrimidin-2-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 125)

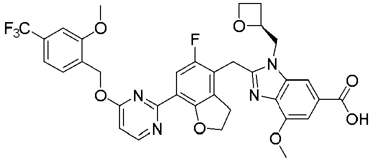

(S)-2-((7-(4-((2-methoxy-4-(trifluoromethyl)benzyl)oxy)pyrimidin-2-yl)-5-fluoro-2,3-dihydrobenzofuran-4-yl)methyl)-4-methoxy-1-(oxetan-2-ylmethyl)-1H-benzo[d]imidazole-6-carboxylic acid (37 mg) was obtained by referring to the synthetic method of Compound 71 using Intermediates 1.1-B, 1.5-AH and 1.2-F as the starting materials. ESI-MS (m/z): 695.2[M+H]⁺, ¹H NMR (600 MHz, DMSO-*d₆*) δ 12.79 (s, 1H), 8.64 (d, *J* = 5.7 Hz, 1H), 7.87 (s, 1H), 7.66-7.64 (m, 2H), 7.34-7.32 (m, 2H), 7.24 (s, 1H), 6.92 (d, *J* = 5.7 Hz, 1H), 5.59 (s, 2H), 5.08-5.04 (m, 1H), 4.72-4.68 (m, 1H), 4.62-4.56 (m, 3H), 4.51-4.44 (m, 2H), 4.37-4.31 (m, 2H), 3.94 (s, 3H), 3.88 (s, 3H), 3.12-3.05 (m, 2H), 2.73-2.67 (m, 1H), 2.39-2.33 (m, 1H).

### Example 66: (S)-2-((7-(5-fluoro-6-((2-fluoro-4-(oxetan-3-yl)benzyl)oxy)pyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 126)

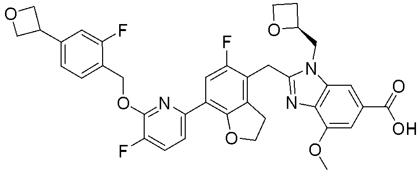

(S)-2-((7-(5-fluoro-6-((2-fluoro-4-(oxetan-3-yl)benzyl)oxy)pyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (18 mg) was obtained by referring to the synthetic method of Compound 71 using Intermediates 1.1-B, 1.5-T and 1.2-F as the starting materials. ESI-MS(m/z): 688.1[M+H]⁺, ¹H NMR (600 MHz, DMSO-*d₆*) δ 12.80 (brs, 1H), 7.87 (s, 1H), 7.83 - 7.80 (m, 1H), 7.79 - 7.75 (m, 1H), 7.71 (d, *J* = 11.4 Hz, 1H), 7.59 (t, *J* = 7.8 Hz, 1H), 7.32 (d, *J* = 11.0 Hz, 1H), 7.26 (d, *J =* 7.8 Hz, 1H), 7.24 (s, 1H), 5.61 (s, 2H), 5.09 - 5.02 (m, 1H), 4.91 (dd, *J* = 8.1, 6.1 Hz, 2H), 4.73 - 4.67 (m, 3H), 4.63 - 4.56 (m, 3H), 4.49 (dd, *J* = 13.7, 7.7 Hz, 1H), 4.45 (d, *J* = 16.8 Hz, 1H), 4.38 - 4.31 (m, 2H), 4.30 - 4.25 (m, 1H), 3.88 (s, 3H), 3.11 (dd, *J* = 14.6, 8.0 Hz, 2H), 2.74 - 2.67 (m, 1H), 2.39 - 2.34 (m, *J* = 9.3 Hz, 1H).

### Example 67: (S)-2-((7-(4-((2-fluoro-4-(oxetan-3-yl)benzyl)oxy)pyrimidin-2-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 127)

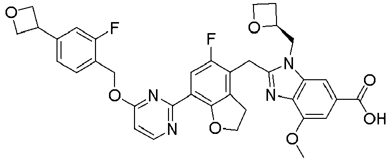

(S)-2-((7-(4-((2-fluoro-4-(oxetan-3-yl)benzyl)oxy)pyrimidin-2-yl)-5-fluoro-2,3-dihydrobenzofuran-4-yl)methyl)-4-methoxy-1-(oxetan-2-ylmethyl)-1H-benzo[d]imidazole-6-carboxylic acid (22 mg) was obtained by referring to the synthetic method of Compound 71 using Intermediates 1.1-B, 1.5-U and 1.2-F as the starting materials. ESI-MS(m/z): 671.2[M+H]⁺, ¹H NMR (600 MHz, DMSO-*d₆*) δ 12.79 (brs, 1H), 8.63 (d, J = 5.7 Hz, 1H), 7.87 (s, 1H), 7.72 (d, J = 11.2 Hz, 1H), 7.63 (t, J = 7.8 Hz, 1H), 7.31 (d, J = 11.1 Hz, 1H), 7.27 - 7.22 (m, 2H), 6.88 (d, J = 5.7 Hz, 1H), 5.59 (s, 2H), 5.06 (d, J = 5.0 Hz, 1H), 4.91 (dd, J = 8.3, 6.1 Hz, 2H), 4.71 (dd, J = 15.6, 6.8 Hz, 1H), 4.64 (t, J = 8.8 Hz, 2H), 4.62 - 4.57 (m, 3H), 4.51 - 4.44 (m, 2H), 4.39 - 4.32 (m, 2H), 4.27 (dt, J = 15.0, 7.6 Hz, 1H), 3.88 (s, 3H), 3.13 - 3.07 (m, 2H), 2.74 - 2.68 (m, 1H), 2.40 - 2.33 (m, 1H).

### Example 68: (S)-2-((7-(5-fluoro-2-((2-fluoro-4-(oxetan-3-yl)benzyl)oxy)pyrimidin-4-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 128)

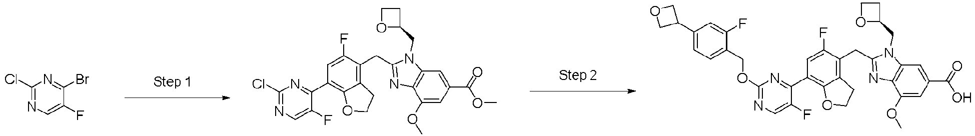

### Step 1: Methyl (S)-2-((7-(2-chloro-5-fluoropyrimidin-4-yl)-5-fluoro-2,3-dihydrobenzofuran-4-yl)methyl)-4-methoxy-1-(oxetan-2-ylmethyl)-1H-benzo[d]imidazole-6-carboxylate

Methyl 4-bromo-2-chloro-5-fluoropyrimidine (23 mg, 0.11 mmol), methyl (S)-2-((5-fluoro-7-(4,4,5,5-tetramethyl-1,3,2-dioxaborolan-2-yl)-2,3-dihydrobenzofuran-4-yl)methyl)-4-methoxy-1-(oxetan-2-ylmethyl)-1H-benzo[d]imidazole-6-carboxylate (prepared by referring to the method in Example 2, 55 mg, 0.1 mmol), Pd(dppf)Cl₂ (7 mg, 0.01 mmol), potassium carbonate (27 mg, 0.2 mmol), 1,4-dioxane (3 mL) and water (1 mL) were added into a 100 mL round bottom flask and the resulting mixture was stirred under a nitrogen atmosphere at 80 °C. After the reaction was completed, the temperature was lowered, the resulting mixture was added into water (100 mL) and extracted with ethyl acetate. The resulting organic phase was dried over anhydrous sodium sulfate and then filtered. The filtrate was concentrated and the resulting crude product was isolated and purified by thin layer chromatography (dichloromethane: methanol =15: 1) to obtain methyl (S)-2-((7-(2-chloro-5-fluoropyrimidin-4-yl)-5-fluoro-2,3-dihydrobenzofuran-4-yl)methyl)-4-methoxy-1-(oxetan-2-ylmethyl)-1H-benzo[d]imidazole-6-carboxylate (49 mg). ESI-MS (m/z): 557.1[M+H]⁺.

### Step 2: (S)-2-((7-(5-fluoro-2-((2-fluoro-4-(oxetan-3-yl)benzyl)oxy)pyrimidin-4-yl)-5-fluoro-2,3-dihydrobenzofuran-4-yl)methyl)-4-methoxy-1-(oxetan-2-ylmethyl)-1H-benzo[d]imidazole-6-carboxylic acid

2-Fluoro-4-(oxetan-3-yl)benzyl alcohol (44 mg, 0.24 mmol), methyl (S)-2-((7-(2-chloro-5-fluoropyrimidin-4-yl)-5-fluoro-2,3-dihydrobenzofuran-4-yl)methyl)-4-methoxy-1-(oxetan-2-ylmethyl)-1*H*-benzo[*d*]imidazole-6-carboxylate (55 mg, 0.1 mmol), potassium tert-butoxide (44 mg, 0.4 mmol) and tert-butanol (3 mL) were added into a 100 mL round bottom flask and the resulting mixture was stirred at 50 °C. After the reaction was completed, the temperature was lowered, water (50 mL) and glacial acetic acid (0.5 mL) were added to the resulting mixture. After the resulting mixture was stirred at room temperature for 20 minutes, the resulting mixture was extracted with dichloromethane (3x50 mL), and the resulting organic phases were combined and concentrated. The resulting crude product was isolated and purified by medium pressure preparative liquid chromatography (mobile phase: acetonitrile/water (0.1% formic acid) from 1/20 to 20/1) to obtain (S)-2-((7-(5-fluoro-2-((2-fluoro-4-(oxetan-3-yl)benzyl)oxy)pyrimidin-4-yl)-5-fluoro-2,3-dihydrobenzofuran-4-yl)methyl)-4-methoxy-1-(oxetan-2-ylmethyl)-1*H*-benzo[*d*]imidazole-6-carboxylic acid (15 mg). ESI-MS (m/z): 689.2[M+H]⁺, ¹H NMR (600 MHz, CDCl₃) *δ* 8.41 (s, 1H), 7.90 (s, 1H), 7.54-7.51 (m, 2H), 7.27 (s, 1H), 7.17-7.14 (m, 2H), 5.49 (s, 2H), 5.19-5.07 (m, 2H), 5.04-4.97 (m, 3H), 4.73 (t, *J =* 6.3 Hz, 2H), 4.66-4.62 (m, 3H), 4.56-4.52 (m, 1H), 4.49-4.45 (m, 1H), 4.39-4.37 (m, 1H), 4.23-4.18 (m, 1H), 4.09 (s, 3H), 3.53-3.49 (m, 1H), 3.26-3.22 (m, 1H), 2.77-2.73 (m, 1H), 2.44-2.37 (m, 1H).

### Example 69: (S)-2-((7-(5-fluoro-4-((2-fluoro-4-(oxetan-3-yl)benzyl)oxy)pyrimidin-2-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 129)

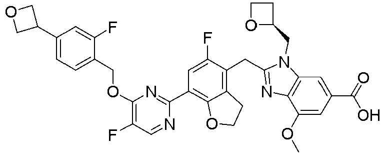

(S)-2-((7-(5-fluoro-4-((2-fluoro-4-(oxetan-3-yl)benzyl)oxy)pyrimidin-2-yl)-5-fluoro-2,3-dihydrobenzofuran-4-yl)methyl)-4-methoxy-1-(oxetan-2-ylmethyl)-1*H*-benzo[*d*]imidazole-6-carboxylic acid (26 mg) was obtained by referring to the synthetic method of Compound 71 using Intermediates 1.1-B, 1.5-W and 1.2-F as the starting materials. ESI-MS (m/z): 689.2[M+H]⁺, ¹H NMR (600 MHz, DMSO-*d₆*) *δ* 12.87 (br s, 1H), 8.72 (d, *J =* 2.8 Hz, 1H), 7.83 (s, 1H), 7.68-7.65 (m, 2H), 7.34 (dd, *J =* 10.8, 1.2 Hz, 1H), 7.28 (dd, *J =* 7.9, 1.4 Hz, 1H), 7.25 (s, 1H), 5.68 (s, 2H), 5.08-5.04 (m, 1H), 4.93-4.90 (m, 2H), 4.70-4.68 (m, 1H), 4.66-4.63 (m, 2H), 4.61 (t, *J =* 6.4 Hz, 2H), 4.58-4.55 (m, 1H), 4.51-4.49 (m, 1H), 4.45 (d, *J =* 16.7 Hz, 1H), 4.37-4.34 (m, 1H), 4.33-4.31 (m, 1H), 4.39-4.27 (m, 1H), 3.87 (s, 3H), 3.13-3.07 (m, 2H), 2.73-2.67 (m, 1H), 2.40-2.34 (m, 1H).

### Example 70: (S)-2-((7-(5-fluoro-2-((2-methoxy-4-(trifluoromethyl)benzyl)oxy)pyrimidin-4-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 130)

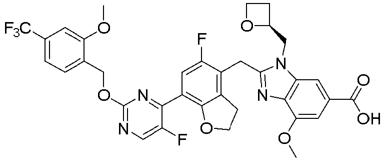

(S)-2-((7-(5-fluoro-2-((2-methoxy-4-(trifluoromethyl)benzyl)oxy)pyrimidin-4-yl)-5-fluoro-2,3-dihydrobenzofuran-4-yl)methyl)-4-methoxy-1-(oxetan-2-ylmethyl)-1H-benzo[d]imidazole-6-carboxylic acid (14 mg) was obtained by referring to the synthetic method of Compound 128 using 2-methoxy-4-(trifluoromethyl)benzyl alcohol as the starting material. ESI-MS (m/z): 713.2[M+H]⁺, ¹H NMR (600 MHz, DMSO-*d*₆) δ 12.81 (s, 1H), 8.76 (s, 1H), 7.87 (s, 1H), 7.61 (d, *J =* 7.7 Hz, 1H), 7.34 (d, *J =* 9.7 Hz, 2H), 7.26-7.19 (m, 2H), 5.47 (s, 2H), 5.11-5.04 (m, 1H), 4.73-4.68 (m, 1H), 4.63-4.56 (m, 3H), 4.51-4.45 (m, 2H), 4.40-4.36 (m, 1H), 4.35-4.30 (m, 1H), 3.92 (s, 3H), 3.89 (s, 3H), 3.16-3.09 (m, 2H), 2.74-2.66 (m, 1H), 2.39-2.36 (m, 1H).

### Example 71: (S)-2-((7-(6-((4-chloro-2-(difluoromethoxy)benzyl)oxy)-5-fluoropyrimidin-4-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 131)

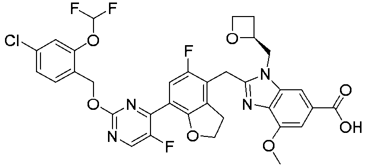

(S)-2-((7-(6-((4-chloro-2-(difluoromethoxy)benzyl)oxy)-5-fluoropyrimidin-4-yl)-5-fluoro-2,3-dihydrobenzofuran-4-yl)methyl)-4-methoxy-1-(oxetan-2-ylmethyl)-1*H*-benzo[*d*]imidazole-6-carboxylic acid (6 mg) was obtained by referring to the synthetic method of Compound 71 using Intermediates 1.1-B, 1.5-Y and 1.2-F as the starting materials. ESI-MS (m/z): 715.2[M+H]⁺, ¹H NMR (600 MHz, DMSO-*d*₆) δ 12.79 (s, 1H), 8.77 (d, *J* = 1.8 Hz, 1H), 7.94 - 7.85 (m, 1H), 7.62 (d, *J =* 8.1 Hz, 1H), 7.42 - 7.38 (m, 2H), 7.35 (t, *J =* 73.4 Hz, 1H), 7.27 - 7.22 (m, 1H), 5.44 (s, 2H), 5.11 - 5.04 (m, 1H), 4.72 (dd, *J =* 15.7, 6.9 Hz, 1H), 4.65 - 4.56 (m, 3H), 4.49 (dd, *J* = 15.6, 8.4 Hz, 2H), 4.39 (d, *J =* 16.8 Hz, 1H), 4.33 (dt, *J =* 9.1, 5.9 Hz, 1H), 3.89 (s, 3H), 3.13 (p, *J =* 7.6 Hz, 2H), 2.71 (dd, *J =* 10.6, 7.5 Hz, 1H), 2.42 - 2.33 (m, 1H).

### Example 72: (S)-2-((7-(5-fluoro-6-((2-fluoro-4-methylbenzyl)oxy)pyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 132)

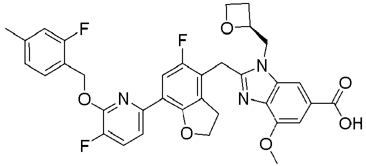

(S)-2-((7-(5-fluoro-6-((2-fluoro-4-methylbenzyl)oxy)pyridin-2-yl)-5-fluoro-2,3-dihydrobenzofuran-4-yl)methyl)-4-methoxy-1-(oxetan-2-ylmethyl)-1H-benzo[d]imidazole-6-carboxylic acid (23 mg) was obtained by referring to the synthetic method of Compound 71 using Intermediates 1.1-B, 1.5-Z and 1.2-F as the starting materials. ESI-MS(m/z): 646.1[M+H]⁺, ¹H NMR (600 MHz, DMSO-*d*₆) δ 12.73 (br s, 1H), 7.87 (s, 1H), 7.82 - 7.79 (m, 1H), 7.76 (d, J = 9.7 Hz, 1H), 7.72 (d, J *=* 11.6 Hz, 1H), 7.46 (t, J = 7.8 Hz, 1H), 7.24 (s, 1H), 7.09 (d, J *=* 11.2 Hz, 1H), 7.04 (d, J = 7.6 Hz, 1H), 5.56 (s, 2H), 5.06 (d, J = 5.2 Hz, 1H), 4.69 (dd, J = 18.9, 10.5 Hz, 3H), 4.58 (d, J = 14.5 Hz, 1H), 4.49 (d, J = 6.4 Hz, 1H), 4.44 (d, J = 16.8 Hz, 1H), 4.38 - 4.31 (m, 2H), 3.88 (s, 3H), 3.15 - 3.08 (m, 2H), 2.73 - 2.67 (m, 1H), 2.39 - 2.35 (m, 1H), 2.32 (s, 3H).

### Example 73: (S)-2-((7-(2-((2-fluoro-4-methylbenzyl)oxy)pyrimidin-4-yl)-5-fluoro-2,3-dihydrobenzofuran-4-yl)methyl)-4-methoxy-1-(oxetan-2-ylmethyl)-1H-benzo[d]imidazole-6-carboxylic acid (Compound 133)

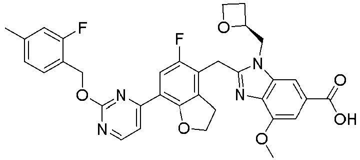

(S)-2-((7-(2-((2-fluoro-4-methylbenzyl)oxy)pyrimidin-4-yl)-5-fluoro-2,3-dihydrobenzofuran-4-yl)methyl)-4-methoxy-1-(oxetan-2-ylmethyl)-1H-benzo[d]imidazole-6-carboxylic acid (36 mg) was obtained by referring to the synthetic method of Compound 71 using Intermediates 1.1-B, 1.5-AA and 1.2-F as the starting materials. ESI-MS(m/z): 629.2[M+H]⁺, ¹H NMR (600 MHz, DMSO-d₆) δ 12.79 (br s, 1H), 8.68 (d, J = 5.2 Hz, 1H), 7.88 - 7.83 (m, 3H), 7.46 (t, J = 7.8 Hz, 1H), 7.24 (d, J = 0.9 Hz, 1H), 7.09 (d, J = 11.2 Hz, 1H), 7.05 (d, J = 7.8 Hz, 1H), 5.48 (s, 2H), 5.08 - 5.04 (m, 1H), 4.74 (t, J = 8.9 Hz, 2H), 4.72 - 4.68 (m, 1H), 4.59 (dd, J = 15.6, 2.5 Hz, 1H), 4.51 - 4.45 (m, 2H), 4.38 (d, J = 16.8 Hz, 1H), 4.33 (dt, J = 9.0, 5.9 Hz, 1H), 3.88 (s, 3H), 3.13 (dd, J = 14.3, 8.3 Hz, 2H), 2.72 - 2.68 (m, 1H), 2.39 - 2.36 (m, 1H), 2.32 (s, 3H).

### Example 74: (S)-2-((7-(5-fluoro-2-((2-fluoro-4-methylbenzyl)oxy)pyrimidin-4-yl)-5-fluoro-2,3-dihydrobenzofuran-4-yl)methyl)-4-methoxy-1-(oxetan-2-yl)-1H-benzo[d]imidazole-6-carboxylic acid (Compound 134)

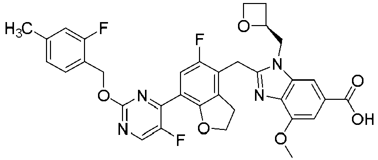

(S)-2-((7-(5-fluoro-2-((2-fluoro-4-methylbenzyl)oxy)pyrimidin-4-yl)-5-fluoro-2,3-dihydrobenzofuran-4-yl)methyl)-4-methoxy-1-(oxetan-2-yl)-1H-benzo[d]imidazole-6-carboxylic acid (16 mg) was obtained by referring to the synthetic method of Compound 71 using Intermediates 1.1-B, 1.5-AB and 1.2-F as the starting materials. ESI-MS (m/z): 647.2[M+H]⁺, ¹H NMR (600 MHz, DMSO-*d*₆) δ 12.80 (s, 1H), 8.76 (d, J = 1.4 Hz, 1H), 7.87 (s, 1H), 7.47 - 7.43 (m, 1H), 7.27 - 7.23 (m, 2H), 7.09 (d, J = 11.2 Hz, 1H), 7.05 (d, J = 7.6 Hz, 1H), 5.42 (s, 2H), 5.10 - 5.04 (m, 1H), 4.74 - 4.68 (m, 1H), 4.64 - 4.57 (m, 3H), 4.53 - 4.45 (m, 2H), 4.40 - 4.36 (m, 1H), 4.34-4.31 (m, 1H), 3.89 (s, 3H), 3.21 - 3.07 (m, 2H), 2.75 - 2.67 (m, 1H), 2.38 - 2.30 (m, 4H).

The following are the efficacy assays and data of the compounds of the present application.

### Test Example 1: Enzymatic assay of Compounds of the present application

The GLP-1R agonistic effects of the test compounds were determined using GLP1R-cAmp-Luc cells. 1) The test compound was dissolved in DMSO and mixed well until the test compound was completely dissolved. The test compounds were dissolved in DMSO to prepare 10 mM stock solutions. All compounds were diluted with DMEM medium (high sugar, Puromycin⁺, Hygromycin B⁺) to an initial concentration of 1250 nM, and then subjected to 5-fold serial dilution to a total of 7 concentration gradients. The GLP1R-cAmp-Luc cells were seeded in white-wall 96-well cell culture plates at a density of 1×10⁵/mL, with 90 µL cell suspension per well; the test compound at each of the 7 concentration gradients was added at 10 µL per well, with the actual initial working concentration of 125 nM; and the cells were placed in a 37 °C, 5% CO₂ cell incubator for 4 hours; 2) 100 µL of luciferin potassium salt solution diluted to 300 µg/mL in PBS was added to each well at a working concentration of 150 µg/mL and incubated at room temperature for 30 minutes; 3) fluorescence signals of firefly luciferin were measured with a multifunctional microplate reader (PerkinElmer, Nivo); and the fluorescence values were processed using GraphPad Prism software to calculate EC₅₀ values.

The test results showed that the compounds of the present application showed strong GLP-1R agonistic effects; and the preferred compounds have superior GLP-1R agonistic effects compared to the control compound (EC₅₀ of positive reference / EC₅₀ of compound of the present application >1), more preferably EC₅₀ of positive reference / EC₅₀ of compound of the present application ≥ 2, more preferably EC₅₀ of positive reference / EC₅₀ of compound of the present application ≥ 3, more preferably EC₅₀ of positive reference / EC₅₀ of compound of the present application ≥ 4, more preferably EC₅₀ of positive reference / EC₅₀ of compound of the present application ≥ 5, more preferably EC₅₀ of positive reference / EC₅₀ of compound of the present application ≥6, more preferably EC₅₀ of positive reference / EC₅₀ of compound of the present application ≥7, more preferably EC₅₀ of positive reference / EC₅₀ of compound of the present application ≥10, more preferably EC₅₀ of positive reference / EC₅₀ of compound of the present application ≥15, more preferably EC₅₀ of positive reference / EC₅₀ of compound of the present application ≥20. For example, 1< EC₅₀ of positive reference / EC₅₀ of compound of the present application <2, or 2≤ EC₅₀ of positive reference / EC₅₀ of compound of the present application <3, or 3≤ EC₅₀ of positive reference / EC₅₀ of compound of the present application <4, or 4≤ EC₅₀ of positive reference / EC₅₀ of compound of the present application <5, or 5≤ EC₅₀ of positive reference / EC₅₀ of compound of the present application <6, or 6≤ EC₅₀ of positive reference / EC₅₀ of compound of the present application <7, or 7≤ EC₅₀ of positive reference / EC₅₀ of compound of the present application <10, or 10≤ EC₅₀ of positive reference / EC₅₀ of compound of the present application <20, or EC₅₀ of positive reference / EC₅₀ of compound of the present application≥20. Exemplary compounds were shown in the following table.

| **Compound** | **Agonistic activity (EC50: nM)** | **EC₅₀ of Positive reference / EC₅₀ of Compound of the Present Application** | **Compound** | **Agonistic activity (EC50: nM)** | **EC₅₀ of Positive reference / EC₅₀ of Compound of the Present Application** |
|---|---|---|---|---|---|
| 1 | C | - | 2 | B | - |
| 6 | C | - | 7 | A | Ratio ≥10 |
| 9 | B | - | 12 | A | Ratio ≥10 |
| 13 | A | Ratio ≥10 | 14 | B | - |
| 18 | B | - | 19 | B | - |
| 20 | B | - | 62 | B | - |
| 71 | A | Ratio ≥10 | 79 | A | 1< ratio <3 |
| 80 | A | Ratio ≥10 | 81 | A | Ratio ≥10 |
| 84 | A | Ratio ≥10 | 86 | B | - |
| 87 | A | 1< ratio <3 | 89 | A | 1< ratio <3 |
| 93 | A | 3≤ ratio <10 | 95 | A | 3≤ ratio <10 |
| 100 | A | Ratio ≥10 | 101 | A | Ratio ≥10 |
| 105 | B | 1< ratio <3 | 106 | A | Ratio ≥10 |
| 107 | B | 1< ratio <3 | 108 | B | 3≤ ratio <10 |
| 109 | B | 3≤ ratio <10 | 110 | A | Ratio ≥10 |
| 111 | B | - | 112 | B | 1< ratio <3 |
| 113 | A | Ratio ≥10 | 114 | A | Ratio ≥10 |
| 115 | A | Ratio ≥10 | 116 | A | Ratio ≥10 |
| 117 | A | Ratio ≥10 | 118 | A | Ratio ≥10 |
| 119 | B | 3≤ ratio <10 | 120 | A | Ratio ≥10 |
| 121 | A | Ratio ≥10 | 122 | B | Ratio ≥10 |
| 124 | A | 3≤ ratio <10 | 125 | A | Ratio ≥10 |
| 126 | A | Ratio ≥10 | 127 | B | 1< ratio <3 |
| 128 | A | Ratio ≥10 | 129 | A | 3≤ ratio <10 |
| 130 | A | Ratio ≥10 | 131 | A | Ratio ≥10 |
| 132 | A | Ratio ≥10 | 133 | A | Ratio ≥10 |
| 134 | A | Ratio ≥10 | | | |

| | | | | | |
|---|---|---|---|---|---|
| Note: A represents EC₅₀ ≤1 nM, B represents 1 nM< EC₅₀ ≤5 nM, and C represents 5 nM< EC₅₀ <10 nM. Positive reference is PF-06882961, which was prepared by referring to Example 4A-01 in CN110325530B. | | | | | |

### Test Example 2: In vivo pharmacokinetic assay in mice

Experimental animals: ICR mice (female, 22-25 g), purchased from Beijing Vital River Laboratory Animal Technology Co., Ltd.

Experimental procedure: ICR mice were administered the test compounds by gavage. Whole blood samples of the mice were collected at 15 min, 0.5 h, 1 h, 2 h, 4 h, 8 h and 24 h after administration and centrifuged to isolate plasma. The compound concentration was measured by LC-MS/MS, and pharmacokinetic parameters such as Area Under the Concentration-Time Curve (AUC) were calculated using Winnolin software.

Oral gavage dose: 10 mg/kg.

Measurement method: Quantitative analysis was carried out in the LC/MS/MS system after pretreatment with a protein precipitation method.

The compounds of the present application have good *in vivo* pharmacokinetics and excellent oral administration performance. The PK parameters of exemplary compounds were shown in the following table.

| Compound | AUCₗₐₛₜ (h*ng/mL) |
|---|---|
| 14 | 12691 |
| 83 | 13998 |
| 87 | 12220 |
| 89 | 14061 |
| 92 | 23746 |
| 93 | 8444 |
| 101 | 22497 |
| 105 | 6896 |
| 118 | 8295 |
| 132 | 19526 |
| 134 | 15730 |

### Test Example 3: HERG Test

The potential inhibitory effects of the test compounds on the hERG channel were assessed using an automated patch-clamp system. In this study, the CHO cell line stably expressing the hERG gene was used, with Cisapride as the positive control.

The CHO-hERG-DUO cell line stably expressing the hERG channel was purchased from B'SYS GmbH. The cells were cultured in F12 (HAM) medium supplemented with 10%FBS, 100 U/mL penicillin-streptomycin, 100 µg/mL hygromycin, and 100 µg/mL G418. The cells were passaged three times per week and maintained at about 80% confluence.

The test compounds were dissolved in DMSO to prepare stock solutions at a final concentration of 10 mM; the stock solution was then serially diluted with DMSO in a 1: 3 ratio (3.33 mM, 1.11 mM and 0.37 mM); and the final concentrations of the test compounds were 30 µM, 10 µM, 3.33 µM, 1.11 µM and 0.37 µM.

A baseline was established using the blank vehicle. After the hERG current stabilized for at least 5 minutes, the compound working solution was perfused. The hERG current was recorded for no less than 5 minutes to reach a steady state, and then 5 scans were captured. If steady state was not reached within 10 minutes, the average peak current of the last 5 scans will replace the steady-state value. The experiments were performed in duplicate (n = 2) and hERG current inhibition by five concentrations of the compound were measured to determine the IC₅₀.

The percent current inhibition was calculated using the following equation. $Peak current inhibition = \left(1-\frac{Peak tail {current}_{compound} - Peak tail {current}_{postive control}}{Peak tail {current}_{Blank vehicle} - Peak tail {current}_{postive control}}\right) \times 100$

The IC₅₀ was calculated using Graphpad Prism 8.0.

The test results indicated that the compounds of the present application have no significant inhibitory effect on the hERG channel at the tested concentrations in this test, with an IC₅₀ > 30 µM, demonstrating that the compounds of the present invention have a low risk of cardiotoxicity. The compounds of the present application have IC₅₀ greater than that of the positive control compound PF-06882961, indicating that the hERG of the compounds of the present application were significantly superior to the control compound PF-06882961. The IC₅₀ of exemplary compounds were shown in the following table:

| Compound | IC₅₀ (µM) |
|---|---|
| 71 | >30 |
| 87 | >30 |

### Test Example 4: Determination of Food Intake Inhibition Rate:

The food intake of C57BL/6 hGLP1R transgenic mice in each cage was measured at 18:00 on the day before administration, and the remaining weight of food was measured at 8: 00 on the next day (day 0) The difference between the two measurements was recorded as the baseline food intake weight. The mice were randomly grouped (test compound group and vehicle group) according to the baseline food intake and body weight. The food intake and body weight of the mice at 0 h were measured before administration. The mice were administered by gavage at 20:00 on day 0, and then the food intake and body weight of the mice were measured at specific time points. The phase (within a defined period of time) food intake, phase food intake rate, cumulative (after administration to a specific time point) food intake, cumulative food intake rate and body weight were finally statistically analyzed to evaluate the food intake inhibitory effect of the compounds. $Cumulative Food intake \left(g\right) = \left(m before\right) - \left(m after\right) .$

"m before" is the weight of food before food intaking for the period of time, and "m after" is the weight of food after food intaking for the period of time. $Food intake inhibition rate \left(%\right) = \left(1-\left(m administration group\right)/\left(m vehicle group\right)\right) * 100 .$

"m administration group" is the cumulative food intake of the administration group during the period of time, and "m vehicle group" is the cumulative food intake of the vehicle group during the period of time.

The compounds of the present application showed good food intake inhibition in C57BL/6 hGLP1R mice. The cumulative food intake inhibition rate of an exemplary compound was shown in the following table:

| Compound | 0-2.5 hours | 0-5 hours | 0-12 hours |
|---|---|---|---|
| 101 (2mpk, administrated once) | 97% | 89% | 75% |

### Test Example 5: Weight-loss Efficacy Study in DIO C57BL/6 hGLP1R Mice

### 1. Test Purpose

To investigate and compare the effects of different compounds on weight loss in diet-induced obese (DIO) C57BL/6 hGLP1R mice.

### 2. Test animals

The animals (species: C57BL/6 humanized transgenic (hGLP1R) mice; Age: 6 weeks; Sex: Male) were fed a high-fat diet (60 kcal% Fat) for no less than 12 weeks to induce obesity, with the body weight of the animals reaching 40 g or more.

### 3. Test Grouping

The mice were weighed prior to administration, one day (one whole night + daytime) food intake was measured for each mouse. The mice were divided equally into groups (test compound group and vehicle group) according to body weight and food intake, with the same number of animals in each group, at least 5 animals per group.

### 4. Test Methods

The mice were administered daily at fixed time points by gavage. The body weight of the mice was measured daily at fixed time points, the body weight change of the mice was calculated, and after measurement, the mice were given the corresponding drug according to the current body weight and the grouping. Body weights of the mice were measured daily, and the rate of change in body weight of the mice was calculated, recorded and plotted.

The compounds of the present application demonstrated good weight-reducing effects on DIO C57BL/6 hGLP1R mice.

## Claims

1. A compound represented by Formula (I'), or a tautomer, stereoisomer, or pharmaceutically acceptable salt thereof, having the following structure: 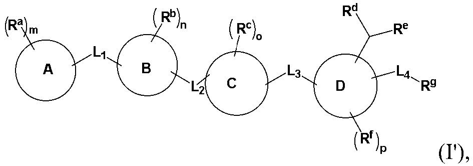 wherein,
ring A is independently selected from the group consisting of phenyl and 5- to 6-membered heteroaryl;
ring B is independently selected from the group consisting of 8- to 10-membered bicyclic heterocyclyl, phenyl, 5- to 6-membered monocyclic heteroaryl, and 8- to 10-membered bicyclic heteroaryl;
ring C is independently selected from the group consisting of C₈₋₁₀ bicyclic fused carbocyclyl, 8- to 10-membered bicyclic fused heterocyclyl, and 8- to 10-membered bicyclic heteroaryl; 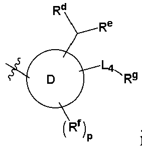 is independently selected from the group consisting of 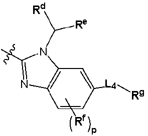 and 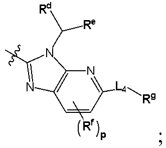
R^{a} is, at each occurrence, independently selected from the group consisting of deuterium, halo, hydroxy, amino, nitro, mercapto, cyano, oxo, -C(O)N(R^{a1})(R^{a2}), N(R^{a1})C(O)(R^{a2}), C₁₋₆ alkyl, C₁₋₆ alkoxy, C₁₋₆ alkylaminyl, C₃₋₆ cycloalkyl, and 3- to 6-membered heterocyclyl, wherein the C₁₋₆ alkyl, the C₁₋₆ alkoxy, the C₁₋₆ alkylaminyl, the C₃₋₆ cycloalkyl, and the 3- to 6-membered heterocyclyl are optionally substituted with one or more R^{a3};
R^{a1} and R^{a2} are, at each occurrence, each independently selected from the group consisting of hydrogen, deuterium, C₁₋₆ alkyl, C₁₋₆ alkoxy, C₃₋₆ cycloalkyl, 3- to 6-membered heterocyclyl, phenyl, and 5- to 6-membered heteroaryl, wherein the C₁₋₆ alkyl, the C₁₋₆ alkoxy, the C₃₋₆ cycloalkyl, the 3- to 6-membered heterocyclyl, the phenyl, and the 5- to 6-membered heteroaryl are optionally substituted with one or more substituents selected from the group consisting of halo, hydroxy, amino, nitro, mercapto, cyano, oxo, C₁₋₆ alkyl, C₁₋₆ alkoxy, C₃₋₆ cycloalkyl, and 3- to 6-membered heterocyclyl;
R^{a3} is, at each occurrence, independently selected from the group consisting of deuterium, halo, hydroxy, amino, nitro, mercapto, cyano, oxo, and -R^{a4};
R^{a4} is independently selected from the group consisting of hydrogen, deuterium, C₁₋₆ alkyl, and C₁₋₆ alkoxy, wherein the C₁₋₆ alkyl and the C₁₋₆ alkoxy are optionally substituted with one or more substituents selected from the group consisting of halo, hydroxy, amino, nitro, mercapto, and cyano;
R^{b} is, at each occurrence, independently selected from the group consisting of deuterium, halo, hydroxy, amino, nitro, mercapto, cyano, oxo, optionally substituted C₁₋₆ alkyl, and optionally substituted C₁₋₆ alkoxy, wherein the term "optionally substituted" means that hydrogen on the substituted group is not substituted, or one or more substitutable sites of the substituted group are independently substituted with R^{b1};
R^{b1} is, at each occurrence, independently selected from the group consisting of deuterium, halo, hydroxy, amino, nitro, mercapto, cyano, oxo, and -R^{b2};
R^{b2} is independently selected from the group consisting of hydrogen, deuterium, C₁₋₆ alkyl, and C₁₋₆ alkoxy, each of which is optionally substituted with one or more substituents selected from the group consisting of halo, hydroxy, amino, nitro, mercapto, cyano, oxo, C₁₋₆ alkyl, and C₁₋₆ alkoxy;
R^{c} is, at each occurrence, independently selected from the group consisting of deuterium, halo, hydroxy, amino, cyano, oxo, optionally substituted C₁₋₆ alkyl, and optionally substituted C₁₋₆ alkoxy, wherein the term "optionally substituted" means that hydrogen on the substituted group is not substituted, or one or more substitutable sites of the substituted group are independently substituted with R^{c1}; or
two R^{c}s, together with the atom(s) to which they are attached, form 3- to 6-membered heterocyclyl or 5- to 6-membered heteroaryl, each of which is optionally substituted with one or more substituents selected from the group consisting of deuterium, halo, hydroxy, amino, cyano, oxo, methyl, ethyl, methoxy, and ethoxy;
R^{c1} is, at each occurrence, independently selected from the group consisting of deuterium, halo, hydroxy, amino, nitro, mercapto, cyano, oxo, and C₁₋₄ alkyl optionally substituted with one or more substituents selected from the group consisting of halo, hydroxy, amino, and cyano;
one of R^{d} and R^{e} is selected from the group consisting of hydrogen and deuterium, and the other is selected from the group consisting of optionally substituted C₃₋₆ cycloalkyl, optionally substituted 3- to 6-membered heterocyclyl, and optionally substituted 5- to 6-membered heteroaryl, wherein the term "optionally substituted" means that hydrogen on the substituted group is not substituted, or one or more substitutable sites of the substituted group are independently substituted with R^{d1};
R^{d1} is, at each occurrence, independently selected from the group consisting of deuterium, halo, hydroxy, amino, nitro, mercapto, cyano, oxo, -C(O)N(R^{d2})(R^{d3}), -N(R^{d2})C(O)(R^{d3}), C₁₋₆ alkyl, and C₁₋₆ alkoxy, wherein the C₁₋₆ alkyl and the C₁₋₆ alkoxy are optionally substituted with one or more substituents selected from the group consisting of deuterium, halo, hydroxy, amino, nitro, mercapto, cyano, oxo, C₁₋₆ alkyl, C₁₋₆ haloalkyl, and C₁₋₆ alkoxy;
R^{d2} and R^{d3} are, at each occurrence, each independently selected from the group consisting of hydrogen, deuterium, C₁₋₆ alkyl, and C₁₋₆ alkoxy, wherein the C₁₋₆ alkyl and the C₁₋₆ alkoxy are optionally substituted with one or more substituents selected from the group consisting of halo, hydroxy, amino, nitro, mercapto, cyano, oxo, C₁₋₆ alkyl, and C₁₋₆ alkoxy;
R^{f} is, at each occurrence, independently selected from the group consisting of deuterium, halo, hydroxy, amino, nitro, mercapto, cyano, oxo, -OR^{f4}, -C(O)N(R^{f4})(R^{f5}), -N(R^{f4})C(O)(R^{f5}), C₁₋₆ alkyl, C₁₋₆ alkoxy, C₁₋₆ alkylaminyl, C₃₋₆ cycloalkyl, 3- to 6-membered heterocyclyl, phenyl, and 5- to 6-membered heteroaryl, wherein the C₁₋₆ alkyl, the C₁₋₆ alkoxy, the C₁₋₆ alkylaminyl, the C₃₋₆ cycloalkyl, the 3- to 6-membered heterocyclyl, the phenyl, and the 5- to 6-membered heteroaryl are optionally substituted with one or more R^{f1};
R^{f1} is, at each occurrence, independently selected from the group consisting of deuterium, halo, hydroxy, amino, cyano, and -R^{f2};
R^{f2} is independently selected from the group consisting of hydrogen, deuterium, C₁₋₆ alkyl, C₁₋₆ alkoxy, C₃₋₆ cycloalkyl, 3- to 6-membered heterocyclyl, phenyl, and 5- to 6-membered heteroaryl, each of which is optionally substituted with one or more substituents selected from the group consisting of halo, hydroxy, amino, nitro, mercapto, cyano, oxo, C₁₋₆ alkyl, C₁₋₆ alkoxy, C₃₋₆ cycloalkyl, 3- to 6-membered heterocyclyl, phenyl, and 5- to 6-membered heteroaryl;
R^{f4} and R^{f5} are, at each occurrence, each independently selected from the group consisting of hydrogen, deuterium, C₁₋₆ alkyl, C₃₋₆ cycloalkyl, 3- to 6-membered heterocyclyl, phenyl, and 5- to 6-membered heteroaryl;
R^{g} is, at each occurrence, independently selected from -C(O)OR^{g1}, wherein R^{g1} is hydrogen;
L₁ is independently selected from the group consisting of a bond, -C(R^{L1})₂-, -O-, and -C(R^{L1})₂O-, wherein R^{L1} is independently selected from the group consisting of hydrogen, deuterium, and C₁₋₆ alkyl optionally substituted with one or more substituents selected from the group consisting of deuterium, halo, hydroxy, amino, nitro, mercapto, and cyano;
L₂ is independently selected from the group consisting of a bond, -C(R^{L2})₂-, -O-, and -C(R^{L1})₂O-, wherein R^{L2} is independently selected from the group consisting of hydrogen, deuterium, and C₁₋₆ alkyl optionally substituted with one or more substituents selected from the group consisting of deuterium, halo, hydroxy, amino, nitro, mercapto, cyano, and C₁₋₆ alkyl;
L₃ is independently selected from the group consisting of a bond, -C(R^{L3})₂-, -O-, -S-, -C(O)-, -C(O)O-, and -OC(O)-, wherein R^{L3} is independently selected from the group consisting of hydrogen, deuterium, and C₁₋₆ alkyl optionally substituted with one or more substituents selected from the group consisting of deuterium, halo, hydroxy, amino, nitro, mercapto, cyano, and C₁₋₆ alkyl;
L₄ is a bond; m, n, o, and p are each independently 0, 1, 2, or 3;
unless otherwise stated, the heteroatom(s) in the heterocyclyl and heteroaryl is(are) independently selected from the group consisting of O, N and S, and the number of the heteroatoms is 1, 2, 3 or 4.

2. The compound, or the tautomer, stereoisomer, or pharmaceutically acceptable salt thereof according to claim 1, wherein:
ring A is independently selected from the group consisting of phenyl and pyridyl; or
ring A is independently selected from phenyl; or 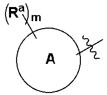 is independently selected from 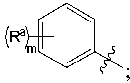 and/or
R^{a} is independently selected from the group consisting of halo, hydroxy, amino, cyano, -C(O)N(C₁₋₄ alkyl)₂, -C(O)NH(C₁₋₄ alkyl), -C(O)NH₂, C₁₋₄ alkyl, C₁₋₄ alkoxy, and 3- to 6-membered heterocyclyl, wherein the C₁₋₄ alkyl, C₁₋₄ alkoxy, and the 3- to 6-membered heterocyclyl are optionally substituted with one or more R^{a3}; and the heteroatom(s) in the 3- to 6-membered heterocyclyl is(are) selected from the group consisting of O and N, and the number of the heteroatom(s) is 1 or 2; or
R^{a} is independently selected from the group consisting of halo, cyano, C₁₋₃ alkyl, C₁₋₃ haloalkyl, C₁₋₃ alkoxy, C₁₋₃ haloalkoxy, and 3- to 6-membered heterocyclyl, wherein the heteroatom in the 3- to 6-membered heterocyclyl is O, and the number of the heteroatom is 1; or
R^{a} is independently selected from the group consisting of halo, cyano, -C(O)NH₂, C₁₋₃ alkyl, C₁₋₃ haloalkyl, C₁₋₃ alkoxy, C₁₋₃ haloalkoxy, and oxetanyl; or
R^{a} is independently selected from the group consisting of halo, cyano, C₁₋₃ alkyl, C₁₋₃ haloalkyl, C₁₋₃ alkoxy, C₁₋₃ haloalkoxy, and oxetanyl; or
R^{a} is independently selected from the group consisting of fluoro, chloro, cyano, -C(O)NH₂, -CF₃, - OCHF₂, methyl, methoxy, and 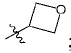 or
R^{a} is independently selected from the group consisting of fluoro, chloro, and cyano; or
R^{a1} and R^{a2} are each independently selected from hydrogen; or
R^{a3} is independently selected from halo; or
R^{a3} is independently selected from the group consisting of fluoro and chloro; or
m is 2; or 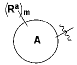 is independently selected from the group consisting of

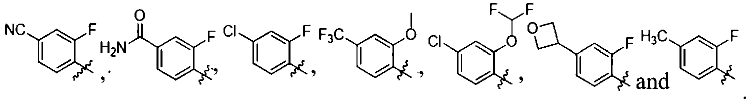

3. The compound, or the tautomer, stereoisomer, or pharmaceutically acceptable salt thereof according to claim 1 or 2, wherein:
L₁ is independently selected from the group consisting of a bond and -CH₂O-; or
L₁ is independently selected from -CH₂O-, wherein the CH₂ group is attached to an end of ring A and the O group is attached to an end of ring B.

4. The compound, or the tautomer, stereoisomer, or pharmaceutically acceptable salt thereof according to any one of claims 1 to 3, wherein:
ring B is independently selected from the group consisting of phenyl and 6-membered heteroaryl, wherein the heteroatom(s) in the heteroaryl is(are) N, and the number of the heteroatoms is 1 or 2; or
ring B is independently selected from the group consisting of phenyl, pyridyl, pyrimidinyl, and 9-membered bicyclic heterocyclyl, wherein the heteroatoms in the 9-membered bicyclic heterocyclyl are O, and the number of the heteroatoms is 2; or
ring B is independently selected from the group consisting of phenyl, pyridyl, pyrimidinyl, and 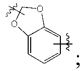 or
ring B is independently selected from the group consisting of 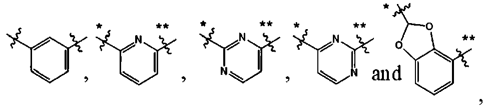 wherein the "*" end denotes the end attached to L₁, and the "**" end denotes the end attached to L₂; or
ring B is independently selected from the group consisting of 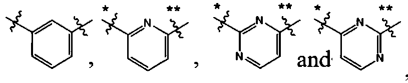 wherein the "*" end denotes the end attached to L₁, and the "**" end denotes the end attached to L₂; or
ring B is independently selected from the group consisting of 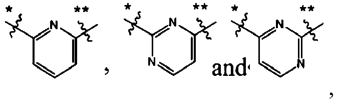 wherein the "*" end denotes the end attached to L₁, and the "**" end denotes the end attached to L₂.

5. The compound, or the tautomer, stereoisomer, or pharmaceutically acceptable salt thereof according to any one of claims 1 to 4, wherein:
R^{b} is independently selected from the group consisting of halo and methyl; or
R^{b} is independently selected from halo; or
R^{b} is independently selected from the group consisting of fluoro, chloro, and bromo; or
R^{b} is independently selected from fluoro; or
n is independently selected from the group consisting of 0 and 1; or
n is 0; or 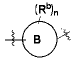 is selected from the group consisting of 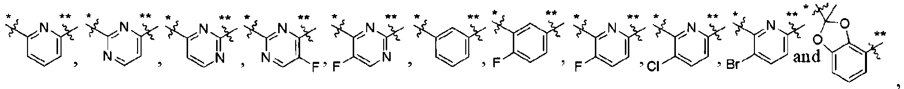 wherein the "*" end denotes the end attached to L₁, and the "**" end denotes the end attached to L₂.

6. The compound, or the tautomer, stereoisomer, or pharmaceutically acceptable salt thereof according to any one of claims 1 to 5, wherein:
ring C is independently selected from the group consisting of C₉ bicyclic fused carbocyclyl, 9-membered bicyclic fused heterocyclyl, 10-membered bicyclic fused heterocyclyl, and 9-membered bicyclic heteroaryl, wherein the heteroatom(s) in the C₉ bicyclic fused carbocyclyl, the 9-membered bicyclic fused heterocyclyl, the 10-membered bicyclic fused heterocyclyl, or the 9-membered bicyclic heteroaryl is(are) independently selected from the group consisting of N and O, and the number of the heteroatoms is 1 or 2; or
ring C is independently selected from the group consisting of C₉ bicyclic fused carbocyclyl, 9-membered bicyclic fused heterocyclyl, and 9-membered bicyclic heteroaryl, wherein the heteroatom(s) in the C₉ bicyclic fused carbocyclyl, the 9-membered bicyclic fused heterocyclyl, or the 9-membered bicyclic heteroaryl is(are) independently selected from the group consisting of N and O, and the number of the heteroatoms is 1 or 2; or
ring C is independently selected from 9-membered bicyclic fused heterocyclyl, wherein the heteroatom(s) in the 9-membered bicyclic fused heterocyclyl is(are) O, and the number of the heteroatoms is 1 or 2; or
ring C is independently selected from the group consisting of 5-membered/6-membered fused heterocyclyl and 6-membered/5-membered fused heterocyclyl, wherein the heteroatom(s) in the 5-membered/6-membered fused heterocyclyl or the 6-membered/5-membered fused heterocyclyl is(are) O, and the number of the heteroatoms is 1 or 2; or
ring C is independently selected from the group consisting of 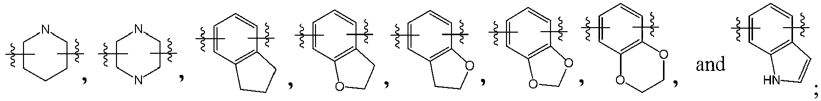 or
ring C is independently selected from the group consisting of 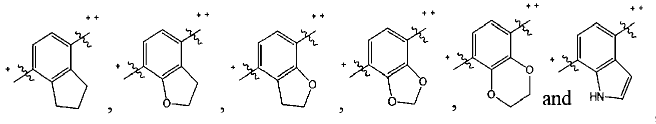 wherein the "+" end denotes the end attached to L₂, and the "++" end denotes the end attached to L₃; or
ring C is independently selected from 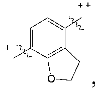 wherein the "+" end denotes the end attached to L₂, and the "++" end denotes the end attached to L₃; and/or
R^{c} is independently selected from the group consisting of halo, hydroxy, amino, cyano, oxo, and optionally substituted C₁₋₆ alkyl, wherein the term "optionally substituted" means that hydrogen on the substituted group is not substituted, or one or more substitutable sites of the substituted group are independently substituted with R^{c1}, wherein R^{c1} is independently selected from the group consisting of halo, hydroxy, amino, and cyano; or
R^{c} is independently selected from the group consisting of halo, hydroxy, oxo, and C₁₋₃ alkyl; or
R^{c} is independently selected from the group consisting of fluoro, chloro, and methyl; or
R^{c} is independently selected from halo; or
R^{c} is independently selected from the group consisting of fluoro and chloro; or
R^{c} is independently selected from fluoro; or
R^{c1} is independently selected from halo (e.g., fluoro, chloro, bromo); or
o is independently selected from the group consisting of 0 and 1; or
o is 0; or 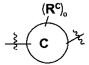 is selected from the group consisting of 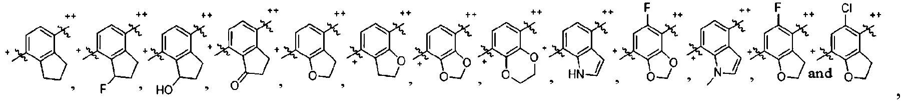 or is selected from the group consisting of 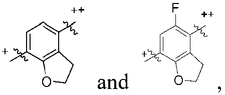 or is selected from 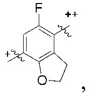 wherein the "+" end denotes the end attached to L₂, and the "++" end denotes the end attached to L₃.

7. The compound, or the tautomer, stereoisomer, or pharmaceutically acceptable salt thereof according to any one of claims 1 to 6, wherein:
L₂ is independently selected from a bond; and/or
L₃ is independently selected from the group consisting of -CH₂-, -C(O)-, and -CH(CH₃)-; or
L₃ is independently selected from -CH₂-.

8. The compound, or the tautomer, stereoisomer, or pharmaceutically acceptable salt thereof according to any one of claims 1 to 7, wherein: 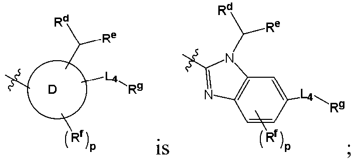 and/or
one of R^{d} and R^{e} is selected from hydrogen, and the other is selected from the group consisting of optionally substituted C₃₋₆ cycloalkyl, optionally substituted 3- to 6-membered heterocyclyl, and optionally substituted 5- to 6-membered heteroaryl, wherein the term "optionally substituted" means that hydrogen on the substituted group is not substituted, or one or more substitutable sites of the substituted group are independently substituted with R^{d1}; or
R^{d} and R^{e} are each independently selected from the group consisting of hydrogen, and optionally substituted 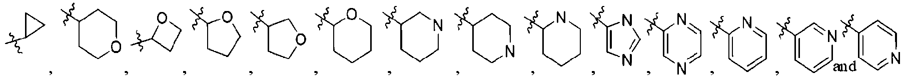 wherein the term "optionally substituted" means that hydrogen on the substituted group is not substituted, or one or more substitutable sites of the substituted group are independently substituted with R^{d1}; or
R^{d} and R^{e} are each independently selected from the group consisting of hydrogen, optionally substituted 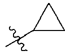 optionally substituted 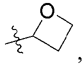 and optionally substituted 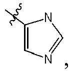 wherein the term "optionally substituted" means that hydrogen on the substituted group is not substituted, or one or more substitutable sites of the substituted group are independently substituted with R^{d1}; or
R^{e} is hydrogen, and R^{d} is selected from the group consisting of optionally substituted 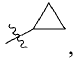 optionally substituted 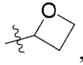 and optionally substituted 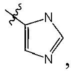 wherein the term "optionally substituted" means that hydrogen on the substituted group is not substituted, or one or more substitutable sites of the substituted group are independently substituted with R^{d1}, where R^{d1} is independently selected from the group consisting of -N(C₁₋₂ alkyl)C(O)( C₁₋₂ alkyl), and C₁₋₃ alkyl optionally substituted with one or more substituents selected from the group consisting of fluoro, chloro, and cyano; or 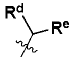 is independently selected from the group consisting of 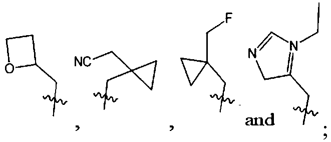 or 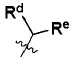 is independently selected from the group consisting of 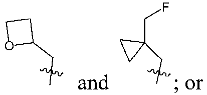 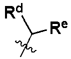 is independently selected from 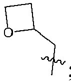 or 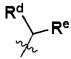 is independently selected from 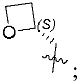 or 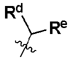 is independently selected from the group consisting of 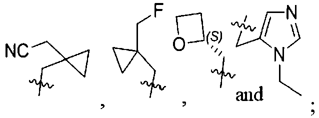 or 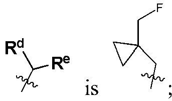 or
R^{d1} is independently selected from C₁₋₄ alkyl optionally substituted with one or more substituents selected from the group consisting of fluoro, chloro, and cyano; or
R^{d1} is independently selected from the group consisting of fluoro, chloro, bromo, hydroxy, amino, cyano, trifluoromethyl, difluoromethyl, monofluoromethyl, trichloromethyl, dichloromethyl, monochloromethyl, methyl, ethyl, n-propyl, isopropyl, n-butyl, methoxy, ethoxy, -CH₂CN, and - CH₂CH₂CN; or
R^{d1} is independently selected from the group consisting of monofluoromethyl, -CH₂CN, methyl, and ethyl; and/or
R^{f} is independently selected from the group consisting of halo, hydroxy, amino, cyano, -OR^{f4}, - C(O)N(R^{f4})(R^{f5}), -N(R^{f4})C(O)(R^{f5}), C₁₋₆ alkyl, and C₁₋₆ alkoxy, wherein the C₁₋₆ alkyl and the C₁₋₆ alkoxy are optionally substituted with one or more R^{f1}; or
R^{f} is independently selected from the group consisting of halo, hydroxy, amino, cyano, -O(C₃₋₆ cycloalkyl), -C(O)NH(C₁₋₃ alkyl), -NHC(O)(C₁₋₃ alkyl), C₁₋₃ alkyl, and C₁₋₃ alkoxy, wherein the C₁₋₃ alkyl and C₁₋₃ alkoxy are optionally substituted with one or more substituents selected from the group consisting of fluoro, chloro and phenyl; or
R^{f} is independently selected from the group consisting of fluoro, chloro, hydroxy, amino, -O-cyclopropyl, -C(O)NHCH₃, -NHC(O)CH₃, -CH₃, -CH₂CH₃, -CH(CH₃)₂, -OCH₃, -OCH₂CH₃, -OCH(CH₃)₂, -OCHF₂, -OCF₃, -OCH₂-phenyl, -OCH₂CF₃, and -OCH₂CHF₂; or
R^{f} is independently selected from halo; or
R^{f} is independently selected from the group consisting of fluoro and chloro; or
R^{f} is independently selected from the group consisting of fluoro, chloro, bromo, C₁₋₄ alkoxy, and C₁₋₃ haloalkoxy; or
R^{f} is independently selected from the group consisting of fluoro, chloro, -OCH₃, -OCH₂CH₃, - OCH(CH₃)₂, -OCHF₂, -OCF₃, -OCH₂CF₃, and -OCH₂CHF₂; or
R^{f} is independently selected from the group consisting of fluoro, -OCH₃, -OCH₂CH₃, -OCH(CH₃)₂, and -OCH₂CHF₂; or
R^{f1} is independently selected from halo; or
R^{f} is independently selected from the group consisting of halo and C₁₋₃ alkoxy, wherein the C₁₋₃ alkoxy is optionally substituted with one or more fluoro; or
p is independently selected from the group consisting of 0 and 1; or
p is 1; or
p is 0; or 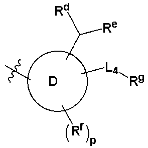 is independently selected from the group consisting of 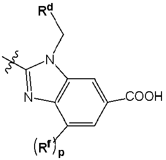 and 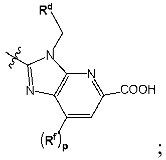 or 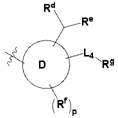 is independently selected from the group consisting of

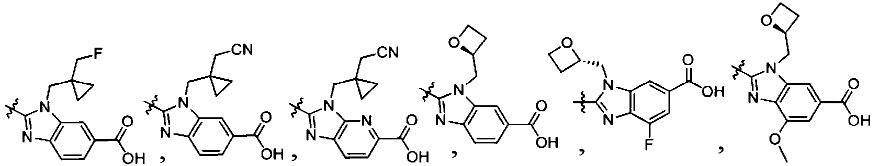

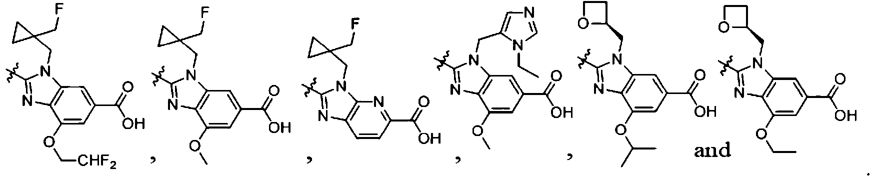

9. The compound, or the tautomer, stereoisomer, or pharmaceutically acceptable salt thereof according to any one of claims 1 to 8, wherein the compound has a structure represented by Formula (II), Formula (II-1), Formula (II-2), Formula (II-3), Formula (III), Formula (III-1), Formula (III-2), Formula (III-3), Formula (IV), Formula (IV-1), Formula (IV-2), Formula (IV-3), Formula (IV-4), Formula (IV-5), Formula (IV-6), Formula (IV-7), Formula (IV-8), Formula (IV-9), Formula (IV-10), Formula (IV-11), Formula (IV-12), Formula (IV-13), Formula (IV-14), Formula (IV-15), Formula (IV-16), Formula (IV-17), Formula (V), Formula (V-1), Formula (V-2), Formula (V-3), Formula (V-4), Formula (V-5), Formula (V-6), Formula (V-7), Formula (V-8), Formula (V-9), Formula (V-10), Formula (V-11), Formula (V-12), Formula (V-13), Formula (V-14), Formula (V-15), Formula (V-16), or Formula (V-17):

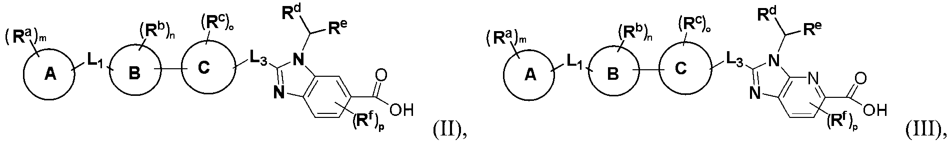

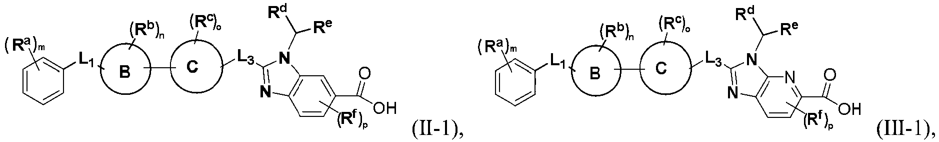

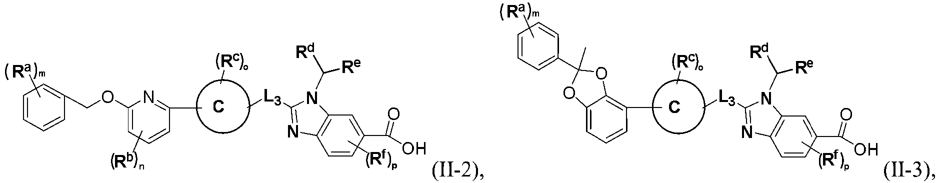

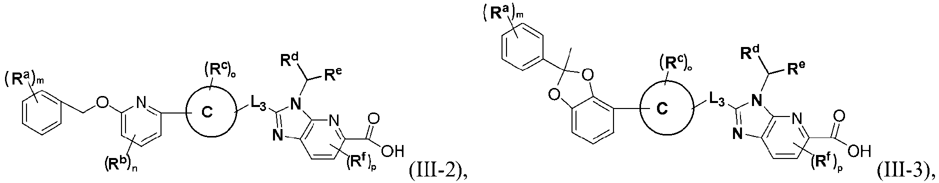

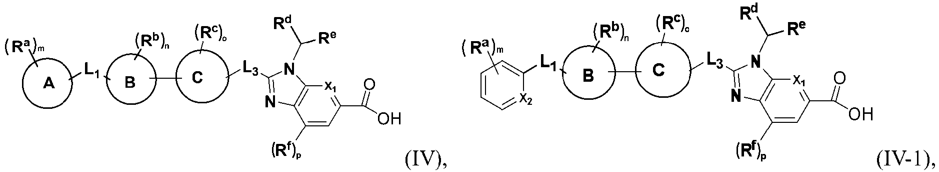

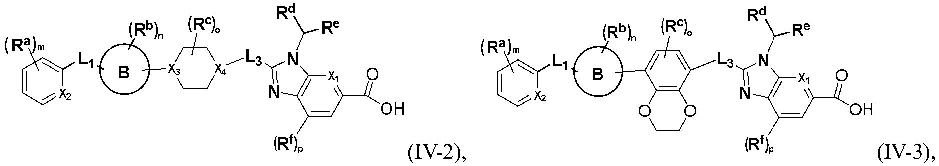

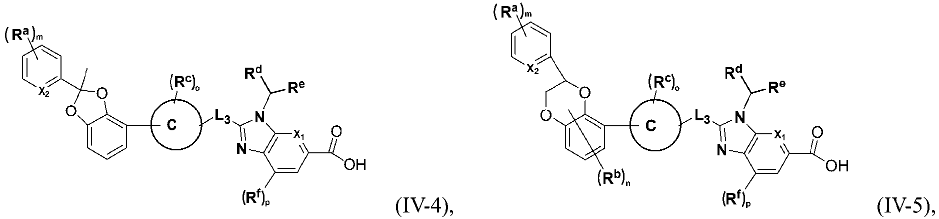

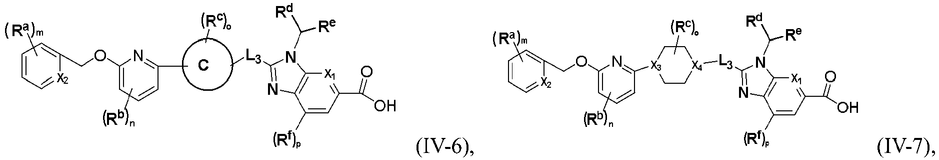

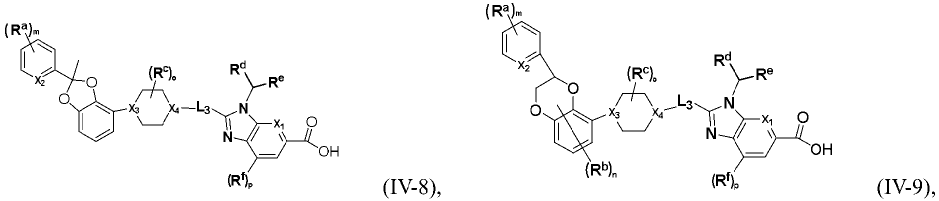

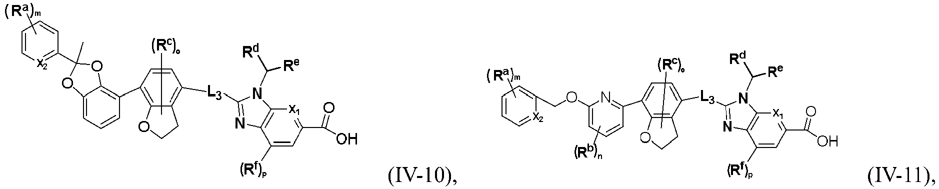

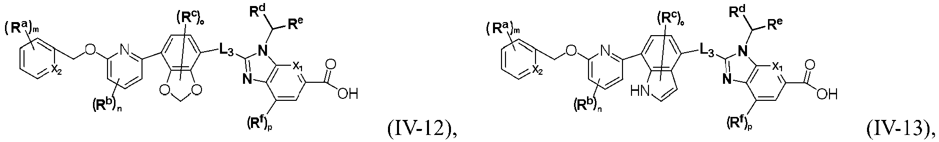

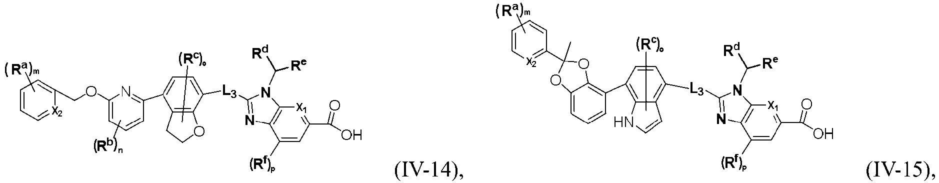

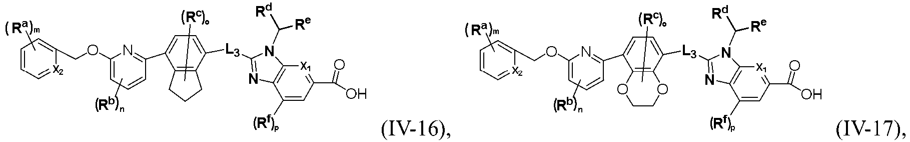

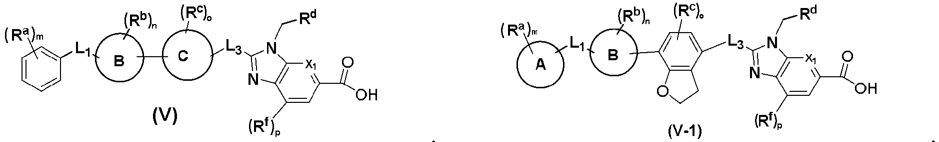

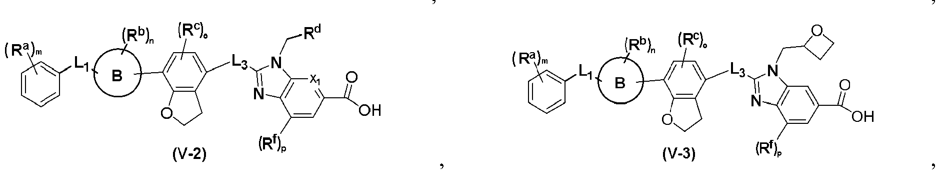

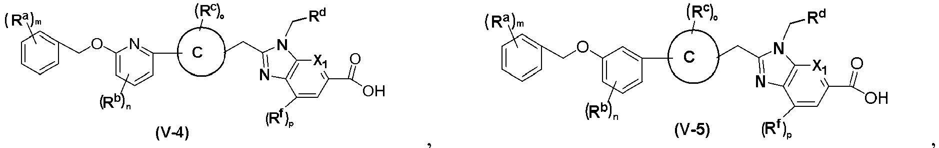

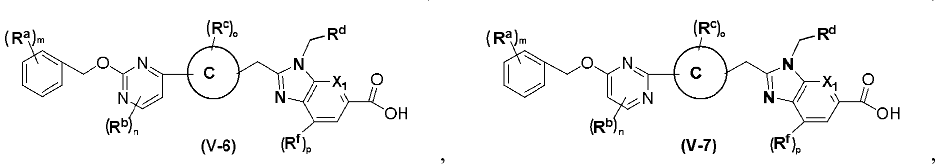

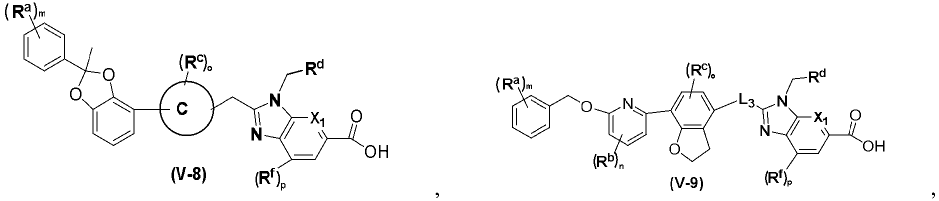

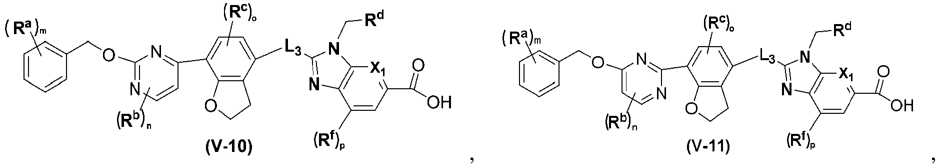

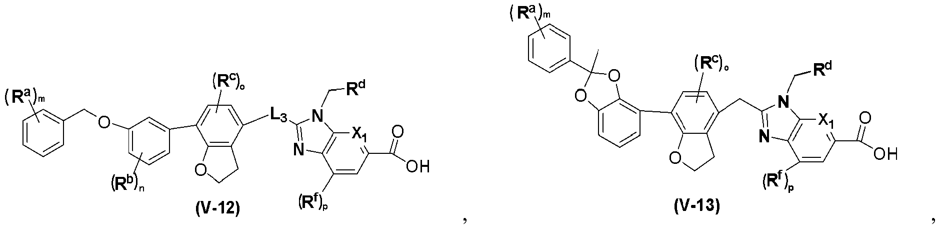

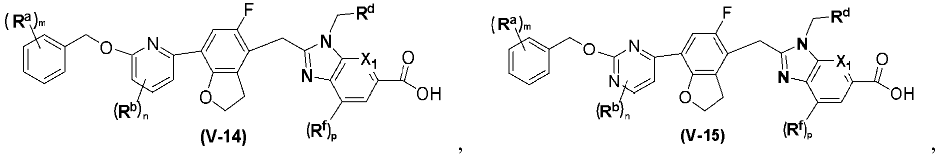

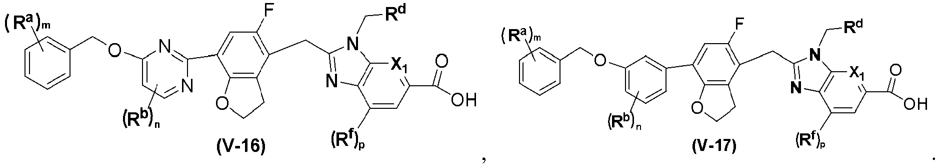

10. The compound, or the tautomer, stereoisomer, or pharmaceutically acceptable salt thereof according to claim 1, wherein the compound is selected from:
| **Compound Structure and Numbering** | **Compound Structure and Numbering** |
|---|---|
| 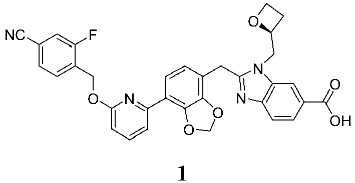 | 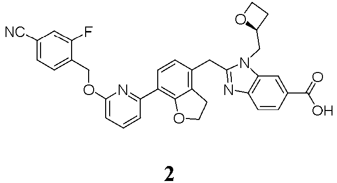 |
| 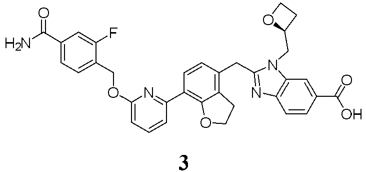 | 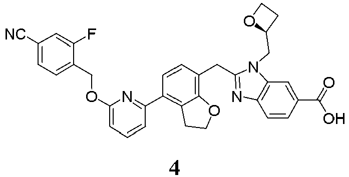 |
| 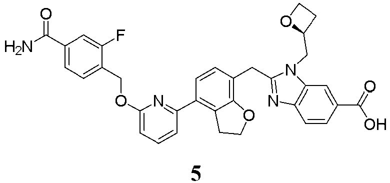 | 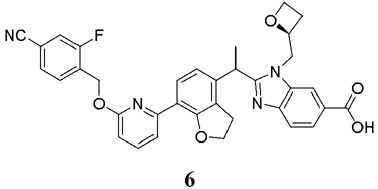 |
| 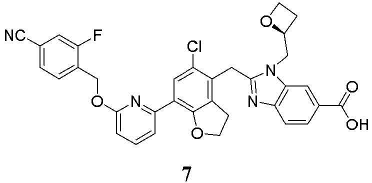 | 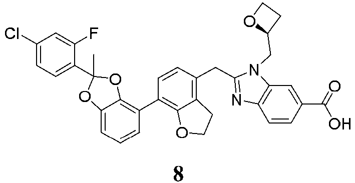 |
| 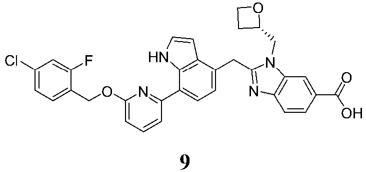 | 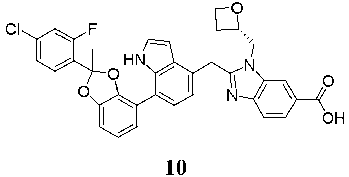 |
| 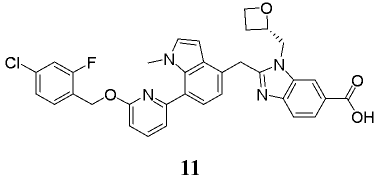 | 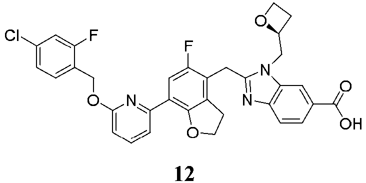 |
| 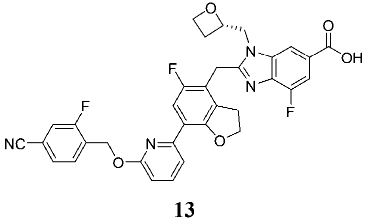 | 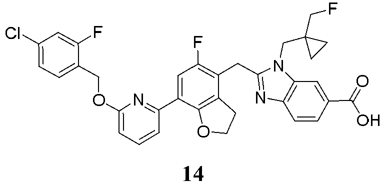 |
| 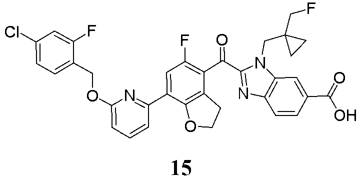 | 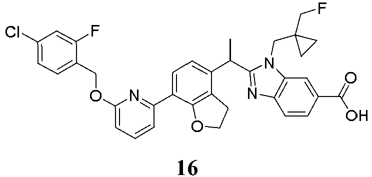 |
| 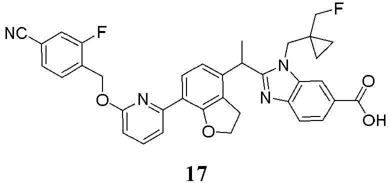 | 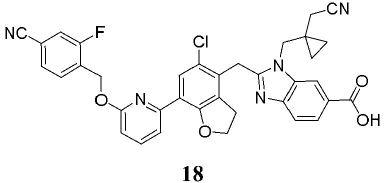 |
| 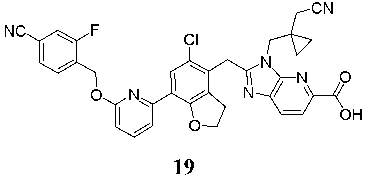 | 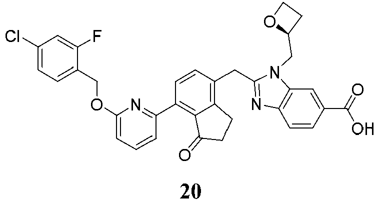 |
| 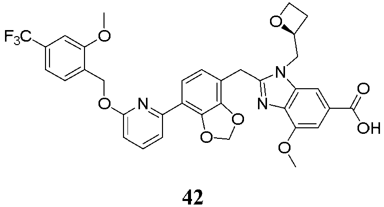 | 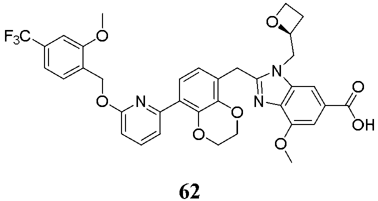 |
| 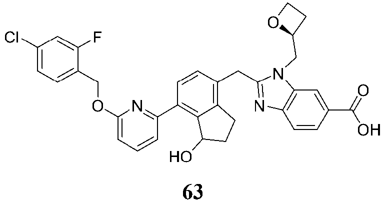 | 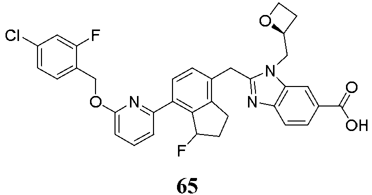 |
| 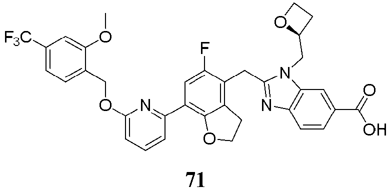 | 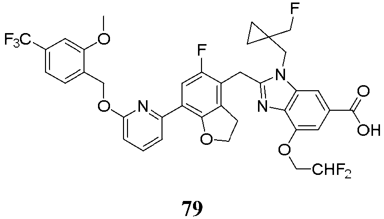 |
| 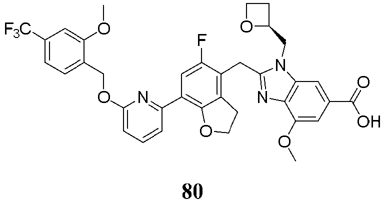 | 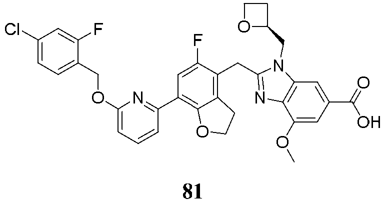 |
| 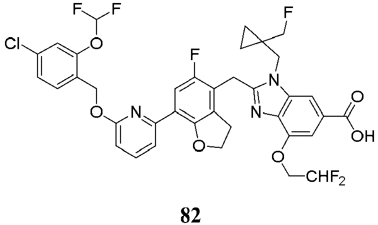 | 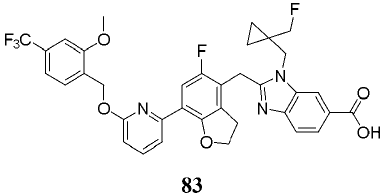 |
| 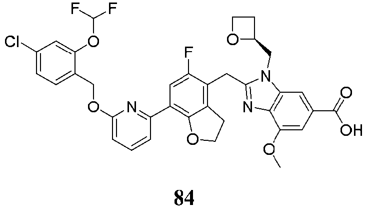 | 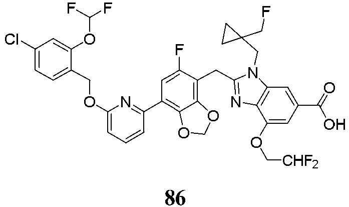 |
| 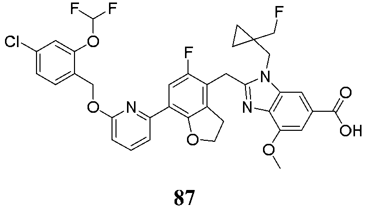 | 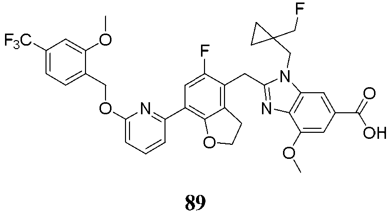 |
| 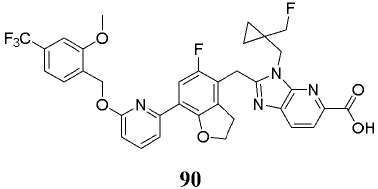 | 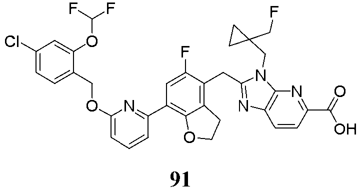 |
| 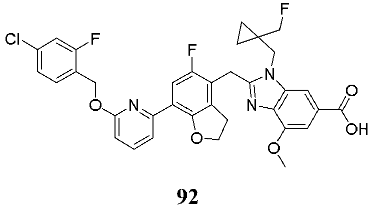 | 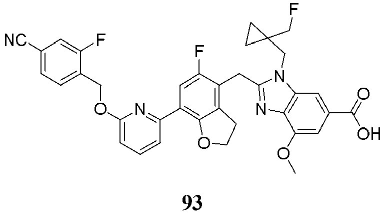 |
| 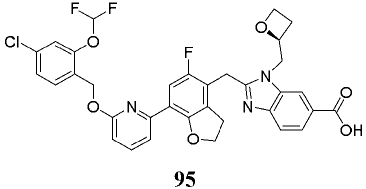 | 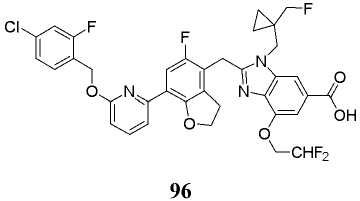 |
| 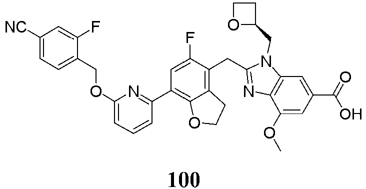 | 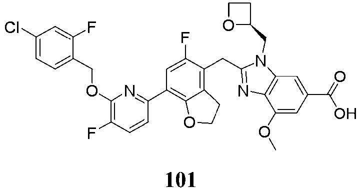 |
| 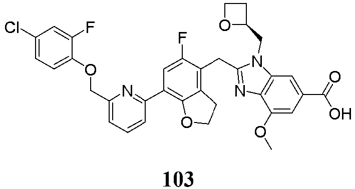 | 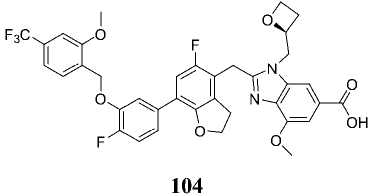 |
| 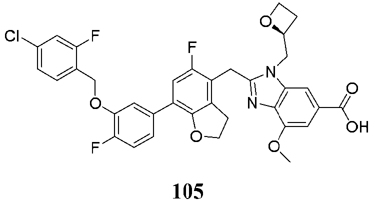 | 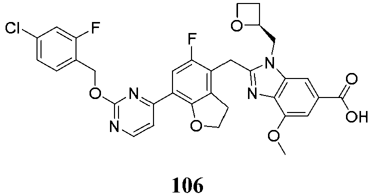 |
| 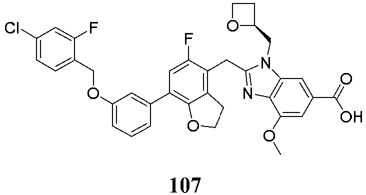 | 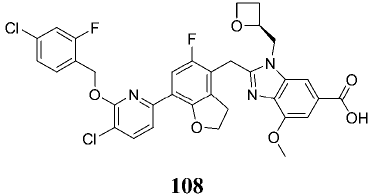 |
| 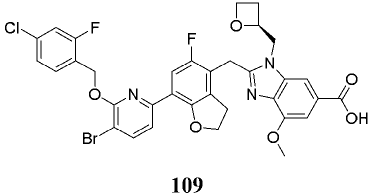 | 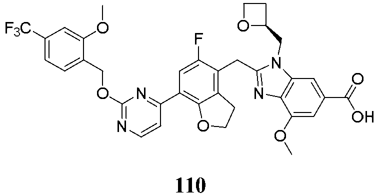 |
| 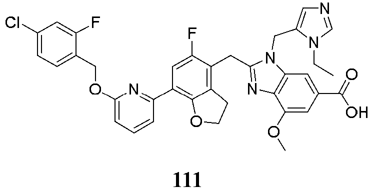 | 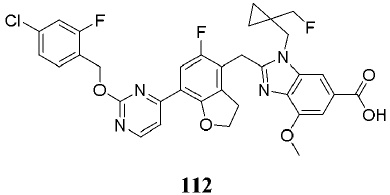 |
| 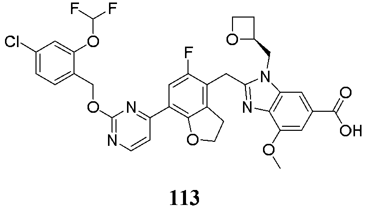 | 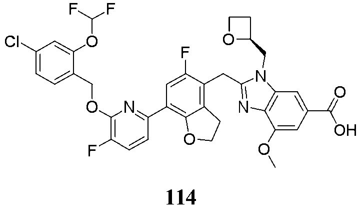 |
| 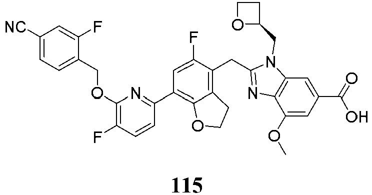 | 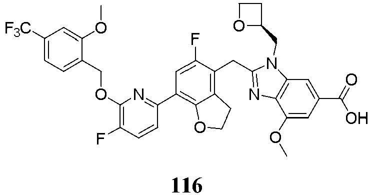 |
| 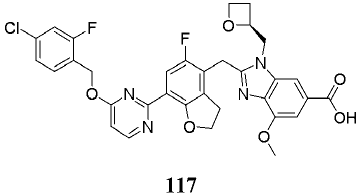 | 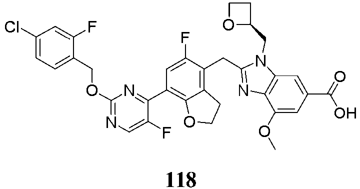 |
| 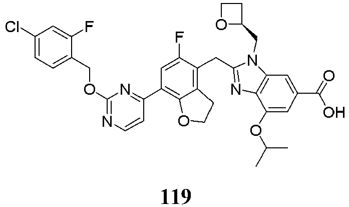 | 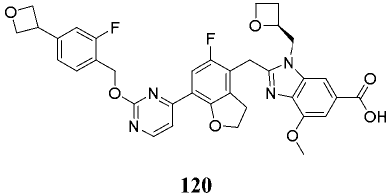 |
| 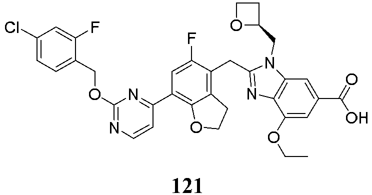 | 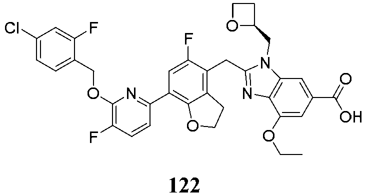 |
| 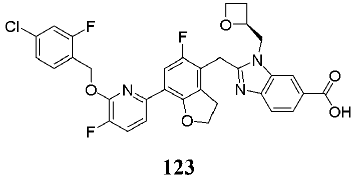 | 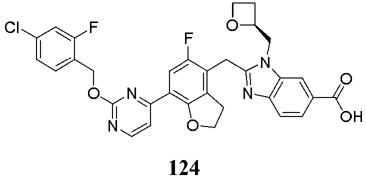 |
| 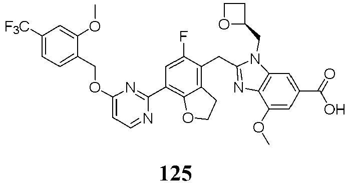 | 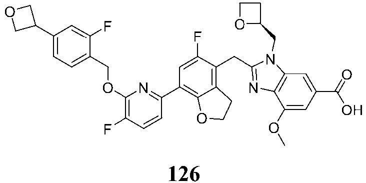 |
| 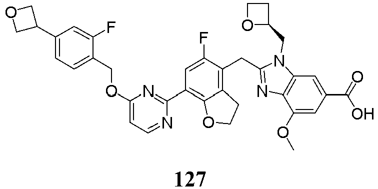 | 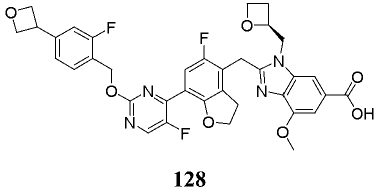 |
| 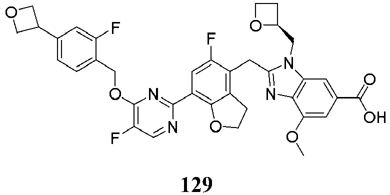 | 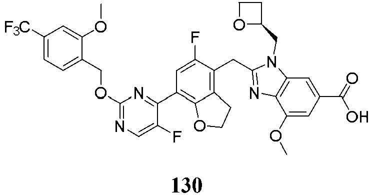 |
| 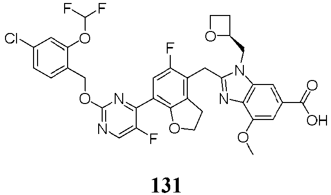 | 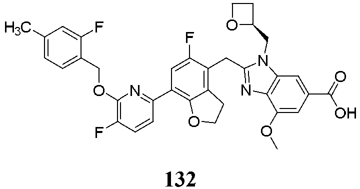 |
| 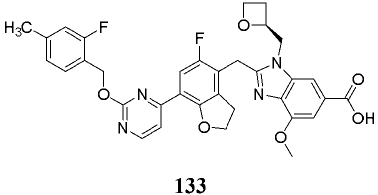 | 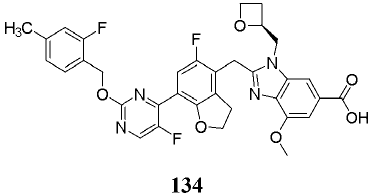 |

11. A pharmaceutical composition, comprising the compound, or the tautomer, stereoisomer, or pharmaceutically acceptable salt thereof according to any one of claims 1 to 10; and optionally further comprising additional one, two or more compounds useful for the same or similar indications.

12. Use of the compound, or the tautomer, stereoisomer, or pharmaceutically acceptable salt thereof according to any one of claims 1 to 10, or the pharmaceutical composition according to claim 11, in the preparation of a medicament for the prevention and/or treatment of diseases, disorders and conditions through the activation of GLP-1R-mediated cascade signaling; preferably, the diseases, disorders and conditions are GLP-1R-related, including diabetes, obesity, overweight, metabolic-related fatty liver diseases, and Alzheimer's disease.
